(19) Europäisches Patentamt
European Patent Office
Office européen des brevets

(11) EP 1 177 195 B1

(12) FASCICULE DE BREVET EUROPEEN

(45) Date de publication et mention de la délivrance du brevet: **19.03.2003 Bulletin 2003/12**

(21) Numéro de dépôt: **00967407.8**

(22) Date de dépôt: **28.04.2000**

(51) Int Cl.[7]: **C07D 487/04**, A61K 31/505

(86) Numéro de dépôt international: **PCT/FR00/01174**

(87) Numéro de publication internationale: **WO 00/066584 (09.11.2000 Gazette 2000/45)**

# (54) 1-AMINO TRIAZOLO 4,3-a! QUINAZOLINE-5-ONES ET/OU -5-THIONES INHIBITRICES DE PHOSPHODIESTERASES IV

1-AMINOTRIAZOLO 4,3-A CHINAZOLIN-5-ONE UND/ODER -THIONE INHIBITOREN VON PHOSPHODIESTERASEN IV

1-AMINO TRIAZOLO 4,3-a! QUINAZOLINE-5-ONES AND OR -5-THIONES INHIBITING PHOSPHODIESTERASE IV

(84) Etats contractants désignés:
**AT BE CH CY DE DK ES FI FR GB GR IE IT LI LU MC NL PT SE**
Etats d'extension désignés:
**AL LT LV MK RO SI**

(30) Priorité: **28.04.1999 FR 9905398**

(43) Date de publication de la demande: **06.02.2002 Bulletin 2002/06**

(73) Titulaire: **WARNER-LAMBERT COMPANY**
**Morris Plains, New Jersey 07950 (US)**

(72) Inventeurs:
- **GAUDILLIERE, Bernard**
  **F-92000 Nanterre (FR)**
- **LAVALETTE, Rémi**
  **F-91160 Longjumeau (FR)**
- **ANDRIANJARA, Charles**
  **F-94260 Fresnes (FR)**
- **BREUZARD, Francine**
  **F-91400 Quincy sous Senart (FR)**

(74) Mandataire: **Hirsch, Denise**
**Pfizer Global Research & Development**
**Fresnes Laboratories**
**3-9, rue de la Loge**
**B.P. 100**
**94265 Fresnes Cedex (FR)**

(56) Documents cités:
**EP-A- 0 076 199**
**EP-A- 0 133 234**
**WO-A-96/39408**
**WO-A-99/06404**
**US-A- 3 850 932**
**US-A- 3 865 824**

- **MALCOLM N. PALFREYMAN ET AL: "phosphodiesterase Type IV Inhibitors" PROGRESS IN MEDICINAL CHEMISTRY, vol. 33, 1996, pages 1-52, XP000650817**

Remarques:
Le dossier contient des informations techniques présentées postérieurement au dépôt de la demande et ne figurant pas dans le présent fascicule.

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Printed by Jouve, 75001 PARIS (FR)

## Description

### Domaine de l'invention

La présente invention est relative à de nouvelles triazolo [4,3-a] quinazoline-5-ones et/ou -5-thiones utiles pour la préparation de médicaments permettant de traiter des affections relevant d'une thérapie par un inhibiteur de phosphodiestérases 4. Ces médicaments sont utiles notamment comme anti-inflammatoires, anti-allergiques, bronchodilatateurs, antiasthmatiques, ou inhibiteurs de TNFα.

### Arrière-plan technologique de l'invention

L'adénosine 3', 5'-monophosphate cyclique (AMPc) est un second messager intracellulaire ubiquitaire, intermédiaire entre un premier messager (hormone, neurotransmetteur, ou autacoïde) et les réponses fonctionnelles cellulaires : le premier messager stimule l'enzyme responsable de la synthèse de l'AMPc ; l'AMPc intervient alors, selon les cellules en cause, dans de très nombreuses fonctions : métaboliques, contractiles, ou sécrétoires.

Les effets de l'AMPc prennent fin lorsqu'il est dégradé par les phosphodiestérases des nucléotides cycliques, enzymes intracellulaires qui catalysent son hydrolyse en adénosine 5'-monophosphate inactive.

On distingue chez les mammifères au moins sept grandes familles de phosphodiestérases des nucléotides cycliques (PDE) numérotées de 1 à 7 selon leur structure, leur comportement cinétique, leur spécificité de substrat, ou leur sensibilité à des effecteurs (Beavo J.A. *et al.* (1990) Trends Pharmacol. Sci. 11, 150-155. Beavo J.A. *et al.* (1994) Molecular Pharmacol. 46, 399-405). Les PDE4 sont spécifiques de l'AMPc.

Des composés inhibiteurs non spécifiques de phosphodiestérases sont connus, qui inhibent plusieurs familles d'enzymes. C'est le cas de certaines méthylxanthines comme la théophylline. Ces composés ont un index thérapeutique faible, notamment en raison de leur action sur des types de PDE présents dans des cellules autres que les cellules cibles. A l'inverse, certaines familles de PDE peuvent être inhibées sélectivement par divers agents pharmacologiques : l'hydrolyse des nucléotides cycliques est ralentie et donc leur concentration augmente dans les seules cellules où se trouve le type de PDE sensible à l'inhibiteur.

Un intérêt particulier se manifeste pour les phosphodiestérases 4 (PDE4), qui ont été identifiées dans de nombreux tissus dont le système nerveux central, le coeur, l'endothélium vasculaire, le muscle lisse vasculaire et celui des voies aériennes, les lignées myéloïdes et lymphoïdes.

Une augmentation de l'AMPc dans les cellules impliquées dans l'inflammation inhibe leur activation : inhibition de la synthèse et de la libération de médiateurs au niveau des mastocytes, des monocytes, des polynucléaires éosinophiles et basophiles, inhibition du chimiotactisme et de la dégranulation des polynucléaires neutrophiles et éosinophiles, inhibition des divisions et de la différenciation des lymphocytes.

Les cytokines, notamment TNF et interleukines, produites par différents types de leukocytes comme les lymphocytes T et les polynucléaires éosinophiles, jouent un rôle important dans le déclenchement des manifestations inflammatoires en particulier en réponse à une stimulation par un allergène au niveau des voies respiratoires.

D'autre part, l'AMPc diminue le tonus des fibres musculaires lisses des voies aériennes ; les inhibiteurs de PDE4 déterminent une bronchorelaxation.

La pneumopathie obstructive chronique (chronic obstructive pulmonary disease ou COPD en anglais) est une pathologie chronique, d'évolution lente, se caractérisant par l'obstruction des voies respiratoires (associée à une inflammation des voies respiratoires et à une numération élevée des neutrophiles). L'altération de la fonction pulmonaire est en grande partie irréversible (bien que des améliorations soient possibles avec un traitement par des bronchodilatateurs).

La présentation clinique de la pneumopathie obstructive chronique peut varier selon la gravité de l'atteinte, allant d'une simple bronchite chronique non invalidante à un état très invalidant à type d'insuffisance respiratoire chronique. Les caractéristiques cliniques principales des patients souffrant d'une pneumopathie obstructive chronique sont la bronchite chronique et/ou l'emphysème (associés à une inflammation des voies respiratoires et/ou une numération élevée des neutrophiles).

Au cours de ces dernières années, des inhibiteurs sélectifs de la phosphodiestérase 4 de seconde génération ont été proposés en tant qu'agents potentiellement efficaces dans le traitement de la pneumopathie obstructive chronique. Voir, entre autres, Doherty, *Chemical Biology* 1999, **3**:466-473 ; Mohammed et al, *Anti-inflammatory & Immunodilatory Investigational Drugs* 1999 **1**(1) :1-28 ; Schmidt et al, *Clinical and Experimental Allergy*, **29**, supplement 2, 99-109.

Ariflo, un inhibiteur de la PDE 4 actif par voie orale, a été proposé pour le traitement de la pneumopathie obstructive chronique. Voir, entre autres : Nieman et al, Am J Respir Crit Care Med 1998, **157** :A413 ; Underwood et al, Eur Respir J 1998, **12** :86s ; Compton et al,

Am J Respir Crit Care Med 1999, **159** :A522. Voir également l'exposé oral de Compton lors de la réunion de

l'"European Respiratory Society" qui s'est tenue à Madrid, le 12 octobre 1999, ainsi que celui de Torphy et Underwood lors du 4ème congrès mondial sur l'inflammation qui s'est tenu à Paris, du 27 au 30 juin 1999. Ariflo est actuellement à l'étude, dans des essais cliniques de phase III, pour le traitement de la pneumopathie obstructive chronique.

Toutefois, il convient de préciser qu'Ariflo présente quelques inconvénients. En effet, des effets indésirables significatifs, de type nausées et vomissements, ont été rapportés après administration d'une dose de 20 mg en prise unique. Voir Murdoch et al, Am J Respir Crit Care Med 1998, **157** :A409. L'apparition d'effets indésirables à des doses si faibles limitera le recours à Ariflo et empêchera l'utilisation de formes pharmaceutiques à dosage unique quotidien, conduisant ainsi à l'inconfort du patient.

L'ostéoporose est une maladie se caractérisant par une diminution de la masse osseuse et la perte de l'architecture squelettique, entraînant ainsi la fracture de l'os. Un grand nombre de femmes, au stade de la post-ménopause, souffrent de cette maladie et le nombre des patients ne cesse de croître.

Il existe deux types de cellules distinctes dans l'os : les ostéoblastes, qui participent à la formation de l'os ; et les ostéoclastes, qui jouent un rôle dans la résorption osseuse. Plus particulièrement, la masse osseuse résulte de la somme de la formation de l'os par les ostéoblastes et de la résorption de l'os par les ostéoclastes. Par conséquent, les molécules inhibant la résorption osseuse induite par les ostéoclastes sont efficaces dans le traitement de l'ostéoporose. La calcitonine, les biphosphonates et vraisemblablement les estrogènes sont des agents luttant contre la résorption et ils sont utilisés en clinique. Les molécules stimulant la formation de l'os par les ostéoblastes constituent également des agents prometteurs dans le traitement de l'ostéoporose. Voir aussi, Yoshihiro et al. Jpn. J. Pharmacolog. 1999, 79, 477 - 483.

Depuis quelques années une recherche extensive a été menée pour l'obtention et la mise au point d'inhibiteurs puissants de PDE4. Elle s'avère difficile du fait que beaucoup des inhibiteurs potentiels de PDE4 ne sont pas dénués d'activité sur les phosphodiestérases des autres familles.

A ce jour, le manque de sélectivité des inhibiteurs de PDE4 représente donc un problème important, étant donné l'étendue des fonctions régulées par l'AMPc. Il existe donc un besoin pour des inhibiteurs puissants et sélectifs de PDE4, c'est-à-dire n'ayant pas d'action vis-à-vis des PDE appartenant à d'autres familles.

Le brevet européen EP 0076199 décrit des composés ayant la formule générale suivante :

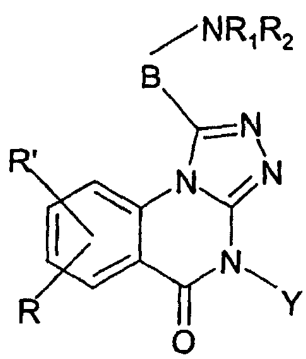

dans laquelle R et R', identiques ou différents, représentent H, halogène, alkyle $C_{1-3}$, alcoxy ou nitro ; Y représente un groupe alkyle, cycloalkyle $C_{3-8}$, alkenyle $C_{2-4}$, aryle ou aralkyle et B représente $(CH_2)_n$ avec n = 1, 2, 3 ou $CH(CH_3)$. L'utilisation de ces composés est proposée pour le traitement de l'asthme, la bronchite et les désordres allergiques.

Le brevet DDR158549 décrit des composés ayant la formule générale suivante :

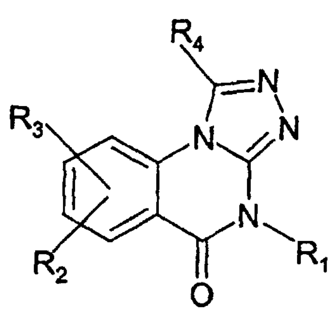

dans laquelle $R_1$ représente H, alkyle ou aryle ; $R_2$ et $R_3$ représentent H, alkyle, halogène, OH, SH, O-alkyle, S-alkyle ; $R_4$ représente H, alkyle, halogénoalkyle, OH, SH, O-alkyle, S-alkyle, $SO_2$-alkyle, $NH_2$, SCN, aryle, $(CH_2)_nCOO$alkyle et n = 0 à 2. L'utilisation de ces composés est proposée à titre de diurétiques et d'antianaphylactiques.

Ram et al., dans J.Prakt.Chem, 1990, 332(5), 629-39 décrivent des composés ayant la formule générale suivante :

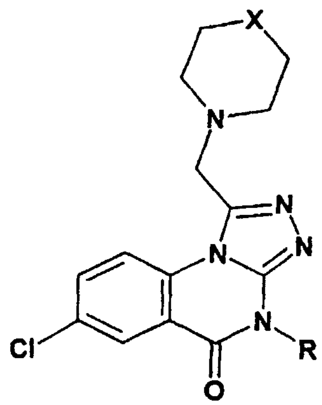

(X)

$R=CH_3$, $C_2H_5$

X= C, NH, N-$CH_3$, N-Ar

L'utilisation de ces composés est proposée pour le traitement de l'hypertension.

## Sommaire de l'invention

L'invention concerne les triazolo [4,3-a] quinazoline-5-ones et/ou -5-thiones de formule I ou II :

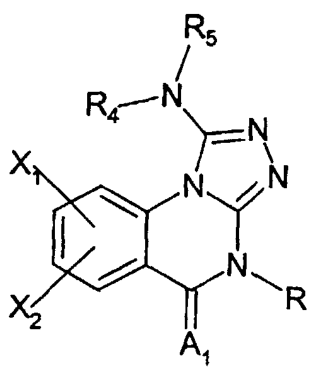

I

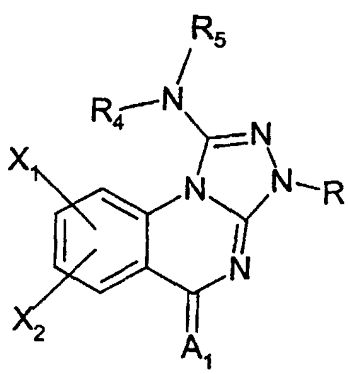

II

I et II étant des isomères de position du groupe R sur les azotes 3 ou 4, dans lesquelles :

- $A_1$ est O ou S ;
- $X_1$ et $X_2$, semblables ou différents, représentent :
  - hydrogène, hydroxy, halogène, amino, nitro, mercapto, cyano, carboxy,
  - alkyle inférieur, alcoxy inférieur ou $-S(O)_mR_8$ dans lequel m est 0, 1 ou 2 et $R_8$ est un alkyle inférieur, éventuellement substitués par un ou plusieurs atomes halogènes,

- $-CO-Q_1-Q_2-Q_3$ dans lequel :

  - $Q_1$- est : une liaison de valence simple, -O-,

    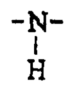 , 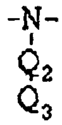 , ou 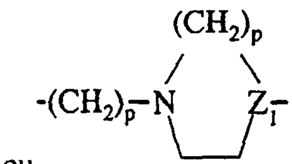 ,

    où p est un nombre entier pouvant varier de 0 à 3, et $Z_1$ est CH, N, O ou S,
  - $Q_2$- est :

    a) $-(CH_2)_q-$, q étant égal à 0, 1, 2, 3, ou 4, ou
    b) $-(CH_2-CH_2-O)_r-$, r étant égal à 2, 3, ou 4, et

  - $Q_3$ est : -H, -OH, alkoxy inférieur, -O-CO- $X_3$ $-NHX_3$ ou

    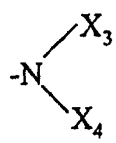

    dans lequel $X_3$ et $X_4$, semblables ou différents, représentent un groupement alkyle inférieur, $X_3$ et $X_4$ pouvant être liés pour former un cycle, comprenant un ou plusieurs hétéroatomes choisis parmi O, S ou N,
- $-NH-R_1$ dans lequel $R_1$ représente un groupement alkyle inférieur, éventuellement substitué par un ou plusieurs groupements choisis parmi halogène, hydroxy, cyano, alcoxy inférieur ou $-CO-Q_1-Q_2-Q_3$, ou
- $-NR_2R_3$ dans lequel $R_2$ et $R_3$, semblables ou différents, représentent un alkyle inférieur, éventuellement substitué par un ou plusieurs groupements hydroxy, halogène, cyano, alcoxy inférieur ou $-CO-Q_1-Q_2-Q_3$, $R_2$ et $R_3$ pouvant être liés pour former un cycle, comprenant un ou plusieurs hétéroatomes choisis parmi O, S ou N et éventuellement ponté par un alkyle inférieur, gem dialkylé ou substitué par un ou plusieurs groupements choisis parmi hydroxy, kéto, alkyle inférieur, alcoxy inférieur ou $-CO-Q_1-Q_2-Q_3$;

- R représente:

  - alkyle inférieur, alcényle inférieur, alcynyle inférieur, aryl alcynyle, 2-, 3- ou 4-pyridylalkyle éventuellement substitué par un alkyle inférieur, un alcoxy inférieur, un

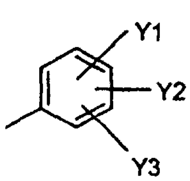 , 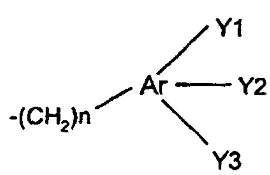 , 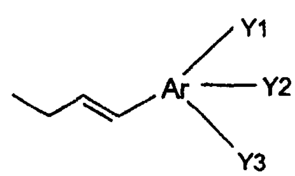

ou

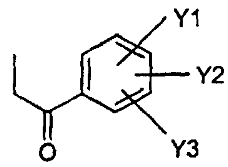

groupement hydroxy, halogène ou amino,
dans lesquels :

- n est un nombre entier de 1 à 5,
- Ar est un cycle aromatique comprenant 5 ou 6 atomes incluant de 0 à 3 hétéroatomes choisis parmi O, S ou N.
- Y1, Y2 et Y3, semblables ou différents représentent :
  - hydrogène, hydroxy, mercapto, amino, nitro, halogène,$-NHR_1$, $-NHR_2R_3$, $-(CH_2)_s^-$ CN, $-(CH_2)_s^-CO-Q_1-Q_2-Q_3$ dans lesquels s est un nombre entier de 0 à 6 ;
  - alkyle inférieur, alcoxy inférieur ou $—S(O)_mR_8$ dans lequel m est 0, 1 ou 2 et $R_8$ est un alkyle inférieur, chacun pouvant être éventuellement substitué par un ou plusieurs atomes halogènes ; et
- $R_4$ et $R_5$, représentent :
  - alkyle inférieur lorsque R4 et R5 sont semblables, aralkyle, cycloalkyle ou cycloalkyl alkyle, lorsque $R_4$ et $R_5$ sont différents,
  - alkyle inférieur, $R_4$ et $R_5$ pouvant être liés pour former un cycle saturé ou comportant une ou plusieurs doubles liaisons comprenant un ou plusieurs hétéroatomes choisis parmi O, S ou N et éventuellement substitué par un alkyle inférieur, un hydroxy ou un alkoxy inférieur ou ponté par un alkyle inférieur, gem dialkylé ou substitué par un ou plusieurs groupements choisis parmi hydroxy, kéto, alkyle inférieur, alcoxy inférieur, phényle alkyle ou $CO-Q_1-Q_2-Q_3$, deux des atomes du cycle ainsi formé pouvant également faire partie d'un autre cycle choisi parmi phényle ou hétéroaryle comportant de 4 à 8 atomes incluant 1 à 4 hétéroatomes ;

éventuellement leurs formes racémiques et leurs isomères, ainsi que leurs sels pharmaceutiquement acceptables.

Les composés de la présente invention sont utiles à titre d'inhibiteurs, notamment d'inhibiteurs sélectifs, de l'enzyme phosphodiestérase, et plus particulièrement l'enzyme PDE4.

L'invention concerne également des composés utilisés principalement à titre d'intermédiaires de synthèse des composés de formule I ou II.

Une première série d'intermédiaires comprend les composés ayant la formule générale III suivante :

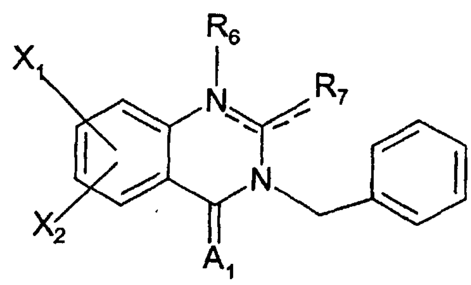

III

dans laquelle :

- $X_1$, $X_2$ et $A_1$ sont tels que définis précédemment ;
- les traits pointillés représentent des doubles liaisons optionnelles ;
- $R_6$ est hydrogène ; et
- $R_7$ est S ou hydrazino ;

$R_7$ pouvant être lié à l'azote en $R_6$ pour former un cycle, particulièrement un triazole, éventuellement substitué par un groupement thioalkyle inférieur, mercapto ou halogène.

Une deuxième série d'intermédiaires comprend les composés ayant la formule générale IV suivante :

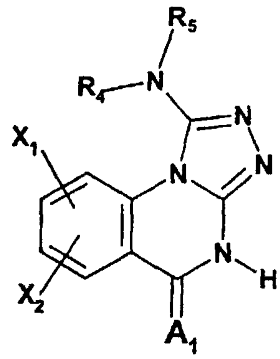

IV

dans laquelle $X_1$, $X_2$, $A_1$, $R_4$ et $R_5$ sont tels que définis précédemment.

Une troisième série d'intermédiaires comprend les composés ayant la formule générale V suivante :

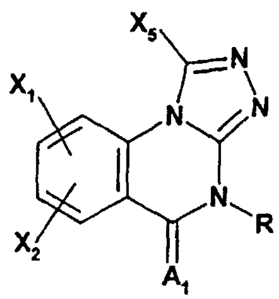

(V)

dans laquelle $X_1$, $X_2$, $A_1$ et R sont tels que définis précédemment et $X_5$ est un groupement halogène, particulièrement F, Br ou Cl, $-OCOX_7$, $-OSO_2X_7$ ou $-SO_2X_7$ dans lesquels $X_7$ est un groupement alkyle inférieur ou aryle .

Une quatrième série d'intermédiaires comprend les composés ayant la formule générale VI suivante :

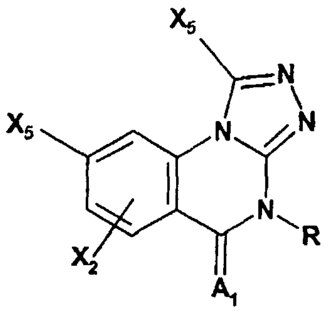

(VI)

dans laquelle $X_2$, $X_5$, $A_1$ et R sont tels que définis précédemment.

Une cinquième série d'intermédiaires comprend les composés ayant la formule générale VII suivante :

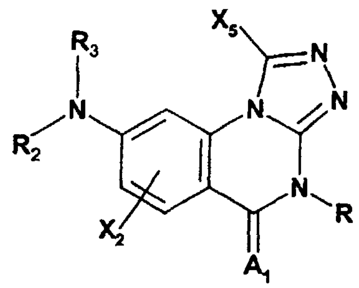

(VII)

dans laquelle $X_2$, $A_1$, $R_2$ et $R_3$ sont tels que définis précédemment $X_5$ est un groupement halogène, particulièrement F, Br ou Cl, $-OCOX_7$, $-OSO_2X_7$ ou $-SO_2X_7$ dans lesquels $X_7$ est un groupement alkyle inférieur ou aryle .

L'invention concerne également un procédé de fabrication des composés de formule I et II. Le procédé est caractérisé en ce qu'il comprend la réaction d'un composé de formule

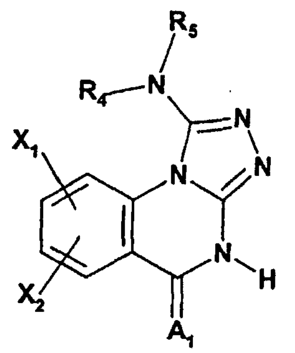

IV

générale IV :

dans laquelle $X_1$, $X_2$, $A_1$, $R_4$ et $R_5$ sont tels que définis précédemment, avec un composé de formule générale

R-X'

dans laquelle R est tel que défini précédemment et X' est un groupement halogène, particulièrement F, Br ou Cl, $-OCOX_7$ ou $-OSO_2X_7$ dans lesquels $X_7$ est un groupement alkyle inférieur ou aryle ;

pour obtenir un mélange des composés de formule générale I et II qui sont ensuite éventuellement séparés.

Les composés de formule générale I peuvent également être préparés par un procédé caractérisé en ce qu'il comprend la réaction d'un composé de formule générale V :

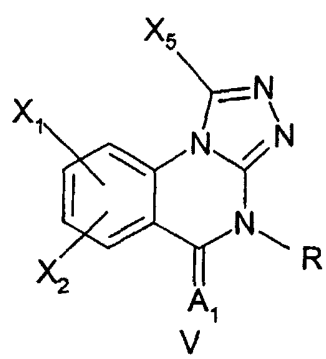

V

dans laquelle $X_1$, $X_2$, $A_1$ et R sont tels que définis précédemment et $X_5$ est un groupement halogène, particulièrement F, Br ou Cl, $-OCOX_7$, $-OSO_2X_7$ ou $-SO_2X_7$ dans lesquels $X_7$ est un groupement alkyle inférieur ou aryle ; avec un composé de formule générale :

$$HNR_4R_5$$

dans laquelle $R_4$ et $R_5$ sont tels que définis précédemment, pour obtenir un composé de formule générale I.

De façon particulière, lorsque $X_1$ est $-NR_2R_3$ et $-NR_2R_3$ et $-NR_4R_5$ sont identiques, les composés de formule I répondant à cette définition peuvent être obtenus en faisant réagir un composé de formule générale VI :

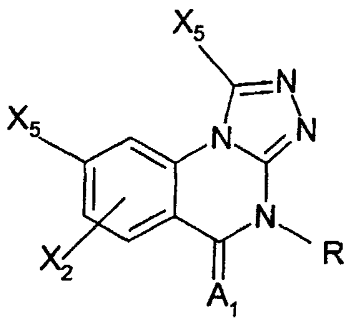

VI

dans laquelle $X_2$, $X_5$, $A_1$ et R sont tels que définis précédemment, avec un composé de formule générale :

$$HNR_2R_3$$

dans laquelle $R_2$ et $R_3$ sont tels que définis précédemment, pour obtenir un composé de formule générale (I):

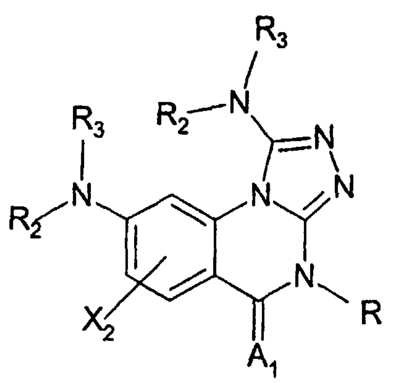

I

Egalement de façon particulière, lorsque $X_1$ est $-NR_2R_3$ et $-NR_2R_3$ et $-NR_4R_5$ sont différents, les composés de formule I répondant à cette définition peuvent être obtenus en faisant réagir un composé de formule générale VII :

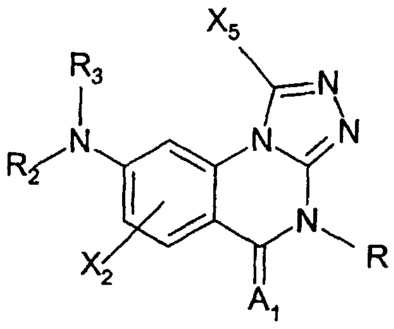

VII

dans laquelle $X_2$, $X_5$, $A_1$, R, $R_2$ et $R_3$ sont tels que définis précédemment, avec un composé de formule générale :

$$HNR_4R_5$$

dans laquelle $R_4$ et $R_5$ sont tels que définis précédemment, pour obtenir un composé de formule générale (I):

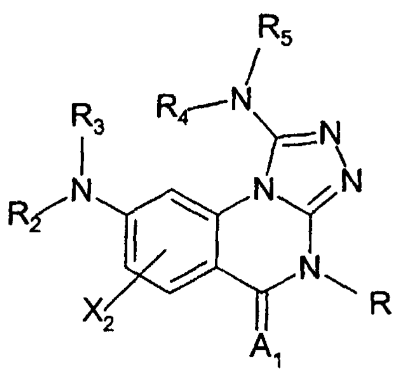

I

Egalement de façon particulière, lorsque $X_1$ est H et $X_2$ est OH, les composés de formule I répondant à cette définition peuvent être obtenus en soumettant un composé de formule générale $Ia_1$:

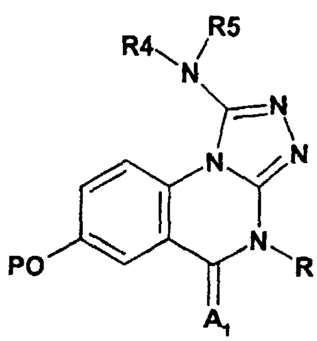

($Ia_1$)

dans laquelle $A_1$, R, $R_4$ et $R_5$ sont tels que définis précédemment et P est un groupement protecteur, à des conditions permettant l'élimination du groupement protecteur P pour obtenir un composé de formule générale I.

Egalement de façon particulière, lorsque $X_1$ est H et $X_2$ est $NH_2$, les composés de formule I répondant à cette définition peuvent être obtenus en soumettant un composé de formule générale $Ia_2$ :

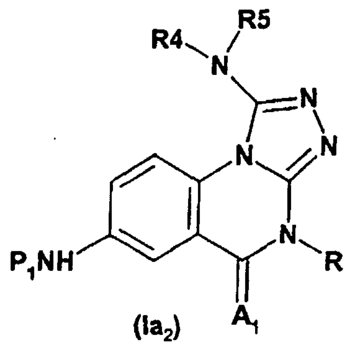

($Ia_2$)

dans laquelle $A_1$, R, $R_4$ et $R_5$ sont tels que définis précédemment et $P_1$ est un groupement protecteur, à des conditions permettant l'élimination du groupement protecteur $P_1$ pour obtenir un composé de formule générale I.

Egalement de façon particulière, lorsque $X_1$ est H et $X_2$ est $NHR_2$ dans lequel $R_2$ est tel que défini précédemment, les composés de formule I répondant à cette définition peuvent être obtenus en faisant réagir un composé de formule générale Ib :

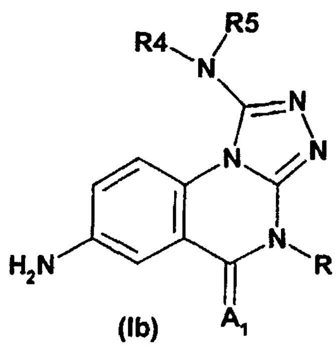

(Ib)

dans laquelle $A_1$, R, $R_4$ et $R_5$ sont tels que définis précédemment, avec un composé de formule $R_2X_5$ dans laquelle $R_2$ et $X_5$ sont tels que définis précédemment, pour obtenir un composé de formule générale I.

De plus, lorsque $X_1$ est H et $X_2$ est $NHR_2$ dans lequel $R_2$ est tel que défini précédemment, les composés de formule I répondant à cette définition peuvent également être obtenus en soumettant un composé de formule générale $Ib_2$ :

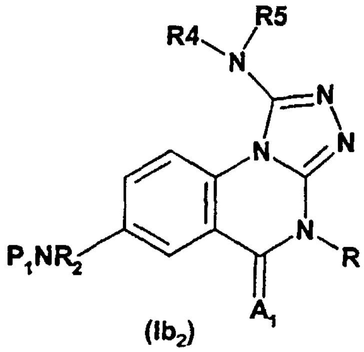

$(Ib_2)$

dans laquelle $A_1$, R, $R_4$ et $R_5$ sont tels que définis précédemment et $P_1$ est un groupement protecteur, à des conditions permettant l'élimination du groupement protecteur, pour obtenir un composé de formule générale I.

Egalement de façon particulière, lorsque $X_1$ est H et $X_2$ est $NR_2R_x$ dans lequel $R_2$ est tel que défini précédemment et $R_x$, représente $R_2$ ou $R_3$ tels que définis précédemment, les composés de formule I répondant à cette définition peuvent être obtenus en faisant réagir un composé de formule générale Ic :

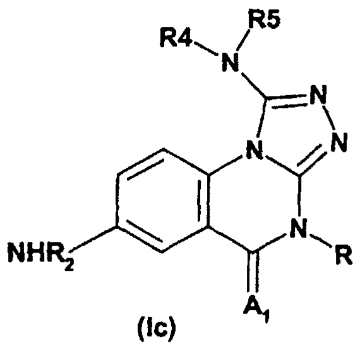

(Ic)

dans laquelle $A_1$, R, $R_2$, $R_4$ et $R_5$ sont tels que définis précédemment, avec un composé de formule $R_xX_5$ dans laquelle $R_x$ et $X_5$ sont tels que définis précédemment, pour obtenir un composé de formule générale I.

Egalement de façon particulière, lorsque R est

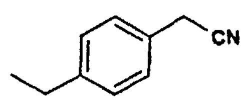

les composés de formule I répondant à cette définition peuvent être obtenus en déshydratant un composé de formule générale Ig :

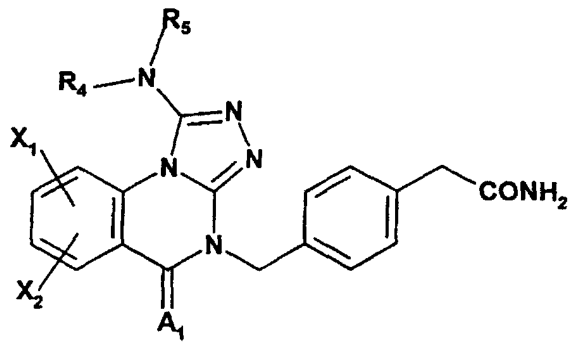

(Ig)

dans laquelle $X_1$, $X_2$, $A_1$, $R_4$ et $R_5$ sont tels que définis précédemment, pour obtenir le composé de formule générale I.

Egalement de façon particulière, lorsque R est

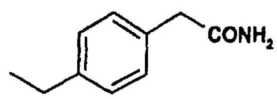

les composés de formule I répondant à cette définition peuvent être obtenus en faisant réagir un composé de formule générale If :

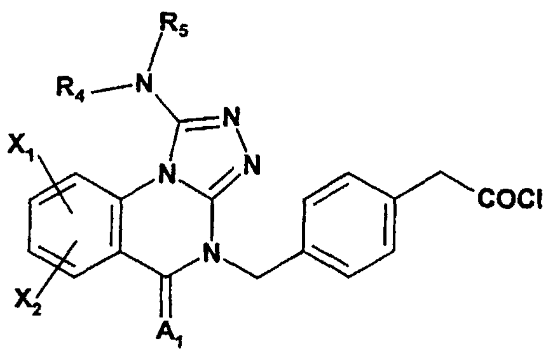

(If)

dans laquelle $X_1$, $X_2$, $A_1$, $R_4$ et $R_5$ sont tels que définis précédemment, avec de l'ammoniaque pour obtenir le composé de formule générale I.

Egalement de façon particulière, lorsque R est

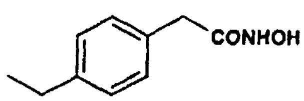

les composés de formule I répondant à cette définition peuvent être obtenus en faisant réagir un composé de formule générale If avec de l'hydroxylamine pour obtenir le composé de formule générale I.

Egalement de façon particulière, lorsque R est

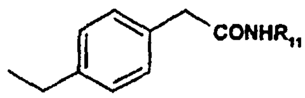

les composés de formule I répondant à cette définition peuvent être obtenus en faisant réagir un composé de formule générale If avec le composé de formule $R_{11}NH_2$ dans laquelle $R_{11}$ a la même signification que $R_2$, pour obtenir le composé de formule générale I.

Egalement de façon particulière, lorsque R est

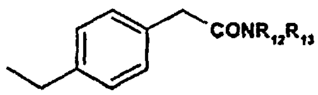

les composés de formule I répondant à cette définition peuvent être obtenus en faisant réagir un composé de formule générale If avec le composé de formule $HNR_{12}R_{13}$ dans laquelle $R_{12}$ et $R_{13}$ ont la même signification que $R_4$ et $R_5$ respectivement, pour obtenir le composé de formule générale I.

L'invention concerne également une composition pharmaceutique comprenant un composé de formule I ou II et un excipient acceptable en pharmacie.

L'invention concerne également l'utilisation d'un composé de formule I ou II pour la préparation d'un médicament destiné au traitement d'une maladie ou d'une affection relevant d'une thérapie par l'inhibition de phosphodiestérases, et plus particulièrement de la PDE4.

L'invention concerne également une méthode de traitement d'une maladie ou d'une affection relevant d'une thérapie par l'inhibition de phosphodiestérases, et plus particulièrement de la PDE4, ladite méthode comprenant l'administration à un patient d'une concentration efficace d'un composé de formule I ou II.

## Description détaillée de l'invention

La présente invention concerne donc les composés de formule générale I ou II :

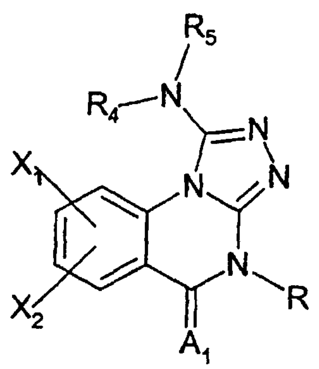

I

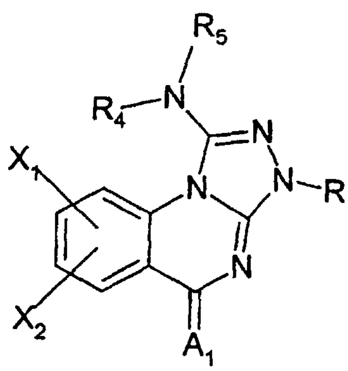

II

dans lesquels $X_1$, $X_2$, $A_1$, R, $R_4$ et $R_5$ sont tels que définis précédemment.

L'invention concerne particulièrement les composés de formule générale I ou II dans lesquels :

$A_1$ représente un atome d'oxygène ;
$X_1$ représente un atome d'hydrogène et $X_2$ est un groupement halogène, amino, alkyle inférieur, hydroxy ou $-NHR_1$, R1 étant tel que défini précédemment.
R représente :

- un groupement alkyle inférieur, alcényle inférieur, aryl alcynyle, 2-, 3- ou 4-pyridylalkyl éventuellement substitué sur le noyau pyridine par un alkyle inférieur, un halogène ou un hydroxy ;

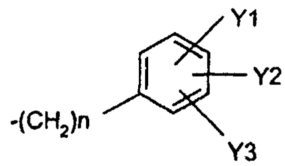

dans lequel :

- n est un nombre entier de 1 à 3,
- Y1, Y2 et Y3 représentent chacun un atome d'hydrogène ou un groupement alcoxy inférieur, plus particulièrement méthoxy,
- Y1 et Y2 représentent chacun un atome d'hydrogène et Y3 représente un groupement alcoxy inférieur, un groupement amino, nitro, hydroxy, un groupement $-(CH_2)_sCO-Q_1-Q_2-Q_3$, un groupement $(CH_2)_s-CN$ dans lesquels s, $Q_1$, $Q_2$, $Q_3$ sont tels que définis précédemment, ou un groupement alkyle inférieur éventuellement substitué par un ou plusieurs atomes d'halogène, la position particulièrement préférée du substituant Y3 étant la position 4, ou,
- Y1 représente un atome d'hydrogène et Y2 et Y3, semblables ou différents, représentent un groupement hydroxy, halogène ou alcoxy inférieur, ou

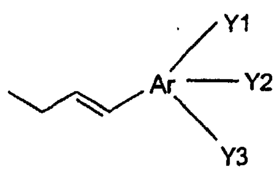

dans lequel :

- Ar est tel que défini précédemment ;
- Y1, Y2 et Y3 représentent chacun un atome d'hydrogène, ou
- Y1 et Y2 représentent chacun un atome d'hydrogène et Y3 est alcoxy inférieur

ou halogène ;

$R_4$ et $R_5$, représentent :

- alkyle inférieur lorsque R4 et R5 sont semblables, aralkyle, cycloalkyle ou cycloalkyl alkyle, lorsque $R_4$ et $R_5$ sont différents,
- alkyle inférieur, $R_4$ et $R_5$ pouvant être liés pour former un cycle saturé ou comportant une ou plusieurs doubles liaisons comprenant un ou plusieurs hétéroatomes choisis parmi O, S ou N et éventuellement substitué par un alkyle inférieur, un hydroxy ou un alkoxy inférieur ou ponté par un alkyle inférieur, gem dialkylé ou substitué par un ou plusieurs groupements choisis parmi hydroxy, kéto, alkyle inférieur, alcoxy inférieur, phényle alkyle ou $CO-Q_1-Q_2-Q_3$, deux des atomes du cycle ainsi formé pouvant également faire partie d'un autre cycle choisi parmi phényle ou hétéroaryle comportant de 4 à 8 atomes incluant 1 à 4 hétéroatomes ;

L'invention concerne plus particulièrement les composés de formule générale I dans lesquels :

$X_1$ représente un atome d'hydrogène,
$X_2$ représente un groupement halogène, amino, alkyle inférieur, hydroxy ou $--NHR_1$;
R représente :

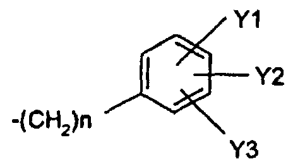

dans lequel :

- n est un nombre entier de 1 à 3,
- Y1, Y2 et Y3 représentent chacun un atome d'hydrogène ou un groupement alcoxy inférieur, plus particulièrement méthoxy et en particulier le 3, 4, 5-triméthoxy,
- Y1 et Y2 représentent chacun un atome d'hydrogène et Y3 représente un groupement alcoxy inférieur, amino, nitro, ou hydroxy, un groupement alkyle inférieur éventuellement substitué par un ou plusieurs atomes d'halogène, un groupement $-(CH_2)_sCO-Q_1-Q_2-Q_3$ dans lequel s est 0 ou 1, $Q_1$ est O, -NH- ou une liaison de valence, $Q_2$ est $-(CH_2)_q-$, q étant égal à 0, 1, 2, 3 ou 4 et $Q_3$ est H, OH ou $-NX_3X_4$ dans lequel $X_3$ et $X_4$ sont tels que définis précédemment, un groupement $(CH_2)_s-CN$ dans lequel s est 0 ou 1, la position particulièrement préférée du substituant Y3 étant la position 4, ou
- Y1 représente un atome d'hydrogène et Y2 et Y3, semblables ou différents, représentent un groupement hydroxy, halogène ou alcoxy inférieur; ou

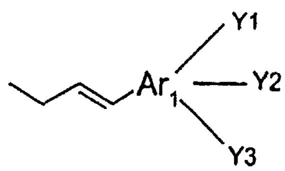

dans lequel :

- $Ar_1$ est un cycle aromatique comprenant 6 atomes pouvant inclure un atome d'azote en position 2, 3 ou 4 et de préférence en position 3 ;
- Y1, Y2 et Y3 représentent chacun un atome d'hydrogène, ou
- Y1 et Y2 représentent chacun un atome d'hydrogène et Y3 est un groupement alcoxy inférieur ou un groupement halogène lorsque $Ar_1$ ne comprend pas d'atome d'azote ; et

$R_4$ et $R_5$, représentent :

- alkyle inférieur lorsque R4 et R5 sont semblables, aralkyle, cycloalkyle ou cycloalkyl alkyle, lorsque $R_4$ et $R_5$ sont différents,
- alkyle inférieur, $R_4$ et $R_5$ pouvant être liés pour former un cycle saturé ou comportant une ou plusieurs doubles liaisons comprenant un ou plusieurs hétéroatomes choisis parmi O, S ou N et éventuellement substitué par un alkyle inférieur, un hydroxy ou un alkoxy inférieur ou ponté par un alkyle inférieur, gem dialkylé ou substitué par un ou plusieurs groupements choisis parmi hydroxy, kéto, alkyle inférieur, alcoxy inférieur, phényle alkyle ou $CO-Q_1-Q_2-Q_3$, deux des atomes du cycle ainsi formé pouvant également faire partie d'un autre cycle choisi parmi phényle ou hétéroaryle comportant de 4 à 8 atomes incluant 1 à 4 hétéroatomes ;

L'invention concerne également des composés de formule générale I ou II dans lesquels :

$X_1$, $X_2$, $A_1$, $R_4$ et $R_5$ sont tels que définis dans le sommaire de l'invention ; et

R représente :

- alcynyle inférieur, aryl alcynyle, 2-, 3- ou 4-pyridylalkyle éventuellement substitué par un alkyle inférieur, un alcoxy inférieur, un groupement hydroxy ou halogène,

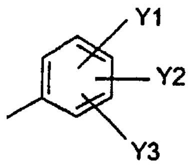 , 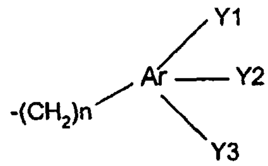 , 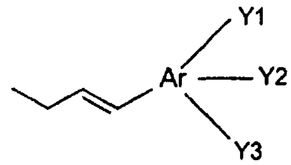 ,

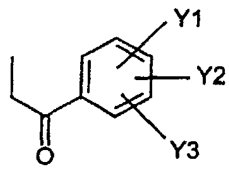 , 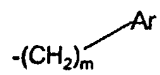 ou 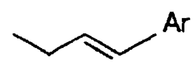

dans lesquels :

- n est un nombre entier de 1 à 5 et m est un nombre entier de 3 à 5 ;
- Ar est un cycle aromatique comprenant 5 ou 6 atomes incluant de 0 à 3 hétéroatomes choisis parmi O, S ou N ;
- Y1, Y2 et Y3, semblables ou différents représentent :
    - hydroxy, mercapto, amino, nitro, halogène, $-(CH_2)_sCO-Q_1-Q_2-Q_3$, $(CH_2)_s-CN$, dans lesquels s est un nombre entier de 0 à 6, alkyle inférieur, alcoxy inférieur ou thioalkyle inférieur, éventuellement substitués par un ou plusieurs atomes halogènes.

Dans une autre de ses réalisations, la présente invention concerne des composés de formule générale I ou II dans lesquels :

$X_1$, $X_2$, $R_4$ et $R_5$ sont tels que définis dans le sommaire de l'invention ; et

R représente :

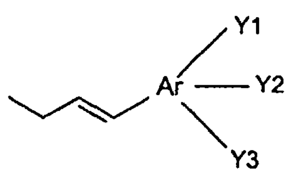

dans lequel :

- Ar est un cycle aromatique comprenant 5 ou 6 atomes incluant de 0 à 3 hétéroatomes choisis parmi O, S ou N (les cycles aromatiques comprenant 6 atomes, dont éventuellement un atome d'azote en position 2, 3 ou 4, de préférence en position 3, étant particulièrement préférés);
- Y1, Y2 et Y3, semblables ou différents représentent :
    - hydrogène, hydroxy, mercapto, amino, nitro, halogène, cyano, $-(CH_2)_sCO-Q_1-Q_2-Q_3$ dans lequel s est un nombre entier de 0 à 6, alkyle inférieur, alcoxy inférieur ou thioalkyle inférieur, éventuellement substitués par un ou plusieurs atomes halogènes.

De façon préférée :

- Y1, Y2 et Y3 représentent chacun un atome d'hydrogène, ou
- Y1 et Y2 représentent chacun un atome d'hydrogène et Y3 est alcoxy inférieur ou halogène.

Dans une autre de ses réalisations, la présente invention concerne des composés de formule générale I ou II dans lesquels :

$X_1$, $X_2$, $A_1$, $R_4$ et $R_5$ sont tels que définis dans le sommaire de l'invention ; et

R représente:

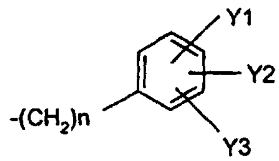

dans lequel :

- n est un nombre entier de 1 à 3 ;
- Y1, Y2 et Y3, semblables ou différents représentent :
  - hydroxy, mercapto, amino, nitro, halogène, $-(CH_2)_sCO-Q_1-Q_2-Q_3$, $(CH_2)_s-CN$ dans lesquels s est un nombre entier de 0 à 6, alkyle inférieur, alcoxy inférieur ou thioalkyle inférieur, éventuellement substitués par un ou plusieurs atomes halogènes.

De façon préférée :

- n est un nombre entier de 1 à 3,
- Y1, Y2 et Y3 représentent chacun un groupement alcoxy inférieur, plus particulièrement méthoxy et en particulier le 3, 4, 5-triméthoxy,
- Y1 et Y2 représentent chacun un atome d'hydrogène et Y3 représente un groupement alcoxy inférieur, cyano, amino, nitro ou hydroxy, un groupement alkyle inférieur éventuellement substitué par un ou plusieurs atomes d'halogène ou un groupement $-(CH_2)_sCO-Q_1-Q_2-Q_3$ dans lequel s est 0 ou 1, $Q_1$ est O, -NH- ou une liaison de valence, $Q_2$ est $-(CH_2)_q-$, q étant égal à 0, 1, 2, 3 ou 4 et $Q_3$ est H, OH ou $-NX_3X_4$ dans lequel $X_3$ et $X_4$ sont tels que définis précédemment, la position particulièrement préférée du substituant Y3 étant la position 4, ou
- Y1 représente un atome d'hydrogène et Y2 et Y3, semblables ou différents, représentent un groupement hydroxy, halogène ou alcoxy inférieur.

Dans une autre de ses réalisations, la présente invention concerne des composés de formule générale I ou II dans lesquels :

$X_1$, $X_2$, $A_1$, $R_4$ et $R_5$ sont tels que définis dans le sommaire de l'invention ; et

R représente :

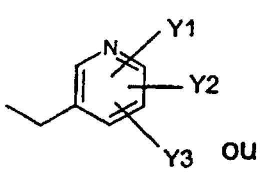 ou 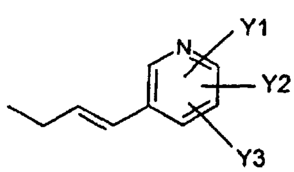

dans lesquels :

- Y1, Y2 et Y3, semblables ou différents représentent :

- hydrogène, hydroxy, mercapto, amino, nitro, halogène, $-(CH_2)_sCO-Q_1-Q_2-Q_3$, $(CH_2)_s-CN$, dans lesquels s est un nombre entier de 0 à 6, alkyle inférieur, alcoxy inférieur ou thioalkyle inférieur, éventuellement substitués par un ou plusieurs atomes halogènes.

De façon préférée :

- Y1, Y2 et Y3 représentent chacun un atome d'hydrogène, ou
- Y1 et Y2 représentent chacun un atome d'hydrogène et Y3 est alcoxy inférieur ou halogène.

Dans une autre de ses réalisations, la présente invention concerne des composés de formule générale I ou II dans lesquels :

$X_1$, $X_2$, $A_1$, R, $R_4$ et $R_5$ sont tels que définis dans le sommaire de l'invention ; et :

- lorsque $X_1$ et $X_2$ représentent hydrogène, R n'est pas alkyle, phényle, benzyle ou allyle,
- lorsque $X_1$ représente hydrogène et $X_2$ représente 7-Cl ou $CH_3$, R n'est pas un alkyle ; et
- lorsque $X_1$ représente hydrogène, $X_2$ n'est pas 8-Cl.

L'invention concerne également un groupe de composés de formule I ou II particulièrement actifs à titre d'inhibiteurs de TNF$\alpha$ et dans lesquels :

- $A_1$ est O ou S ;
- $X_1$ et $X_2$, semblables ou différents, représentent :
  - hydrogène, hydroxy, halogène, amino, nitro, mercapto, cyano, carboxy,
  - alkyle inférieur, alcoxy inférieur ou $-S(O)_mR_8$ dans lequel m est 0, 1 ou 2 et $R_8$ est un alkyle inférieur, éventuellement substitués par un ou plusieurs atomes halogènes.
  - De façon préférée, $X_1$ est H et $X_2$ est halogène, notamment, 7-Br, ou alkyle inférieur, notamment 7-$CH_3$.
- R représente:

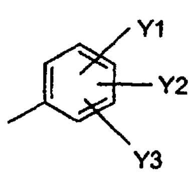

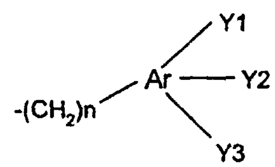

ou

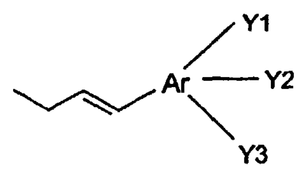

dans lesquels :

- n est un nombre entier de 1 à 5,
- Ar est un cycle aromatique comprenant 5 ou 6 atomes incluant de 0 à 3 hétéroatomes choisis parmi O, S ou N,
- Y1, Y2 et Y3, semblables ou différents représentent :
- hydrogène, hydroxy, mercapto, amino, nitro, halogène, $-(CH_2)_sCO-Q_1-Q_2-Q_3$, $(CH_2)_s-CN$ dans lesquels s est un nombre entier de 0 à 6 ;
- alkyle inférieur, alcoxy inférieur ou $-S(O)_mR_8$ dans lequel m est 0, 1 ou 2 et $R_8$ est un alkyle inférieur, éventuellement substitués par un ou plusieurs atomes halogènes.

Les substituants particulièrement préférés formant le groupement R incluent cinnamyl, 3-pyridyl allyl, paracyano benzyle, diméthoxy benzyle et 3-pyridyl méthyl.

- $R_4$ et $R_5$, semblables ou différents, représentent :

alkyle inférieur, $R_4$ et $R_5$ pouvant être liés pour former un cycle saturé ou comportant une ou plusieurs doubles liaisons comprenant un ou plusieurs hétéroatomes choisis parmi O, S ou N et éventuellement ponté par un

alkyle inférieur, gem dialkylé ou substitué par un ou plusieurs groupements choisis parmi hydroxy, kéto, alkyle inférieur, alcoxy inférieur, phényle alkyle ou $CO-Q_1-Q_2-Q_3$. Les substituants particulièrement préférés formant le groupe $NR_4R_5$ incluent diméthylamino, pyrrolidine et azepanyl.

Les composés particulièrement préférés à titre d'inhibiteurs de TNFα incluent les molécules suivantes :

| | |
|---|---|
| 3 | 7-Bromo-1-dimethylamino-4-((E)-3-pyridin-3-yl-allyl)-4H-[ 1,2,4]triazolo[4,3-a]quinazolin-5-one |
| 104 | 1-Dimethylamino-7-methyl-4-(3-pyridin-3-yl-allyl)-4H-[1,2,4]triazolo[4,3-a]qumazolin-5-one |
| 94 | 4-(3,4-Dimethoxy-benzyl)-7-methyl-1-pyrrolidin-1-yl-4H-[ 1,2,4]triazolo[4,3-a]quinazolin-5-one |
| 101 | 4-(1-Dimethylamino-7-methyl-5-oxo-5H-[1,2,4]triazolo[4,3-a]quinazolin-4-ylmethyl)-benzonitrile |
| 85 | 7-Bromo-1-dimethylamino-4-(3-phenyl-allyl)-4H-[1,2,4]triazolo[4,3-a]quinazolin-5-one |
| 98 | 7-Methyl-4-(3-phenyl-allyl)-1-pyrrolidin-1-yl-4H-[1,2,4]triazolo[4,3-a]quinazolin-5-one |
| 79 | 4-(7-Bromo-1-dimethylamino-5-oxo-5H-[1,2,4]triazolo[4,3-a]quinazolin-4-ylmethyl)-benzonitrile |
| 91 | 1-Azepan-1-yl-7-methyl-4-pyridin-3-ylmethyl-4H-[1,2,4]triazolo[4,3-a]quinazolin-5-one |
| 93 | 4-(7-Methyl-5-oxo-1-pyrrolidin-1-yl-5H-[1,2,4]triazolo[4,3-a]quinazolin-4-ylmethyl)-benzonitrile |
| 103 | 1-Dimethylamino-7-methyl-4-((E)-3-phenyl-allyl)-4H-[ 1,2,4]triazolo[4,3-a]quinazolin-5-one |
| 46 | 4-(7-Bromo-5-oxo-1-pyrrolidin-1-yl-5H-[1,2,4]triazolo[4,3-a]quinazolin-4-ylmethyl)-benzonitrile |
| 35 | 1-Azepan-1-yl-7-bromo-4-(3,4-dimethoxy-benzyl)-4H-[1,2,4]triazolo[4,3-a]quinazolin-5-one |

Parmi les groupements définis ci-dessus les substituants suivants sont particulièrement préférés :

- De façon générale pour les groupements $X_1$, $X_2$, $X_3$, $X_4$, R, $R_1$, $R_2$, $R_3$, $R_4$, $R_5$ :
  - halogène : F, Cl, Br, I, de préférence Br et Cl,
  - alkyle inférieur : linéaire ou ramifié comportant de 1 à 6, de préférence de 1 à 3 atomes de carbone,
  - alcoxy inférieur : linéaire ou ramifié comportant de 1 à 5, de préférence de 1 à 3 atomes de carbone,
  - alkylthio inférieur : linéaire ou ramifié comportant de 1 à 5, de préférence de 1 à 3 atomes de carbone,
  - alcényle inférieur : comportant de 3 à 6, de préférence de 3 à 4 atomes de carbone, plus particulièrement allyle,
  - alcynyle inférieur: comportant de 3 à 9 atomes de carbone, plus particulièrement propargyle et phényl-propargyle,
  - 2-, 3- ou 4-pyridylalkyle dans lequel l'alkyle comporte de 1 à 5, de préférence de 1 à 3 atomes de carbone,
  - aryle : comportant de 5 à 8, de préférence 5 ou 6 atomes,
  - aralkyle dans lequel l'alkyle comporte de 1 à 6, de préférence de 1 à 4 atomes de carbone,
  - cycloalkyle : comportant de 3 à 8, de préférence de 3 à 6 atomes de carbone,
  - cycloalkyl alkyle dans lequel l'alkyle comporte de 1 à 6, de préférence de 1 à 3 atomes de carbone et le cycloalkyle comporte de 3 à 8, de préférence de 3 à 6 atomes de carbone,
  - alkyle inférieur, alcoxy inférieur ou alkylthio inférieur éventuellement substitués par un ou plusieurs atomes halogènes: on préférera les groupements trisubstitués de type $-(CH_2)_p-CF_3$, $-O-(CH_2)_p-CF_3$ ou $-S-(CH_2)_p-CF_3$, dans lesquels p est un nombre entier de 0 à 3.

- De façon particulière pour les groupements $X_1$ et $X_2$ :
  - $-NH-R_1$, ou $-NR_2R_3$ : lorsque l'alkyle inférieur est substitué par un ou plusieurs groupements choisis parmi halogène, hydroxy, cyano, alcoxy inférieur ou $CO-Q_1-Q_2-Q_3$, le nombre de substituants varie entre 1 et 4, de préférence entre 1 et 2,
  - $-NR_2R_3$ : lorsque $R_2$ et $R_3$ sont liés pour former un cycle, ce cycle est caractérisé en ce qu'il comprend de préférence :
    - entre 1 et 4, plus particulièrement entre 1 et 2 hétéroatomes choisis parmi O, S ou N, les substituants cycliques de ce type étant, de façon préférée, les cycles saturés de type $C_mN$ dans lequel m est un nombre entier de 2 à 7, de préférence de 4 à 6, les cycles particulièrement préférés étant choisis parmi le groupe comportant pyrrolidine, piperidine, homopipéridine ou cyclooctylamine et
    - entre 0 et 4, de façon préférée entre 0 et 2, plus particulièrement entre 1 et 2 substituants choisis parmi hydroxy, kéto, alkyle inférieur, alcoxy inférieur ou $-CO-Q_1-Q_2-Q_3$,

  - les groupements $X_1$ et $X_2$ sont particulièrement situés en position 7 et 8 du cycle aromatique des composés

de formule I et II auquel ils sont liés.

- De façon particulière pour le groupement R :
  - les substituants Y1, Y2 et Y3 sont particulièrement situés en position 3 et/ou 4 du cycle aromatique auquel ils sont liés.
- De façon particulière pour les groupements $R_4$ et $R_5$ :
  - lorsque $R_4$ et $R_5$ sont liés pour former un cycle, ce cycle est caractérisé en ce qu'il comprend de préférence :
    - entre 1 et 4 hétéroatomes choisis parmi O, S ou N, les substituants cycliques de ce type étant, de façon préférée, les cycles saturés de type $C_mN$, m étant un nombre entier de 2 à 7, les cycles particulièrement préférés étant choisis parmi le groupe comportant pyrrolidine, piperidine, homopipéridine ou cyclooctylamine, et
    - entre 0 et 4, de façon préférée entre 0 et 2 substituants choisis parmi hydroxy, kéto, alkyle inférieur, alcoxy inférieur ou -CO-$Q_1$-$Q_2$-$Q_3$.

Parmi les composés préférés de la présente invention, on retrouve les composés suivants :

| | |
|---|---|
| 1 | 1-(Azepan-1-yl)-7-chloro-4-(3-phenylallyl)-4H-[1,2,4]triazolo[4,3-a]quinazolin-5-one |
| 2 | 1-(azepan-1-yl)-7-chloro-3-(3-phenylallyl)-3H-[1,2,4]triazolo[4,3-a]quinazolin-5-one |
| 3 | 7-Bromo-1-dimethylamino-4-((E)-3-pyridin-3-yl-allyl)-4H-[1,2,4]triazolo[4,3-a]quinazolin-5-one |
| 4 | 7-Bromo-4-pyridin-3-ylmethyl-1-pyrrolidin-1-yl-4H-[1,2,4]triazolo[4,3-a]quinazolin-5-one |
| 5 | 7-Bromo-3-pyridin-3-ylmethyl-1-pyrrolidin-1-yl-3H-[1,2,4]triazolo[4,3-a]quinazolin-5-one |
| 6 | 1-Azepan-1-yl-4-(3-phenyl-allyl)-4H-[1,2,4]triazolo[4,3-a]quinazolin-5-one |
| 7 | 1-(azepan-1-yl)-7-chloro-4-allyl-4H-[1,2,4]triazolo[4,3-a]- quinazolin-5-one |
| 8 | 1-(azepan-1-yl)-7-chloro-4-(4-methylbenzyl)-4H-[1,2,4]triazolo[4,3-a]quinazolin-5-one |
| 9 | 1-(azepan-1-yl)-7-chloro-4-(2-chlorobenzyl)-4H-[1,2,4]triazolo[4,3-a]quinazolin-5-one |
| 10 | 1-(azepan- 1-yl)-7-chloro-4-(3-chlorobenzyl)-4H-[1,2,4]triazolo[4,3-a]quinazolin-5-one |
| 11 | 1-(azepan-1-yl)-7-chloro-4-(4-chlorobenzyl)-4H-[1,2,4]triazolo[4,3-a]quinazolin-5-one |
| 12 | 1-(azepan-1-yl)-7-chloro-4-(4-bromobenzyl)-4H-[1,2,4]triazolo[4,3-a]quinazolin-5-one |
| 13 | 1 -(azepan-1-yl)-7-chloro-4-(4-fluorobenzyl)-4H-[ 1,2,4]triazolo[4,3-a]quinazolin-5-one |
| 14 | 1-(azepan-1-yl)-7-chloro-4-(4-(trifluoromethyl)benzyl)-4H-[ 1,2,4]triazolo[4,3-a]quinazolin-5-one |
| 15 | 1-(azepan-1-yl)-7-chloro-4-(4-cyanobenzyl)-4H-[1,2,4]triazolo[4,3-a]quinazolin-5-one |
| 16 | 1-(azepan-1-yl)-7-chloro-4-(2-methoxybenzyl)-4H-[1,2,4]triazolo[4,3-a]quinazolin-5-one |
| 17 | 1-(azepan-1-yl)-7-chloro-4-(3-methoxybenzyl)-4H-[1,2,4]triazolo[4,3-a]quinazolin-5-one |
| 18 | 1-(azepan-1-yl)-7-chloro-4-(4-methoxybenzyl)-4H-[1,2,4]triazolo[4,3-a]quinazolin-5-one |
| 19 | 1-(azepan-1-yl)-7-chloro-4-(3,4-dichlorobenzyl)-4H-[1,2,4]triazolo[4,3-a]quinazolin-5-one |
| 20 | 1-(azepan-1-yl)-7-chloro-4-(3,4-dimethoxybenzyl)-4H-[ 1,2,4]triazolo[4,3-a]quinazolin-5-one |
| 21 | 1-(azepan-1-yl)-7-chloro-4-(2-pyridylmethyl)-4H-[1,2,4]triazolo[4,3-a]quinazolin-5-one |
| 22 | 1-(azepan-1-yl)-7-chloro-4-(3-pyridylmethyl)-4H-[1,2,4]triazolo[4,3-a]quinazolin-5-one |
| 23 | 1-(azepan-1-yl)-7-chloro-4-(4-pyridylmethyl)-4H-[1,2,4]triazolo[4,3-a]quinazolin-5-one |
| 24 | 1-(azepan-1-yl)-7-chloro-4-(2-phenylethyl)-4H-[1,2,4]triazolo[4,3-a]quinazolin-5-one |
| 25 | 1-(azepan-1-yl)-7-chloro-4-[2-(4-methoxyphenyl)ethyl]-4H-[1,2,4]triazolo[4,3-a]quinazolin-5-one |
| 26 | 1-(azepan-1-yl)-7-chloro-4-(3-phenylpropyl)-4H-[1,2,4]triazolo[4,3-a]quinazolin-5-one |
| 27 | 1-Azepan-1-yl-7-chloro-4-(2-oxo-2-phenyl-ethyl)-4H-[1,2,4]triazolo[4,3-a]quinazolin-5-one |
| 28 | 1-(azepan-1-yl)-7-chloro-4-[2-(4-methoxyphenyl)-2-oxoethyl]-4H-[1,2,4]triazolo[4,3-a]quinazolin-5-one |
| 29 | 1-(azepan-1-yl)-7-chloro-4-[2-(4-chlorophenyl)-2-oxoethyl]-4H-[1,2,4]triazolo[4,3-a]quinazolin-5-one |
| 30 | 5-[(1-(azepan-1-yl)-7-chloro-5-oxo-5H-[1,2,4]triazolo[4,3-a]-quinazolin-4-yl)acetyl]-2-methoxybenzoic acid methyl ester |
| 31 | 7-Chloro-4-pyridin-3-ylmethyl-1-pyrrolidin-1-yl-4H-[1,2,4]triazolo[4,3-a]quinazolin-5-one |
| 32 | 1-(azepan-1-yl)-7-bromo-4-(4-chloro-benzyl)-4H-[1,2,4]triazolo[4,3-a]quinazolin-5-one |
| 33 | 1-Azepan-1-yl-7-bromo-4-(4-fluoro-benzyl)-4H-[1,2,4]triazolo[4,3-a]quinazolin-5-one |

(suite)

| | |
|---|---|
| 34 | 4-(1-Azepan-1-yl-7-bromo-5-oxo-5H-[1,2,4]triazolo[4,3-a]quinazolin-4-ylmethyl)-benzonitrile |
| 35 | 1-Azepan-1-yl-7-bromo-4-(3,4-dimethoxy-benzyl)-4H-[1,2,4]triazolo[4,3-a]quinazolin-5-one |
| 36 | 1-(azepan-1-yl)-7-bromo-4-(3-pyridinylmethyl)-4H-[1,2,4]triazolo[4,3-a]quinazolin-5-one |
| 37 | 1-(azepan-1-yl)-7-bromo-4-(3-phenylallyl)-4H-[1,2,4]triazolo[4,3-a]quinazolin-5-one |
| 38 | 1-Azepan-1-yl-7-bromo-4-[3-(4-chloro-phenyl)-allyl]-4H-[ 1,2,4]triazolo[4,3-a]quinazolin-5-one |
| 39 | 1-Azepan-1-yl-7-bromo-4-[3-(4-methoxy-phenyl)-allyl]-4H-[ 1,2,4]triazolo[4,3-a]quinazolin-5-one |
| 40 | 1-Azepan-1-yl-7-bromo-4-((E)-3-pyridin-3-yl-allyl)-4H-[1,2,4]triazolo[4,3-a]quinazolin-5-one |
| 41 | 1-Azepan-1-yl-7-bromo-4-(3-pyridin-4-yl-allyl)-4H-[1,2,4]triazolo[4,3-a]quinazolin-5-one |
| 42 | 7-Bromo-4-(4-methyl-benzyl)-1-pyrrolidin-1-yl-4H-[1,2,4]triazolo[4,3-a]quinazolin-5-one |
| 43 | 7-Bromo-4-(4-chloro-benzyl)-1-pyrrolidin-1-yl-4H-[1,2,4]triazolo[4,3-a]quinazolin-5-one |
| 44 | 7-Bromo-4-(4-fluoro-benzyl)-1-pyrrolidin-1-yl-4H-[1,2,4]triazolo[4,3-a]quinazolin-5-one |
| 45 | 3-(7-Bromo-5-oxo-1-pyrrolidin-1-yl-5H-[1,2,4]triazolo[4,3-a]quinazolin-4-ylmethyl)-benzonitrile |
| 46 | 4-(7-Bromo-5-oxo-1-pyrrolidin-1-yl-5H-[1,2,4]triazolo[4,3-a]quinazolin-4-ylmethyl)-benzonitrile |
| 47 | 4-(7-Bromo-5-oxo-1-pyrrolidin-1-yl-5H-[1,2,4]triazolo[4,3-a]quinazolin-4-ylmethyl)-benzoic acid methyl ester |
| 48 | 7-Bromo-4-(4-nitro-benzyl)-1-pyrrolidin-1-yl-4H-[1,2,4]triazolo[4,3-a]quinazolin-5-one |
| 49 | 7-Bromo-4-(4-methoxy-benzyl)-1-pyrrolidin-1-yl-4H-[1,2,4]triazolo[4,3-a]quinazolin-5-one |
| 50 | Acetic acid 4-(7-bromo-5-oxo-1-pyrrolidin-1-yl-5H-[1,2,4]triazolo[4,3-a]quinazolin-4-ylmethyl)-phenyl ester |
| 51 | 7-Bromo-4-(4-hydroxy-benzyl)-1-pyrrolidin-1-yl-4H-[1,2,4]triazolo[4,3-a]quinazolin-5-one |
| 52 | 7-Bromo-4-(3,4-dimethoxy-benzyl)-1-pyrrolidin-1-yl-4H-[ 1,2,4]triazolo[4,3-a]quinazolin-5-one |
| 53 | 4-Benzo[1,3]dioxol-5-ylmethyl-7-bromo-1-pyrrolidin-1-yl-4H-[ 1,2,4]triazolo[4,3-a]quinazolin-5-one |
| 54 | 7-Bromo-4-(3,5-dimethoxy-benzyl)-1-pyrrolidin-1-yl-4H-[ 1,2,4]triazolo[4,3-a]quinazolin-5-one |
| 55 | 7-Bromo-1-pyrrolidin-1-yl-4-(3,4,5-trimethoxy-benzyl)-4H-[1,2,4]triazolo[4,3-a]quinazolin-5-one |
| 56 | [4-(7-Bromo-5-oxo-1-pyrrolidin-1-yl-5H-[1,2,4]triazolo[4,3-a]quinazolin-4-ylmethyl)-phenyl]-acetic acid |
| 57 | 1-(pyrrolidin-1-yl)-7-bromo-4-(3-phenylallyl)-4H-[1,2,4]triazolo[4,3-a]quinazolin-5-one |
| 58 | 7-Bromo-4-(3-phenyl-allyl)-1-pyrrolidin-1-yl-4H-[1,2,4]triazolo[4,3-a]quinazolin-5-one |
| 59 | 7-Bromo-4-[(E)-3-(4-chloro-phenyl)-allyl]-1-pyrrolidin-1-yl-4H-[ 1,2,4]triazolo[4,3-a]quinazolin-5-one |
| 60 | 7-Bromo-4-[3-(4-methoxy-phenyl)-allyl]-1-pyrrolidin-1-yl-4H-[ 1,2,4]triazolo[4,3-a]quinazolin-5-one |
| 61 | 7-Bromo-4-(3-pyridin-3-yl-allyl)-1-pyrrolidin-1-yl-4H-[ 1,2,4]triazolo[4,3-a]quinazolin-5-one |
| 62 | 7-Bromo-4-((E)-3-pyridin-4-yl-allyl)-1-pyrrolidin-1-yl-4H-[ 1,2,4]triazolo[4,3-a]quinazolin-5-one |
| 63 | 7-Bromo-4-(1H-imidazol-4-ylmethyl)-1-pyrrolidin-1-yl-4H-[ 1,2,4]triazolo[4,3-a]quinazolin-5-one |
| 64 | 7-Bromo-4-(3,5-dimethyl-isoxazol-4-ylmethyl)-1-pyrrolidin-1-yl-4H-[1,2,4]triazolo[4,3-a]quinazolin-5-one |
| 65 | 7-Bromo-4-cyclopentylmethyl-1-pyrrolidin-1-yl-4H-[1,2,4]triazolo[4,3-a]quinazolin-5-one |
| 66 | 7-Bromo-4-butyl-1-pyrrolidin-1-yl-4H-[1,2,4]triazolo[4,3-a]quinazolin-5-one |
| 67 | 7-Bromo-1-pyrrolidin-1-yl-4-(2,2,2-trifluoro-ethyl)-4H-[1,2,4]triazolo[4,3-a]quinazolin-5-one |
| 68 | 7-Bromo-4-(2-hydroxy-ethyl)-1-pyrrolidin-1-yl-4H-[1,2,4]triazolo[4,3-a]quinazolin-5-one |
| 69 | 7-Bromo-4-(2-diethylamino-ethyl)-1-pyrrolidin-1-yl-4H-[1,2,4]triazolo[4,3-a]quinazolin-5-one |
| 70 | 7-Bromo-4-prop-2-ynyl-1-pyrrolidin-1-yl-4H-[1,2,4]triazolo[4,3-a]quinazolin-5-one |
| 71 | 7-Bromo-4-(2-phenoxy-ethyl)-1-pyrrolidin-1-yl-4H-[1,2,4]triazolo[4,3-a]quinazolin-5-one |
| 72 | 7-Bromo-4-(2-phenylsulfényl-ethyl)-1-pyrrolidin-1-yl-4H-[ 1,2,4]triazolo[4,3-a]quinazolin-5-one |
| 73 | (7-Bromo-5-oxo-1-pyrrolidin-1-yl-5H-[1,2,4]triazolo[4,3-a]quinazolin-4-yl)-phenyl-acetic acid methyl ester |
| 74 | 4-(7-Bromo-5-oxo-1-piperidin-1-yl-5H-[1,2,4]triazolo[4,3-a]quinazolin-4-ylmethyl)-benzonitrile |
| 75 | 7-Bromo-4-(3,4-dimethoxy-benzyl)-1-piperidin-1-yl-4H-[ 1,2,4]triazolo[4,3-a]quinazolin-5-one |
| 76 | 1-(piperidin-1-yl)-7-bromo-4-(3-phenylallyl)-4H-[1,2,4]triazolo[4,3-a]quinazolin-5-one |
| 77 | 7-Bromo-4-(3-pyridin-3-yl-allyl)-1-thiomorpholin-4-yl-4H-[1,2,4]triazolo[4,3-a]quinazolin-5-one |
| 78 | Bromo-dimethylamino-(4-methyl-benzyl)-4H-[1,2,4]triazolo[4,3-a]quinazolin-5-one |
| 79 | 4-(7-Bromo-1-dimethylamino-5-oxo-5H-[1,2,4]triazolo[4,3-a]quinazolin-4-ylmethyl)-benzonitrile |
| 80 | 7-Bromo-1-dimethylamino-4-(4-hydroxy-benzyl)-4H-[1,2,4]triazolo[4,3-a]quinazolin-5-one |
| 81 | 4-(7-Bromo-1-dimethylamino-5-oxo-5H-[1,2,4]triazolo[4,3-a]quinazolin-4-ylmethyl)-benzoic acid methyl ester |
| 82 | [4-(7-Bromo-1-dimethylamino-5-oxo-5H-[1,2,4]triazolo[4,3-a]quinazolin-4-ylmethyl)-phenyl]-acetic acid |

(suite)

| | |
|---|---|
| 83 | [4-(7-Bromo-1-dimethylamino-5-oxo-5H-[1,2,4]triazolo[4,3-a]quinazolin-4-ylmethyl)-phenyl]-acetonitrile |
| 84 | 7-Bromo-1-dimethylamino-4-pyridin-3-ylmethyl-4H-[1,2,4]triazolo[4,3-a]quinazolin-5-one |
| 85 | 7-Bromo-1-dimethylamino-4-(3-phenyl-allyl)-4H-[1,2,4]triazolo[4,3-a]quinazolin-5-one |
| 86 | 7-Bromo-1-dimethylamino-4-(3-phenyl-allyl)-4H-[1,2,4]triazolo[4,3-a]quinazolin-5-one |
| 87 | 7-Bromo-1-dimethylamino-4-(3-pyridin-4-yl-allyl)-4H-[ 1,2,4]triazolo[4,3-a]quinazolin-5-one |
| 88 | 7-Bromo-1-dimethylamino-4-prop-2-ynyl-4H-[1,2,4]triazolo[4,3-a]quinazolin-5-one |
| 89 | 7-Bromo-1-dimethylamino-4-(3-phenyl-prop-2-ynyl)-4H-[ 1,2,4]triazolo[4,3-a]quinazolin-5-one |
| 90 | (7-Bromo-1-dimethylamino-5-oxo-5H-[1,2,4]triazolo[4,3-a]quinazolin-4-yl)-phenyl-acetic acid methyl ester |
| 91 | 1-Azepan-1-yl-7-methyl-4-pyridin-3-ylmethyl-4H-[ 1,2,4]triazolo[4,3-a]quinazolin-5-one |
| 92 | 1-Azepan-1-yl-7-methyl-4-(3-phenyl-allyl)-4H-[1,2,4]triazolo[4,3-a]quinazolin-5-one |
| 93 | 4-(7-Methyl-5-oxo-1-pyrrolidin-1-yl-5H-[1,2,4]triazolo[4,3-a]quinazolin-4-ylmethyl)-benzonitrile |
| 94 | 4-(3,4-Dimethoxy-benzyl)-7-methyl-1-pyrrolidin-1-yl-4H-[1,2,4]triazolo[4,3-a]quinazolin-5-one |
| 95 | 4-(7-Methyl-5-oxo-1-pyrrolidin-1-yl-5H-[1,2,4]triazolo[4,3-a]quinazolin-4-ylmethyl)-benzoic acid methyl ester |
| 96 | [4-(7-Methyl-5-oxo-1-pyrrolidin-1-yl-5H-[1,2,4]triazolo[4,3-a]quinazolin-4-ylmethyl)-phenyl]-acetic acid |
| 97 | 7-Methyl-4-pyridin-3-ylmethy]-1-pyrrolidin-1-yl-4H-[1,2,4]triazolo[4,3-a]quinazolin-5-one |
| 98 | 7-Methyl-4-(3-phenyl-allyl)-1-pyrrolidin-1-yl-4H-[1,2,4]triazolo[4,3-a]quinazolin-5-one |
| 99 | [4-(7-Methyl-5-oxo-1-thiomorpholin-4-yl-5H-[1,2,4]triazolo[4,3-a]quinazolin-4-ylmethyl)-phenyl]-acetic acid |
| 100 | 7-Methyl-4-(3-pyridin-3-yl-allyl)-1-thiomorpholin-4-yl-4H-[1,2,4]triazolo[4,3-a]quinazolin-5-one |
| 101 | 4-(1-Dimethylamino-7-methyl-5-oxo-5H-[1,2,4]triazolo[4,3-a]quinazolin-4-ylmethyl)-benzonitrile |
| 102 | [4-(1-Dimethylamino-7-methyl-5-oxo-5H-[1,2,4]triazolo[4,3-a]quinazolin-4-ylmethyl)-phenyl]-acetic acid |
| 103 | 1-Dimethylamino-7-methyl-4-((E)-3-phenyl-allyl)-4H-[ 1,2,4]triazolo[4,3-a]quinazolin-5-one |
| 104 | 1-Dimethylamino-7-methyl-4-(3-pyridin-3-yl-allyl)-4H-[1,2,4]triazolo[4,3-a]quinazolin-5-one |
| 105 | 1-Dimethylamino-7-methyl-4-(3-pyridin-4-yl-allyl)-4H-[ 1,2,4]triazolo[4,3-a]quinazolin-5-one |
| 106 | 1-(azepan-1-yl)-8-methyl-4-(3-phenylallyl)-4H-[1,2,4]triazolo[4,3-a]quinazolin-5-one |
| 107 | 4-(4-Cyano-benzyl)-1-dimethylamino-5-oxo-4,5-dihydro-[ 1,2,4]triazolo[4,3-a]quinazoline-7-carbonitrile |
| 108 | 7-Hydroxy-4-((E)-3-phenyl-allyl)-1-pyrrolidin-1-yl-4H-[ 1,2,4]triazolo[4,3-a]quinazolin-5-one |
| 109 | 1-(azepan-1-yl)-3-(3-phenylallyl)- 3H-[1,2,4]triazolo[4,3-a]- quinazolin-5-one |
| 110 | 3-Allyl-1-azepan-1-yl-7-chloro-3H-[1,2,4]triazolo[4,3-a]quinazolin-5-one |
| 111 | 1-(azepan-1-yl)-7-chloro-3-benzyl-3H-[1,2,4]triazolo[4,3-a]-quinazolin-5-one |
| 112 | 1-Azepan-1-yl-7-chloro-3-(4-methyl-benzyl)-3H-[1,2,4]triazolo[4,3-a]quinazolin-5-one |
| 113 | 1-(azepan-1-yl)-7-chloro-3-(2-chlorobenzyl)-3H-[1,2,4]triazolo[4,3-a]quinazolin-5-one |
| 114 | 1 -(azepan-1-yl)-7-chloro-3-(3-chlorobenzyl)-3H-[1,2,4]triazolo[4,3-a]quinazolin-5-one |
| 115 | 1-(azepan-1-yl)-7-chloro-3-(4-chlorobenzyl)-3H-[1,2,4]triazolo[4,3-a]quinazolin-5-one |
| 116 | 1-(azepan-1-yl)-7-chloro-3-(4-bromobenzyl)-3H-[1,2,4]triazolo[4,3-a]quinazolin-5-one |
| 117 | 1-(azepan-1-yl)-7-chloro-3-(4-fluorobenzyl)-3H-[1,2,4]triazolo[4,3-a]quinazolin-5-one |
| 118 | 1-(azepan-1-yl)-7-chloro-3-(4-(trifluoromethyl)benzyl)-3H-[ 1,2,4]triazolo[4,3-a]quinazolin-5-one |
| 119 | 1-(azepan-1-yl)-7-chloro-3-(4-cyanobenzyl)-3H-[1,2,4]triazolo[4,3-a]quinazolin-5-one |
| 120 | 1-(azepan-1-yl)-7-chloro-3-(2-methoxybenzyl)-3H-[1,2,4]triazolo[4,3-a]quinazolin-5-one |
| 121 | 1-(azepan-1-yl)-7-chloro-3-(3-methoxybenzyl)-3H-[1,2,4]triazolo[4,3-a]quinazolin-5-one |
| 122 | 1-(azepan-1-yl)-7-chloro-3-(4-methoxybenzyl)-3H-[1,2,4]triazolo[4,3-a]quinazolin-5-one |
| 123 | 1-(azepan-1-yl)-7-chloro-3-(3,4-dichlorobenzyl)-3H-[1,2,4]triazolo[4,3-a]quinazolin-5-one |
| 124 | 1-(azepan-1-yl)-7-chloro-3-(3,4-dimethoxybenzyl)-3H [ 1,2,4]triazolo[4,3-a]quinazolin-5-one |
| 125 | 1-(azepan-1-yl)-7-chloro-3-(2-pyridylmethyl)-3H-[1,2,4]triazolo[4,3-a]quinazolin-5-one |
| 126 | 1-(azepan-1-yl)-7-chloro-3-(3-pyridylmethyl)-3H-[1,2,4]triazolo[4,3-a]quinazolin-5-one |
| 127 | 1-(azepan-1-yl)-7-chloro-3-(2-phenylethyl)-3H-[1,2,4]triazolo[4,3-a]quinazolin-5-one |
| 128 | 1-(azepan-1-yl)-7-chloro-3-[2-(4-methoxyphenyl)ethyl]-3H-[ 1,2,4]triazolo[4,3-a]quinazolin-5-one |
| 129 | 1-(azepan-1-yl)-7-chloro-3-(3-phenylpropyl)-3H-[1,2,4]triazolo[4,3-a]quinazolin-5-one |
| 130 | 1-Azepan-1-yl-7-chloro-3-(2-oxo-2-phenyl-ethyl)-3H-[1,2,4]triazolo[4,3-a]quinazolin-5-one |
| 131 | 1-(azepan-1-yl)-7-chloro-3-[2-(4-methoxyphenyl)-2-oxoethyl]-3H-[ 1,2,4]triazolo[4,3-a]quinazolin-5-one |
| 132 | 1-(azepan-1-yl)-7-chloro-3-[2-(4-chlorophenyl)-2-oxoethyl]-3H-[1,2,4]triazolo[4,3-a]quinazolin-5-one |

(suite)

| | |
|---|---|
| 133 | 5-[(1-(azepan-1-yl)-7-chloro-5-oxo-5H-[1,2,4]triazolo[4,3-a]-quinazolin-3-yl)acetyl]-2-methoxybenzoic acid methyl ester |
| 134 | 1-(azepan-1-yl)-7-bromo-3-(4-chlorobenzyl)-3H-[1,2,4]triazolo[4,3-a]quinazolin-5-one |
| 135 | 1 -(azepan-1-yl)-7-bromo-3-(4-fluorobenzyl)-3H-[1,2,4]triazolo[4,3-a]quinazolin-5-one |
| 136 | 4-(1-(azepan-1-yl)-7-bromo-5-oxo-5H-[1,2,4]triazolo[4,3-a]- quinazolin-3-ylmethyl)- benzonitrile |
| 137 | 1-(azepan-1-yl)-7-bromo-3-(3,4-dimethoxybenzyl)-3H-[ 1,2,4]triazolo[4,3-a]quinazolin-5-one |
| 138 | [4-(7-Bromo-5-oxo-1-perhydro-azepin-1-yl-5H-[1,2,4]triazolo[4,3-a]quinazolin-3-ylmethyl)-phenyl]-acetic acid |
| 139 | 1-(azepan-1-yl)-7-bromo-3-(pyridin-3-ylmethyl)-3H-[1,2,4]triazolo[4,3-a]quinazolin-5-one |
| 140 | 1-Azepan-1-yl-7-bromo-3-((E)-3-phenyl-allyl)-3H-[1,2,4]triazolo[4,3-a]quinazolin-5-one |
| 141 | 7-Bromo-3-((E)-3-phenyl-allyl)-1-piperidin-1-yl-3H-[1,2,4]triazolo[4,3-a]quinazolin-5-one |
| 142 | 7-bromo-3-(4-chlorobenzyl)-1-(pyrrolidin-1-yl)-3H-[1,2,4]triazolo[4,3-a]quinazolin-5-one |
| 143 | 7-bromo-3-(4-fluorobenzyl)-1-(pyrrolidin-1-yl)-3H-[1,2,4]triazolo[4,3-a]quinazolin-5-one |
| 144 | 4-(7-bromo-5-oxo-1-(pyrrolidin-1-yl)-5H-[1,2,4]triazolo[4,3-a]-quinazolin-3-ylmethyl)- benzonitrile |
| 145 | 4-(7-bromo-5-oxo-1-(pyrrolidin-1-yl)-5H-[1,2,4]triazolo[4,3-a]-quinazolin-3-ylmethyl)benzoic acid methyl ester |
| 146 | 7-Bromo-3-(4-methoxy-benzyl)-1-pyrrolidin-1-yl-3H-[1,2,4]triazolo[4,3-a]quinazolin-5-one |
| 147 | Acetic acid 4-(7-bromo-5-oxo-1-pyrrolidin-1-yl-5H-[1,2,4]triazolo[4,3-a]quinazolin-3-ylmethyl)-phenyl ester |
| 148 | 7-Bromo-1-dimethylamino-3-(4-hydroxy-benzyl)-3H-[1,2,4]triazolo[4,3-a]quinazolin-5-one |
| 149 | 3-(benzo[1,3]dioxol-5-ylmethyl)-7-bromo-1-(pyrrolidin-1-yl)-3H-[1,2,4]triazolo[4,3-a]quinazolin-5-one |
| 150 | 7-bromo-3-(3,5-dimethoxy-benzyl)-1-(pyrrolidin-1-yl)-3H-[1,2,4]triazolo- [4,3-a]quinazolin-5-one |
| 151 | 7-bromo-1-(pyrrolidin-1-yl)-3-(3,4,5-trimethoxybenzyl)-3H-[ 1,2,4]triazolo[4,3-a]quinazolin-5-one |
| 152 | 7-Bromo-3-(1H-imidazol-4-ylmethyl)-1-pyrrolidin-1-yl-3H-[1,2,4]triazolo[4,3-a]quinazolin-5-one |
| 153 | 7-bromo-3-(n-butyl)-1-(pyrrolidin-1-yl)-3H-[1,2,4]triazolo[4,3-a]-quinazolin-5-one |
| 154 | (7-Bromo-5-oxo-1-pyrrolidin-1-yl-5H-[1,2,4]triazolo[4,3-a]quinazolin-3-yl)-phenyl-acetic acid methyl ester |
| 155 | 7-Bromo-1-dimethylamino-3-(3-phenyl-allyl)-3H-[1,2,4]triazolo[4,3-a]quinazolin-5-one |
| 156 | (7-Bromo-1-dimethylamino-5-oxo-5H-[1,2,4]triazolo[4,3-a]quinazolin-3-yl)-phenyl-acetic acid methyl ester |
| 157 | 1-(azepan- 1-yl)-7-methyl-3-(3-phenylallyl)-3H-[1,2,4]triazolo[4,3-a]quinazolin-5-one |
| 158 | 7-methyl-3-(3-phenylallyl)-1-(pyrrolidin-1-yl)-3H-[1,2,4]triazolo[4,3-a]quinazolin-5-one |
| 159 | 1-(azepan-1-yl)-3,8-dimethyl-3H-[1,2,4]triazolo[4,3-a]- quinazolin-5-one |
| 160 | 1-Azepan-1-yl-8-methyl-3-((E)-3-phenyl-allyl)-3H-[1,2,4]triazolo[4,3-a]quinazolin-5-one |
| 161 | 7-hydroxy-3-(3-phenylallyl)-1-(pyrrolidin-1-yl)-3H-[1,2,4]triazolo[4,3-a]quinazolin-5-one |
| 162 | 1,8-bis(azepan-1-yl)-3-(3-phenylallyl)-3H-[1,2,4]triazolo[4,3-a]quinazolin-5-one |
| 163 | 1-(azepan-1-yl)-4-benzyl-7-bromo-4H-[1,2,4]triazolo[4,3-a]- quinazolin-5-one |
| 164 | 4-benzyl-7-bromo-1-(pyrrolidin-1-yl)-4H-[1,2,4]triazolo[4,3-a]-quinazolin-5-one |
| 165 | 4-Benzyl-7-bromo-1-(butyl-methyl-amino)-4H-[1,2,4]triazolo[4,3-a]quinazolin-5-one |
| 166 | 4-benzyl-1-(pyrrolidin-1-yl)-4H-[1,2,4]triazolo[4,3-a]quinazolin-5-one |
| 167 | 7-chloro-1-dibutylamino-4-methyl-4H-[1,2,4]triazolo[4,3-a]- quinazolin-5-one |
| 168 | 7-chloro-4-methyl-1-(piperidin-1-yl)-4H-[1,2,4]triazolo[4,3-a]-quinazolin-5-one |
| 169 | 7-Chloro-4-methyl-1-(4-methyl-piperazin-1-yl)-4H-[1,2,4]triazolo[4,3-a]quinazolin-5-one |
| 170 | 7-Chloro-4-methyl-1-(1,8,8-trimethyl-3-aza-bicyclo[3.2.1]oct-3-yl)-4H-[ 1,2,4]triazolo[4,3-a]quinazolin-5-one |
| 171 | 1-(azepan-1-yl)-7-chloro-4-phenyl-4H-[1,2,4]triazolo[4,3-a]- quinazolin-5-one |
| 172 | 1-(azepan-1-yl)-4-benzyl-7-chloro-4H-[1,2,4]triazolo[4,3-a]- quinazolin-5-one |
| 173 | 4-benzyl-7-chloro-1-(pyrrolidin-1-yl)-4H-[1,2,4]triazolo[4,3-a]-quinazolin-5-one |
| 174 | 4-benzyl-7-chloro-1-(piperidin-1-yl)-4H-[1,2,4]triazolo[4,3-a]-quinazolin-5-one |
| 175 | 1-(azepan-1-yl)-8-chloro-4-methyl-4H-[1,2,4]triazolo[4,3-a]- quinazolin-5-one |
| 176 | 1-(azepan-1-yl)-4-benzyl-8-chloro-4H-[1,2,4]triazolo[4,3-a]- quinazolin-5-one |
| 177 | 1-(azepan-1-yl)-7-bromo-4-methyl-4H-[1,2,4]triazolo[4,3-a]- quinazolin-5-one |
| 178 | 4-benzyl-7-bromo-1-(piperidin-1-yl)-4H-[1,2,4]triazolo[4,3-a]-quinazolin-5-one |
| 179 | 4-Benzyl-7-bromo-1-dimethylamino-4H-[1,2,4]triazolo[4,3-a]quinazolin-5-one |

(suite)

| | |
|---|---|
| 180 | 4-Benzyl-7-bromo-1-morpholin-4-yl-4H-[1,2,4]triazolo[4,3-a]quinazolin-5-one |
| 181 | 4-Benzyl-7-bromo-1-thiomorpholin-4-yl-4H-[1,2,4]triazolo[4,3-a]quinazolin-5-one |
| 182 | 4-Benzyl-7-bromo-1-(4-methyl-piperazin-1-yl)-4H-[1,2,4]triazolo[4,3-a]quinazolin-5-one |
| 183 | 4-Benzyl-7-bromo-1-(4-phenyl-piperazin-1-yl)-4H-[1,2,4]triazolo[4,3-a]quinazolin-5-one |
| 184 | 4-Benzyl-1-(4-benzyl-piperazin-1-yl)-7-bromo-4H-[1,2,4]triazolo[4,3-a]quinazolin-5-one |
| 185 | 4-Benzyl-7-bromo-1-(3,6-dihydro-2H-pyridin-1-yl)-4H-[ 1,2,4]triazolo[4,3-a]quinazolin-5-one |
| 186 | 4-Benzyl-7-bromo-1-(2,5-dihydro-pyrrol-1-yl)-4H-[1,2,4]triazolo[4,3-a]quinazolin-5-one |
| 187 | 4-Benzyl-7-bromo-1-(3-hydroxy-pyrrolidin-1-yl)-4H-[1,2,4]triazolo[4,3-a]quinazolin-5-one |
| 188 | 4-Benzyl-7-bromo-1-methylamino-4H-[1,2,4]triazolo[4,3-a]quinazolin-5-one |
| 189 | 4-Benzyl-7-iodo-1-pyrrolidin-1-yl-4H-[1,2,4]triazolo[4,3-a]quinazolin-5-one |
| 190 | 1-Azepan-1-yl-4-benzyl-7-methyl-4H-[1,2,4]triazolo[4,3-a]quinazolin-5-one |
| 191 | 4-Benzyl-7-methyl-1-pyrrolidin-1-yl-4H-[1,2,4]triazolo[4,3-a]quinazolin-5-one |
| 192 | 4-Benzyl-1-dimethylamino-7-methyl-4H-[ 1,2,4]triazolo[4,3-a]quinazolin-5-one |
| 193 | 4-Benzyl-7-methyl-1-thiomorpholin-4-yl-4H-[1,2,4]triazolo[4,3-a]quinazolin-5-one |
| 194 | 1-Azepan-1-yl-4-benzyl-8-methyl-4H-[1,2,4]triazolo[4,3-a]quinazolin-5-one |
| 195 | 1-Azepan-1-yl-4-benzyl-7-methoxy-4H-[1,2,4]triazolo[4,3-a]quinazolin-5-one |
| 196 | 4-Benzyl-7-methoxy-1-pyrrolidin-1-yl-4H-[1,2,4]triazolo[4,3-a]quinazolin-5-one |
| 197 | 4-Benzyl-5-oxo-1-pyrrolidin-1-yl-4,5-dihydro-[1,2,4]triazolo[4,3-a]quinazoline-7-carbonitrile |
| 198 | 1-Azepan-1-yl-4-benzyl-7-nitro-4H-[1,2,4]triazolo[4,3-a]quinazolin-5-one |
| 199 | 1-(azepan-1-yl)-4-benzyl-7-chloro-4H-[1,2,4]triazolo[4,3-a]- quinazolin-5-one |
| 200 | 1-(azepan-1-yl)-4-methyl-4H-[1,2,4]triazolo[4,3-a]quinazolin-5-one |
| 201 | 1-(azepan-1-yl)-4-benzyl-4H-[1,2,4]triazolo[4,3-a]quinazolin-5-one |
| 202 | 1-(azepan-1-yl)-6-chloro-4-methyl-4H-[1,2,4]triazolo[4,3-a]- quinazolin-5-one |
| 203 | 1 -(azepan-1-yl)-7-chloro-4-methyl-4H-[1,2,4]triazolo[4,3-a]- quinazolin-5-one |
| 204 | 1-(azepan-1-yl)-7-chloro-4-ethyl-4H-[1,2,4]triazolo[4,3-a]- quinazolin-5-one |
| 205 | 7-chloro-4-methyl-1-(pyrrolidin-1-yl)-4H-[1,2,4]triazolo[4,3-a]-quinazolin-5-one |
| 206 | 7-chloro-4-methyl-1-(morpholin-4-yl)-4H-[1,2,4]triazolo[4,3-a]-quinazolin-5-one |
| 207 | 1-(azocan-1-yl)-7-chloro-4-methyl-4H-[1,2,4]triazolo[4,3-a]- quinazolin-5-one |
| 208 | 7-chloro-1-(3,4-dihydro-2H-quinolin-1-yl)-4-methyl-4H-[ 1,2,4]triazolo[4,3-a]quinazolin-5-one |
| 209 | 7-chloro-1-(3,4-dihydro-1H-isoquinolin-2-yl)-4-methyl-4H-[1,2,4]triazolo[4,3-a]quinazolin-5-one |
| 210 | 1-(4-benzylpiperidin-1-yl)-7-chloro-4-methyl-4H-[1,2,4]triazolo[4,3-a]quinazolin-5-one |
| 211 | 7-chloro-4-methyl-1-(1,3,3-trimethyl-6-azabicyclo[3,2,1]oct-6-yl)-4H-[1,2,4]triazolo[4,3-a]- quinazolin-5-one |
| 212 | 1-(azepan-1-yl)-7-fluoro-4-methyl-4H-[1,2,4]triazolo[4,3-a]- quinazolin-5-one |
| 213 | 1-(azepan-1-yl)-7-iodo-4-methyl-4H-[1,2,4]triazolo[4,3-a]- quinazolin-5-one |
| 214 | 1-(azepan-1-yl)-7-methoxy-4-methyl-4H-[1,2,4]triazolo[4,3-a]-quinazolin-5-one |
| 215 | 4-Benzyl-7-bromo-1-(ethyl-methyl-amino)-4H-[1,2,4]triazolo[4,3-a]quinazolin-5-one |
| 216 | 4-Benzyl-1-diethylamino-7-methyl-4H-[1,2,4]triazolo[4,3-a]qumazolin-5-one |
| 217 | 4-Benzyl-7-bromo-1-pyrrol-1-yl-4H-[1,2,4]triazolo[4,3-a]quinazolin-5-one |
| 218 | 4-(4-Amino-benzyl)-7-bromo-1-pyrrolidin-1-yl-4H-[1,2,4]triazolo[4,3-a]quinazolin-5-one |
| 219 | 4-Benzyl-7-hydroxy-1-pyrrolidin-1-yl-4H-[1,2,4]triazolo[4,3-a]quinazolin-5-one |
| 220 | 4-(7-Hydroxy-5-oxo-1-pyrrolidin-1-yl-5H-[1,2,4]triazolo[4,3-a]quinazolin-4-ylmethyl)-benzonitrile |
| 221 | N-(4-Benzyl-5-oxo-1-pyrrolidin-1-yl-4,5-dihydro-[1,2,4]triazolo[4,3-a]quinazolin-7-yl)-acetamide |
| 222 | N-[5-Oxo-4-(3-phenyl-allyl)-1-pyrrolidin-1-yl-4,5-dihydro-[1,2,4]triazolo[4,3-a]quinazolin-7-yl]-acetamide |
| 223 | 7-Amino-4-((E)-3-phenyl-allyl)-1-pyrrolidin-1-yl-4H-[1,2,4]triazolo[4,3-a]quinazolin-5-one |
| 224 | 7-Amino-1-azepan-1-yl-4-benzyl-4H-[1,2,4]triazolo[4,3-a]quinazolin-5-one |
| 225 | 7-Amino-4-benzyl-1-pyrrolidin-1-yl-4H-[1,2,4]triazolo[4,3-a]quinazolin-5-one |
| 226 | 4-(7-Amino-5-oxo-1-pyrrolidin-1-yl-5H-[1,2,4]triazolo[4,3-a]quinazolin-4-ylmethyl)-benzonitrile |
| 227 | 7-Amino-4-((E)-3-pyridin-3-yl-allyl)-1-pyrrolidin-1-yl-4H-[1,2,4]triazolo[4,3-a]quinazolin-5-one |
| 228 | 4-(7-Amino-1-dimethylamino-5-oxo-5H-[1,2,4]triazolo[4,3-a]quinazolin-4-ylmethyl)-benzonitrile |
| 229 | 7-Amino-1-dimethylamino-4-((E)-3-phenyl-allyl)-4H-[1,2,4]triazolo[4,3-a]quinazolin-5-one |
| 230 | 4-Benzyl-7-methylamino-1-pyrrolidin-1-yl-4H-[1,2,4]triazolo[4,3-a]quinazolin-5-one |

(suite)

| | |
|---|---|
| 231 | 4-(7-Methylamino-5-oxo-1-pyrrolidin-1-yl-5H-[1,2,4]triazolo[4,3-a]quinazolin-4-ylmethyl)-benzonitrile |
| 232 | 4-Benzyl-8-methylamino-1-pyrrolidin-1-yl-4H-[1,2,4]triazolo[4,3-a]quinazolin-5-one |
| 233 | 4-Benzyl-7-ethylamino-1-pyrrolidin-1-yl-4H-[1,2,4]triazolo[4,3-a]quinazolin-5-one |
| 234 | 4-Benzyl-7-isopropylamino-1-pyrrolidin-1-yl-4H-[1,2,4]triazolo[4,3-a]quinazolin-5-one |
| 235 | N-(4-Benzyl-5-oxo-1-pyrrolidin-1-yl-4,5-dihydro-[1,2,4]triazolo[4,3-a]quinazolin-7-yl)-methanesulfonamide |
| 236 | 4-Benzyl-7-dimethylamino-1-pyrrolidin-1-yl-4H-[1,2,4]triazolo[4,3-a]quinazolin-5-one |
| 237 | 4-Benzyl-1-dimethylamino-5-oxo-4,5-dihydro-[1,2,4]triazolo[4,3-a]quinazoline-7-carbonitrile |
| 238 | 4-Benzyl-5-oxo-1-pyrrolidin-1-yl-4,5-dihydro-[1,2,4]triazolo[4,3-a]quinazoline-7-carboxylic acid |
| 239 | [4-(7-Bromo-5-oxo-1-pyrrolidin-1-yl-5H-[1,2,4]triazolo[4,3-a]quinazolin-4-ylmethyl)-phenyl]-acetic acid methyl ester |
| 240 | 2-[4-(7-Bromo-5-oxo-1-pyrrolidin-1-yl-5H-[1,2,4]triazolo[4,3-a]quinazolin-4-ylmethyl)-phenyl]-N-methyl-acetamide |
| 241 | 2-[4-(7-Bromo-1-dimethylamino-5-oxo-5H-[1,2,4]triazolo[4,3-a]quinazolin-4-ylmethyl)-phenyl]-acetamide |
| 242 | 2-[4-(7-Bromo-5-oxo-1-pyrrolidin-1-yl-5H-[1,2,4]triazolo[4,3-a]quinazolin-4-ylmethyl)-phenyl]-N,N-dimethyl-acetamide |
| 243 | 2-[4-(7-Bromo-5-oxo-1-pyrrolidin-1-yl-5H-[1,2,4]triazolo[4,3-a]quinazolin-4-ylmethyl)-phenyl]-N-hydroxy-acetamide |
| 244 | 4-(1-Dimethylamino-7-methyl-5-thioxo-5H-[1,2,4]triazolo[4,3-a]quinazolin-4-ylmethyl)-benzonitrile |
| 245 | 4-(7-Bromo-1-dimethylamino-5-thioxo-5H-[1,2,4]triazolo[4,3-a]quinazolin-4-ylmethyl)-benzonitrile |
| 246 | 1-Dimethylamino-7-methyl-4-(3-pyridin-3-yl-allyl)-4H-[1,2,4]triazolo[4,3-a]quinazoline-5-thione |
| 247 | 4-benzyl-7-(N,N-dimethylsulfonylamino)-1-(pyrrolidin-1-yl)-4H-[1,2,4] triazolo [4,3-a] quinazolin-5-one |

Parmi les composés mentionnés ci-dessus, les composés suivants sont préférés :

| | |
|---|---|
| 1 | 1-(Azepan-1-yl)-7-chloro-4-(3-phenylallyl)-4H-[1,2,4]triazolo[4,3-a]quinazolin-5-one |
| 3 | 7-Bromo-1-dimethylamino-4-((E)-3-pyridin-3-yl-allyl)-4H-[1,2,4]triazolo[4,3-a]quinazolin-5-one |
| 11 | 1-(azepan-1-yl)-7-chloro-4-(4-chlorobenzyl)-4H-[1,2,4]triazolo[4,3-a]quinazolin-5-one |
| 13 | 1-(azepan-1-yl)-7-chloro-4-(4-fluorobenzyl)-4H-[1,2,4]triazolo[4,3-a]quinazolin-5-one |
| 20 | 1-(azepan-1-yl)-7-chloro-4-(3,4-dimethoxybenzyl)-4H-[ 1,2,4]triazolo[4,3-a]quinazolin-5-one |
| 22 | 1-(azepan-1-yl)-7-chloro-4-(3-pyridylmethyl)-4H-[1,2,4]triazolo[4,3-a]quinazolin-5-one |
| 32 | 1-(azepan-1-yl)-7-bromo-4-(4-chlorophenylmethyl)-4H-[1,2,4]triazolo[4,3-a]quinazolin-5-one |
| 34 | 4-(1-Azepan-1-yl-7-bromo-5-oxo-5H-[1,2,4]triazolo[4,3-a]quinazolin-4-ylmethyl)-benzonitrile |
| 35 | 1-Azepan-1-yl-7-bromo-4-(3,4-dimethoxy-benzyl)-4H-[1,2,4]triazolo[4,3-a]quinazolin-5-one |
| 37 | 1-(azepan-1-yl)-7-bromo-4-(3-phenylallyl)-4H-[1,2,4]triazolo[4,3-a]quinazolin-5-one |
| 40 | 1-Azepan-1-yl-7-bromo-4-((E)-3-pyridin-3-yl-allyl)-4H-[1,2,4]triazolo[4,3-a]quinazolin-5-one |
| 41 | 1-Azepan-1-yl-7-bromo-4-(3-pyridin-4-yl-allyl)-4H-[1,2,4]triazolo[4,3-a]quinazolin-5-one |
| 42 | 7-Bromo-4-(4-methyl-benzyl)-1-pyrrolidin-1-yl-4H-[1,2,4]triazolo[4,3-a]quinazolin-5-one |
| 43 | 7-Bromo-4-(4-chloro-benzyl)-1-pyrrolidin-1-yl-4H-[1,2,4]triazolo[4,3-a]quinazolin-5-one |
| 44 | 7-Bromo-4-(4-fluoro-benzyl)-1-pyrrolidin-1-yl-4H-[1,2,4]triazolo[4,3-a]quinazolin-5-one |
| 46 | 4-(7-Bromo-5-oxo-1-pyrrolidin-1-yl-5H-[1,2,4]triazolo[4,3-a]quinazolin-4-ylmethyl)-benzonitrile |
| 47 | 4-(7-Bromo-5-oxo-1-pyrrolidin-1-yl-5H-[1,2,4]triazolo[4,3-a]quinazolin-4-ylmethyl)-benzoic acid methyl ester |
| 48 | 7-Bromo-4-(4-nitro-benzyl)-1-pyrrolidin-1-yl-4H-[1,2,4]triazolo[4,3-a]quinazolin-5-one |
| 49 | 7-Bromo-4-(4-methoxy-benzyl)-1-pyrrolidin-1-yl-4H-[1,2,4]triazolo[4,3-a]quinazolin-5-one |
| 50 | Acetic acid 4-(7-bromo-5-oxo-1-pyrrolidin-1-yl-5H-[1,2,4]triazolo[4,3-a]quinazolin-4-ylmethyl)-phenyl ester |
| 51 | 7-Bromo-4-(4-hydroxy-benzyl)-1-pyrrolidin-1-yl-4H-[ 1,2,4]triazolo[4,3-a]quinazolin-5-one |
| 52 | 7-Bromo-4-(3,4-dimethoxy-benzyl)-1-pyrrolidin-1-yl-4H-[ 1,2,4]triazolo[4,3-a]quinazolin-5-one |
| 57 | 1-(pyrrolidin-1-yl)-7-bromo-4-(3-phenylallyl)-4H-[1,2,4]triazolo[4,3-a]quinazolin-5-one |
| 59 | 7-Bromo-4-[(E)-3-(4-chloro-phenyl)-allyl]-1-pyrrolidin-1-yl-4H-[1,2,4]triazolo[4,3-a]quinazolin-5-one |
| 60 | 7-Bromo-4-[3-(4-methoxy-phenyl)-allyl]-1-pyrrolidin-1-yl-4H-[1,2,4]triazolo[4,3-a]quinazolin-5-one |
| 61 | 7-Bromo-4-(3-pyridin-3-yl-allyl)-1-pyrrolidin-1-yl-4H-[1,2,4]triazolo[4,3-a]quinazolin-5-one |

(suite)

| | |
|---|---|
| 62 | 7-Bromo-4-((E)-3-pyridin-4-yl-allyl)-1-pyrrolidin-1-yl-4H-[1,2,4]triazolo[4,3-a]quinazolin-5-one |
| 75 | 7-Bromo-4-(3,4-dimethoxy-benzyl)-1-piperidin-1-yl-4H-[ 1,2,4]triazolo[4,3-a]quinazolin-5-one |
| 76 | 1-(piperidin-1-yl)-7-bromo-4-(3-phenylallyl)-4H-[1,2,4]triazolo[4,3-a]quinazolin-5-one |
| 78 | 7-Bromo-1-dimethylamino-4-(4-methyl-benzyl)-4H-[1,2,4]triazolo[4,3-a]quinazolin-5-one |
| 79 | 4-(7-Bromo-1-dimethylamino-5-oxo-5H-[1,2,4]triazolo[4,3-a]quinazolin-4-ylmethyl)-benzonitrile |
| 80 | 7-Bromo-1-dimethylamino-4-(4-hydroxy-benzyl)-4H-[1,2,4]triazolo[4,3-a]quinazolin-5-one |
| 81 | 4-(7-Bromo-1-dimethylamino-5-oxo-5H-[1,2,4]triazolo[4,3-a]quinazolin-4-ylmethyl)-benzoic acid methyl ester |
| 83 | [4-(7-Bromo-1-dimethylamino-5-oxo-5H-[1,2,4]triazolo[4,3-a]quinazolin-4-ylmethyl)-phenyl]-acetonitrile |
| 85 | 7-Bromo-1-dimethylamino-4-(3-phenyl-allyl)-4H-[1,2,4]triazolo[4,3-a]quinazolin-5-one |
| 89 | 7-Bromo-1-dimethylamino-4-(3-phenyl-prop-2-ynyl)-4H-[1,2,4]triazolo[4,3-a]quinazolin-5-one |
| 92 | 1-Azepan-1-yl-7-methyl-4-(3-phenyl-allyl)-4H-[1,2,4]triazolo[4,3-a]quinazolin-5-one |
| 94 | 4-(3,4-Dimethoxy-benzyl)-7-methyl-1-pyrrolidin-1-yl-4H-[1,2,4]triazolo[4,3-a]quinazolin-5-one |
| 96 | [4-(7-Methyl-5-oxo-1-pyrrolidin-1-yl-5H-[1,2,4]triazolo[4,3-a]quinazolin-4-ylmethyl)-phenyl]-acetic acid |
| 98 | 7-Methyl-4-(3-phenyl-allyl)-1-pyrrolidin-1-yl-4H-[1,2,4]triazolo[4,3-a]quinazolin-5-one |
| 102 | [4-(1-Dimethylamino-7-methyl-5-oxo-5H-[1,2,4]triazolo[4,3-a]quinazolin-4-ylmethyl)-phenyl]-acetic acid |
| 103 | 1 -Dimethylamino-7-methyl-4-((E)-3-phenyl-allyl)-4H-[ 1,2,4]triazolo[4,3-a]quinazolin-5-one |
| 104 | 1-Dimethylamino-7-methyl-4-(3-pyridin-3-yl-allyl)-4H-[1,2,4]triazolo[4,3-a]quinazolin-5-one |
| 138 | [4-(7-Bromo-5-oxo-1-perhydro-azepin-1-yl-5H-[1,2,4]triazolo[4,3-a]quinazolin-3-ylmethyl)-phenyl]-acetic acid |
| 164 | 4-benzyl-7-bromo-1-(pyrrolidin-1-yl)-4H-[1,2,4]triazolo[4,3-a]-quinazolin-5-one |
| 186 | 4-Benzyl-7-bromo-1-(2,5-dihydro-pyrrol-1-yl)-4H-[1,2,4]triazolo[4,3-a]quinazolin-5-one |
| 189 | 4-Benzyl-7-iodo-1-pyrrolidin-1-yl-4H-[1,2,4]triazolo[4,3-a]quinazolin-5-one |
| 190 | 1-Azepan-1-yl-4-benzyl-7-methyl-4H-[1,2,4]triazolo[4,3-a]quinazolin-5-one |
| 218 | 4-(4-Amino-benzyl)-7-brome-1-pyrrolidin-1-yl-4H-[1,2,4]triazolo[4,3-a]quinazolin-5-one |
| 223 | 7-Amino-4-((E)-3-phenyl-allyl)-1-pyrrolidin-1-yl-4H-[1,2,4]triazolo[4,3-a]quinazolin-5-one |
| 224 | 7-Amino-1-azepan-1-yl-4-benzyl-4H-[1,2,4]triazolo[4,3-a]quinazolin-5-one |
| 227 | 7-Amino-4-((E)-3-pyridin-3-yl-allyl)-1-pyrrolidin-1-yl-4H-[ 1,2,4]triazolo[4,3-a]quinazolin-5-one |
| 229 | 7-Amino-1-dimethylamino-4-((E)-3-phenyl-allyl)-4H-[1,2,4]triazolo[4,3-a]quinazolin-5-one |
| 230 | 4-Benzyl-7-methylamino-1-pyrrolidin-1-yl-4H-[1,2,4]triazolo[4,3-a]quinazolin-5-one |
| 231 | 4-(7-Methylamino-5-oxo-1-pyrrolidin-1-yl-5H-[1,2,4]triazolo[4,3-a]quinazolin-4-ylmethyl)-benzonitrile |
| 232 | 4-Benzyl-8-methylamino-1-pyrrolidin-1-yl-4H-[1,2,4]triazolo[4,3-a]quinazolin-5-one |
| 233 | 4-Benzyl-7-ethylamino-1-pyrrolidin-1-yl-4H-[1,2,4]triazolo[4,3-a]quinazolin-5-one |
| 234 | 4-Benzyl-7-isopropylamino-1-pyrrolidin-1-yl-4H-[1,2,4]triazolo[4,3-a]quinazolin-5-one |
| 239 | [4-(7-Bromo-5-oxo-1-pyrrolidin-1-yl-5H-[1,2,4]triazolo[4,3-a]quinazolin-4-ylmethyl)-phenyl]-acetic acid methyl ester |
| 240 | 2-[4-(7-Bromo-5-oxo-1-pyrrolidin-1-yl-5H-[1,2,4]triazolo[4,3-a]quinazolin-4-ylmethyl)-phenyl]-N-methyl-acetamide |
| 241 | 2-[4-(7-Bromo-1-dimethylamino-5-oxo-5H-[1,2,4]triazolo[4,3-a]quinazolin-4-ylmethyl)-phenyl]-acetamide |
| 242 | 2-[4-(7-Bromo-5-oxo-1-pyrrolidin-1-yl-5H-[1,2,4]triazolo[4,3-a]quinazolin-4-ylmethyl)-phenyl]-N,N-dimethyl-acetamide |
| 243 | 2-[4-(7-Bromo-5-oxo-1-pyrrolidin-1-yl-5H-[1,2,4]triazolo[4,3-a]quinazolin-4-ylmethyl)-phenyl]-N-hydroxy-acetamide |
| 246 | 1-Dimethylamino-7-methyl-4-(3-pyridin-3-yl-allyl)-4H-[ 1,2,4]triazolo[4,3-a]quinazoline-5-thione |

Parmi les composés mentionnés ci-dessus, les composés suivants sont particulièrement préférés :

| | |
|---|---|
| 3 | 7-Bromo-1-dimethylamino-4-((E)-3-pyridin-3-yl-allyl)-4H-[1,2,4]triazolo[4,3-a]quinazolin-5-one |
| 20 | 1-(azepan-1-yl)-7-chloro-4-(3,4-dimethoxybenzyl)-4H-[ 1,2,4]triazolo[4,3-a]quinazolin-5-one |
| 22 | 1-(azepan-1-yl)-7-chloro-4-(3-pyridylmethyl)-4H-[1,2,4]triazolo[4,3-a]quinazolin-5-one |
| 34 | 4-(1-Azepan-1-yl-7-bromo-5-oxo-5H-[1,2,4]triazolo[4,3-a]quinazolin-4-ylmethyl)-benzonitrile |

(suite)

| | |
|---|---|
| 35 | 1-Azepan-1-yl-7-bromo-4-(3,4-dimethoxy-benzyl)-4H-[1,2,4]triazolo[4,3-a]quinazolin-5-one |
| 37 | 1-(azepan-1-yl)-7-bromo-4-(3-phenylallyl)-4H-[1,2,4]triazolo[4,3-a]quinazolin-5-one |
| 40 | 1-Azepan-1-yl-7-bromo-4-((E)-3-pyridin-3-yl-allyl)-4H-[ 1,2,4]triazolo[4,3-a]quinazolin-5-one |
| 41 | 1-Azepan-1-yl-7-bromo-4-(3-pyridin-4-yl-allyl)-4H-[1,2,4]triazolo[4,3-a]quinazolin-5-one |
| 42 | 7-Bromo-4-(4-methyl-benzyl)-1-pyrrolidin-1-yl-4H-[1,2,4]triazolo[4,3-a]quinazolin-5-one |
| 43 | 7-Bromo-4-(4-chloro-benzyl)-1-pyrrolidin-1-yl-4H-[1,2,4]triazolo[4,3-a]quinazolin-5-one |
| 52 | 7-Bromo-4-(3,4-dimethoxy-benzyl)-1-pyrrolidin-1-yl-4H-[1,2,4]triazolo[4,3-a]quinazolin-5-one |
| 57 | 1-(pyrrolidin-1-yl)-7-bromo-4-(3-phenylallyl)-4H-[1,2,4]triazolo[4,3-a]quinazolin-5-one |
| 61 | 7-Bromo-4-(3-pyridin-3-yl-allyl)-1-pyrrolidin-1-yl-4H-[ 1,2,4]triazolo[4,3-a]quinazolin-5-one |
| 76 | 1-(piperidin-1-yl)-7-bromo-4-(3-phenylallyl)-4H-[1,2,4]triazolo[4,3-a]quinazolin-5-one |
| 79 | 4-(7-Bromo-1-dimethylamino-5-oxo-5H-[1,2,4]triazolo[4,3-a]quinazolin-4-ylmethyl)-benzonitrile |
| 81 | 4-(Bromo-dimethylamino-oxo-5H-[1,2,4]triazolo[4,3-a]quinazolin-4-ylmethyl)-benzoic acid methyl ester |
| 85 | 7-Bromo-1-dimethylamino-4-(3-phenyl-allyl)-4H-[1,2,4]triazolo[4,3-a]quinazolin-5-one |
| 89 | 7-Bromo-1-dimethylamino-4-(3-phenyl-prop-2-ynyl)-4H-[1,2,4]triazolo[4,3-a]quinazolin-5-one |
| 92 | 1-Azepan-1-yl-7-methyl-4-(3-phenyl-allyl)-4H-[1,2,4]triazolo[4,3-a]quinazolin-5-one |
| 94 | 4-(3,4-Dimethoxy-benzyl)-7-methyl-1-pyrrolidin-1-yl-4H-[ 1,2,4]triazolo[4,3-a]quinazolin-5-one |
| 98 | 7-Methyl-4-(3-phenyl-allyl)-1-pyrrolidin-1-yl-4H-[1,2,4]triazolo[4,3-a]quinazolin-5-one |
| 223 | 7-Amino-4-((E)-3 -phenyl-allyl)-1-pyrrolidin-1-yl-4H-[1,2,4]triazolo[4,3-a]quinazolin-5-one |
| 227 | 7-Amino-4-((E)-3-pyridin-3-yl-allyl)-1-pyrrolidin-1-yl-4H-[ 1,2,4]triazolo[4,3-a]quinazolin-5-one |
| 230 | 4-Benzyl-7-methylamino-1-pyrrolidin-1-yl-4H-[1,2,4]triazolo[4,3-a]quinazolin-5-one |
| 231 | 4-(7-Methylamino-5-oxo-1-pyrrolidin-1-yl-5H-[1,2,4]triazolo[4,3-a]quinazolin-4-ylmethyl)-benzonitrile |
| 239 | [4-(7-Bromo-5-oxo-1-pyrrolidin-1-yl-5H-[1,2,4]triazolo[4,3-a]quinazolin-4-ylmethyl)-phenyl]-acetic acid methyl ester |
| 240 | 2-[4-(7-Bromo-5-oxo-1-pyrrolidin-1-yl-5H-[1,2,4]triazolo[4,3-a]quinazolin-4-ylmethyl)-phenyl]-N-methyl-acetamide |
| 242 | 2-[4-(7-Bromo-5-oxo-1-pyrrolidin-1-yl-5H-[1,2,4]triazolo[4,3-a]quinazolin-4-ylmethyl)-phenyl]-N,N-dimethyl-acetamide |
| 246 | 1-Dimethylamino-7-methyl-4-(3-pyridin-3-yl-allyl)-4H-[1,2,4]triazolo[4,3-a]quinazoline-5-thione |

L'invention concerne également les sels acceptables en pharmacie des composés de formule I ou II. On trouvera une revue des sels acceptables en pharmacie dans J. Pharm. Sci., 1977, 66, 1-19. Toutefois, par sel pharmacologiquement acceptable d'un composé de formule I ou II présentant une fonction basique on entend les sels d'addition des composés de formule I ou II que l'on forme à partir d'acides minéraux ou organiques non toxiques comme par exemple les sels d'acides bromhydrique, chlorhydrique, sulfurique, phosphorique, nitrique, acétique, succinique, tartrique, citrique, maléique, hydroxymaléique, benzoïque, fumarique, toluène-sulfonique, isethionique et autres. Les divers sels d'ammonium quaternaires des dérivés I ou II sont également inclus dans cette catégorie des composés de l'invention. Et par sel pharmacologiquement acceptable d'un composé de formule I ou II présentant une fonction acide on entend les sels usuels des composés de formule I ou II que l'on forme à partir de bases minérales ou organiques non toxiques comme par exemple les hydroxydes des métaux alcalins et alcalino-terreux (sodium, potassium, magnésium et calcium), les amines (dibenzyléthylènediamine, triméthylamine, pipéridine, pyrrolidine, benzylamine et autres) ou encore les hydroxydes d'ammoniums quaternaires comme l'hydroxyde de tétraméthylammonium.

Tels que mentionnés précédemment, les composés de formule I et II de la présente invention sont des inhibiteurs de l'enzyme phosphodiestérase et particulièrement de l'enzyme phosphodiestérase 4 (PDE4).

A ce titre, leur utilisation est préconisée dans le traitement de maladies ou d'affections relevant d'une thérapie par l'inhibition de PDE4. A titre d'exemple, l'utilisation des composés de la présente invention peut être préconisée lors du traitement de la septicémie, de la défaillance polyviscérale, de l'asthme, de la bronchite chronique, de l'emphysème, de la pneumopathie obstructive chronique (ou COPD), de la rhinite allergique, de la dermatite atopique, de l'hypertension pulmonaire, de l'insuffisance cardiaque ou pulmonaire, de l'insuffisance cardiaque congestive, du psoriasis, de maladies inflammatoires du système digestif telles que la rectocolite hémorragique et la maladie de Crohn, de maladies liées à un taux élevé de TNF-$\alpha$ telles que le syndrome de détresse respiratoire aiguë chez l'adulte et la pancréatite aiguë, de l'arthrite rhumatoïde, de l'ostéoporose, de la sclérose en plaques et de la dépression.

Les inhibiteurs de PDE4 de la présente invention peuvent également être utilisés pour le traitement de l'atteinte pulmonaire aigüe, de l'atteinte neuronale causée par ischémie (ischemia-induced neuronal damage), du diabète, de la

leucémie lymphoïde chronique, et pour atténuer le développement de phénomènes de tolérance ou de dépendance à la morphine. Les composés de l'invention peuvent également contribuer à diminuer les pertes de mémoire du comportement (behavioral memory) telles qu'observées par exemple chez les patients souffrant de la maladie d'Alzheimer. On peut aussi envisager l'utilisation des composés de la présente invention dans le domaine de l'urologie, plus particulièrement dans le traitement d'affections de la prostate telle que l'hypertrophie bénigne de la prostate ou pour la prévention d'accouchements prématurés, par exemple en inhibant le déclenchement de contractions avant terme, de préférence par l'action d'un inhibiteur de PDE4 au niveau du myomètre.

**Analyse structure-activité des composés de formule I et II**

Les inventeurs, sans souhaiter être liés de manière formelle par une théorie définitive, sont d'avis que les paramètres structurels évoqués ci-dessous peuvent être considérés afin de guider la personne versée dans l'art dans le choix de la combinaison de substituants qui, au-delà des composés préférés décrits dans la présente demande, pourrait permettre non seulement une optimisation de l'activité inhibitrice de PDE4, mais également une meilleure optimisation de paramètres additionnels importants tels que la solubilité, la biodisponibilité et la toxicité des composés envisagés.

Tout d'abord, les inventeurs sont d'avis que le site catalytique de l'enzyme PDE4 est de taille suffisamment importante pour accommoder globalement un éventail assez varié de changements structuraux au niveau des substituants des composés de l'invention pouvant se lier à ce site. A cet égard, les inventeurs considèrent que les composés de la présente invention ont probablement la capacité d'interagir au moins en trois points distincts du site catalytique de l'isoenzyme PDE4. Un premier point d'interaction se situerait au niveau du noyau aromatique comportant les substituants $X_1$ et $X_2$. Un deuxième point d'interaction se trouve vraisemblablement au niveau du substituant R alors que le troisième point d'interaction est probablement situé au niveau du groupement $NR_4R_5$. La fonctionnalité potentielle de chacun de ces points de liaison est proposée ci-dessous.

Il est cependant important de préciser ici que les points d'interaction évoqués ci-dessus ne le sont pas nécessairement par ordre croissant ou décroissant d'importance au niveau de leur incidence sur l'activité inhibitrice des composés de l'invention. En fait, il semble possible que chacun de ces points d'interaction participe de manière différente aux propriétés pharmacologiques globales de ces composés.

Le premier point d'interaction évoqué précédemment se situerait donc au niveau du noyau aromatique comportant les substituants $X_1$ et $X_2$. Ce noyau aromatique participerait à la liaison des composés de l'invention au site catalytique de l'enzyme PDE4, liaison qu'il semble possible de moduler par le choix des substituants $X_1$ et $X_2$.

Les expériences effectuées jusqu'à présent par les inventeurs tendent à démontrer que les substituants $X_1$ et $X_2$ présentement préférés sont ceux pour lesquels $X_1$ est hydrogène et $X_2$ est choisi parmi halogène, plus particulièrement Br et Cl, méthyle, hydroxy, amino et alkylamino. On constate donc que parmi les substituants préférés de $X_2$, on retrouve à la fois des donneurs (e.g. méthyl) et des attracteurs (e.g. Br, Cl) d'électrons. Il semble donc improbable que $X_2$ puisse être choisi uniquement en fonction des propriétés électroniques du substituant préconisé. Les inventeurs sont d'avis que les critères de sélection importants se situent d'abord au niveau de la position du substituant sur le noyau aromatique et ensuite au niveau de certains paramètres tels que l'encombrement stérique du substituant ou la présence d'un atome donneur ou accepteur de proton.

Il semble cependant acquis que la position des substituants $X_1$ et $X_2$ sur le noyau aromatique puisse avoir une influence sur l'activité finale des composés de l'invention. A titre d'exemple, les composés comportant un substituant autre que l'hydrogène en position 7 sont en général plus actifs que les mêmes composés comportant ce substituant en position 8. Il semble donc probable que le choix et la position des substituants $X_1$ et $X_2$ permette de déplacer le noyau aromatique à l'intérieur d'une cavité du site catalytique de PDE4 et par voie de conséquence de moduler l'activité inhibitrice des composés de l'invention. De plus, il semble que les composés comportant un substituant en position 7 soient plus sélectifs du sous type PDE4 vis à vis des autres isoenzymes PDE5, PDE3 et PDE1 que les composés comportant un substituant en position 8. Ces derniers ont une activité inhibitrice de PDE4 (bien qu'inférieure) mais ils semblent moins sélectifs vis à vis des autres isoenzymes. Cependant, il semble aussi assez clair que bien que $X_1$ et $X_2$ puissent être choisis parmi un nombre considérable de substituants, on obtiendra une meilleure tolérance au niveau de ce choix si le substituant R est bien ciblé.

Le deuxième point d'interaction des composés de la présente invention à l'enzyme PDE4 se situerait au niveau du substituant R. Les inventeurs pensent qu'il s'agit vraisemblablement du point d'ancrage le plus important de la molécule sur l'enzyme. Il semble en effet probable que ce deuxième point d'interaction se situe au niveau d'une vaste cavité à l'intérieur du site catalytique de PDE4. Il est donc primordial que le substituant R puisse s'ancrer au site catalytique. Cependant, le choix des groupements inclus dans la définition de R donnée ci-dessus, semble démontrer une certaine flexibilité au niveau de l'ancrage de R à ce deuxième site de liaison. Autrement dit, il semblerait possible d'obtenir une activité inhibitrice de PDE4 avec des composés possédant des substituants R assez différents d'un point de vue structural. A titre d'exemple, on préférera l'utilisation d'un substituant comprenant un noyau aromatique, de

préférence lui-même substitué, et séparé de l'hétérocycle principal par une chaîne comportant entre 1 et 4 atomes, notamment des atomes de carbone, ledit substituant présentant une orientation dans l'espace relativement variable. Cette observation semble ouvrir la voie à la possibilité de moduler de façon plus subtile les propriétés globales des composés de l'invention.

Les inventeurs sont en effet d'avis que bien que le substituant R demeure vraisemblablement un élément déterminant de l'activité inhibitrice de PDE4 des composés de l'invention, il est probablement possible de le varier et ainsi agir sur des paramètres pharmacologiques supplémentaires importants sans altérer de façon substantielle cette activité inhibitrice. A titre d'exemple, des composés comportant respectivement au niveau du substituant R un groupement $-CH_2CH = CH-C_6H_5$ ou un groupement benzyle substitué, de préférence en position 4 (les autres substituants étant identiques pour les deux composés), ont une activité inhibitrice de PDE4 du même ordre de grandeur.

Le troisième site d'interaction des composés de l'invention à la PDE4 se situe vraisemblablement au niveau du groupement $-NR_4R_5$. Les inventeurs sont d'avis qu'il s'agit probablement d'un site de liaison beaucoup plus spécifique que les deux sites décrits ci-dessus bien que le déplacement du substituant R dans la cavité enzymatique puisse cependant influer sur la spécificité de ce troisième site. Les composés de l'invention possédant les meilleures activités inhibitrices sont ceux pour lesquels $R_4$ et $R_5$, qui représentent chacun un alkyle inférieur, sont liés pour former un cycle, de préférence un cycle possédant entre 5 et 8 atomes de carbone, plus particulièrement un cycle possédant 5 ou 7 atomes de carbone. La marge de manoeuvre de la personne versée dans l'art au niveau de la variation de ce groupement semble donc plus limitée.

En résumé, les expérimentations effectuées par les inventeurs avec les composés de la présente invention semblent démontrer que la taille du site catalytique de la PDE4 est suffisamment importante pour accommoder plusieurs changements structuraux au niveau des trois sites de liaison décrits précédemment. Toutefois, la marge de manoeuvre la plus importante semble tout de même se situer au niveau de la variation du substituant R.

**Formulation galénique des composés de l'invention**

Les composés de l'invention sont administrés sous forme de compositions appropriées à la nature et à l'importance de l'affection à traiter. La posologie journalière chez l'homme est habituellement comprise entre 2 mg et 1 g de produit qui peut être absorbé en une ou plusieurs prises. Les compositions sont préparées par des méthodes courantes pour l'homme de l'art et comprennent de façon générale 0,5 à 60 % en poids de principe actif (composé de formule I) et 40 à 99,5 % en poids de véhicule pharmaceutique approprié. Les compositions de la présente invention sont donc préparées dans des formes compatibles avec la voie d'administration souhaitée. A titre d'exemple, les formes pharmaceutiques suivantes peuvent être envisagées, bien que la liste fournie ci-dessous ne soit pas limitative :

1) Formes pour administration par voie orale :

Solutions buvables, suspensions, sachets de poudre pour solution buvable, sachets de poudre pour suspension buvable, gélules, gélules gastro-résistantes, formes à libération prolongée, émulsions, capsules ou gélules HPMR, lyophilisats à faire fondre sous la langue.

2) Formes pour administration par voie parentérale :

Voie intraveineuse :

Solutions aqueuses, solutions eau / co-solvant, solutions utilisant un ou des solubilisants, suspensions colloïdales, émulsions, suspensions nanoparticulaires utilisables pour l'injection de formes à libération prolongée, formes dispersées et liposomes

Voie sous-cutanée / intramusculaire :

En plus des formes utilisables en voie intraveineuse qui sont également utilisables pour les voies sous-cutanées et intramusculaires, d'autres types de formes telles que les suspensions, les formes dispersées, les gels à libération prolongée ainsi que les implants à libération prolongée peuvent également être utilisés.

3) Formes pour administration par voie topique :

Parmi les formes topiques les plus habituelles, on distingue les crèmes, les gels (phases aqueuses gélifiées par des polymères), les patchs, qui sont des pansements à coller directement sur la peau et qui peuvent être utilisés pour traiter des dermatoses sans pénétration percutanée de la substance active, les sprays, les émulsions et les

solutions.

4) Formes pour administration par voie pulmonaire :

On distingue dans cette catégorie des formes de type solutions pour aérosols, poudres pour inhaleurs, et autres formes appropriées.

5) Formes pour administration par voie nasale :

Il s'agit surtout ici de solutions pour gouttes.

6) Formes pour administration par voie rectale :

On retiendra entre autres les suppositoires et les gels.

On peut également envisager l'utilisation de formes permettant l'administration de solutions ophtalmiques ou permettant l'administration du principe actif par voie vaginale.

Une autre catégorie importante de forme pharmaceutique pouvant être utilisée dans le contexte de la présente invention concerne les formes permettant d'améliorer la solubilité du principe actif. A titre d'exemple, on peut envisager l'utilisation de solutions aqueuses de cyclodextrine, et plus particulièrement des formes comprenant l'hydroxypropyle beta cyclodextrine. Une revue détaillée de ce type de forme pharmaceutique est présentée dans l'article paru sous la référence *Journal of Pharmaceutical Sciences, 1142-1169, 85 (11), 1996*, et incorporé par référence à la présente demande.

Les différentes formes pharmaceutiques préconisées ci-haut sont décrites de façon détaillée dans l'ouvrage «Pharmacie galénique» de A. LEHIR *(Ed. Masson, 1992 (6ème édition)* incorporé par référence à la présente demande.

**Composés intermédiaires**

La présente invention concerne également les composés intermédiaires de formule générale III :

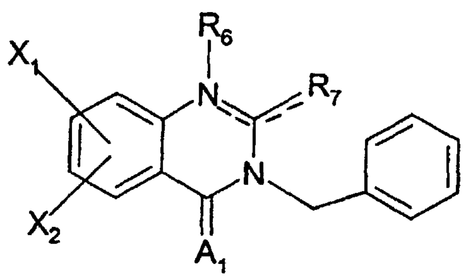

III

dans lesquels $X_1$, $X_2$, $A_1$, $R_6$ et $R_7$ sont tels que définis précédemment.

L'invention concerne particulièrement les composés intermédiaires de formule générale III dans lesquels :

$X_1$ et $X_2$ sont tels que définis précédemment, et
$R_7$ est lié à l'azote en $R_6$ pour former un triazole, substitué en position 1 par un groupement Br, Cl, mercapto ou thioalkyle inférieur, de préférence $CH_3$-S-.

Parmi les groupements définis ci-dessus les substituants suivants sont particulièrement préférés :

- De façon générale pour les groupements $X_1$, $X_2$, $R_6$ et $R_7$ :
  - halogène : F, Cl, Br, I, de préférence Br et Cl,
  - alkyle inférieur : linéaire ou ramifié comportant de 1 à 6, de préférence de 1 à 3 atomes de carbone,
  - alcoxy inférieur : linéaire ou ramifié comportant de 1 à 5, de préférence de 1 à 3 atomes de carbone,
  - thioalkyle inférieur : linéaire ou ramifié comportant de 1 à 5, de préférence de 1 à 3 atomes de carbone.

- De façon particulière pour les groupements $X_1$ et $X_2$ :

  $X_1$ et $X_2$ sont particulièrement situés en position 6 et 7 du cycle quinazolinone principal.

- De façon particulière pour les groupements $R_6$ et $R_7$ :

  lorsque $R_7$ est lié à l'azote en $R_6$ pour former un cycle, le cycle formé est de préférence un triazole, substitué en position 1 par un groupement Br, Cl, mercapto ou thioalkyle inférieur, de préférence $CH_3$-S-.

Une deuxième série d'intermédiaires comprend les composés ayant la formule générale IV suivante :

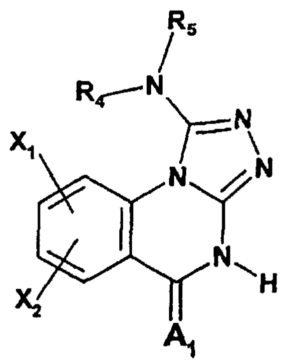

IV

dans laquelle $X_1$, $X_2$, $A_1$, $R_4$ et $R_5$ sont tels que définis précédemment.

Pour les groupements ci-dessus, les substituants suivants sont particulièrement préférés :

- De façon générale pour les groupements $X_1$, $X_2$, $R_4$ et $R_5$ :

  - halogène : F, Cl, Br, I, de préférence Br et Cl,
  - alkyle inférieur : linéaire ou ramifié comportant de 1 à 6, de préférence de 1 à 3 atomes de carbone,
  - alcoxy inférieur : linéaire ou ramifié comportant de 1 à 5, de préférence de 1 à 3 atomes de carbone,
  - alkyle inférieur, $R_4$ et $R_5$ pouvant être liés pour former un cycle saturé ou comportant une ou plusieurs doubles liaisons comprenant un ou plusieurs hétéroatomes choisis parmi O, S ou N et éventuellement ponté par un alkyle inférieur, gem dialkylé ou substitué par un ou plusieurs groupements choisis parmi hydroxy, kéto, alkyle inférieur, alcoxy inférieur, phényle alkyle ou CO-$Q_1$-$Q_2$-$Q_3$, deux des atomes du cycle ainsi formé pouvant également faire partie d'un autre cycle choisi parmi phényle ou hétéroaryle comportant de 4 à 8 atomes incluant 1 à 4 hétéroatomes.

- De façon particulière pour les groupements $X_1$ et $X_2$ :

  $X_1$ et $X_2$ sont particulièrement situés en position 6 et 7 du cycle quinazolinone principal.

- De façon particulière pour les groupements $R_4$ et $R_5$ :

  $R_4$ et $R_5$ sont alkyle inférieur, $R_4$ et $R_5$ pouvant être liés pour former un cycle saturé ou comportant une ou plusieurs doubles liaisons comprenant un ou plusieurs hétéroatomes choisis parmi O, S ou N, substitué par un ou plusieurs groupements choisis parmi hydroxy, kéto, alkyle inférieur ou alcoxy inférieur. Les substituants particulièrement préférés formant le groupe $NR_4R_5$ incluent pyrrolidine, 3-hydroxy pyrrolidine, thiamorpholine, diméthyl amino, azepanyl et pipéridinyl.

Une troisième série d'intermédiaires comprend les composés ayant la formule générale V suivante :

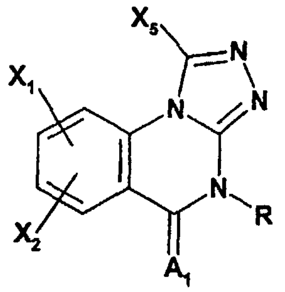

(V)

dans laquelle $X_1$, $X_2$, $X_5$, $A_1$ et R sont tels que définis précédemment.

Pour les groupements ci-dessus, les substituants suivants sont particulièrement préférés :

- De façon générale pour les groupements $X_1$, $X_2$ et $X_5$ :
  - halogène : F, Cl, Br, I, de préférence Br et Cl,
  - alkyle inférieur : linéaire ou ramifié comportant de 1 à 6, de préférence de 1 à 3 atomes de carbone,
  - alcoxy inférieur : linéaire ou ramifié comportant de 1 à 5, de préférence de 1 à 3 atomes de carbone.
- De façon particulière pour les groupements $X_1$ et $X_2$ :

  $X_1$ et $X_2$ sont particulièrement situés en position 6 et 7 du cycle quinazolinone principal.
- De façon particulière pour le groupement $X_5$ : $X_5$ est F, Br ou Cl.

Une quatrième série d'intermédiaires comprend les composés ayant la formule générale VI suivante :

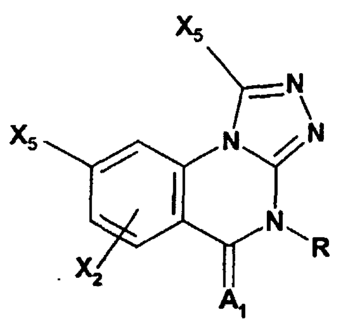

(VI)

dans laquelle $X_2$, $X_5$, $A_1$ et R sont tels que définis précédemment.

Pour les groupements ci-dessus, les substituants suivants sont particulièrement préférés :

- De façon générale pour les groupements $X_2$ et $X_5$ :
  - halogène : F, Cl, Br, I, de préférence Br et Cl,
  - alkyle inférieur : linéaire ou ramifié comportant de 1 à 6, de préférence de 1 à 3 atomes de carbone,
  - alcoxy inférieur : linéaire ou ramifié comportant de 1 à 5, de préférence de 1 à 3 atomes de carbone.
- De façon particulière pour le groupement $X_2$ :

  $X_2$ est particulièrement situé en position 7 du cycle quinazolinone principal.

- De façon particulière pour le groupement $X_5$ : $X_5$ est F, Br ou Cl.

Une cinquième série d'intermédiaires comprend les composés ayant la formule générale VII suivante :

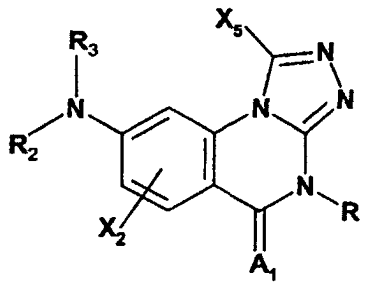

(VII)

dans laquelle $X_2$, $X_5$ $A_1$, $R_2$ et $R_3$ sont tels que définis précédemment.

Pour les groupements ci-dessus, les substituants suivants sont particulièrement préférés :

- De façon générale pour les groupements $X_2$, $X_5$, $R_2$ et $R_3$ :

  - halogène : F, Cl, Br, I, de préférence Br et Cl,
  - alkyle inférieur : linéaire ou ramifié comportant de 1 à 6, de préférence de 1 à 3 atomes de carbone,
  - alcoxy inférieur : linéaire ou ramifié comportant de 1 à 5, de préférence de 1 à 3 atomes de carbone
  - hydrogène, alkyle inférieur, éventuellement substitué par un ou plusieurs groupements hydroxy, halogène, cyano, alcoxy inférieur ou $-CO-Q_1-Q_2-Q_3$, $R_2$ et $R_3$ pouvant être liés pour former un cycle, comprenant un ou plusieurs hétéroatomes choisis parmi O, S ou N et éventuellement ponté par un alkyle inférieur, gem dialkylé ou substitué par un ou plusieurs groupements choisis parmi hydroxy, kéto, alkyle inférieur, alcoxy inférieur ou $-CO-Q_1-Q_2-Q_3$.

- De façon particulière pour le groupement $X_2$ :

  $X_2$ est particulièrement situé en position 7 du cycle quinazolinone principal.

- De façon particulière pour le groupement $X_5$ : $X_5$ est F, Br ou Cl.

- De façon particulière pour les groupement $R_2$ et $R_3$ :

  $R_2$ et $R_3$, semblables ou différents, sont hydrogène, alkyle inférieur ou $R_2$ et $R_3$ sont liés pour former un cycle, comprenant un ou plusieurs hétéroatomes choisis parmi O, S ou N et éventuellement substitué par un ou plusieurs groupements choisis parmi hydroxy, kéto, alkyle inférieur, alcoxy inférieur ou $-CO-Q_1-Q_2-Q_3$. Parmi les réalisations particulièrement préférées du substituant $NR_2R_3$, on retrouve les groupements azepanyl, pyrrolidine, $NH_2$ et $NHCH_3$.

**Procédés de synthèse des composés de formules I et II**

**A)** Les composés de la présente invention peuvent être obtenus par la mise en oeuvre de plusieurs procédés de synthèse. Quelques-uns de ces procédés de synthèse sont décrits ci-dessous.

Les composés de la présente invention peuvent d'abord être obtenus de façon convergente par la méthode représentée sur le schéma 1.

## SCHEMA 1

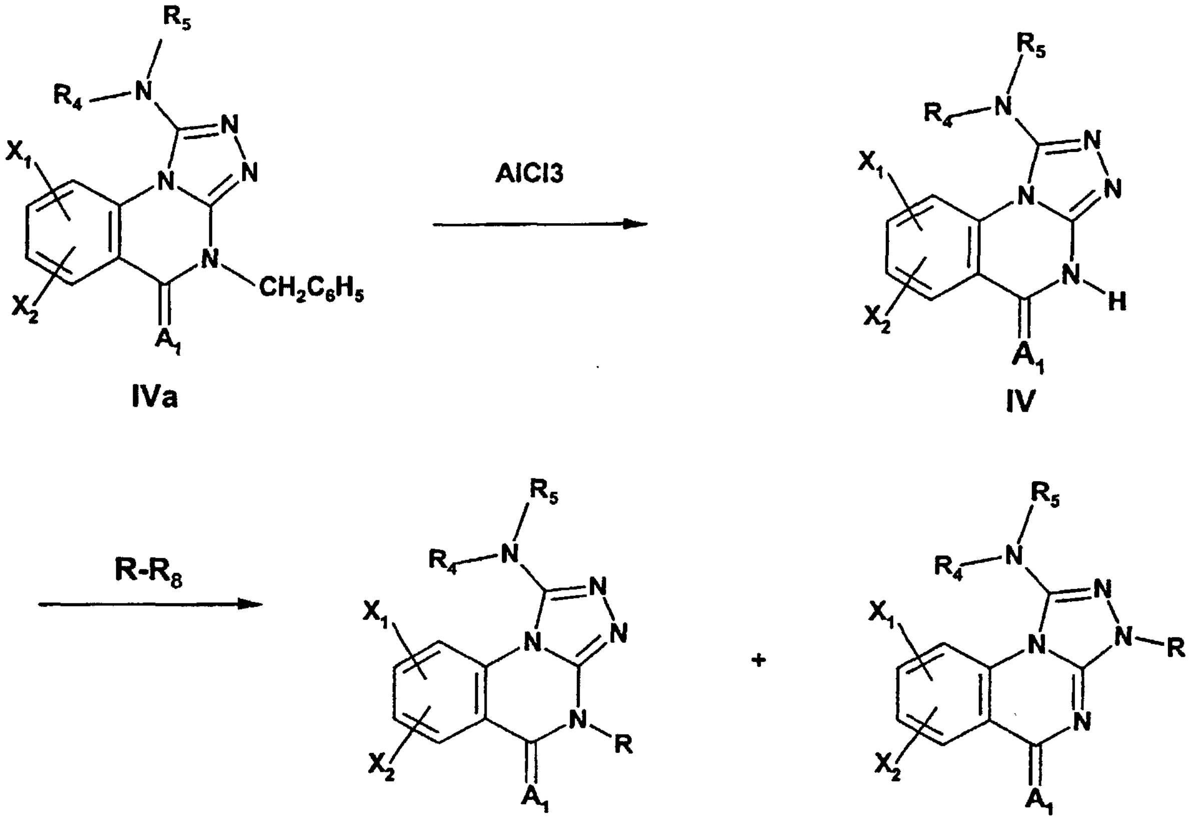


IVa

IV

(I) + (II)

dans lequel X1, X2, A1, R, R4 et R5 sont tels que définis précédemment, et R8 représente Cl, Br, OSO2CH3, OS02CF3 ou OSO2Ar.

La 4-benzyl 1-amino triazolo [4,3-a] quinazoline-5-one et/ou -5-thione (IVa) est traitée par du trichlorure d'aluminium dans un solvant aromatique tel que le benzène ou le toluène pour donner le composé correspondant N-débenzylé (IV). Celui-ci est ensuite traité par un halogénure ou un sulfonate choisi en fonction du substituant R désiré en conditions basiques ; par exemple de l'hydrure de sodium dans un solvant tel que le 1, 2-diméthoxyéthane (DME) ou du carbonate de césium dans le diméthylformamide, pour conduire aux 1-amino triazolo [4,3-a] quinazoline-5-ones de formule (I) et (II).

En fait, en fonction des conditions basiques utilisées, l'alkylation est peu régiosélective dans certains cas. On obtient alors un mélange de $N_4$ et $N_3$, régioisomères, respectivement (I) et (II).

Les 2 composés sont généralement séparés par des méthodes classiques de chromatographie.

**B)** Un autre exemple d'une méthode de synthèse utilisée pour construire le motif triazolo [4,3-a] quinazoline-5-one et/ou -5-thione de formule (I) convenablement substitué est illustré au schéma 2 :

## SCHEMA 2

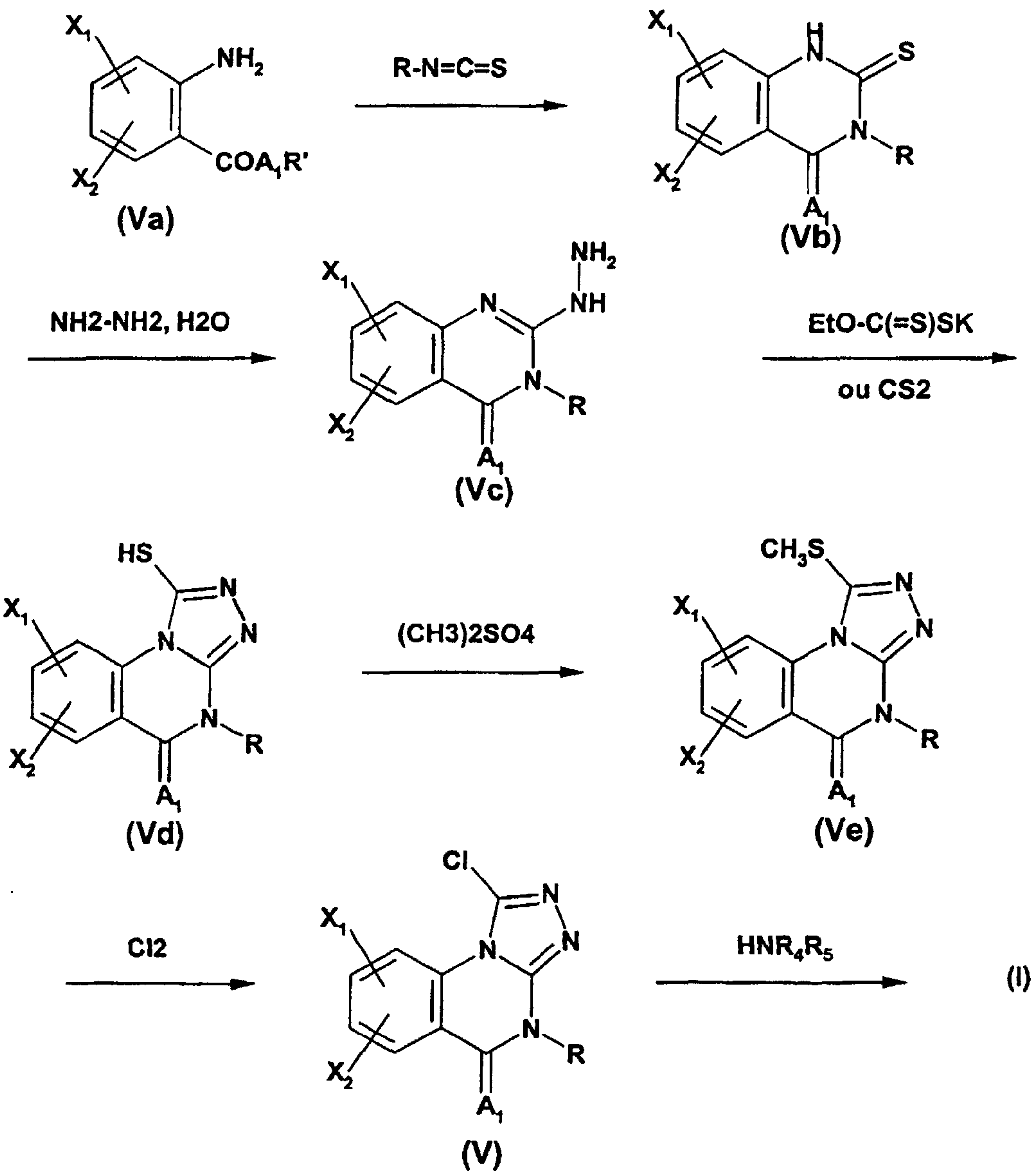


(Va) (Vb) (Vc) (Vd) (Ve) (V) (I)

dans lequel $X_1$, $X_2$, $A_1$, R, $R_4$, et $R_5$ sont tels que définis précédemment, et,
R' représente un groupement alkyle inférieur linéaire ou ramifié comportant de 1 à 6, de préférence de 1 à 3 atomes de carbone.

Un acide ou ester anthranilique convenablement substitué sur le cycle aromatique (Va) est d'abord transformé en 2-thio quinazoline-4-one et/ou —4-thione correspondante (Vb) par cyclisation au moyen d'isothiocyanate d'alkyle, d'aryle ou d'aralkyle, dans un solvant tel que l'acide acétique ou la pyridine.
La thio quinazoline-4-one et/ou —4-thione (Vb) est traitée par de l'hydrate d'hydrazine pour fournir la 2-hydrazino quinazoline-4-one et/ou —4-thione (Vc) qui est à son tour cyclisée en 1-mercapto triazolo [4,3-a] quinazoline-5-one et/ou —5-thione (Vd) par action de xanthogenate de potassium ou d'autres réactifs tels que $CS_2$.
Par action d'un agent alkylant tel que le sulfate de diméthyle, le thiol (VI) est transformé en 1-méthylthio dérivé (Ve) lequel est ensuite converti au moyen de chlore, en 1-chloro triazolo [4,3-a] quinazoline-5-one et/ou—5-thione (V).
Ce dernier est traité par une amine primaire ou secondaire pour conduire finalement à la 1-amino triazolo [4,3-a] quinazoline-5-one de formule (I).

**C)** Une autre méthode avantageuse dans certains cas est représentée sur le schéma 3.

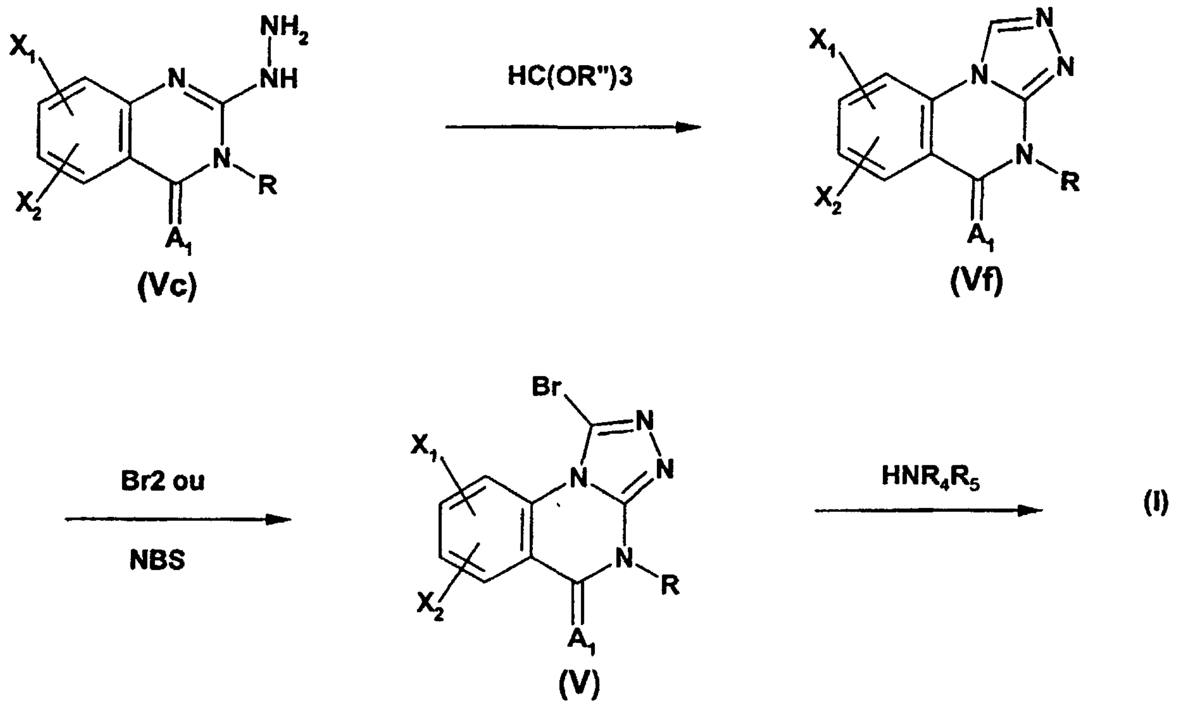


**SCHEMA 3**

dans lequel $X_1$, $X_2$, $A_1$, R, $R_4$ et $R_5$ sont tels que définis précédemment, et
R" représente un alkyle inférieur linéaire ou ramifié comportant de 1 à 6, de préférence de 1 à 3 atomes de carbone, tel que $CH_3$ ou $C_2 H_5$.

La 2-hydrazino quinazoline-4-one et/ou — 4-thione (Vc), obtenue à partir d'un anthranilate en 2 étapes (comme illustré dans le schéma 2), est cyclisée au moyen d'un orthoformiate d'alkyle, en milieu acide, en triazolo [4,3-a] quinazoline-5-one et/ou — 5-thione (Vf).
Celui-ci est ensuite bromé par du Brome ou du N-Bromosuccinimide (NBS) pour donner la 1-bromotriazolo [4,3-a] quinazoline-5-one et/ou — 5-thione (V).
Ce dérivé bromé est finalement traité par une solution éthanolique d'une aminé primaire ou secondaire pour conduire à la 1-amino triazolo [4,3-a] quinazoline-5-one et/ou — 5-thione de formule (I).

D) Quand X1 représente H et X2 représente une fonction phénolique réactive OH, ce groupement doit en général être protégé pendant les dernières étapes de la synthèse des composés (I). A titre d'exemple, le schéma 4 montre la synthèse d'un tel composé hydroxylé en position 7. La 4-benzyl-7-hydroxy-4H-[1,2,4]triazolo[4,3-a]quinazolin-5-one et/ou — 5-thione(Vg), obtenue par une méthode représentée sur le schéma 3, est traitée par un composé permettant l'insertion d'un groupement protecteur d'oxygène (P) sur la fonction OH. L'homme du métier pourra choisir sans difficulté le groupement protecteur approprié. Le groupement protecteur peut être choisi entre autres parmi triméthyl silyl, méthoxyméthyl, tolylsulfonyl, méthylsulfonyl (mésyl) ou encore méthoxyéthylméthoxy (MEM). A titre d'exemple, le composé (Vg) est traité par du chlorure de tosyle, dans un solvant tel que le chlorure de méthylène, en présence d'une base ou d'une amine telle que la triéthylamine, pour donner le phénol O-tosylé correspondant (Vf). Celui-ci est traité par du brome pour conduire à la 4-benzyl-1-bromo-7-(4-tolylsulfonyl)-4H-[1,2,4]triazolo[4,3-a]quinazolin-5-one et/ou — 5-thione ($V_3$), lequel réagit avec une amine HNR4R5 au reflux, de préférence en présence d'une base comme le bicarbonate de sodium, dans un solvant tel que le diméthylformamide, pour fournir la 1-amino-4-benzyl-7-(4-tolylsulfonyl) — 4H-[1,2,4]triazolo[4,3-a]quinazolin-5-one et/ou — 5-thione ($IVa_1$).

On peut alors remplacer le groupe benzyle en position 4 par un autre groupe R, par exemple en débenzylant le composé ($IVa_1$) obtenu précédemment au moyen de chlorure d'aluminium dans un solvant comme le benzène, puis en alkylant l'intermédiaire obtenu ($IV_1$) par traitement avec un halogénure ou un sulfonate $R\text{-}X_5$, dans des conditions basiques, pour obtenir les 1-amino-7-(4-tolylsulfonyl) — 4H-[1,2,4]triazolo[4,3-a]quinazolin-5-one et/ou — 5-thione (Ia) diversement substituées en position 4. Celles-ci sont de préférence détosylées en dérivés 7-hydroxy (I) par exemple par chauffage pendant quelques heures dans de la pyrrolidine.

**SCHEMA 4**

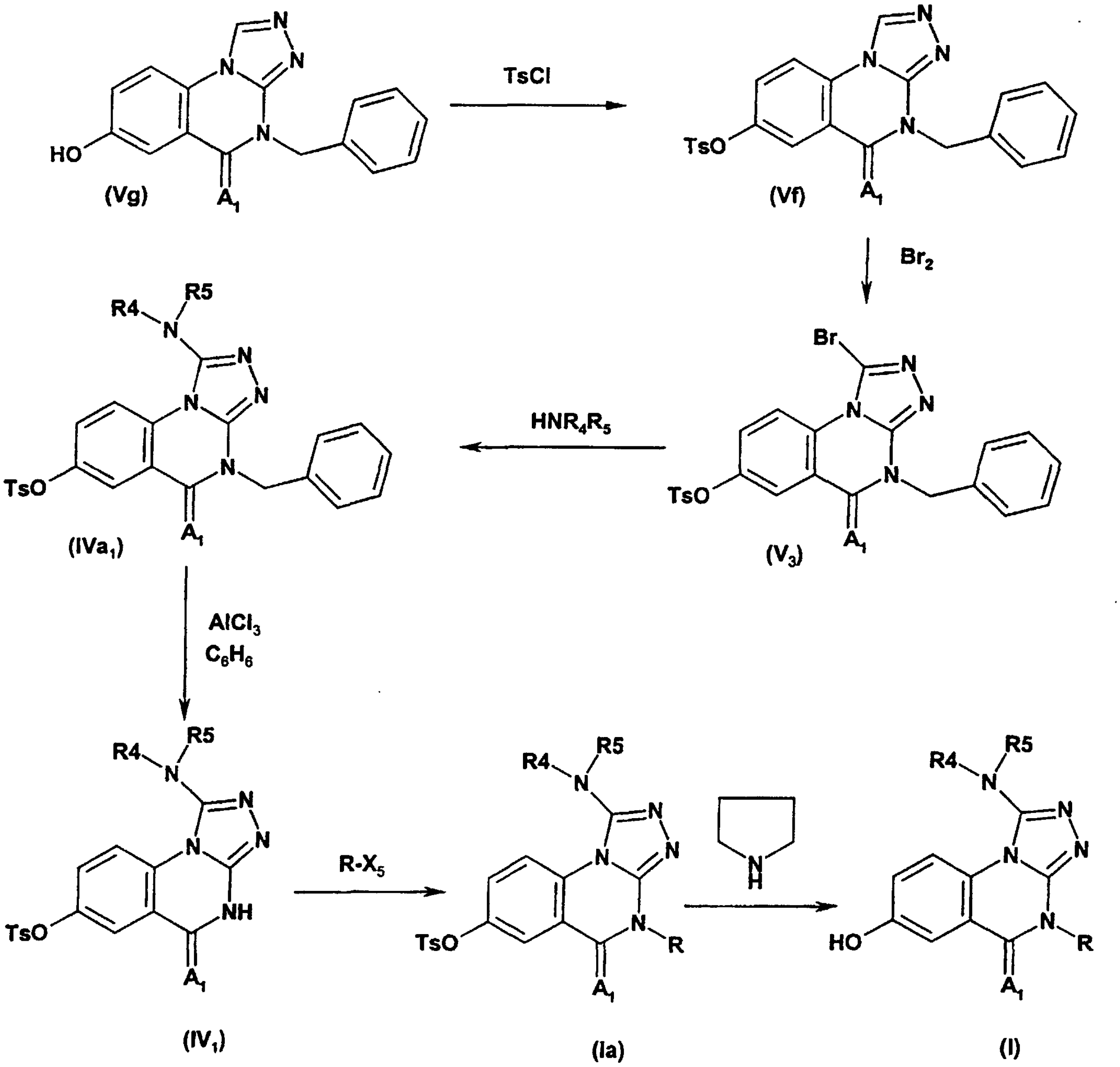


dans lequel $A_1$, $R_4$ et $R_5$ sont tels que définis précédemment.

E) Quand X1 représente H et X2 représente une fonction anilino réactive : NH2, NHR2 ou $NR_2R_x$ (R2 tel que défini précédemment et $R_x$ représente $R_2$ ou $R_3$ tels que définis précédemment), le groupement amino NH2 doit en général être protégé pendant les dernières étapes de la synthèse des composés (I). A titre d'exemple, le schéma 5 montre la synthèse d'un tel composé aminé en position 7. La 7-acétamido-4-benzyl-4H-[1,2,4]triazolo[4,3-a]quinazolin-5-one et/ou — 5-thione ($Vf_1$), obtenue par une méthode représentée sur le schéma 3, est traitée par du brome pour conduire à la 7-acétamido-4-benzyl-1-bromo-4H-[1,2,4]triazolo[4,3-a]quinazolin-5-one et/ou — 5-thione ($V_4$). Celle-ci est mise à réagir avec une amine HNR4R5 au reflux, de préférence en présence d'une base comme le bicarbonate de sodium, dans un solvant tel que le diméthylformamide, pour fournir la 7-acétamido-1-amino-4-benzyl-4H-[1,2,4] triazolo[4,3-a]quinazolin-5-one et/ou — 5-thione ($IVa_2$). Dans l'exemple décrit ci-dessus, le groupement protecteur ($P_1$) de la fonction NH est un groupement acétyle. L'homme du métier peut cependant choisir un autre groupement protecteur, par exemple le méthylsulfonyl, le tolylsulfonyl ou le phtalimido.

On peut alors remplacer le groupe benzyle en position 4 par un autre groupe R, par exemple en débenzylant le composé ($IVa_2$) obtenu précédemment, au moyen de formiate d'ammonium et de Palladium sur Charbon, dans un solvant tel que le tétrahydrofuranne, puis en alkylant l'intermédiaire obtenu ($IV_2$) par traitement avec un halogénure ou un sulfonate R-$X_5$, dans des conditions basiques, pour obtenir les 7-acétamido-1-amino-4H-[1,2,4]triazolo[4,3-a] quinazolin-5-one et/ou — 5-thione (I) diversement substituées en position 4. Celles-ci peuvent être N-déacétylées en

composés finaux (Ib) portant une fonction NH2 en position 7, par des méthodes classiques comme par exemple un chauffage à reflux dans une solution aqueuse d'acide chlorhydrique. Ces composés peuvent être à leur tour traités, suivant le cas, par un réactif R2-$X_5$ (R2 et $X_5$ ayant la signification donnée précédemment) pour conduire à un composé final (Ic) N-monosubstitué, qui peut lui-même ensuite être traité par un réactif $R_xX_5$ pour conduire à un composé final (1d) N,N-disubstitué. Il est également possible de traiter les 7-acétamido-1-amino-4H-[1,2,4]triazolo[4,3-a]quinazolin-5-one et/ou — 5-thione (I) diversement substituées en position 4 d'abord par un réactif $R_2X_5$ pour obtenir le composé ($1b_2$) qui est ensuite N-déacétylé pour obtenir le composé (Ic).

SCHEMA 5

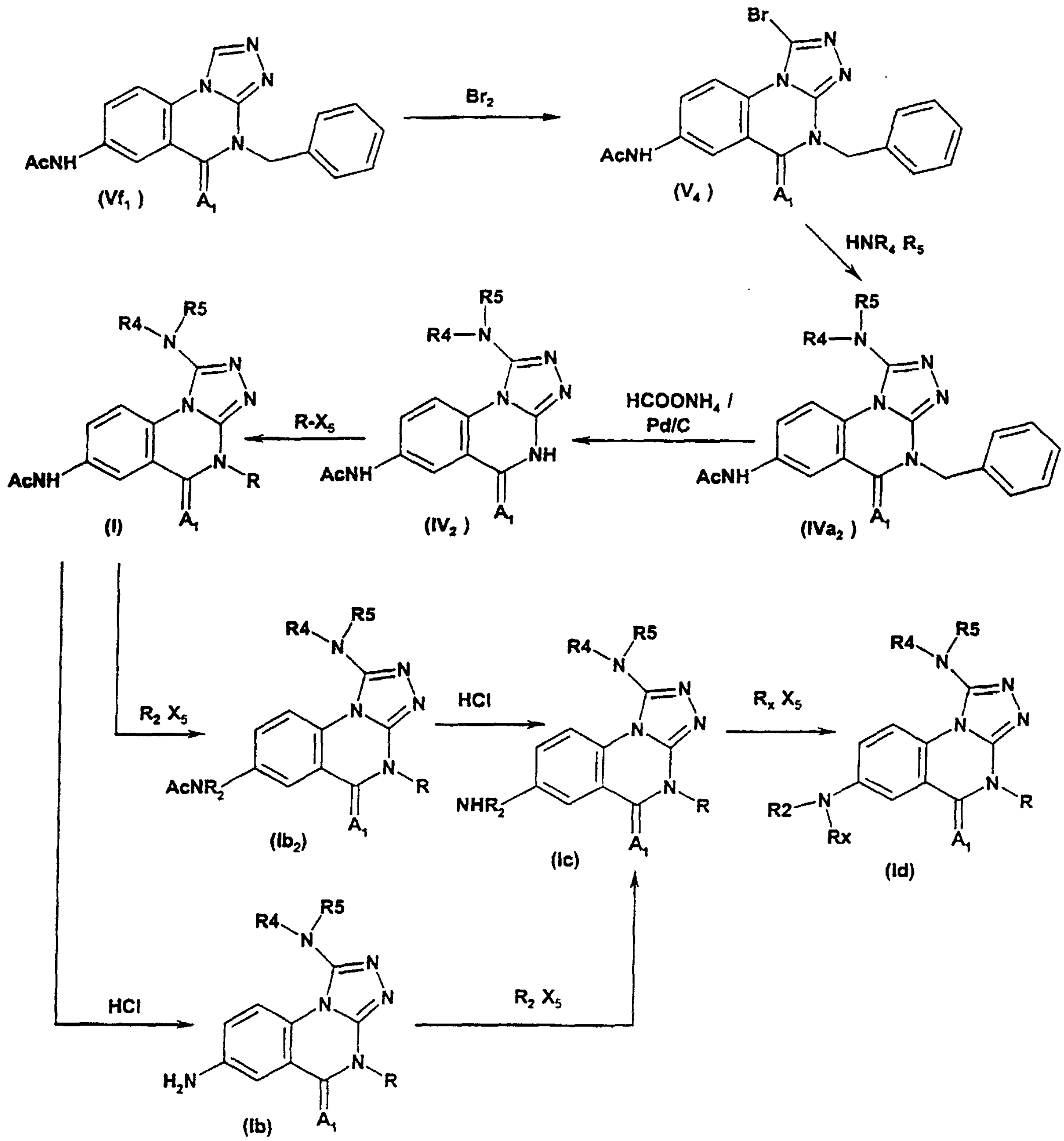


F) Quand le substituant R en position 4 des composés (I) représente un groupe 4-(carboxyméthyl)-benzyle, il peut être avantageux de transformer la fonction acide carboxylique en dérivé ester, amide, nitrile ou acide hydroxamique. Pour cela, les méthodes représentées sur le schéma 6 peuvent être appliquées à un acide de formule générale (Id). Celui-ci est transformé en chlorure d'acide (Ie), lequel est directement condensé soit avec l'ammoniaque pour donner un amide primaire (If), soit avec une amine primaire ou secondaire pour donner respectivement un amide secondaire (Ih) ou tertiaire (Ii) (Dans ces structures, R11 a la même signification que R2 et R12, R13 ont les mêmes significations que R4, R5 respectivement).

L'acide hydroxamique (Ij) peut être obtenu par réaction du chlorure d'acide (Ie) avec l'hydroxylamine. L'amide primaire (If) peut aussi être déshydraté par des méthodes classiques et connues en soi, comme par exemple au moyen du pentoxyde de phosphore, pour conduire au nitrile correspondant (Ig).

SCHEMA 6

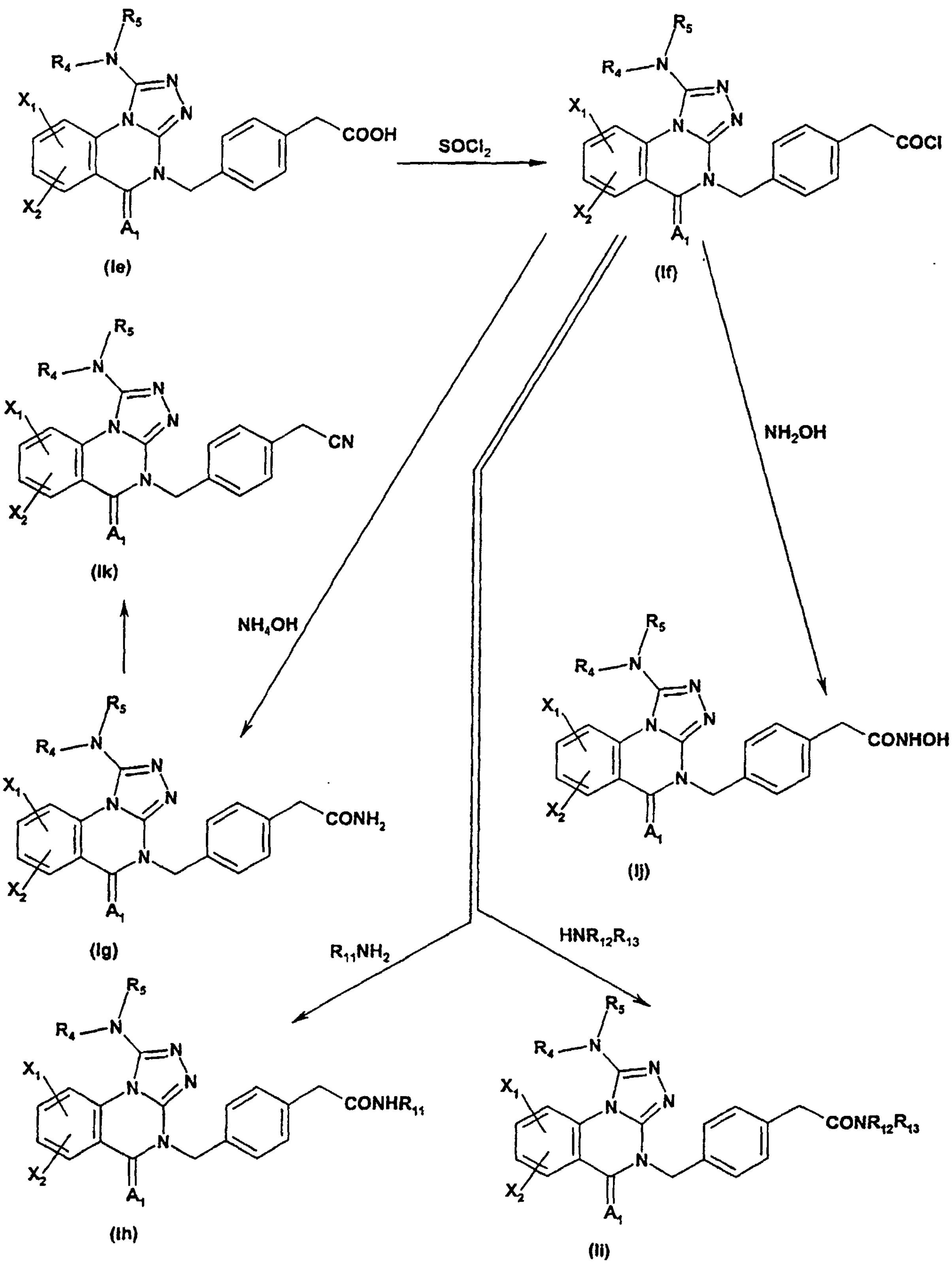


(Ie) (If) (Ik) (Ij) (Ig) (Ih) (Ii)

dans lequel $X_1$, $X_2$, $A_1$, $R_4$ et $R_5$ sont tels que définis précédemment.

G) Les composés de structure (I) dans laquelle $X_1$ ou $X_2$ représente un groupe amino $NR_2R_3$ en position 8

identique au groupement $NR_4R_5$, peuvent également être obtenus par chauffage de l'intermédiaire 1-bromo correspondant (VI ; $X_5$ = hal) en présence d'un excès d'amine $HNR_4R_5$, sans solvant ou dans un solvant tel que le diméthylformamide comme illustré au schéma 7.

## SCHEMA 7

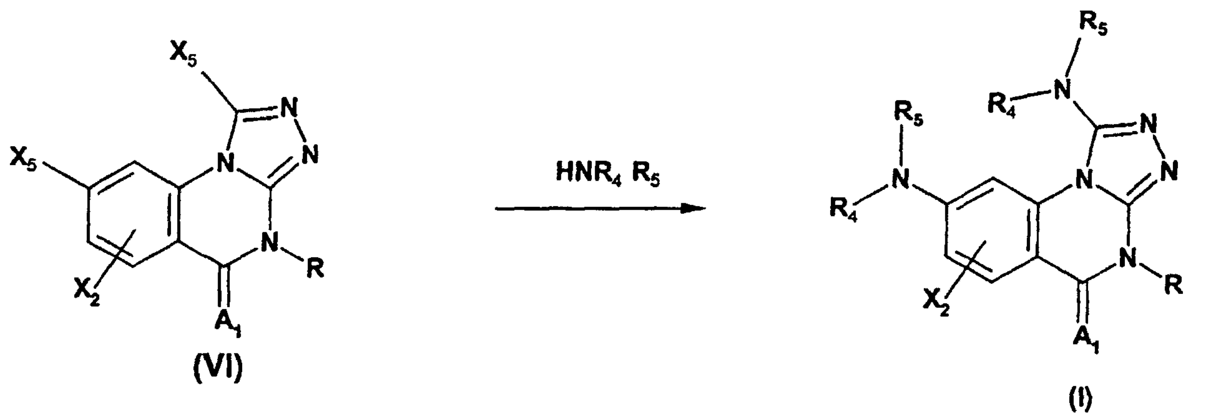


dans lequel $X_2$, $X_5$, $A_1$, R, $R_4$ et $R_5$ sont tels que définis précédemment.

Il est cependant préférable d'éviter pour ce type de réaction les substituants R comportant un groupement halogène susceptible de réagir de façon compétitive avec le réactif $HNR_4R_5$.

**H)** Dans le cas où les 2 groupes amino $NR_2R_3$ et $NR_4R_5$ sont différents, une voie de synthèse légèrement modifiée est indiquée sur le schéma 8.

## SCHEMA 8

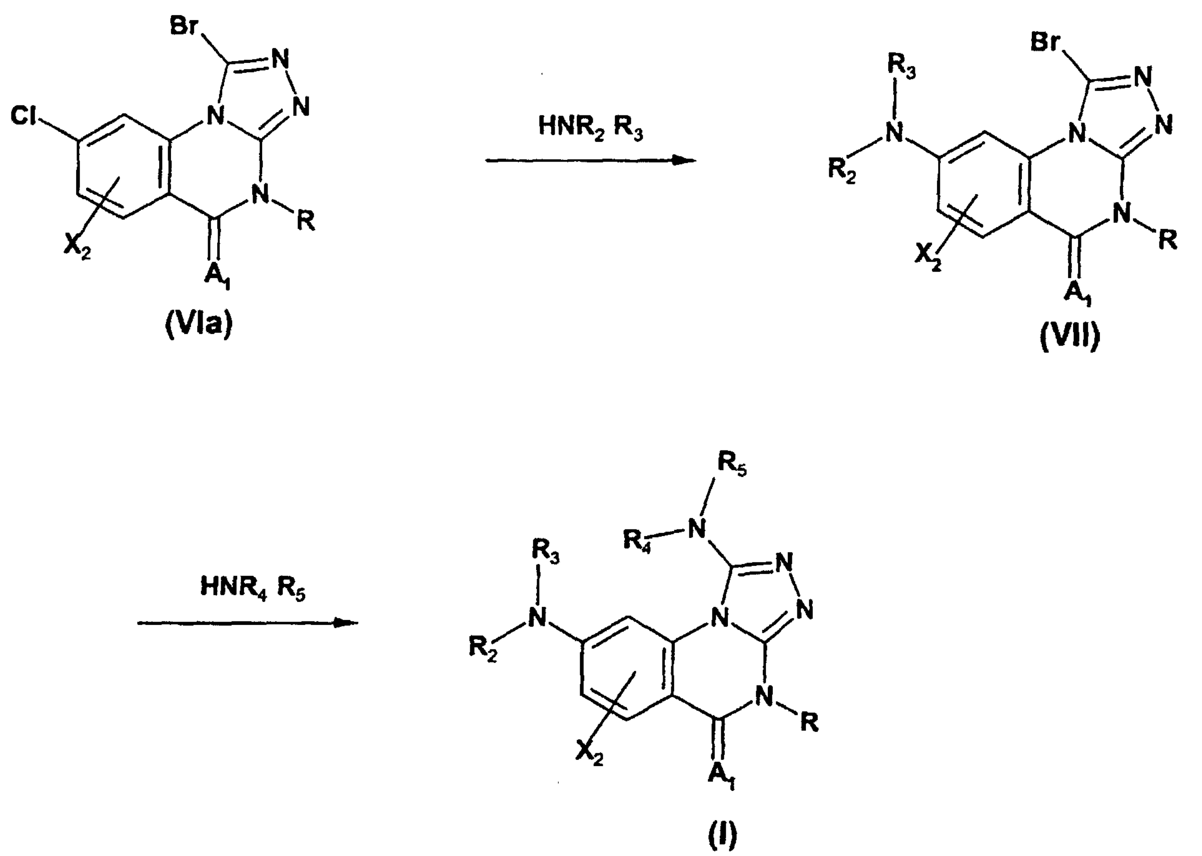


(VIa) (VII) (I)

dans lequel $X_2$, $A_1$, R, $R_2$, $R_3$, $R_4$ et $R_5$ sont tels que définis précédemment. Le substituant amino $NR_2R_3$ se trouve en position 8.

Une 1-Bromo 8-chlorotriazolo [4,3-a] quinazoline-5-one et/ou —5-thione (VIa) convenablement substituée en 4, et préparée comme précédemment par bromation du dérivé non substitué en 1, est traitée par un léger excès d'amine $HNR_2R_3$, dans un solvant tel que le diméthylformamide pour conduire à l'intermédiaire (VII).
Cet intermédiaire est à son tour chauffé dans un excès d'amine $HNR_4R_5$, dans un solvant tel que le diméthylformamide pour conduire au composé (I).

De façon surprenante, les inventeurs ont constaté que la réactivité de l'atome d'halogène en position 8 est beaucoup plus importante que la réactivité de l'autre atome d'halogène de l'intermédiaire. Ceci permet donc une première réaction sélective au niveau de cet halogène en position 8, qui peut être suivie par une réaction au niveau du deuxième halogène . L'exemple ci-dessus illustre l'utilisation du chlore en position 8. Il est cependant possible d'utiliser d'autres halogènes tels que le brome et le fluor, ce dernier s'étant avéré particulièrement réactif.

### Exemples

### A. Composés de type (I) et (II)

### Exemples 1 et 2

**METHODE A** : 1-Azepanyl-7-chloro-4-(3-phénylallyl)-4H-[1,2,4]triazolo[4,3-a]quinazolin-5-one (ex. 1)

(I): $X_1$ = 7 - Cl ; $X_2$ = H ;

R= 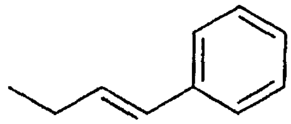 ; $NR_4R_5$= 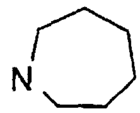

et

1-Azepanyl-7-chloro-3-(3-phénylallyl)-3H-[1,2,4]triazolo[4,3-a]quinazolin-5-one (ex. 2)

(II): $X_1$ = 7 - Cl ; $X_2$ = H ;

R= 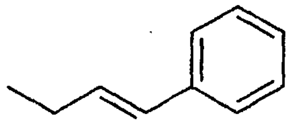 ; $NR_4R_5$= 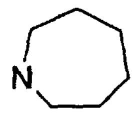

et

Dans un réacteur protégé de l'humidité, on place 2,5 g (7,87 mmol) de 1-Azepan-yl-7-chloro-4H-[1,2,4] triazolo [4,3-a] quinazolin-5-one, en suspension dans 35 ml de 1,2-diméthoxyéthane puis on agite.
Sous atmosphère inerte, on additionne alors 240 mg d'une suspension d'hydrure de sodium à 75 % (représentant 7,90 mmol NaH).
Le mélange est chauffé à 60° C, sous agitation, pendant 6 heures.
Le mélange obtenu est chauffé ensuite à 60° C pendant 20 heures, sous agitation.
Après refroidissement, la suspension est versée dans 200 ml d'eau glacée.
On extrait 3 fois à l'acétate d'éthyle ; les phases organiques réunies sont lavées avec une solution aqueuse saturée de chlorure de sodium, séchées sur sulfate de sodium puis le solvant est évaporé sous vide.
On obtient 3,5 g de mélange brut des 2 régio isomères (théorie : 3,4 g).
Les 2 isomères sont séparés par chromatographie flash sur colonne de silice avec élution au mélange chlorure de méthylène 99 / méthanol 1.
On obtient, dans l'ordre d'élution :

1) 0,58 g du composé de l'exemple 1
Rendement = 17%
F (Tottoli) = 125°C
CCM ($CH_2$ $Cl_2$ 98 / $CH_3$ OH 2) = 0,60
RMN$^1$ H δ (ppm) $CDCl_3$ : 1,7 - 2,0 (m, 8H) ; 3,3 - 3,5 (m, 4H) ; 5,05 (d, 2H) ; 6,45 (dt, 1H) ; 6,9 (d, 1H) ; 7,15 - 7,3 (m, 3H) ; 7,35 (d, 2H) ; 7,75 (d, 1H) ; 8,35 (s, 1H) ; 8,4 (d, 1H).
2) 2,1 g du composé de l'exemple 2
Rendement = 61,5 %
F (Tottoli) = 188°C
CCM ($CH_2$ $Cl_2$ 98 / $CH_3$ OH 2) : Rf = 0,35.
RMN $^1$H δ (ppm) $CDCl_3$ :
1,7 - 2,0 (m, 8H) ; 3,4 (m, 4H) ; 4,9 (d, 2H) ; 6,35 (dt, 1 H) ; 6,75 (d, 1H) ; 7,2 - 7,45 (m, 5H) ; 7,65 (d, 1H); 8,2 (d, 1H); 8,45 (s, 1H)

**Exemple 3 :**

**METHODE B** : 7-bromo-1-(N,N-dimethylamino)-4-[3-(3-pyridyl)-allyl]-4H-[1,2,4]triazolo[4,3-a]quinazolin-5-one (ex. 3)

(I): $X_1$ = 7 - Br ; $X_2$ = H ;

R= 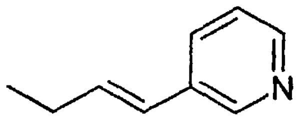 ; NR4R5= 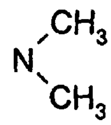

Dans un réacteur équipé d'une agitation magnétique et d'un réfrigérant, on place 7,4 g (0,024 mol) de 7-bromo-1-(N,N-dimethylamino)-4H-[1,2,4]triazolo[4,3-a]quinazolin-5-one en solution dans 200 ml de 1,2-diméthoxyéthane puis on agite. On additionne 17,0g (0.052mol) de carbonate de césium puis agite à température ambiante pendant 15 minutes. 4,5g ((0,024mol) de chlorhydrate de chlorure de 3-(3-pyridyl)-allyle sont alors ajoutés par fractions, puis le mélange est chauffé à 70°C, sous agitation, pendant 3 heures. Le solvant est évaporé sous vide puis le résidu est mis en suspension dans 300 ml d'eau glacée. Après extractions répétées à l'acétate d'éthyle, les phases organiques réunies sont lavées avec une solution aqueuse saturée de chlorure de sodium, séchées sur sulfate de sodium puis le solvant est évaporé sous vide.
Le résidu est chromatographié sur une colonne de silice avec élution au mélange CH2Cl2 98 / CH3OH 2 / NH4OH 0,2. On récupère 6,3g d'isomère (I) pur en CCM. Celui-ci est recristallisé dans 20ml d'isopropanol pour donner 5,3g de composé de l'exemple 3 :

Rendement = 52%
RMN[1] H δ (ppm) $CDCl_3$ : 2,95 (s, 6H) ; 5,1 (d, 2H) ; 6,45 (dt, 1H) ; 6,8 (d, 1H) ; 7,15 (m, 1H) ; 7,65 (d, 1H) ; 7,9 (d, 1H) ; 8,25 (d, 1H) ; 8,4 - 8,6 (m, 3H).

**Exemples 4 et 5 :**

**METHODE C :** 7-bromo-1-(pyrrolidin-1-yl)-4-[(3-pyridyl)-methyl]-4H-[1,2,4]triazolo[4,3-a]quinazolin-5-one (ex. 4)

(1): $X_1$ = 7 - Br ; $X_2$ = H ;

R= 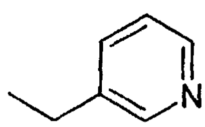 ; NR4R5= 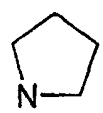

7-bromo-1-(pyrrolidin-1-yl)-3-[(3-pyridyl)-methyl]-3H-[1,2,4]triazolo[4,3-a]quinazolin-5-one (ex. 5)
(II) : X1 = 7-Br ; X2 = H ;

R= 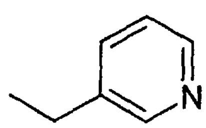 ; NR4R5= 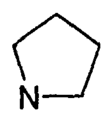

Dans un réacteur protégé de l'humidité, équipé d'un système d'agitation, on place 2,0g (0,006 mol) de 1-(pyrrolidin-1-yl)-7-bromo-4H-[1,2,4]triazolo[4,3-a]quinazolin-5-one en solution dans 125 ml de dimethylsulfoxyde (DMSO) puis on additionne 1,0g (0,018 mol) de potasse finement broyée. Le mélange est agité à température ambiante pendant 1h30, jusqu'à obtention d'une solution légèrement trouble. On ajoute alors 0,82g (0,005 mol) de chlorhydrate de chlorure de 3-picolyle en une fois puis maintient l'agitation à température ambiante pendant 4 heures.
Le mélange obtenu est versé dans de l'eau glacée et la suspension résultante extraite 3 fois à l'acétate d'éthyle. Les extraits organiques joints sont lavés avec une solution saturée de NaCl, séchés sur Na2SO4 puis concentrés sous vide. On obtient 2,0g de mélange brut des 2 régioisomères qui sont séparés par chromatographie sur colonne de silice

avec élution au mélange CH2C12 98 - CH3OH 2 - NH4OH 0,4.

On obtient, dans l'ordre d'élution :

1) 1,2g du produit majoritaire qui est recristallisé dans le méthanol pour donner, après séchage sous vide, 1,1g du composé de l'exemple 4
Rendement = 57 %
F (Tottoli) = 206-207°C
CCM ($CH_2$ $Cl_2$ 97 / $CH_3$ OH 3 / NH4OH 0,3) : Rf= 0,30
RMN$^1$ H δ (ppm) $CDCl_3$ : 1,95 - 2,1 (m, 4H) ; 3,35- 3,45 (m, 4H) ; 5,45 (s, 2H) ; 7,2 - 7,3 (dd, 1H) ; 7,85 (d, 1H) ; 8,0 (d, 1H) ; 8,2 (d, 1H) ; 8,45 - 8,55 (m, 2H) ; 8,9 (s, 1H).

2) 0,25g du produit minoritaire qui est recristallisé dans le méthanol pour donner, après séchage sous vide, 0,17g du composé de l'exemple 5
Rendement = 12%
F (Tottoli) = 261-262°C
CCM ($CH_2$ $Cl_2$ 97 / $CH_3$ OH 3 / NH4OH 0,3) : Rf= 0,20
RMN$^1$ H δ (ppm) $CDCl_3$ : 1,9 - 2,05 (m, 4H) ; 3,2 - 3,4 (m, 4H) ; 5,25 (s, 2H) ; 7,1 - 7,2 (m, 1H) ; 7,7 (d, 1H) ; 7,8 (d, 1H) ; 7,9 (d, 1H) ; 8,45 - 8,60 (m, 2H) ; 8,65 (s, 1H).

Les composés (I) des exemples 6 à 108 et les composés (II) des exemples 109 à 162, dans lesquels $X_2$ = $H_1$ sont préparés selon le procédé de l'exemple 1 :

Composés (I) : Tableau 1
Composés (II) : Tableau 2

## TABLEAU 1

| N° Composé | X1 | R | NR4R5 | Rdt (%) | PF (°C) | Méthode |
|---|---|---|---|---|---|---|
| 6 | H | (E) C6H5CH=CHCH2 | 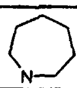 | 11 | 144 | A |
| 7 | 7-Cl | CH2=CHCH2 | 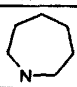 | 9 | - | A |
| 8 | 7-Cl | 4-CH3C6H4CH2 | 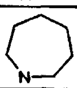 | 16 | 163 | A |
| 9 | 7-Cl | 2-ClC6H4CH2 | 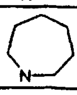 | 6 | 160-162 | A |

| N° Composé | X1 | R | NR4R5 | Rdt (%) | PF (°C) | Méthode |
|---|---|---|---|---|---|---|
| 10 | 7-Cl | 3-ClC6H4CH2 | 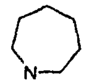 | 35 | 157 | A |
| 11 | 7-Cl | 4-ClC6H4CH2 | 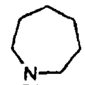 | 20 | 166 | A |
| 12 | 7-Cl | 4-BrC6H4CH2 | 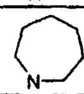 | 25 | 104-110 | A |
| 13 | 7-Cl | 4-FC6H4CH2 | 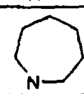 | 48 | 150 | A |
| 14 | 7-Cl | 4-CF3C6H4CH2 | 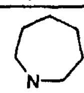 | 22 | 138 | A |
| 15 | 7-Cl | 4-CNC6H4CH2 | 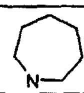 | 49 | 165-168 | A |
| 16 | 7-Cl | 2-(CH3O)C6H4CH2 | 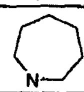 | 6 | 98-100 | A |
| 17 | 7-Cl | 3-(CH3O)C6H4CH2 | 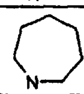 | 22 | 138 | A |
| 18 | 7-Cl | 4-(CH3O)C6H4CH2 | 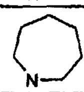 | 26 | 138 | A |
| 19 | 7-Cl | 3,4-Cl2C6H3CH2 | 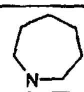 | 19 | - | A |
| 20 | 7-Cl | 3,4-(CH3O)2C6H3CH2 | 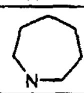 | 41 | 172 | A |
| 21 | 7-Cl | (2-pyridyl)CH2 | 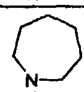 | 16 | 152 | A |
| 22 | 7-Cl | (3-pyridyl)CH2 | 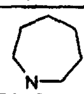 | 29 | 155 | A |
| 23 | 7-Cl | (4-pyridyl)CH2 | 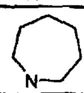 | 64 | 137 | A |
| 24 | 7-Cl | C6H5CH2CH2 | 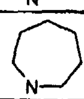 | 5 | 105 | A |
| 25 | 7-Cl | 4-(CH3O)C6H4(CH2)2 | 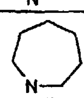 | 12 | 136 | A |
| 26 | 7-Cl | C6H5(CH2)3 | 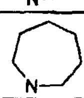 | 17 | - | A |
| 27 | 7-Cl | C6H5C(=O)CH2 | 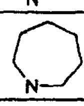 | 26,5 | 105-107 | A |

| N° Composé | X1 | R | NR4R5 | Rdt (%) | PF (°C) | Méthode |
|---|---|---|---|---|---|---|
| 28 | 7-Cl | 4-(CH3O)C6H4C(=O)CH2 | 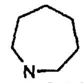 | 30 | 191 | A |
| 29 | 7-Cl | 4-ClC6H4C(=O)CH2 | 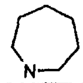 | 36 | 190 | A |
| 30 | 7-Cl | 4-(CH3O)-3-(COOCH3)-C6H3C(=O)CH2 | 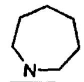 | 18 | 140 | A |
| 31 | 7-Cl | (3-pyridyl)-CH2 | 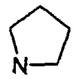 | 39 | 176 | C |
| 32 | 7-Br | 4-ClC6H4CH2 | 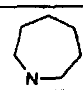 | 8 | 179 | A |
| 33 | 7-Br | 4-FC6H4CH2 | 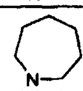 | 21 | 158 | A |
| 34 | 7-Br | 4-CNC6H4CH2 | 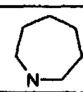 | 21 | 190 | A |
| 35 | 7-Br | 3,4-(CH3O)2C6H3CH2 | 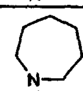 | 23,5 | 185 | A |
| 36 | 7-Br | (3-pyridyl)-CH2 | 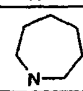 | 4 | 180 | C |
| 37 | 7-Br | (E) C6H5CH=CHCH2 | 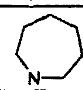 | 64 | 155 | B |
| 38 | 7-Br | (E) 4-Cl-C6H4CH=CHCH2 | 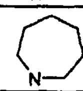 | 25 | 176 | B |
| 39 | 7-Br | (E) 4-(CH30)C6H4CH=CHCH2 | 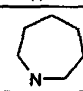 | 30 | 129 | B |
| 40 | 7-Br | (E) (3-pyridyl)CH=CHCH2 | 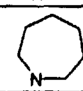 | 12 | 185 | B |
| 41 | 7-Br | (E) (4-pyridyl)CH=CHCH2 | 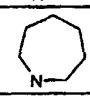 | 39 | 216 | B |
| 42 | 7-Br | 4-CH3C6H4CH2 | 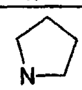 | 53 | 215 | B |
| 43 | 7-Br | 4-ClC6H4CH2 | 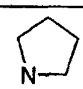 | 12 | 105 | A |
| 44 | 7-Br | 4-FC6H4CH2 | 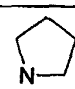 | 42 | 166 | A |
| 45 | 7-Br | 3-CNC6H4CH2 | 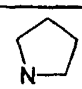 | 52 | 206 | B |

| N° Composé | X1 | R | NR4R5 | Rdt (%) | PF (°C) | Méthode |
|---|---|---|---|---|---|---|
| 46 | 7-Br | 4-CNC6H4CH2 | 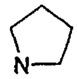 | 19 | 116 | A |
| 47 | 7-Br | 4-(COOCH3)C6H4CH2 | 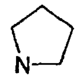 | 54 | 205 | A |
| 48 | 7-Br | 4-NO2C6H4CH2 | 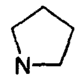 | 52 | 200 | B |
| 49 | 7-Br | 4-(CH3O)C6H4CH2 | 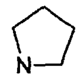 | 39 | 169 | B |
| 50 | 7-Br | 4-(OCOCH3)C6H4CH2 | 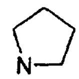 | 21 | 195 | B |
| 51 | 7-Br | 4-OHC6H4CH2 | 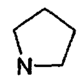 | 13 | 288 | B |
| 52 | 7-Br | 3,4-(CH3O)2C6H3CH2 | 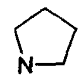 | 15 | 151 | A |
| 53 | 7-Br | 3,4-(OCH2O)C6H3CH2 | 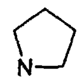 | 21 | 194 | A |
| 54 | 7-Br | 3,5-(CH3O)2C6H3CH2 | 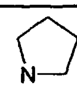 | 31 | - | A |
| 55 | 7-Br | 3,4,5-(CH3O)3C6H2CH2 | 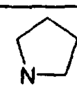 | 35 | 141-143 | A |
| 56 | 7-Br | 4-(CH2COOH)C6H4CH2 | 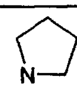 | 17 | 260 | B |
| 57 | 7-Br | (E) C6H5CH=CHCH2 | 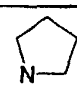 | 57 | 152-155 | A |
| 58 | 7-Br | (Z) C6H5CH=CHCH2 | 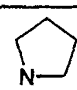 | 24 | 110 | B |
| 59 | 7-Br | (E) (4-ClC6H4)-CH=CHCH2 | 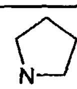 | 45 | 187 | B |
| 60 | 7-Br | (E) (4-CH3O)C6H4CH=CHCH2 | 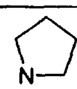 | 32 | 171 | B |
| 61 | 7-Br | (E) (3-pyridyl)-CH=CHCH2 | 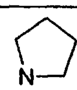 | 10 | 102 | B |
| 62 | 7-Br | (E) (4-pyridyl)-CH=CHCH2 | 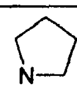 | 38 | 167 | B |
| 63 | 7-Br | 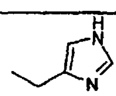 | 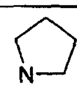 | 4 | 290(dec) | B |

| N° Composé | X1 | R | NR4R5 | Rdt (%) | PF (°C) | Méthode |
|---|---|---|---|---|---|---|
| 64 | 7-Br | 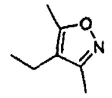 | 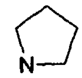 | 60 | 221 | B |
| 65 | 7-Br | 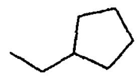 | 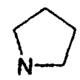 | 32 | 155 | B |
| 66 | 7-Br | n-butyl | 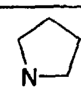 | 39 | 135 | B |
| 67 | 7-Br | CH2CF3 | 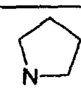 | 14 | 202 | B |
| 68 | 7-Br | CH2CH2OH | 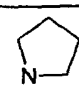 | 25 | 240 | B |
| 69 | 7-Br | CH2CH2N(C2H5)2 | 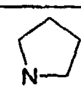 | 50 | 215 (HCl) | C |
| 70 | 7-Br | 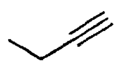 | 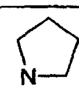 | 36 | 204 | B |
| 71 | 7-Br | CH2CH2OC6H5 | 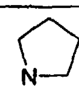 | 25 | 171 | B |
| 72 | 7-Br | CH2CH2SC6H5 | 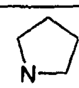 | 20 | 122 | B |
| 73 | 7-Br | CH(C6H5)COOCH3 | 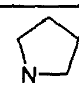 | 14 | 184 | B |
| 74 | 7-Br | 4-CNC6H4CH2 | 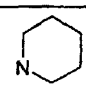 | 72 | 200 | B |
| 75 | 7-Br | 3,4-(CH3O)2C6H3CH2 | 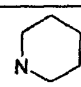 | 67 | 178 | B |
| 76 | 7-Br | (E) C6H5CH=CHCH2 | 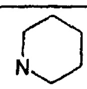 | 8 | - | A |
| 77 | 7-Br | (E) (3-pyridyl)CH=CHCH2 | 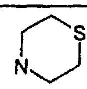 | 48 | 177 | B |
| 78 | 7-Br | 4-CH3C6H4CH2 | 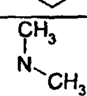 | 56 | 223 | B |
| 79 | 7-Br | 4-CNC6H4CH2 | 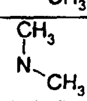 | 56 | 207 | B |
| 80 | 7-Br | 4-OHC6H4CH2 | 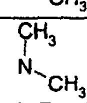 | 15 | 284 | B |
| 81 | 7-Br | 4-(COOCH3)C6H4CH2 | 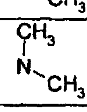 | 35 | 197 | B |

| N° Composé | X1 | R | NR4R5 | Rdt (%) | PF (°C) | Méthode |
|---|---|---|---|---|---|---|
| 82 | 7-Br | 4-(CH2COOH)C6H4CH2 | 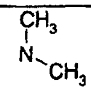 | 8 | 246 | B |
| 83 | 7-Br | 4-(CH2CN)C6H4CH2 | 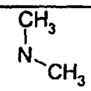 | <1 | 230 | B |
| 84 | 7-Br | (3-pyridyl)-CH2 | 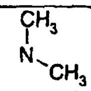 | 28 | 142 | B |
| 85 | 7-Br | (E) C6H5CH=CHCH2 | 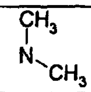 | 63 | 171 | B |
| 86 | 7-Br | (Z) C6H5CH=CHCH2 | 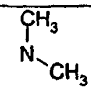 | 28 | 167 | B |
| 87 | 7-Br | (E) (4-pyridyl)-CH=CHCH2 | 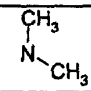 | 48 | 115 | B |
| 88 | 7-Br | 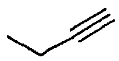 | 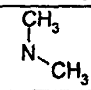 | <1 | 234 | B |
| 89 | 7-Br | C6H5C≡CCH2 | 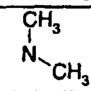 | 15 | 159 | B |
| 90 | 7-Br | CH(C6H5)COOCH3 | 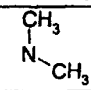 | 18 | 243 | B |
| 91 | 7-CH3 | (3-pyridyl)-CH2 | 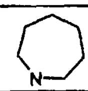 | 64 | 175 | C |
| 92 | 7-CH3 | (E) C6H5CH=CHCH2 | 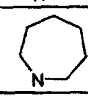 | 16 | 195 | A |
| 93 | 7-CH3 | 4-CNC6H4CH2 | 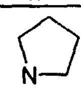 | 84 | 166 | B |
| 94 | 7-CH3 | 3,4-(CH3O)2C6H3CH2 | 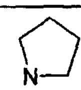 | 52 | 184 | B |
| 95 | 7-CH3 | 4-(COOCH3)C6H4CH2 | 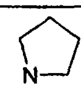 | 44 | 230 | B |
| 96 | 7-CH3 | 4-(CH2COOH)C6H4CH2 | 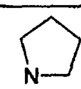 | 21 | 262 | B |
| 97 | 7-CH3 | (3-pyridyl)-CH2 | 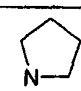 | 10 | 139 | C |
| 98 | 7-CH3 | (E) C6H5CH=CHCH2 | 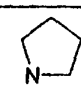 | 17 | 173 | A |
| 99 | 7-CH3 | 4-(CH2COOH)C6H4CH2 | 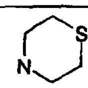 | 10 | - | B |

| N° Composé | X1 | R | NR4R5 | Rdt (%) | PF (°C) | Méthode |
|---|---|---|---|---|---|---|
| 100 | 7-CH3 | (E) (3-pyridyl)CH=CHCH2 | 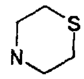 | 51 | 230 | B |
| 101 | 7-CH3 | 4-CNC6H4CH2 | 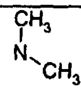 | 73 | 201 | B |
| 102 | 7-CH3 | 4-(CH2COOH)C6H4CH2 | 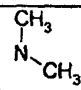 | 3 | - | B |
| 103 | 7-CH3 | (E) C6H5CH=CHCH2 | 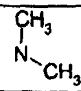 | 50 | 171 | B |
| 104 | 7-CH3 | (E) (3-pyridyl)CH=CHCH2 | 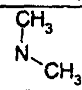 | 53 | 155 | B |
| 105 | 7-CH3 | (E) (4-pyridyl)-CH=CHCH2 | 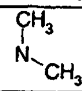 | 66 | 119 | B |
| 106 | 8-CH3 | (E) C6H5CH=CHCH2 | 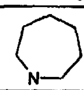 | 52 | - | A |
| 107 | 7-CN | 4-CNC6H4CH2 | 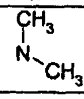 | 43 | 147-149 | B |
| 108 | 7-OH | (E) C6H5CH=CHCH2 | 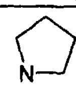 | 3 | 295(dec) | A |

- **<u>Composé 6</u> :**

R.M.N.$^1$H δ (ppm) : 1,7 – 1,85 (m, 8H) ; 3,3 – 3,4 (m, 4H) ; 4,95 (d, 2H) ; 6,4 – 6,5 (dt, 1H) ; 6,7 – 6,75 (d, 1H) ; 7,25 (t, 1H) ; 7,3 (t, 2H) ; 7,45 (d, 2H) ; 7,6 (t, 1H) ; 7,95 (t, 1H) ; 8,25 (d, 1H) ; 8,4 (d, 1H)

Solvant : DMSO

- **<u>Composé 7</u> :**

R.M.N.$^1$H δ (ppm) : 1,5 – 1,9 (m, 8H) ; 3,3 (m, 4H) ; 4,8 (d, 2H) ; 5,2 (d, 1H) ; 5,4 (d, 1H) ; 5,95 (m, 1H) ; 7,65 (d, 1H) ; 8,25 (s, 1H) ; 8,3 (d, 1H)

Solvant : $CDCl_3$

- **<u>Composé 8</u> :**

R.M.N.$^1$H δ (ppm): 1,7 – 2,0 (m, 8H) ; 2,3 (s, 3H) ; 3,35 (m, 4H) ; 5,4 (s, 2H) ; 7,1 (d, 2H) ; 7,6 (d, 2H) ; 7,7 (d, 1H) ; 8,35 (m, 2H)

Solvant : $CDCl_3$

- **Composé 9** :

R.M.N.$^1$H δ (ppm) : 1,75 – 1,95 (m, 8H) ; 3,4 (m, 4H) ; 5,6 (s, 2H) ; 7,05 – 7,25 (m, 3H) ; 7,4 (d, 1H) ; 7,75 (d, 1H) ; 8,35 (s, 1H) ; 8,45 (d, 1H)

Solvant : $CDCl_3$

- **Composé 10** :

R.M.N.$^1$H δ (ppm) : 1,6 – 2,0 (m, 8H) ; 3,35 (m, 4H) ; 5,4 (s, 2H) ; 7,2 (m, 2H) ; 7,55 (s, 1H) ; 7,65 (s, 1H) ; 7,7 (d, 1H) ; 8,35 (m, 2H)

Solvant : $CDCl_3$

- **Composé 11** :

R.M.N.$^1$H δ (ppm) : 1,65 – 1,9 (m, 8H) ; 3,3 (m, 4H) ; 5,35 (s, 2H) ; 7,2 (d, 2H) ; 7,55 (d, 2H) ; 7,65 (d, 1H) ; 8,25 (m, 2H)

Solvant : $CDCl_3$

- **Composé 12** :

R.M.N.$^1$H δ (ppm) : 1,7 – 2 (m, 8H) ; 3,4 (m, 4H) ; 5,4 (s, 2H) ; 7,4 (d, 2H) ; 7,55 (d, 2H) ; 7,7 (d, 1H) ; 8,3 (s, 1H) ; 8,35 (d, 1H)

Solvant : $CDCl_3$

- **Composé 13** :

R.M.N.$^1$H δ (ppm) : 1,75 – 1,95 (m, 8H) ; 3,4 (m, 4H) ; 5,4 (s, 2H) ; 7,0 (m, 2H) ; 7,7 (m, 3H) ; 8,35 (m, 2H)

Solvant : $CDCl_3$

- **Composé 14** :

R.M.N.$^1$H δ (ppm) : 1,7 – 1,95 (m, 8H) ; 3,4 (m, 4H) ; 5,5 (s, 2H) ; 7,55 (d, 2H) ; 7,7 (d, 1H) ; 7,8 (d, 2H) ; 8,3 – 8,45 (m, 2H)

Solvant : $CDCl_3$

- **Composé 15** :

R.M.N.$^1$H δ (ppm) : 1,65 – 2 (m, 8H) ; 3,4 (m, 4H) ; 5,45 (s, 2H) ; 7,55 (d, 2H) ; 7,7 – 7,85 (m, 3H) ; 8,25 – 8,45 (m, 2H)

Solvant : $CDCl_3$

- **Composé 16** :

R.M.N.$^1$H δ (ppm) : 1,75 – 1,95 (m, 8H) ; 3,4 (m, 4H) ; 3,9 (s, 3H) ; 5,5 (s, 2H) ; 6,8 (t, 1H) ; 6,9 (d, 1H) ; 7,1 (d, 1H) ; 7,2 (t, 1H) ; 8,35 (s, 1H) ; 8,4 (d, 1H)

Solvant : $CDCl_3$

- **Composé 17** :

R.M.N.$^1$H δ (ppm) : 1,75 – 1,95 (m, 8H) ; 3,4 (m, 4H) ; 3,8 (s, 3H) ; 5,5 (s, 2H) ; 6,8 (m, 1H) ; 7,25 (m, 3H) ; 7,75 (d, 1H) ; 8,4 (m, 2H)

Solvant : $CDCl_3$

- **Composé 18** :

R.M.N.$^1$H δ (ppm) : 1,75 – 1,95 (m, 8H) ; 3,4 (m, 4H) ; 3,75 (s, 3H) ; 5,4 (s, 2H) ; 6,85 (d, 2H) ; 7,7 (m, 3H) ; 8,35 (m, 2H)

Solvant : $CDCl_3$

- **Composé 19** :

R.M.N.$^1$H δ (ppm) : 1,7 – 2,0 (m, 8H) ; 3,35 (m, 4H) ; 5,4 (s, 2H) ; 7,3 (d, 1H) ; 7,5 (d, 1H) ; 7,75 (m, 2H) ; 8,3 (s, 1H) ; 8,35 (d, 1H)

Solvant : $CDCl_3$

- **Composé 20** :

R.M.N.$^1$H δ (ppm) : 1,75 – 1,95 (m, 8H) ; 3,4 (m, 4H) ; 3,85 (s, 3H) ; 3,90 (s, 3H) ; 5,4 (s, 2H) ; 6,75 (d, 1H) ; 7,35 (m,2H) ; 7,7 (d, 1H) ; 8,35 (m, 2H)

Solvant : $CDCl_3$

- **Composé 21** :

R.M.N.$^1$H δ (ppm) : 1,7 – 1,95 (m, 8H) ; 3,4 (m, 4H) ; 5,6 (s, 2H) ; 7,15 (m, 1H) ; 7,4 (d, 1H) ; 7,6 (m, 1H) ; 7,75 (d, 1H) ; 8,35 (s, 1H) ; 8,4 (d, 1H) ; 8,45 (m, 1H)

Solvant : $CDCl_3$

- **Composé 22** :

R.M.N.$^1$H δ (ppm) : 1,6 – 1,95 (m, 8H) ; 3,35 (m, 4H) ; 5,4 (s, 2H) ; 7,2 (m, 1H) ; 7,7 (d, 1H) ; 8,0 (m, 1H) ; 8,3 (m, 2H) ; 8,5 (m, 1H) ; 8,9 (s, 1H)

Solvant : $CDCl_3$

- **Composé 23** :

R.M.N.$^1$H δ (ppm) : 1,6 – 1,9 (m, 8H) ; 3,3 (m, 4H) ; 5,35 (s, 2H) ; 7,4 (d, 2H) ; 7,65 (d, 1H) ; 8,25 (s, 1H) ; 8,3 (d, 1H) ; 8,45 (d, 2H)

Solvant : $CDCl_3$

- **Composé 24** :

R.M.N.$^1$H δ (ppm) : 1,7 – 2,1 (m, 8H) ; 3,15 (t, 2H) ; 3,4 (m, 4H) ; 4,5 (t, 2H) ; 7,2-7,45 (m, 5H) ; 7,7 (d, 1H) ; 8,35 (s, 1H) ; 8,35 (s, 1H) ; 8,4 (d, 1H)

Solvant : $CDCl_3$

- **Composé 25** :

R.M.N.$^1$H δ (ppm) : 1,7 – 1,95 (m, 8H) ; 3,05 (t, 2H) ; 3,4 (m, 4H) ; 3,8 (s, 3H) ; 4,45 (t, 2H) ; 6,85 (d, 2H) ; 7,25 (d, 2H) ; 7,7 (d, 1H) ; 8,3 (s, 1H) ; 8,4 (d, 1H)

Solvant : $CDCl_3$

- **Composé 26** :

R.M.N.$^1$H δ (ppm) : 1,7 – 2,0 (m, 8H) ; 2,2 (qn, 2H) ; 2,75 (t, 2H) ; 3,35 (m, 4H) ; 4,35 (t, 2H) ; 7,0 – 7,2 (m, 5H) ; 7,7 (d, 1H) ; 8,3 (s, 1H) ; 8,35 (d, 1 H)

Solvant : $CDCl_3$

- **Composé 27** :

R.M.N.1H δ (ppm) : 1.65-1.85(m,8H) ; 3.35(m,4H) ; 5.7(s,2H) ; 7.6(t,2H) ; 7.75(t,1H) ; 8.05(d,1H) ; 8.15(m,3H) ; 8.4(d,1H)

Solvant : DMSO

- **Composé 28** :

R.M.N.$^1$H δ (ppm) : 1,75 – 1,95 (m, 8H) ; 3,4 (m, 4H) ; 3,9 (s, 3H) ; 5,7 (s, 2H) ; 7,0 (d, 2H) ; 7,8 (d, 1H) ; 8,05 (d, 2H) ; 8,35 (s, 1H) ; 8,45 (d, 1 H)

Solvant : $CDCl_3$

- **Composé 29** :

R.M.N.$^1$H δ (ppm) : 1,75 – 1,95 (m, 8H) ; 3,4 (m, 4H) ; 5,7 (s, 2H) ; 7,45 (d, 2H) ; 7,8 (d, 1H) ; 8 (d, 2H) ; 8,3 (s, 1H) ; 8,4 (d, 1H)

Solvant : $CDCl_3$

- **Composé 30** :

R.M.N.$^1$H δ (ppm) : 1,75 – 1,95 (m, 8H) ; 3,4 (m, 4H) ; 3,9 (s, 3H) ; 4 (s, 3H) ; 5,7 (s, 2H) ; 7,1 (d, 1H) ; 7,8 (d, 1H) ; 8,2 (d, 1 H) ; 8, 35 (s, 1H) ; 8,45 (s, 1H) ; 8,5 (s, 1H)

Solvant : $CDCl_3$

- **Composé 31** :

R.M.N.$^1$H δ (ppm) : 2,0 – 2,1 (m, 4H) ; 3,35 - 3,45 (m, 4H) ; 5,45 (s, 2H) ; 7,2 - 7,3 (dd, 1H) ; 7,75 (d, 1H) ; 8,05 (d, 1H) ; 8,25 (d, 1H) ; 8,35 (s, 1 H) ; 8, 55 (d, 1H) ; 8,9 (s, 1H)

Solvant : $CDCl_3$

- **Composé 32** :

R.M.N.$^1$H δ (ppm) : 1,7 – 1,95 (m, 8H) ; 3,4 (m, 4H) ; 5,45 (s, 2H) ; 7,25 (d, 2H) ; 7,75 (d, 2H) ; 7,9 (d, 1H) ; 8,25 (d, 1H) ; 8,45 (s, 1H)

Solvant : $CDCl_3$

- **Composé 33** :

R.M.N.$^1$H δ (ppm) : 1,7 – 2,0 (m, 8H) ; 3,3 – 3,45 (m, 4H) ; 5,4 (s, 2H) ; 6,9 – 7,0 (m, 2H) ; 7,65 - 7,75 (m, 2H) ; 7,9 (d, 1H) ; 8,3 (d, 1H) ; 8,5 (s, 1H)

Solvant : $CDCl_3$

- **Composé 34** :

R.M.N.$^1$H δ (ppm) : 1,8 – 2 (m, 8H) ; 3,35 – 3,5 (m, 4H) ; 5,5 (s, 2H) ; 7,6 (dd, 2H) ; 7,8 (dd,2H) ; 7,9 (m, 1H) ; 8,3 (dd, 1H) ; 8,5 (d, 1H)

Solvant : $CHCl_3$

- **Composé 35** :

R.M.N.$^1$H δ (ppm) : 1,7 – 2 (m, 8H) ; 3,3 – 3,45 (m, 4H) ; 3,8 (s, 3H) ; 3,85 (s, 3H) ; 5,4 (s, 2H) ; 6,8 (d, 1H) ; 7,25 – 7,35 (m, 2H) ; 7,8 (d, 1H) ; 8,3 (d, 1H) ; 8,5 (s, 1H)

Solvant : $CDCl_3$

- **Composé 36** :

R.M.N.$^1$H δ (ppm) : 1,8 – 1,95 (m, 8H) ; 3,4 (m, 4H) ; 5,45 (s, 2H) ; 7,25 (m, 1H) ; 7,9 (d, 1H) ; 8,1 (d, 1H) ; 8,35 (d, 1H) ; 8,5 (m, 2H) ; 8,95 (s, 1H)

Solvant : $CDCl_3$

- **Composé 37** :

R.M.N.$^1$H δ (ppm) : 1,7 – 1,95 (m, 8H) ; 3,4 (m, 4H) ; 5,05 (d, 2H) ; 6,45 (dt, 1H) ; 6,9 (d, 1H) ; 7,15 – 7,3 (m, 3H) ; 7,35 (d, 2H) ; 7,9 (d, 1H) ; 8,3 (d, 1H) ; 8,5 (s, 1H)

Solvant : $CDCl_3$

- **Composé 38** :

R.M.N.$^1$H δ (ppm) : 1,7 – 2 (m, 8H) ; 3,3 – 3,5 (m, 4H) ; 5,05 (d, 2H) ; 6,35 – 6,45 (m, 1H) ; 6,75 – 6,85 (d, 1H) ; 7,2 – 7,35 (m, 4H) ; 7,85 (m, 1H) ; 8,3 (m, 1H) ; 8,5 (s, 1H)

Solvant : $CDCl_3$

- **Composé 39** :

R.M.N.$^1$H δ (ppm) : 1,7 – 1,95 (m, 8H) ; 3,3 – 3,45 (m, 4H) ; 3,75 (s, 3H) ; 5,05 (m, 2H) ; 6,25 – 6,35 (m, 1H) ; 6,8 (m, 3H) ; 7,3 (m, 2H) ; 7,85 (m, 1H) ; 8,3 (m, 1H) ; 8,5 (s, 1H)

Solvant : $CDCl_3$

- **Composé 40** :

R.M.N.$^1$H δ (ppm) : 1,7 – 2 (m, 8H) ; 3,3 – 3,5 (m, 4H) ; 5,05 (d, 2H) ; 6,45 – 6,55 (m, 1H) ; 6,85 (d, 1H) ; 7,2 (m, 1H) ; 7,65 (m, 1H) ; 7,9 (m, 1H) ; 8,35 (d, 1H) ; 8,45 (m, 1H) ; 8,5 (d, 1H) ; 8,6 (d, 1H)

Solvant : $CDCl_3$

- **Composé 41** :

R.M.N.$^1$H δ (ppm) : 1,7 – 2 (m, 8H) ; 3,3 – 3,5 (m, 4H) ; 5,05 (d, 2H) ; 6,55 – 6,7 (m, 1H) ; 6,8 (d, 1H) ; 7,2 (d, 2H) ; 7,9 (m, 1H) ; 8,3 (d, 1H) ; 8,5 (m, 3H)

Solvant : $CDCl_3$

- **Composé 42** :

R.M.N.$^1$H δ (ppm) : 2 - 2,1 (m, 4H) ; 2,3 (s, 3H) ; 3,3 – 3,45 (m, 4H) ; 5,4 (s, 2H) ; 7,1 (d, 2H) ; 7,6 (d, 2H) ; 7,8 (d, 1H) ; 8,1 (d, 1H) ; 8,5 (s, 1H)

Solvant : $CDCl_3$

- **Composé 43** :

R.M.N.$^1$H δ (ppm) : 1,9 – 2,05 (m, 4H) ; 3,25 – 3,4 (m, 4H) ; 5,35 (s, 2H) ; 7,2 (d, 2H) ; 7,6 (d, 2H) ; 7,8 (d, 1H) ; 8,1 (d, 1H) ; 8,4 (s, 1H)

Solvant : $CDCl_3$

- **Composé 44** :

R.M.N.$^1$H δ (ppm) : 2,0 – 2,1 (m, 4H) ; 3,35 – 3,45 (m, 4H) ; 5,4 (s, 2H) ; 6,9 – 7,0 (m, 2H) ; 7,6 – 7,7 (m, 2H) ; 7,85 (d, 1H) ; 8,1 (d, 1H) ; 8,5 (s, 1H)

Solvant : $CDCl_3$

- **Composé 45** :

R.M.N.$^1$H δ (ppm) : 2 – 2,15 (m, 4H) ; 3,35 – 3,5 (m, 4H) ; 5,45 (s, 2H) ; 7,45 (t, 1H) ; 7,55 (d, 1H) ; 7,85 – 8,0 (m, 3H) ; 8,2 (d, 1H) ; 8,5 (s, 1H)

Solvant : $CDCl_3$

- **Composé 46** :

R.M.N.$^1$H δ (ppm) : 1,95 – 2,1 (m, 4H) ; 3,35 – 3,5 (m, 4H) ; 5,45 (s, 2H) ; 7,6 (d, 2H) ; 7,8 (d, 2H) ; 7,9 (d, 1H) ; 8,15 (d, 1H) ; 8,5 (s, 1H)

Solvant : $CDCl_3$

- **Composé 47** :

R.M.N.$^1$H δ (ppm) : 2 – 2,1 (m, 4H) ; 3,35 – 3,45 (m, 4H) ; 3,9 (s, 3H) ; 5,5 (s, 2H) ; 7,7 (d, 2H) ; 7,9 (d, 1H) ; 8,0 (d, 2H) ; 8,15 (d, 1H) ; 8,5 (s, 1H)

Solvant : $CDCl_3$

- **Composé 48** :

R.M.N.$^1$H δ (ppm) : 2 – 2,15 (m, 4H) ; 3,3 – 3,45 (m, 4H) ; 5,5 (s, 2H) ; 7,75 - 7,9 (m, 3H) ; 8,1 – 8,2 (m, 3H) ; 8,5 (s, 1H)

Solvant : $CDCl_3$

- **Composé 49** :

R.M.N.$^1$H δ (ppm) : 2 – 2,15 (m, 4H) ; 3,35 – 3,5 (m, 4H) ; 3,75 (s, 3H) ; 5,4 (s, 2H) ; 6,8 (d, 2H) ; 7,65 (d, 2H) ; 7,8 (d, 1H) ; 8,15 (d, 1H) ; 8,5 (s, 1H)

Solvant : $CDCl_3$

- **Composé 50** :

R.M.N.$^1$H δ (ppm) : 2 – 2,15 (m, 4H) ; 2,25 (s, 3H) ; 3,35 – 3,45 (m, 4H) ; 5,45 (s, 2H) ; 7,0 (d, 2H) ; 7,75 (d, 2H) ; 7,85 (d, 1H) ; 8,15 (d, 1H) ; 8,5 (s, 1H)

Solvant : $CDCl_3$

- **Composé 51** :

R.M.N.$^1$H δ (ppm) : 1,9 – 2,1 (m, 4H) ; 3,2 – 3,45 (m, 4H) ; 5,2 (s, 2H) ; 6,7 (d, 2H) ; 7,35 (d, 2H) ; 8 (d, 1H) ; 8,2 (d, 1H) ; 8,3 (s, 1H) ; 9,25 (s, 1H)

Solvant : $CDCl_3$

- **Composé 52** :

R.M.N.$^1$H δ (ppm) : 2,0 – 2,1 (m, 4H) ; 3,35 – 3,45 (m, 4H) ; 3,85 (s, 3H) ; 3,9 (s, 3H) ; 5,4 (s, 2H) ; 6,8 (d, 1H) ; 7,2 – 7,35 (m, 2H) ; 7,8 (d, 1H) ; 8,15 (d, 1H) ; 8,5 (s, 1H)

Solvant : $CDCl_3$

- **Composé 53** :

R.M.N.$^1$H δ (ppm) : 2,0 – 2,1 (m, 4H) ; 3,3 – 3,4 (m, 4H) ; 5,35 (s, 2H) ; 5,9 (s, 2H) ; 6,7 (d, 1H) ; 7,15 – 7,3 (m, 2H) ; 7,85 (d, 1H) ; 8,1 (d, 1H) ; 8,5 (s, 1H)

Solvant : $CDCl_3$

**Composé 54 :**

R.M.N.$^1$H δ (ppm) : 2 – 2,1 (m, 4H) ; 3,35 – 3,4 (m, 4H) ; 3,75 (s, 6H) ; 5,4 (s, 2H) ; 6,35 (s, 1H) ; 6,8 (s, 2H) ; 7,85 (d, 1H) ; 8,2 (d, 1H) ; 8,5 (s, 1H)

Solvant : $CDCl_3$

- **Composé 55 :**

R.M.N.$^1$H δ (ppm) : 2 – 2,1 (m, 4H) ; 3,35 – 3,45 (m, 4H) ; 3,8 (s, 3H) ; 3,85 (s, 6H) ; 5,4 (s, 2H) ; 7 (s, 2H) ; 7,85 (d, 1H) ; 8,2 (d, 1H) ; 8,5 (s, 1H)

Solvant : $CDCl_3$

- **Composé 56 :**

R.M.N.$^1$H δ (ppm) : 1,95 - 2,1 (m, 4H) ; 3,25 – 3,45 (m, 4H) ; 3,55 (s, 2H) ; 5,4 (s, 2H) ; 7,25 (d, 2H) ; 7,35 (d, 2H) ; 8,15 (d, 1H) ; 8,2 (d, 1H) ; 8,35 (s, 1H) ; 12,2 – 12,5 (m, 1H)

Solvant : $CDCl_3$

- **Composé 57 :**

R.M.N.$^1$H δ (ppm) : 2,1 (m, 4H) ; 3,4 (m, 4H) ; 5,05 (d, 2H) ; 6,4 (dt, 1H) ; 6,9 (d, 1H) ; 7,15 – 7,3 (m, 3H) ; 7,35 (d, 2H) ; 7,9 (d, 1H) ; 8,15 (d, 1H) ; 8,5 (s, 1H)

Solvant : $CDCl_3$

- **Composé 58 :**

R.M.N.$^1$H δ (ppm) : 2,0 – 2,15 (m, 4H) ; 3,35 – 3,5 (m, 4H) ; 5,2 (d, 2H) ; 5,7 – 5,8 (m, 1H) ; 6,7 (d, 1H) ; 7,2 – 7,45 (m, 5H) ; 7,9 (d, 1H) ; 8,2 (d, 1H) ; 8,6 (s, 1H)

Solvant : $CDCl_3$

- **Composé 59 :**

R.M.N.$^1$H δ (ppm) : 2,05 (m, 4H) ; 3,4 (m, 4H) ; 5 (d, 2H) ; 6,4 (m, 1H) ; 6,85 (d, 1H) ; 7,15 – 7,3 (m, 4H) ; 7,85 (m, 1H) ; 8,15 (d, 1H) ; 8,45 (s, 1H)

Solvant : $CDCl_3$

- **Composé 60** :

R.M.N.$^1$H δ (ppm) : 1,95 – 2,10 (m, 4H) ; 3,4 (m, 4H) ; 3,75 (s, 3H) ; 4,95 (m, 2H) ; 6,25 – 6,35 (m, 1H) ; 6,75 – 6,9 (m, 3H) ; 7,2 – 7,3 (m, 2H) ; 7,85 (m, 1H) ; 8,15 (m, 1H) ; 8,45 (m, 1H)

Solvant : $CDCl_3$

- **Composé 61** :

R.M.N.$^1$H δ (ppm) : 1,95 – 2,15 (m, 4H) ; 3,3 – 3,5 (m, 4H) ; 5,05 (m, 2H) ; 6,45 – 6,55 (m, 1H) ; 6,75 – 6,9 (d, 1H) ; 7,2 (m, 1H) ; 7,6 – 7,7 (m, 1H) ; 7,85 – 7,95 (m, 1H) ; 8,15 (m, 1H) ; 8,4 (m, 1H) ; 8,5 (m, 1H) ; 8,6 (m, 1H)

Solvant : $CDCl_3$

- **Composé 62** :

R.M.N.$^1$H δ (ppm) : 1,9 – 2,05 (m, 4H) ; 3,3 – 3,45 (m, 4H) ; 5,05 (d, 2H) ; 6,55 – 6,7 (m, 1H) ; 6,8 (d, 1H) ; 7,25 (m, 2H) ; 7,9 (m, 1H) ; 8,2 (m, 1H) ; 8,45 - 8,55 (m, 3H)

Solvant : $CDCl_3$

- **Composé 63** :

R.M.N.$^1$H δ (ppm) : 1,8 – 1,9 (m, 2H) ; 3,25 (m, 2H) ; 5,1 (s, 2H) ; 6,9 (s, 1H) ; 7,4 (s, 1H) ; 8 (d, 1H) ; 8,1 (d, 1H) ; 8,2 (s, 1H) ; 11, 8 (m, 1H)

Solvant : DMSO

- **Composé 64** :

R.M.N.$^1$H δ (ppm) : 2,05 – 2,15 (m, 4H) ; 2,4 (s, 3H) ; 2,6 (s, 3H) ; 3,4 (m, 4H) ; 5,2 (s, 2H) ; 7,9 (d, 1H) ; 8,2 (d, 1H) ; 8,4 (s, 1H)

Solvant : $CDCl_3$

- **Composé 65** :

R.M.N.$^1$H δ (ppm) : 1,25 – 1,75 (m, 8H) ; 1,9 – 2,05 (m, 4H) ; 2,5 – 2,7 (m, 1H) ; 3,3 – 3,4 (m, 4H) ; 4,2 (d, 2H) ; 7,8 (d, 1H) ; 8,1 (d, 1H) ; 8,45 (s, 1H)

Solvant : $CDCl_3$

- **Composé 66 :**

R.M.N.$^1$H δ (ppm) : 1 (t, 3H) ; 1,4 – 1,55 (m, 2H) ; 1,8 – 1,9 (m, 2H) ; 2,0 – 2,1 (m, 4H) ; 3,4 – 3,5 (m, 4H) ; 4,3 (t, 2H) ; 7,9 (d, 1H) ; 8,2 (d, 1H) ; 8,5 (s, 1H)

Solvant : $CDCl_3$

- **Composé 67 :**

R.M.N.$^1$H δ (ppm) : 2 – 2,15 (m, 4H) ; 3,35 – 3,5 (m, 4H) ; 5,0 (q, 2H) ; 7,9 (d, 1H) ; 8,2 (d, 1H) ; 8,55 (s, 1H)

Solvant : $CDCl_3$

- **Composé 68 :**

R.M.N.$^1$H δ (ppm) : 2 (m, 4H) ; 3,15 (m, 1H) ; 3,3 (m, 4H) ; 4,05 (m, 2H) ; 4,5 (m, 2H) ; 7,08 (m, 1H) ; 8,15 (m, 1H) ; 8,4 (s, 1H)

Solvant : $CDCl_3$

- **Composé 69 :**

R.M.N.$^1$H δ (ppm) : 1,1 (t, 6H) ; 2,0 – 2,1 (m, 4H) ; 2,65 (q, 4H) ; 2,9 (t, 2H) ; 3,35 – 3,45 (m, 4H) ; 4,4 (t, 2H) ; 7,9 (d, 1H) ; 8,2 (d, 1H) ; 8,5 (s, 1H)

Solvant : $CDCl_3$

- **Composé 70 :**

R.M.N.$^1$H δ (ppm) : 2 – 2,15 (m, 4H) ; 2,3 (s, 1H) ; 3,35 – 3,5 (m, 4H) ; 5,1 (s, 2H) ; 7,9 (d, 1H) ; 8,2 (d, 1H) ; 8,5 (s, 1H)

Solvant : $CDCl_3$

- **Composé 71 :**

R.M.N.$^1$H δ (ppm) : 2,1 (m, 4H) ; 3,4 (m, 4H) ; 4,45 (m, 2H) ; 4,75 (m, 2H) ; 6,9 (m, 3H) ; 7,2 – 7,3 (m, 2H) ; 7,9 (m, 1H) ; 8, 2 (m, 1H) ; 8,5 (s, 1H)

Solvant : $CDCl_3$

- **Composé 72 :**

R.M.N.$^1$H δ (ppm) : 2,1 (m, 4H) ; 3,45 (m, 6H) ; 4,6 (m, 2H) ; 7,1 (m, 1H) ; 7,2 (m, 2H) ; 7,4 (m, 2H) ; 7,85 (m, 1H) ; 8,1 (m, 1H) ; 8,45 (s, 1H)

Solvant : $CDCl_3$

- **Composé 73 :**

R.M.N.$^1$H δ (ppm) : 1,9 - 2,05 (m, 4H) ; 3,3 - 3,4 (m, 4H) ; 3,08 (s, 3H) ; 6,7 (s, 1H) ; 7,2 – 7,35 (m, 3H) ; 7,7 – 7,85 (m, 3H) ; 8,1 (d, 1H) ; 8, 4 (s, 1H)

Solvant : $CDCl_3$

- **Composé 74 :**

R.M.N.$^1$H δ (ppm) : 1,4 – 1,6 (m, 1H) ; 1,7 - 2 (m, 5H) ; 3 – 3,1 (m, 2H) ; 3,3 – 3,4 (m, 2H) ; 5,5 (s, 2H) ; 7,6 (d, 2H) ; 7,8 (d, 2H) ; 7,9 (d, 1H) ; 8,3 (d, 1H) ; 8,5 (s, 1H)

Solvant : $CDCl_3$

- **Composé 75 :**

R.M.N.$^1$H δ (ppm) : 1,4 (m, 1H) ; 1,7 – 1,95 (m, 5H) ; 3 – 3,1 (m, 2H) ; 3,3 – 3,4 (m, 2H) ; 3,8 (s, 3H) ; 3,9 (s, 3H) ; 5,4 (s, 2H) ; 6, 8 (d, 1H) ; 7,25 – 7,35 (m, 2H) ; 7,9 (d, 1H) ; 8,3 (d, 1H) ; 8,40 (s, 1H)

Solvant : $CDCl_3$

- **Composé 76 :**

R.M.N.$^1$H δ (ppm) : 1,35 – 2,1 (m, 6H) ; 3,05 (t, 2H) ; 3,35 (m, 2H) ; 5,1 (d, 2H) ; 6,5 (dt, 1H) ; 6,9 (d, 1H) ; 7,1 – 7,5 (m, 5H) ; 7,9 (d, 1H) ; 8,3 (d, 1H) ; 8,55 (s, 1H)

Solvant : $CDCl_3$

- **Composé 77 :**

R.M.N.$^1$H δ (ppm) : 2,9 (m, 4H) ; 3,45 (m, 2H) ; 3,6 (m, 2H) ; 5,1 (m, 2H) ; 6,5 (m, 1H) ; 6,85 (d, 1H) ; 7,2 (m, 1H) ; 7,65 (m, 1H) ; 7,9 (m, 1H) ; 8,25 (m, 1H) ; 8,45 ( m, 1H) ; 8,5 (m, 1H) ; 8,55 (s, 1H)

Solvant : $CDCl_3$

- **Composé 78 :**

R.M.N.$^1$H δ (ppm) : 2,3 (s, 3H) ; 2,9 (s, 6H) ; 5,4 (s, 2H) ; 7,1 (d, 2H) ; 7,6 (d, 2H) ; 7,85 (d, 1H) ; 8,2 (d, 1H) ; 8,5 (s, 1H)

Solvant : $CDCl_3$

- **Composé 79 :**

R.M.N.$^{1}$H δ (ppm) : 2,95 (s, 6H) ; 5,45 (s, 2H) ; 7,55 (d, 2H) ; 7,75 (d, 2H) ; 7,9 (d, 1H) ; 8,2 (d, 1H) ; 8,5 (s, 1H)

Solvant : $CDCl_3$

- **Composé 80 :**

R.M.N.$^{1}$H δ (ppm) : 2,85 (s, 6H) ; 5,2 (s, 2H) ; 6,7 (d, 2H) ; 7,3 (d, 2H) ; 8 (d, 1H) ; 8,2 – 8,3 (m, 2H) ; 9,3 (s, 1H)

Solvant : $CDCl_3$

- **Composé 81 :**

R.M.N.$^{1}$H δ (ppm) : 2,9 (s, 6H) ; 3,9 (s, 3H) ; 5,45 (s, 2H) ; 7,7 (m, 2H) ; 7,85 (m, 1H) ; 7,9 (m, 2H) ; 8,2 (d, 1H) ; 8,5 (s, 1H)

Solvant : $CDCl_3$

- **Composé 82 :**

R.M.N.$^{1}$H δ (ppm) : 2,85 (s, 6H) ; 3,6 (s, 2H) ; 5,35 (s, 2H) ; 7,25 (d, 2H) ; 7,5 (d, 2H) ; 8,15 (d, 1H) ; 8,3 (d, 1H) ; 8, 35 (s, 1H) ; 12,2 – 12,45 (m, 1H)

Solvant : DMSO

- **Composé 83 :**

R.M.N.$^{1}$H δ (ppm) : 2,9 (s, 6H) ; 3,7 (s, 2H) ; 5,45 (s, 2H) ; 7,25 (m, 2H) ; 7,7 (m, 2H) ; 7,85 (m, 1H) ; 8,2 (d, 1H) ; 8,5 (s, 1H)

Solvant : $CDCl_3$

- **Composé 84 :**

R.M.N.$^{1}$H δ (ppm) : 2,9 (s, 6H) ; 5,5 (s, 2H) ; 7,25 (m, 1H) ; 7,85 (m, 1H) ; 8,05 (m, 1H) ; 8,25 (d, 1H) ; 8,5 (m, 2H) ; 8,9 (s, 1H)

Solvant : $CDCl_3$

- **Composé 85 :**

R.M.N.$^{1}$H δ (ppm) : 2,9 (s, 6H) ; 5,05 (d, 2H) ; 6,4 – 6,55 (dt, 1H) ; 6,9 (d, 1H) ; 7,2 – 7,4 (m, 5H) ; 7,9 (d, 1H) ; 8,25 (d, 1H) ; 8,55 (s, 1H)

Solvant : $CDCl_3$

- **Composé 86 :**

R.M.N.$^1$H δ (ppm) : 2,95 (s, 6H) ; 5,29 (d, 2H) ; 5,7 – 5,8 (m, 1H) ; 6,7 (d, 1H) ; 7,2 – 7,45 (m, 5H) ; 7,9 (d, 1H) ; 8,25 (d, 1H) ; 8,5 (s, 1H)

Solvant : $CDCl_3$

- **Composé 87 :**

R.M.N.$^1$H δ (ppm) : 2,9 (s, 6H) ; 5,05 (d, 2H) ; 6,55 – 6,7 (m, 1H) ; 6,85 (d, 1H) ; 7,2 (m, 2H) ; 7,85 (m, 1H) ; 8,25 (d, 1H) ; 8,5 (m, 3H)

Solvant : $CDCl_3$

- **Composé 88 :**

R.M.N.$^1$H δ (ppm) : 2,8 (s, 6H) ; 3,2 (s, 1H) ; 4,9 (s, 2H) ; 8,1 (m, 1H) ; 8,2 (d, 1H) ; 8,3 (s, 1H)

Solvant : DMSO

- **Composé 89 :**

R.M.N.$^1$H δ (ppm) : 2,9 (s, 6H) ; 5,2 (s, 2H) ; 7,2 (m, 3H) ; 7,4 (m, 2H) ; 7,85 (m, 1H) ; 8,2 (d, 1H) ; 8,55 (s, 1H)

Solvant : $CDCl_3$

- **Composé 90 :**

R.M.N.$^1$H δ (ppm) : 2,95 (s, 6H) ; 3,85 (s, 3H) ; 6,8 (s, 1H) ; 7,3 – 7,4 (m, 3H) ; 7,75 – 7,9 (m, 3H) ; 8,2 (d, 1H) ; 8,5 (s, 1H)

Solvant : $CDCl_3$

- **Composé 91 :**

R.M.N.$^1$H δ (ppm) : 1,75 – 1,9 (m, 8H) ; 2,5 (s, 3H) ; 3,4 – 3,5 (m, 4H) ; 5,5 (s, 2H) ; 7,2 – 7,3 (dd, 1H) ; 7,6 – 7,65 (d, 1H) ; 8,05 – 8,01 (d, 1H) ; 8,2 (s, 1H) ; 8,3 – 8,35 (d, 1H) ; 8,55 (d, 1H) ; 8, 95 (s, 1H)

Solvant : $CDCl_3$

- **Composé 92**:

R.M.N.$^1$H δ (ppm) : 1,75 – 2 (m, 8H) ; 2,5 (s, 3H) ; 3,4 – 3,5 (m, 4H) ; 5,1 (d, 1H) ; 5,4 – 5,55 (dt, 1H) ; 6,9 – 7 (d, 1H) ; 7,2 – 7,3 (m, 4H) ; 7,4 (d, 2H) ; 7,6 (d, 1H) ; 8,2 (s, 1H) ; 8,3 (d, 1H)

Solvant : $CDCl_3$

- **Composé 93** :

R.M.N.$^1$H δ (ppm) : 2 – 2,1 (m, 4H) ; 2,5 (s, 3H) ; 3,3 – 3,4 (m, 4H) ; 5,5 (s, 2H) ; 7,6 (m, 3H) ; 7,8 (d, 2H) ; 8,1 - 8,2 (m, 2H)

Solvant : $CDCl_3$

- **Composé 94** :

R.M.N.$^1$H δ (ppm) : 2 – 2,1 (m, 4H) ; 2,5 (s, 3H) ; 3,4 – 3,5 (m, 4H) ; 3,8 (s, 3H) ; 3,9 (s, 3H) ; 5,4 (s, 2H) ; 6,8 (d, 1H) ; 7,3 - 7,4 (m, 2H) ; 7,5 (d, 1H) ; 8,1 – 8,2 (m, 2H)

Solvant : $CDCl_3$

- **Composé 95** :

R.M.N.$^1$H δ (ppm) : 2,1 – 2,2 (m, 4H) ; 2,5 (s, 3H) ; 3,4 – 3,5 (m, 4H) ; 3,9 (s, 3H) ; 5,5 (s, 2H) ; 7,6 (m, 1H) ; 7,7 (m, 2H) ; 7,95 - 8 (m, 2H) ; 8,1 – 8,2 (m, 2H)

Solvant : $CDCl_3$

- **Composé 96** :

R.M.N.$^1$H δ (ppm) : 2 (m, 4H) ; 2,5 (s, 3H) ; 3,3 – 3,4 (m, 4H) ; 3,6 (s, 2H) ; 5,3 (s, 2H) ; 7,3 (m, 2H) ; 7,45 (m, 2H) ; 7,8 (m, 2H) ; 8,1 (s, 1H) ; 8,2 (d, 2H) ; 12,4 (m, 1H)

Solvant : DMSO

- **Composé 97** :

R.M.N.$^1$H δ (ppm) : 1,95 (m, 4H) ; 2,5 (s, 3H) ; 3,35 (m, 4H) ; 5,4 (s, 2H) ; 7,35 (dd, 1H) ; 7,55 (d, 1H) ; 8,05 (s, 1H) ; 8,15 (d, 1H) ; 8,5 (d, 1H) ; 8,7 (s, 1H)

Solvant : DMSO

- **Composé 98** :

R.M.N.$^1$H δ (ppm) : 2 – 2,1 (m, 4H) ; 2,45 (s, 3H) ; 3,3 – 3,45 (m, 4H) ; 5,05 (d, 2H) ; 6,4 – 6,5 (dt, 1H) ; 6,85 – 6,95 (d, 1H) ; 7,1 – 7,45 (m, 5H) ; 7,6 (d, 1H) ; 8,1 – 8,2 (m, 2H)

Solvant : $CDCl_3$

- **Composé 99** :

R.M.N.$^1$H δ (ppm) : 2,4 – 3,75 (m, 13H) ; 5,35 (s, 2H) ; 7,1 – 7,5 (m, 4H) ; 7,8 (d, 1H) ; 8,1 (s, 1H) ; 8,25 (d, 1H)

Solvant : DMSO

- **Composé 100** :

R.M.N.$^1$H δ (ppm) : 2,5 (s, 3H) ; 2,9 (m, 4H) ; 3,45 (m, 2H) ; 3,65 (m, 2H) ; 5,1 (m, 2H) ; 6,5 (m, 1H) ; 6,85 (d, 1H) ; 7,2 (m, 1H) ; 7,6 (m, 1H) ; 7,7 (m, 1H) ; 8,2 (m, 2H) ; 8,45 (d, 1H) ; 8,6 (1s, 1H)

Solvant : $CDCl_3$

- **Composé 101** :

R.M.N.$^1$H δ (ppm) : 2,5 (s, 3H) ; 2,95 (s, 6H) ; 5,5 (s, 2H) ; 7,6 (m, 3H) ; 7,8 (m, 2H) ; 8,15 –8,25 (m, 2H)

Solvant : $CDCl_3$

- **Composé 102** :

R.M.N.$^1$H δ (ppm) : 2,2 (s, 3H) ; 2,6 (s, 6H) ; 3,25 (s, 2H) ; 5,1 (s, 2H) ; 7 (m, 2H) ; 7,15 (m, 2H) ; 7,5 (m, 1H) ; 7,8 (s, 1H) ; 8 (d, 1H) ; 12 (m, 1H)

Solvant : DMSO

- **Composé 103** :

R.M.N.$^1$H δ (ppm) : 2,5 (s, 3H) ; 3 (s, 6H) ; 5,1 (d, 2H) ; 6,4 – 6,5 (dt, 1H) ; 6,9 (d, 1H) ; 7,15 – 7,4 (m, 6H) ; 7,6 (d, 1H) ; 8,2 (m, 1H)

Solvant : $CDCl_3$

- **<u>Composé 104</u> :**

R.M.N.$^1$H δ (ppm) : 2,5 (s, 3H) ; 2,95 (s, 6H) ; 5,1 (d, 2H) ; 6,45 – 6,55 (dt, 1H) ; 6,8 – 6,85 (d, 1H) ; 7,2 (m, 1H) ; 7,6 (dd, 1H) ; 7,7 (dd, 1H) ; 8,2 – 8,25 (m, 2H) ; 8,4 (d, 1H) ; 8,6 (s, 1H)

Solvant : $CDCl_3$

- **<u>Composé 105</u> :**

R.M.N.$^1$H δ (ppm) : 2,5 (s, 3H) ; 2,95 (s, 6H) ; 5,1 (d, 2H) ; 6,6 – 6,7 (dt, 1H) ; 6,8 (d, 1H) ; 7,2 (d, 2H) ; 7,6 (d, 1H) ; 8,2 – 8,25 (dd, 2H) ; 8,5 (d, 2H)

Solvant : $CDCl_3$

- **<u>Composé 106</u> :**

R.M.N.$^1$H δ (ppm) : 1,7 - 2 (m, 8H) ; 2,55 (m, 3H) ; 3,35 – 3,6 (m, 4H) ; 5,1 (d, 2H) ; 6,45 (dt, 1H) ; 6,85 (d, 1H) ; 7,1 – 7,45 (m, 6H) ; 8,25 (m, 2H)

Solvant : $CDCl_3$

- **<u>Composé 107</u> :** R.M.N.$^1$H δ (ppm) : 2,9 (d, 6H) ; 5,5 (s, 2H) ; 7,6 (m, 2H) ; 7,7 (m, 2H) ; 8,0 (m, 1H) ; 8,4 (m, 1H) ; 8,7 (s, 1H)

Solvant : $CDCl_3$

- **<u>Composé 108</u> :**

R.M.N.$^1$H δ (ppm) : 1,9 (m, 4H) ; 3,25 (m, 4H) ; 6,85 (d, 2H) ; 6,3 – 6,4 (dt, 1H) ; 6,6 – 6,7 (d, 1H) ; 7,15 – 7,3 (m, 4H) ; 7,35 – 7,4 (d, 2H) ; 7,5 (s, 1H) ; 8,05 (d, 1H) ; 10,1 (m, 1H),

Solvant : DMSO

**TABLEAU 2**

| N° Composé | X1 | R | NR4R5 | Rdt (%) | PF (°C) | Méthode |
|---|---|---|---|---|---|---|
| 109 | H | (E) C6H5CH=CHCH2 | 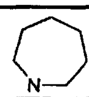 | 28 | 176 | A |
| 110 | 7-Cl | CH2=CHCH2 | 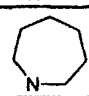 | 24 | 173 | A |
| 111 | 7-Cl | C6H5CH2 | 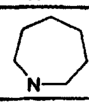 | 58 | 148 | A |

| N° Composé | X1 | R | NR4R5 | Rdt (%) | PF (°C) | Méthode |
|---|---|---|---|---|---|---|
| 112 | 7-Cl | 4-CH3C6H4CH2 | 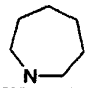 | 50 | 182 | A |
| 113 | 7-Cl | 2-ClC6H4CH2 | 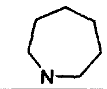 | 77 | 228 | A |
| 114 | 7-Cl | 3-ClC6H4CH2 | 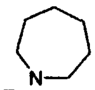 | 31 | 166 | A |
| 115 | 7-Cl | 4-ClC6H4CH2 | 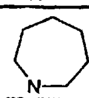 | 60 | 245 | A |
| 116 | 7-Cl | 4-BrC6H4CH2 | 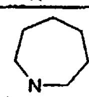 | 38 | 244 | A |
| 117 | 7-Cl | 4-FC6H4CH2 | 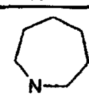 | 42,5 | 224 | A |
| 118 | 7-Cl | 4-CF3C6H4CH2 | 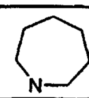 | 39 | 232 | A |
| 119 | 7-Cl | 4-CNC6H4CH2 | 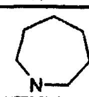 | 46 | > 260 | A |
| 120 | 7-Cl | 2-(OCH3)C6H4CH2 | 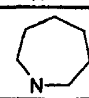 | 57 | 184 | A |
| 121 | 7-Cl | 3-(OCH3)C6H4CH2 | 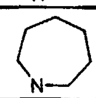 | 46 | 163 | A |
| 122 | 7-Cl | 4-(OCH3)C6H4CH2 | 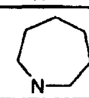 | 32,5 | 164-165 | A |
| 123 | 7-Cl | 3,4-Cl2C6H3CH2 | 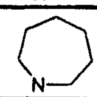 | 60 | 212 | A |
| 124 | 7-Cl | 3,4-(OCH3)2C6H3CH2 | 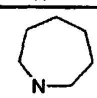 | 39 | 153 | A |
| 125 | 7-Cl | (2-pyridyl)CH2 | 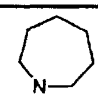 | 9 | 153 | A |
| 126 | 7-Cl | (3-pyridyl)CH2 | 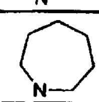 | 8 | 184 | C |
| 127 | 7-Cl | C6H5CH2CH2 | 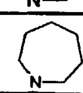 | 7 | 196 | A |
| 128 | 7-Cl | 4(CH3O)C6H4(CH2)2 | 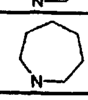 | 61 | 196 | A |

| N° Composé | X1 | R | NR4R5 | Rdt (%) | PF (°C) | Méthode |
|---|---|---|---|---|---|---|
| 129 | 7-Cl | C6H5(CH2)3 | 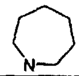 | 36 | 130 | A |
| 130 | 7-Cl | C6H5C(=O)CH2 | 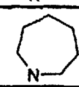 | 38,5 | 230-232 | A |
| 131 | 7-Cl | 4(CH3O)C6H4C(=O)CH2 | 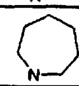 | 42 | 238 | A |
| 132 | 7-Cl | 4-ClC6H4C(=O)CH2 | 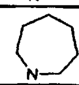 | 59 | 238 | A |
| 133 | 7-Cl | 4(CH3O)-3-(COOCH3)-C6H3C(=O)CH2 | 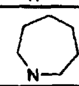 | 30 | 136 | A |
| 134 | 7-Br | 4-ClC6H4CH2 | 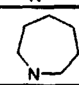 | 57 | 247 | A |
| 135 | 7-Br | 4-FC6H4CH2 | 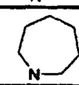 | 54 | 216 | A |
| 136 | 7-Br | 4-CNC6H4CH2 | 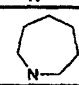 | 53 | 293 | A |
| 137 | 7-Br | 3,4-(CH3O)2C6H3CH2 | 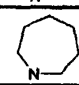 | 61 | 174 | A |
| 138 | 7-Br | 4-(CH2COOH)C6H4CH2 | 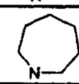 | 1 | 269 | B |
| 139 | 7-Br | (3-pyridyl)CH2 | 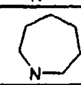 | 4 | 192 | C |
| 140 | 7-Br | (E) C6H5CH=CHCH2 | 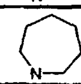 | 70 | 198 | A |
| 141 | 7-Br | (Z) C6H5CH=CHCH2 | 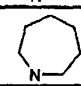 | 57 | 187 | A |
| 142 | 7-Br | 4-ClC6H4CH2 | 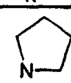 | 18 | 185 | A |
| 143 | 7-Br | 4-FC6H4CH2 | 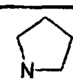 | 16 | 233 | A |
| 144 | 7-Br | 4-CNC6H4CH2 | 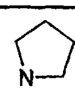 | 52 | 222 | A |
| 145 | 7-Br | 4-(COOCH3)C6H4CH2 | 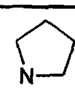 | 31 | 193 | A |
| 146 | 7-Br | 4-(CH3O)C6H4CH2 | 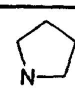 | 14 | 164 | B |
| 147 | 7-Br | 4-(OCOCH3)C6H4CH2 | 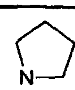 | 24 | 199 | B |

| N° Composé | X1 | R | NR4R5 | Rdt (%) | PF (°C) | Méthode |
|---|---|---|---|---|---|---|
| 148 | 7-Br | 4-OHC6H4CH2 | 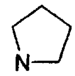 | 15 | 283 | B |
| 149 | 7-Br | 3,4-(OCH2O)C6H4CH2 | 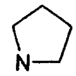 | 57 | 234 | A |
| 150 | 7-Br | 3,5-(CH3O)2C6H4CH2 | 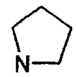 | 21 | 168 | A |
| 151 | 7-Br | 3,4,5-(CH3O)3C6H2CH2 | 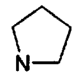 | 21 | 199-201 | A |
| 152 | 7-Br | 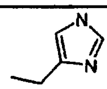 | 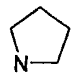 | 4 | - | B |
| 153 | 7-Br | n-butyl | 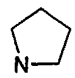 | 13 | 130 | B |
| 154 | 7-Br | CH(C6H5)COOCH3 | 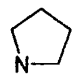 | 55 | 187 | A |
| 155 | 7-Br | (E) C6H5CH=CHCH2 | 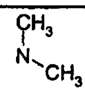 | 10 | 206 | B |
| 156 | 7-Br | CH(C6H5)COOCH3 | 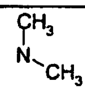 | 32 | 83 | B |
| 157 | 7-CH3 | (E) C6H5CH=CHCH2 | 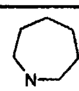 | 43 | 193 | A |
| 158 | 7-CH3 | (E) C6H5CH=CHCH2 | 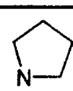 | 35 | 225 | A |
| 159 | 8-CH3 | CH3 | 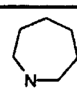 | 70 | - | A |
| 160 | 8-CH3 | (E) C6H5CH=CHCH2 | 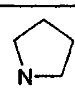 | 18 | - | A |
| 161 | 7-OH | (E) C6H5CH=CHCH2 | 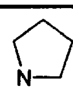 | 10 | 255 | A |
| 162 | 7- 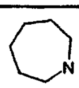 | (E) C6H5CH=CHCH2 | 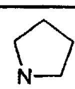 | 28 | - | A |

**Composé 109** :

R.M.N.$^1$H δ (ppm) : 1,7 – 1,85 (m, 8H) ; 3,3 – 3,45 (m, 4H) ; 5,85 (d, 2H) ; 6,35 – 6,45 (dt, 1H) ; 6,65 – 6,75 (d, 1H) ; 7,25 (t, 1H) ; 7,35 (t, 1H) ; 7,45 (d, 1H) ; 7,6 (t, 1H) ; 7,85 (t, 1H) ; 8,2 (d, 1H) ; 8,3 (d, 1H)

Solvant : $CDCl_3$

- **Composé 110** :

R.M.N.$^1$H δ (ppm) : 1,65 – 1,95 (m, 8H) ; 3,35 (m, 4H) ; 4,8 (d, 2H) ; 5,25 – 5,4 (m, 2H) ; 5,9 – 6,1 (m, 1H) ; 7,55 – 8,4 (m, 3H)

Solvant : $CDCl_3$

- **Composé 111** :

R.M.N.$^1$H δ (ppm): 1,7 – 1,9 (m, 8H) ; 3,35 (m, 4H) ; 5,25 (s, 2H) ; 7,2 – 7,4 (m, 3H) ; 7,45 (d, 2H) ; 7,6 (d, 1H) ; 8,15 (d, 1H) ; 8,4 (s, 1H)

Solvant : $CDCl_3$

- **Composé 112** :

R.M.N.$^1$H δ (ppm) : 1,7 – 1,9 (m, 8H) ; 2,3 (s, 3H) ; 3,35 (m, 4H) ; 5,25 (s, 2H) ; 7,1 – 8,45 (m, 7H)

Solvant : $CDCl_3$

- **Composé 113** :

R.M.N.$^1$H δ (ppm) : 1,7 – 1,95 (m, 8H) ; 3,4 (m, 4H) ; 5,45 (s, 2H) ; 7,15 – 7,3 (m, 3H) ; 7,4 (d, 1H) ; 7,65 (d, 1H) ; 8,2 (d, 1H) ; 8,45 (s, 1H)

Solvant : $CDCl_3$

- **Composé 114** :

R.M.N.$^1$H δ (ppm) : 1,7 – 1,9 (m, 8H) ; 3,4 (m, 4H) ; 5,25 (s, 2H) ; 7,2 – 7,4 (m, 3H) ; 7,45 (s, 1H) ; 7,6 (d, 1H) ; 8,15 (d, 1H) ; 8,45 (s, 1H)

Solvant : $CDCl_3$

- **Composé 115** :

R.M.N.$^1$H δ (ppm) : 1,65 – 1,85 (m, 8H) ; 3,3 (m, 4H) ; 5,15 (s, 2H) ; 7,25 (d, 2H) ; 7,35 (d, 2H) ; 7,55 (d, 1H) ; 8,05 (d, 1H) ; 8,3 (s, 1H)

Solvant : $CDCl_3$

- **Composé 116** :

R.M.N.$^1$H δ (ppm) : 1,7 – 1,95 (m, 8H) ; 3,35 (m, 4H) ; 5,25 (d, 2H) ; 7,35 (d, 2H) ; 7,45 (d, 2H) ; 7,6 (d, 1H) ; 8,1 (d, 1H) ; 8,4 (s, 1H)

Solvant : $CDCl_3$

- **Composé 117** :

R.M.N.$^1$H δ (ppm) : 1,7 – 1,9 (m, 8H) ; 3,35 (m, 4H) ; 5,35 (s, 2H) ; 7,5 – 7,7 (m, 5H) ; 8,15 (d, 1H) ; 8,4 (s, 1H)

Solvant : $CDCl_3$

- **Composé 118** :

R.M.N.$^1$H δ (ppm) : 1,7 – 1,9 (m, 8H) ; 3,4 (m, 4H) ; 5,35 (s, 2H) ; 7,5 – 7,7 (m, 5H) ; 8,1 (d, 1H) ; 8,4 (s, 1H)

Solvant : $CDCl_3$

- **Composé 119** :

R.M.N.$^1$H δ (ppm) : 1,7 – 1,9 (m, 8H) ; 3,4 (m, 4H) ; 3,9 (s, 3H) ; 5,35 (s, 2H) ; 6,9 (m, 2H) ; 7,2 (d, 1H) ; 7,3 (t, 1H) ; 7,6 (d, 1H) ; 8,2 (d, 1H) ; 8,4 (s, 1H)

Solvant : $CDCl_3$

- **Composé 120** :

R.M.N.$^1$H δ (ppm) : 1,7 – 2,0 (m, 8H) ; 3,35 (m, 4H) ; 3,75 (s, 3H) ; 5,4 (s, 2H) ; 6,8 (m, 1H) ; 7,15 – 7,3 (m, 3H) ; 7,7 (d, 1H) ; 8,35 (m, 2H)

Solvant : $CDCl_3$

- **Composé 121** :

R.M.N.$^1$H δ (ppm) : 1,7 – 1,9 (m, 8H) ; 3,35 (m, 4H) ; 3,8 (s, 3H) ; 5,2 (s, 2H) ; 6,85 (d, 2H) ; 7,45 (d, 2H) ; 7,65 (d, 1H) ; 8,15 (d, 1H) ; 8,45 (s, 1H)

Solvant : $CDCl_3$

- **Composé 122** :

1,7 – 1,9 (m, 8H) ; 3,4 (m, 4H) ; 5,2 (s, 2H) ; 7,3 (d, 1H) ; 7,4 (d, 1H) ; 7,5 (s, 1H) ; 7,6 (d, 1H) ; 8,15 (d, 1H) ; 8,4 (s, 1H)

Solvant : $CDCl_3$

- **Composé 123** :

R.M.N.$^1$H δ (ppm) : 1,7 – 1,9 (m, 8H) ; 3,4 (m, 4H) ; 3,85 (s, 3H) ; 3,90 (s, 3H) ; 5,2 (s, 2H) ; 6,85 (d, 1H) ; 7,1 (m, 2H) ; 7,65 (d, 1H) 8,2 (d, 1H) ; 8,45 (s, 1H)

Solvant : $CDCl_3$

- **Composé 124** :

R.M.N.$^1$H δ (ppm) : 1,65 – 195 (m, 8H) ; 3,4 (m, 4H) ; 5,45 (s, 2H) ; 7,2 (m, 1H) ; 7,3 (d, 1H) ; 7,65 (m, 2H) ; 8,2 (d, 1H) ; 8,4 (s, 1H) ; 8,55 (d, 1H)

Solvant : $CDCl_3$

- **Composé 125** :

R.M.N.$^1$H δ (ppm) : 1,7 – 1,95 (m, 8H) ; 3,4 (m, 4H) ; 5,3 (s, 2H) ; 7,25 (m, 1H) ; 7,6 (d, 1H) ; 7,85 (d, 1H) ; 8,15 (d, 1H) ; 8,45 (s, 1H) ; 8,6 (d, 1H) ; 8,75 (s, 1H)

Solvant : $CDCl_3$

- **Composé 126** :

R.M.N.$^1$H δ (ppm) : 1,55 – 1,9 (m, 8H) ; 3,1 (t, 2H) ; 3,25 (m, 4H) ; 4,25 (t, 2H) ; 7,05 – 7,25 (m,5H) ; 7,55 (d, 1H) ; 8,1 (d, 1H) ; 8,35 (s, 1H)

Solvant : $CDCl_3$

- **Composé 127** :

R.M.N.$^1$H δ (ppm) : 1,75 – 1,9 (m, 8H) ; 3,15 (t, 2H) ; 3,35 (m, 4H) ; 3,75 (s, 3H) ; 4,35 (t, 2H) ; 6,8 (d, 2H) ; 7,15 (d, 2H) ; 7,6 (d, 1H) ; 8,15 (d, 1H) ; 8,4 (s, 1H)

Solvant : $CDCl_3$

- **Composé 128** :

R.M.N.$^1$H δ (ppm) : 1,7 – 1,95 (m, 8H) ; 2,2 (m, 2H) ; 2,7 (t, 2H) ; 3,35 (m, 4H) ; 4,2 (t, 2H) ;
7 – 7,3 (m, 5H) ; 7,65 (d, 1H) ; 8,1 (d, 1H) ; 8,45 (s, 1H)
Solvant : $CDCl_3$

- **Composé 129** :

R.M.N.$^1$H δ (ppm) : 1,7 – 1,9 (m, 8H) ; 3,4 (m, 4H) ; 3,9 (s, 3H) ; 5,6 (s, 2H) ; 7,0 (d, 2H) ; 7,7 (d, 1H) ; 8 (d, 2H) ; 8,25 (d, 1H) ; 8,45 (s, 1H)
Solvant : $CDCl_3$

- **Composé 130** :

R.M.N.1H δ (ppm) : 1.6-1.9(m,8H) ; 3.4(m,4H) ; 5.8(s,2H) ; 7.6(t,2H) ; 7.75(t,1H) ; 7.95(d,1H) ; 8.1(m,3H) ; 8.3(d,1H)
Solvant : DMSO

- **Composé 131** :

R.M.N.$^1$H δ (ppm) : 1,7 – 1,95 (m, 8H) ; 3,4 (m, 4H) ; 5,55 (s, 2H) ; 7,45 (d, 2H) ; 7,65 (d, 1H) ; 7,9 (d, 2H) ; 8,2 (d, 1H) ; 8,4 (s, 1 H)
Solvant : $CDCl_3$

- **Composé 132** :

R.M.N.$^1$H δ (ppm) : 1,7 – 1,9 (m, 8H) ; 3,4 (m, 4H) ; 3,9 (s, 3H) ; 4,0 (s, 3H) ; 5,6 (s, 2H) ; 7,1 (d, 1H) ; 7,7 (d, 1H) ; 8,1 (d, 1H) ; 8,2 (d, 1 H) ; 8,4 (m, 2H)
Solvant : $CDCl_3$

- **Composé 133** :

R.M.N.$^1$H δ (ppm) : 1,75 – 1,9 (m, 8H) ; 3,4 (m, 4H) ; 3,9 (s, 3H) ; 4,0 (s, 3H) ; 5,6 (s, 2H) ; 7,1 (m, 1H) ; 7,7 (m, 1H) ; 8,15 –8,45 (m, 4H)
Solvant : $CDCl_3$

- **Composé 134**:

R.M.N.$^1$H δ (ppm) : 1,75 – 1,9 (m, 8H) ; 3,35 (m, 4H) ; 5,25 (s, 2H) ; 7,25 - 8,6 (m, 7 H)

Solvant : $CDCl_3$

- **<u>Composé 135</u> :**

R.M.N.$^1$H δ (ppm) : 1,65 – 1,95 (m, 8H) ; 3,3 – 3,45 (m, 4H) ; 5,25 (s, 2H) ; 6,95 – 7,1 (m, 2H) ; 7,4 – 7,55 (m, 2H) ; 7,8 (d, 1H) ; 8,1 (d, 1H) ; 8,6 (s, 1H)

Solvant : $CDCl_3$

- **<u>Composé 136</u> :**

R.M.N.$^1$H δ (ppm) : 1,75 – 1,95 (m, 8H) ; 3,3 – 3,45 (m, 4H) ; 5,3 (s, 2H) ; 7,5 – 7,7 (m, 4H) ; 7,8 (m, 1H) ; 8,1 (dd, 1H) ; 8,6 (s, 1H)

Solvant : $CDCl_3$

- **<u>Composé 137</u> :**

R.M.N.$^1$H δ (ppm) : 1,7 – 1,9 (m, 8H) ; 3,25 – 3,4 (m, 4H) ; 3,8 (s, 3H) ; 3,82 (s, 3H) ; 5,2 (s, 2H) ; 6,8 (d, 1H) ; 7,05 – 7,1 (m, 2H) ; 7,75 (d, 1H) ; 8,05 (d, 1H) ; 8,6 (s, 1H)

Solvant : $CDCl_3$

- **<u>Composé 138</u> :**

R.M.N.$^1$H δ (ppm) : 1,6 – 1,85 (m, 8H) ; 3,2 - 3,4 (bs, 4H) ; 3,55 (s, 2H) ; 5,2 (s, 2H) ; 7,2 (m, 2H) ; 7,3 (m, 2H) ; 8 (m, 1H) ; 8,2 (m, 1H) ; 8,25 (s, 1H) ; 12,3 (bs, 1H)

Solvant : DMSO

- **<u>Composé 139</u> :**

R.M.N.$^1$H δ (ppm) : 1,75 – 1,9 (m, 8H) ; 3,4 (m, 4H) ; 5,35 (s, 2H) ; 7,3 (m, 1H) ; 7,8 (d, 1H) ; 7,9 (d, 1H) ; 8,1 (d, 1H) ; 8,65 (m, 2H) ; 8,8 (s, 1H)

Solvant : $CDCl_3$

- **<u>Composé 140</u> :**

R.M.N.$^1$H δ (ppm) : 1,8 – 1,95 (m, 8H) ; 3,4 (m, 4H) ; 4,9 (d, 2H) ; 6,35 (m, 1H) ; 6,75 (d, 1H) ; 7,25 – 7,45 (m, 5H) ; 7,8 – 8,65 (m, 3H)

Solvant : $CDCl_3$

- **Composé 141** :

R.M.N.$^1$H δ (ppm) : 1,35 – 2,05 (m, 6H) ; 2,95 (t, 2H) ; 3,4 (d, 2H) ; 4,9 (d, 2H) ; 6,35 (dt, 1H) ; 6,75 (d, 1H) ; 7,25 – 7,45 (m, 5H) ; 7,85 (d, 1H) ; 8,15 (d, 1H) ; 8,65 (s, 1H)

Solvant : $CDCl_3$

- **Composé 142** :

R.M.N.$^1$H δ (ppm) : 2 – 2,1 (m, 4H) ; 3,3 - 3,4 (m, 4H) ; 5,25 (s, 2H) ; 7,25 (d, 2H) ; 7,4 (d, 2H) ; 7,75 (d, 1H) ; 7,95 (d, 1H) ; 8,55 (s, 1H)

Solvant : $CDCl_3$

- **Composé 143** :

R.M.N.$^1$H δ (ppm) : 1,95 – 2,1 (m, 4H) ; 3,3 - 3,45 (m, 4H) ; 5,2 (s, 2H) ; 6,95 – 7,1 (m, 2H) ; 7,35 – 7,5 (m, 2H) ; 7,75 (d, 1H) ; 7,95 (d, 1H) ; 8,6 (s, 1H)

Solvant : $CDCl_3$

- **Composé 144** :

R.M.N.$^1$H δ (ppm) : 2 – 2,15 (m, 4H) ; 3,3 - 3,45 (m, 4H) ; 5,3 (s, 2H) ; 7,55 – 7,7 (m, 4H) ; 7,8 – 7,9 (d, 1H) ; 8,0 (d, 1H) ; 8,6 (s, 1H)

Solvant : $CDCl_3$

- **Composé 145** :

R.M.N.$^1$H δ (ppm) : 2 – 2,1 (m, 4H) ; 3,3 - 3,4 (m, 4H) ; 3,9 (s, 3H) ; 5,3 (s, 2H) ; 7,5 (d, 2H) ; 7,8 (d, 1H) ; 7,9 – 8,05 (m, 3H) ; 8,6 (s, 1H)

Solvant : $CDCl_3$

- **Composé 146** :

R.M.N.$^1$H δ (ppm) : 2 – 2,1 (m, 4H) ; 3,3 - 3,4 (m, 4H) ; 3,8 (s, 3H) ; 5,2 (s, 2H) ; 6,9 (d, 2 H) ; 7,45 (d, 2H) ; 7,8 (d, 1H) ; 7,95 (d, 1H) ; 8,6 (s, 1H)

Solvant : $CDCl_3$

- **Composé 147** :

R.M.N.$^1$H δ (ppm) : 2 – 2,1 (m, 4H) ; 2,3 (s, 3H) ; 3,3 – 3,4 (m, 4H) ; 5,25 (s, 2H) ; 7,05 (d, 2H) ; 7,5 (d, 2H) ; 7,8 (d, 1H) ; 8,0 (d, 1H) ; 8,6 (s, 1H)

Solvant : $CDCl_3$

- **Composé 148** :

R.M.N.$^1$H δ (ppm) : 2,7 (s, 6H) ; 5 (s, 2H) ; 6,6 (d, 2H) ; 7,1 (d, 2H) ; 7,9 (d, 1H) ; 8,0 (d, 1H) ; 8,1 (s, 1H) ; 9,35 (s, 1H)

Solvant : $CDCl_3$

- **Composé 149** :

R.M.N.$^1$H δ (ppm) : 2 – 2,15 (m, 4H) ; 3,3 - 3,45 (m, 4H) ; 5,15 (s, 2H) ; 5,9 (s, 2H) ; 6,75 (d, 1H) ; 6,9 – 7,0 (m, 2H) ; 7,8 (d, 1H) ; 7,9 (d, 1H) ; 8,6 (s, 1H)

Solvant : $CDCl_3$

- **Composé 150** :

R.M.N.$^1$H δ (ppm) : 2,0 – 2,1 (m, 4H) ; 3,3 - 3,4 (m, 4H) ; 3,75 (s, 6H) ; 5,2 (s, 2H) ; 6,4 (s, 1H) ; 6,65 (s, 2H) ; 7,8 (d, 1H) ; 7,95 (d, 1H) ; 8,65 (s, 1H)

Solvant : $CDCl_3$

- **Composé 151** :

R.M.N.$^1$H δ (ppm) : 2,0 – 2,1 (m, 4H) ; 3,3 - 3,4 (m, 4H) ; 3,85 (s, 3H) ; 3,9 (s, 6H) ; 5,2 (s, 2H) ; 6,8 (s, 2H) ; 7,8 (d, 1H) ; 7,95 (d, 1H) ; 8,65 (s, 1H)

Solvant : $CDCl_3$

- **Composé 152** :

R.M.N.$^1$H δ (ppm) : 2 (m, 4H) ; 3,35 (m, 4H) ; 5,2 (s, 2H) ; 7,15 (s, 1H) ; 7,6 (s, 1H) ; 8 – 8,15 (m, 2H) ; 8,3 (s, 1H) ; 12 (m, 1H)

Solvant : DMSO

- **Composé 153** :

R.M.N.$^1$H δ (ppm) : 0,95 (t, 3H) ; 1,35 – 1,5 (m, 2H) ; 1,8 – 1,9 (m, 2H) ; 2,0 – 2,1 (m, 4H) ; 3,4 - 3,5 (m, 4H) ; 4,1 (t, 2H) ; 7,8 (d, 1H) ; 8,0 (d, 1H) ; 8,6 (s, 1H)

Solvant : $CDCl_3$

- **Composé 154** :

R.M.N.$^1$H δ (ppm) : 2,45 – 2,55 (m, 4H) ; 3,25 - 3,4 (m, 4H) ; 3,7 (s, 3H) ; 6,6 (s, 1H) ; 7,35 – 7,50 (m, 3H) ; 7,55 (d, 2H) ; 8 – 8,1 (m, 2H) ; 8,3 (s, 1H)

Solvant : DMSO

- **Composé 155** :

R.M.N.$^1$H δ (ppm) : 2,9 (s, 6H) ; 4,8 (d, 2H) ; 6,2 – 6,3 (dt, 1H) ; 6,7 (d, 1H) ; 7,1 – 7,35 (m, 5H) ; 7,75 (d, 1H) ; 8,0 (d, 1H) ; 8,6 (s, 1H)

Solvant : $CDCl_3$

- **Composé 156** :

R.M.N.$^1$H δ (ppm) : 2,9 (s, 6H) ; 3,8 (s, 3H) ; 6,6 (s, 1H) ; 7,35 – 7,45 (m, 3H) ; 7,55 (d, 2H) ; 7,8 (d, 1H) ; 8 (d, 1H) : 8,6 (s, 1H)

Solvant : $CDCl_3$

- **Composé 157** :

R.M.N.$^1$H δ (ppm) : 1,8 – 1,95 (m, 8H) ; 2,5 (s, 3H) ; 3,4 – 3,5 (m, 4H) ; 4,9 (d, 2H) ; 6,3 – 6,45 (dt, 1H) ; 6,7 – 6,8 (d, 1H) ; 7,2 – 7,3 (m, 3H) ; 7,35 (d, 2H) ; 7,55 (d, 2H) ; 8,1 (d, 1H) ; 8,3 (s, 1H)

Solvant : $CDCl_3$

- **Composé 158** :

R.M.N.$^1$H δ (ppm) : 2,1 (m, 4H) ; 2,5 (s, 3H) ; 3,4 (m, 4H) ; 4,9 (d, 2H) ; 6,3 – 6,45 (dt, 1H) ; 6,7 – 6,8 (d, 1H) ; 7,2 – 7,4 (m, 5H) ; 7,5 (m, 1H) ; 8 (d, 1H) ; 8,3 (d, 1H)

Solvant : $CDCl_3$

- **Composé 159** :

R.M.N.$^1$H δ (ppm) : 1,7 – 1,9 (m, 8H) ; 2,45 (s, 3H) ; 3,25 – 3,35 (m, 4H) ; 3,65 (s, 3H) ; 7,2 – 7,3 (m, 2H) ; 8 (m, 1H) ; 8,2 - 8,3 (m, 1H)

Solvant : $CDCl_3$

- **Composé 160** :

R.M.N.$^1$H δ (ppm) : 1,8 – 2 (m, 8H) ; 2,55 (s, 3H) ; 3,3 - 3,5 (m, 4H) ; 4,9 (m, 2H) ; 6,3 – 6,4 (m, 1H) ; 6,7 – 6,8 (d, 1H) ; 7,2 – 7,4 (m, 6H) ; 8,1 (s, 1H) ; 8,35 (m, 1H)

Solvant : $CDCl_3$

- **Composé 161** :

R.M.N.$^1$H δ (ppm) : 2 (m, 4H) ; 3,4 (m, 4H) ; 4,8 (d, 2H) ; 6,35 – 6,4 (dt, 1H) ; 6,7 (d, 1H) ; 7,2 – 7,4 (m, 4H) ; 7,45 (d, 2H) ; 7,55 (s, 1H) ; 8 (d, 1H) ; 10 (m, 1H)

Solvant : $CDCl_3$

- **Composé 162** :

R.M.N.$^1$H δ (ppm) : 1,5 – 2,1 (m, 16H) ; 3,3 - 3,7 (m, 8H) ; 4,9 (d, 2H) ; 6,3 – 6,4 (dt, 1H) ; 6,7 – 6,8 (d, 1H) ; 6,8– 6,9 (d, 1H) ; 7,2 – 7,5 (m, 6H) ; 8,25 (d, 1H)

Solvant : $CDCl_3$

**Exemple 163**

**METHODE A** : 1-Azepanyl-4-benzyl-7-bromo-4H-[1,2,4] triazolo [4,3-a] quinazolin-5-one.

(I) : X1 = 7-Br ; X2 = H ;

R = 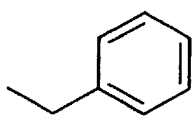 ; NR4R5 = 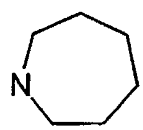

Dans un ballon de 50 ml protégé de l'humidité, on place 4,0 g (10,7 mmol) de 4-benzyl-1,7-dibromo-4H-[1,2,4] triazolo [4,3-a]quinazolin-5-one (préparé par la méthode de l'exemple 256) en suspension dans 25 ml d'hexaméthylène imine.

Le mélange est ensuite chauffé à reflux, sous agitation, pendant 16 heures.

Après refroidissement, la solution obtenue est concentrée sous vide pour donner 4,8 g de résidu qui est purifié par chromatographie flash sur colonne de silice, avec élution au mélange $CH_2$ $Cl_2$ 99,6 / $CH_3$ OH 0,4.

Les fractions pures en CCM sont réunies, évaporées à sec et le produit obtenu (4,0 g) est recristallisé dans l'éthanol.

On obtient 3,2 g du composé de l'exemple 163 sous forme de cristaux.

Rendement = 66 %.

F (Tottoli) = 175° C

CCM ($CH_2$ $Cl_2$ 99 / $CH_3$ OH 1) : Rf = 0,40

RMN $^1$H δ (ppm) CDCl3 :

1,7 - 1,85 (m, 8H) ; 3,3 (m, 4H) ; 5,3 (s, 2H) ; 7,2 - 7,35 (m, 3H) ; 7,45 -d, 2H) ; 8,0 (d, 1H) ; 8,15 (s, 1H); 8,4 (d, 1H)

**Exemple 164**

**METHODE B**: 1-(Pyrrolidin-1-yl)-4-benzyl-7-bromo-4H-[1,2,4] triazolo [4,3-a] quinazolin-5-one.

(I) : X1 = 7-Br ; X2 = H ;

R = 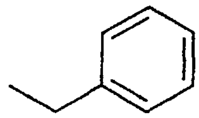 ; NR4R5 = 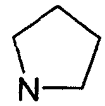

Dans un réacteur protégé de l'humidité, on place 37,0 g (85 mmol) de 4-benzyl-1,7-dibromo-4H-[1,2,4] triazolo [4,3-a]quinazolin-5-one en solution dans 750 ml de diméthylformamide (DMF) et additionne 14,3 g (340 mmol) de bicarbonate de sodium puis 12,1 g (340 mmol) de pyrrolidine.
Le mélange est ensuite chauffé à reflux, sous agitation, pendant 6 heures.
Après refroidissement, le solvant est évaporé sous vide, le résidu obtenu est repris par un mélange eau / acétate d'éthyle et l'insoluble y est trituré puis filtré et séché : on obtient ainsi 18,3g d'un premier jet du composé de l'exemple 164, pur en CCM.
Les phases aqueuse et organique sont séparées, la phase acétate d'éthyle est lavée à l'eau et séchée sur Na2S04. Après concentration du solvant sous vide, on obtient 14,2g d'un deuxième jet du composé de l'exemple 164, également pur en CCM.
Rendement (en produit brut) = 90% ; le produit sera utilisé tel quel pour l'étape suivante.
Un échantillon de 0.35g est recristallisé dans le méthanol pour donner 0.32g du composé pur sous forme de cristaux.
F (Tottoli) = 173°C
CCM (CH2Cl2 99 / CH3OH 1) = 0,35
R.M.N.$^1$H δ (ppm) : 2,1 (m, 4H) ; 3,4 (m, 4H) ; 5,45 (s, 2H) ; 7,3 (m, 3H) ; 7,65 (d, 2H) ; 7,85 (d, 1H); 8,15 (d, 1H); 8,45 (s, 1H)
Solvant : $CDCl_3$

**Exemple 165**

**METHODE C :** 1-[N-(n-butyl), N-methylamino]-4-benzyl-7-bromo-4H-[1,2,4] triazolo [4,3-a] quinazolin-5-one.

(I) : X1 =7-Br;X2=H

R = 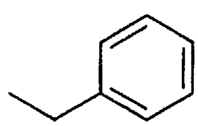 ; NR4R5 = 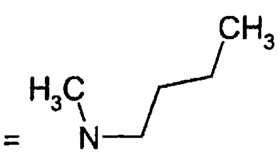

Dans un réacteur à pression, on place 2,5 g (5,75 mmol) de 4-benzyl-1,7-dibromo-4H-[1,2,4] triazolo [4,3-a] quinazolin-5-one en suspension dans 30 ml d'éthanol. On additionne 5,0g de n-butyl-méthylamine (57,5 mmol), ferme hermétiquement le réacteur puis chauffe au bain d'huile à 160°C pendant 8 heures. Après refroidissement et abandon pendant 2 jours, l'huile résiduelle (2,8g) est chromatographiée sur colonne de silice avec élution au mélange CH2C12 99,5 - CH3OH 0,5. On obtient 1,8 g du composé de l'exemple 165.
Rendement = 70 %.
CCM ($CH_2$ $Cl_2$ 98,5 / $CH_3$ OH 1,5) : Rf= 0,45
R.M.N.$^1$H δ (ppm) : 0,9 (t, 3H) ; 1,25 - 1,4 (m, 2H) ; 1,55 - 1,7 (m, 2H) ; 2,85 (s, 3H) ; 2;9 - 3,5 (m, 2H) ; 5,5 (s, 2H) ; 7,2 - 7,35 (m, 3H) ; 7,7 (d, 2H) ; 7,9 (d, 1H) ; 8,25 (d, 1H) ; 8,5 (s, 1H)
Solvant : $CDCl_3$
Les composés (I) des exemples 166 à 198 (tableau 3) sont préparés selon l'une des méthodes A, B ou C décrites dans les exemples 163 à 165.

## TABLEAU 3

| N° Composé | X1 | R | NR4R5 | Rdt (%) | PF (°C) | Méthode |
|---|---|---|---|---|---|---|
| 166 | H | C6H5CH2 | 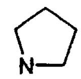 | 70 | 167 | B |
| 167 | 7-Cl | CH3 | 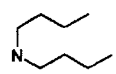 | 17 | 112 | A |
| 168 | 7-Cl | CH3 | 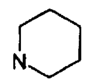 | 35 | 192 | A |
| 169 | 7-Cl | CH3 | 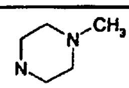 | 50 | 180-182 | A |

| N° Composé | X1 | R | NR4R5 | Rdt (%) | PF (°C) | Méthode |
|---|---|---|---|---|---|---|
| 170 | 7-Cl | CH3 | 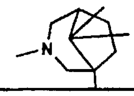 | 60 | 185 | A |
| 171 | 7-Cl | C6H5 | 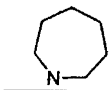 | 5 | 179 | A |
| 172 | 7-Cl | C6H5CH2 | 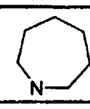 | 88 | 162 | A |
| 173 | 7-Cl | C6H5CH2 | 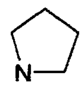 | 78 | 163 | B |
| 174 | 7-Cl | C6H5CH2 | 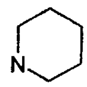 | 68 | 178 | B |
| 175 | 8-Cl | CH3 | 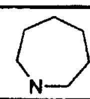 | 11 | 179 | A |
| 176 | 8-Cl | C6H5CH2 | 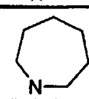 | 1 | - | B |
| 177 | 7-Br | CH3 | 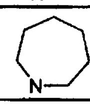 | 72 | 174 | A |
| 178 | 7-Br | C6H5CH2 | 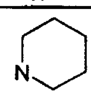 | 67 | 183-185 | A |
| 179 | 7-Br | C6H5CH2 | 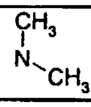 | 53 | 171 | B |
| 180 | 7-Br | C6H5CH2 | 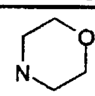 | 50 | 189 | B |
| 181 | 7-Br | C6H5CH2 | 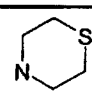 | 49 | 235 | B |
| 182 | 7-Br | C6H5CH2 | 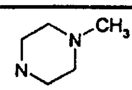 | 60 | 230 | B |
| 183 | 7-Br | C6H5CH2 | 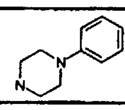 | 51 | 238 | B |
| 184 | 7-Br | C6H5CH2 | 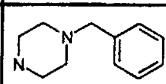 | 50 | 226 | B |
| 185 | 7-Br | C6H5CH2 | 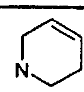 | 82 | 172 | B |
| 186 | 7-Br | C6H5CH2 | 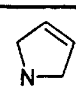 | 85 | 210 | B |

| N° Composé | X1 | R | NR4R5 | Rdt (%) | PF (°C) | Méthode |
|---|---|---|---|---|---|---|
| 187 | 7-Br | C6H5CH2 | 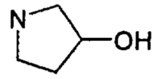 | 79 | 176 | B |
| 188 | 7-Br | C6H5CH2 | NHCH3 | 52 | 238 | C |
| 189 | 7-I | C6H5CH2 | 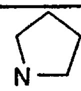 | 100 | 184 | B |
| 190 | 7-CH3 | C6H5CH2 | 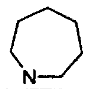 | 90 | 183 | B |
| 191 | 7-CH3 | C6H5CH2 | 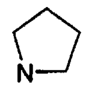 | 60 | 189 | B |
| 192 | 7-CH3 | C6H5CH2 | 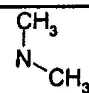 | 75 | 186 | B |
| 193 | 7-CH3 | C6H5CH2 | 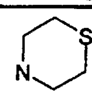 | 78 | 265 | B |
| 194 | 8-CH3 | C6H5CH2 | 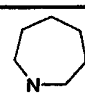 | 50 | 202 | A |
| 195 | 7-OCH3 | C6H5CH2 | 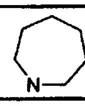 | 42 | 153 | B |
| 196 | 7-OCH3 | C6H5CH2 | 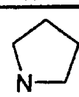 | 65 | 154 | B |
| 197 | 7-CN | C6H5CH2 | 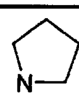 | 77 | 219 | B |
| 198 | 7-NO2 | C6H5CH2 | 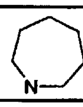 | 32 | 206 | A |

- **Composé 166 :**

R.M.N.$^1$H δ (ppm) : 2 (m, 4H) ; 3,3 (m, 4H) ; 5,35 (s, 2H) ; 7,2 – 7,3 (m, 3H) ; 7,4 (d, 2H) ; 7,6 (t, 1H) ; 7,9 (t, 1H) ; 8,2 (m, 2H)

Solvant : DMSO

- **Composé 167 :**

R.M.N.$^1$H δ (ppm) : 0,8 (m, 6H) ; 1,15 – 1,25 (m, 4H) ; 1,35 – 1,55 (m, 4H) ; 3 (m, 2H) ; 3,2 (m, 2H) ; 3,7 (s, 3H) ; 7,65 (m, 1H) ; 8,3 (m, 1H) ; 8,45 (m, 1H)

Solvant : $CDCl_3$

- **Composé 168 :**

R.M.N.1H δ (ppm) : 1,3-1,9 (m, 6H) ; 2,9 (t, 2H) ; 3,3 (m, 2H) ; 3,5 (s, 3H) ; 8,0 (d, 1H) ; 8,1 (d, 1H) ; 8,3 (d, 1H)

Solvant : CDC13

- **Composé 170 :**

R.M.N.1H δ (ppm) : 0,7 (s, 3H) ; 0,8 (s, 3H) ; 1,0 (s, 3H) ; 1,5-1,9 (m, 5H) ; 2,55 (d, 1H) ; 2,85 (d, 1H) ; 3,15 (m, 4H) ; 3,4 (m, 4H) ; 7,9 (d, 1 H) ; 8,0 (s, 1H) ; 8,4 (m, 1H)

Solvant : DMSO

- **Composé 171** :

R.M.N.$^1$H δ (ppm) : 1,7 – 1,8 (m, 8H) ; 3,3 (m, 4H(+$H_2O$) ; 7,45 – 7,6 (m, 5H) ; 8,05 (m, 1H) ; 8,15 (s, 1H) ; 8,45 (d, 1H)

Solvant : DMSO

- **Composé 172** :

R.M.N.$^1$H δ (ppm) : 1,7 – 1,85 (m, 8H) ; 3,3 (s, 4H) ; 5,3 (s, 2H) ; 7,25 – 7,5 (m, 5H) ; 8,0 (m, 1H) ; 8,15 (d, 1H) ; 8,4 (d, 1H)

Solvant : DMSO

- **Composé 173** :

R.M.N.$^1$H δ (ppm) : 2,05 (m, 4H) ; 3,4 (m, 4H) ; 5,45 (s, 2H) ; 7,2 – 7,35 (m, 3H) ; 7,65 – 7,75 (m, 3H) ; 8,2 (dd, 1H) ; 8,35 (s, 1H)

Solvant : $CDCl_3$

- **Composé 174** :

R.M.N.$^1$H δ (ppm) : 1,4 – 1,6 (m, 1H) ; 1,7 - 2 (m, 4H) ; 3 - 3,15 (m, 2H) ; 3,3 – 3,45 (m, 2H) ; 5,45 (s, 2H) ; 7,25 – 7,35 (m, 3H) ; 7,7 – 7,8 (m, 3H) ; 8,3 – 8,4 (m, 2H)

Solvant : $CDCl_3$

- **Composé 175** :

R.M.N.$^1$H δ (ppm) : 1,85 – 1,95 (m, 4H) ; 3,4 (m, 4H + $H_2O$) ; 3,65 (s, 3H) ; 7,7 (d, 1H) ; 8,3 (d, 1H) ; 8 ,55 (s, 1H)

Solvant : DMSO

- **Composé 176** :

R.M.N.$^1$H δ (ppm) : 1,8 – 2 (m, 8H) ; 3,3 – 3,5 (m, 4H) ; 5,4 (s, 2H) ; 7,2 – 7,35 (m, 3H) ; 7,4 – 7,45 (m, 2H) ; 7,65 – 7,7 (m, 2H) ; 8,25 –8,3 (m, 2H) ; 8,6 (s, 1H)

Solvant : $CDCl_3$

- **Composé 177** :

R.M.N.$^1$H δ (ppm) : 1,7 – 1,85 (m, 8H) ; 3,4 (m, 4H) ; 3,7 (s, 3H) ; 7,75 (m, 1H) ; 8,25 (m, 1H) ; 8,4 (m, 1H)

Solvant : $CDCl_3$

- **Composé 178** :

R.M.N.$^1$H δ (ppm) : 1,35 – 1,95 (m, 6H) ; 3,05 (t, 2H) ; 3,35 (d, 2H) ; 5,45 (s, 2H) ; 7,3 (m, 3H) ; 7,75 (d, 2H) ; 7,95 (d, 1H) ; 8,3 (d, 1H) ; 8,5 (s, 1H)

Solvant : $CDCl_3$

- **Composé 179** :

R.M.N.$^1$H δ (ppm) : 2,9 (s, 6H) ; 5,5 (s, 2H) ; 7,25 – 7,35 (m, 3H) ; 7,7 (d, 2H) ; 7,85 (d, 1H) ; 8,2 (d, 1H) ; 8,5 (s, 1H)

Solvant : $CDCl_3$

- **Composé 180** :

R.M.N.$^1$H δ (ppm) : 3,2 – 3,4 (m, 4H) ; 3,75 – 3,9 (m, 2H) ; 3,9 – 4,1 (m, 2H) ; 5,5 (s, 2H) ; 7,2 – 7,35 (m, 3H) ; 7,7 (d, 2H) ; 7,9 (d, 1H) ; 8,25 (d, 1H) ; 8,5 (s, 1H)

Solvant : $CDCl_3$

- **Composé 181** :

R.M.N.$^1$H δ (ppm) : 2,8 – 3,0 (m, 4H) ; 3,35 – 3,5 (m, 2H) ; 3,5 – 3,7 (m, 2H) ; 5,45 (s, 2H) ; 7,2 – 7,35 (m, 3H) ; 7,7 (d, 2H) ; 7,9 (d, 1H) ; 8,2 (d, 1H) ; 8,5 (s, 1H)

Solvant : $CDCl_3$

- **Composé 182** :

R.M.N.$^1$H δ (ppm) : 2,3 – 2,45 (m, 5H) ; 2,9 – 3,0 (m, 2H) ; 3,25 – 3,35 (m, 4H) ; 5,5 (s, 2H) ; 7,2 – 7,35 (m, 3H) ; 7,7 (d, 2H) ; 7,9 (d, 1H) ; 8,25 (d, 1H) ; 8,5 (s, 1H)

Solvant : $CDCl_3$

- **Composé 183** :

R.M.N.$^1$H δ (ppm) : 3,0 – 3,2 (m, 2H) ; 3,35 – 3,5 (m, 4H) ; 3,6 – 3,75 (m, 2H) ; 5,5 (s, 2H) ; 6,9 – 7,05 (m, 3H) ; 7,2 – 7,35 (m, 5H) ; 7,7 (d, 2H) ; 7,85 (d, 1H) ; 8,3 (d, 1H) ; 8,55 (s, 1H)

Solvant : $CDCl_3$

- **Composé 184** :

R.M.N.$^1$H δ (ppm) : 2,4 (m, 2H) ; 3 (m, 2H) ; 3,3 (m, 4H) ; 5,5 (s, 2H) ; 7,3 (m, 8H) ; 7,7 (m, 2H) ; 7,9 (d, 1H) ; 8,2 (d, 1H) ; 8,5 (s, 1H)

Solvant : $CDCl_3$

- **Composé 185** :

R.M.N.$^1$H δ (ppm) : 2,2 – 2,65 (m, 2H) ; 3,2 – 3,9 (m, 4H) ; 5,45 (s, 2H) ; 5,8 – 5,9 (m, 1H) ; 5,9 – 6,0 (m, 1H) ; 7,2 – 7,35 (m, 3H) ; 7,7 (d, 2H) ; 7,9 (d, 1H) ; 8,25 (d, 1H) ; 8,5 (s, 1H)

Solvant : $CDCl_3$

- **Composé 186** :

R.M.N.$^1$H δ (ppm) : 4,3 (s, 4H) ; 5,5 (s, 2H) ; 5,95 (s, 2H) ; 7,25 – 7,4 (m, 3H) ; 7,7 (d, 2H) ; 7,9 (d, 1H) ; 8,25 (d, 1H) ; 8,5 (s, 1H)

Solvant : $CDCl_3$

- **Composé 187** :

R.M.N.$^1$H δ (ppm) : 2 - 2,1 (m, 1H) ; 2,3 – 2,4 (m, 1H) ; 3,2 – 3,6 (m, 5H) ; 4,6 – 4,7 (m, 1H) ; 5,45 (s, 2H) ; 7,2 – 7,3 (m, 3H) ; 7,65 (d, 1H) ; 7,85 (d, 1H) ; 8,3 (d, 2H) ; 8,5 (s, 1H)

Solvant : $CDCl_3$

- **Composé 188** :

R.M.N.$^1$H δ (ppm) : 3,05 (s, 3H) ; 3,9 – 4,0 (m, 1H) ; 5,35 (s, 2H) ; 7,15 – 7,25 (m, 3H) ; 7,6 (d, 2H) ; 7,7 (d, 1H) ; 7,95 (d, 1H) ; 8,4 (s, 1H)

Solvant : $CDCl_3$

- **Composé 189** :

R.M.N.$^1$H δ (ppm) : 2 (m, 4H) ; 3,4 (m, 4H) ; 5,3 (s, 2H) ; 7,3 (m, 3H) ; 7,4 (m, 2H) ; 8.0 (m, 1H) ; 8,2 (m, 1H) ; 8,5 (m, 1H)

Solvant : DMSO

- **Composé 190** :

R.M.N.$^1$H δ (ppm) : 1,75 – 1,95 (m, 8H) ; 2,45 (s, 3H) ; 3,35 – 3,45 (m, 4H) ; 5,45 (s, 2H) ; 7,2 – 7,35 (m, 3H) ; 7,45 (dd, 1H) ; 7,7 (dd, 2H) ; 8,15 (s, 1H) ; 8,3 (d, 1H)

Solvant : $CDCl_3$

- **Composé 191** :

R.M.N.$^1$H δ (ppm) : 2 (m, 4H) ; 2,5 (s, 3H) ; 3,3 (m, 4H) ; 5,3 (s, 2H) ; 7,2 – 7,55 (m, 5H) ; 7,7 (d, 1H) ; 8 (s, 1H) ; 8,15 (d, 1H)

Solvant : $CDCl_3$

- **Composé 192** :

R.M.N.$^1$H δ (ppm) : 2,45 (s, 3H) ; 2,9 (s, 6H) ; 5,45 (s, 2H) ; 7,2 – 7,3 (m, 3H) ; 7,45 (d, 1H) ; 7,7 (d, 2H) ; 8,2 (d, 2H)

Solvant : $CDCl_3$

- **Composé 193** :

R.M.N.$^1$H δ (ppm) : 2,5 (s, 3H) ; 2,8 – 3,05 (m, 4H) ; 3,35 – 3,75 (m, 4H) ; 5,5 (s, 2H) ; 7,15 – 7,4 (m, 3H) ; 7,6 (d, 1H) ; 7,7 (d, 2H) ; 8,1 – 8,25 (m, 2H)

Solvant : $CDCl_3$

- **Composé 194** :

R.M.N.$^1$H δ (ppm) : 1,8 – 1,95 (m, 8H) ; 2,55 (s, 3H) ; 3,4 (m, 4H) ; 5,4 (s, 2H) ; 7,25 – 7,35 (m, 4H) ; 7,7 (m, 2H) ; 8,25 (m, 2H)

Solvant : $CDCl_3$

- **Composé 195** :

R.M.N.$^1$H δ (ppm) : 1,8 – 1,95 (m, 8H) ; 3,35 – 3,40 (m, 4H) ; 3,9 (s, 3H) ; 5,4 (s, 2H) ; 7,25 – 7,35 (m, 4H) ; 7,7 (dd, 2H) ; 7,8 (d, 1H) ; 8,35 (d, 1H)

Solvant : $CDCl_3$

- **Composé 196** :

R.M.N.$^1$H δ (ppm) : 2 (m, 4H) ; 3,35 (m, 4H) ; 3,9 (s, 3H) ; 5,35 (s, 2H) ; 7,25 – 7,35 (m, 3H) ; 7,45 (d, 2H) ; 7,55 (d, 1H) ; 7,7 (s, 1H) ; 8,2 (d, 1H)

Solvant : DMSO

- **Composé 197** :

R.M.N.$^1$H δ (ppm) : 2,4 (m, 4H) ; 3,2 (m, 4H) ; 5,2 (s, 2H) ; 7,1 – 7,25 (m, 3H) ; 7,35 (m, 2H) ; 8,25 (m, 2H) ; 8,5 (s, 1H)

Solvant : $CDCl_3$

- **Composé 198** :

R.M.N.$^1$H δ (ppm) : 1,7 – 1,85 (m, 8H) ; 3,3 (s, 4H + $H_2O$) ; 5,35 (s, 2H) ; 7,3 (m, 3H) ; 7,5 (m, 2H) ; 8,55 (d, 1H) ; 8,75 (d, 1H) ; 8,9 (s, 1H)

Solvant : DMSO

**Exemple 199** :1-Azepanyl-4-benzyl-7-chloro-4H-[1,2,4] triazolo [4,3-a] quinazolin-5-one.

(I) ;X1 = 7-C1 ; X2 = H

R = 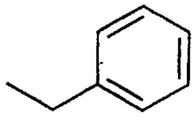 ; NR4R5 = 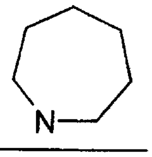

Dans un ballon de 50 ml muni d'une agitaion et d'un réfrigérant, on place 0,44 g (1,27 mmol) de 4-benzyl-1,7-dichloro-4H-[1,2,4]triazolo[4,3-a]quinazolin-5-one (exemple 254) en suspension dans 2,5 ml d'hexamethylèneimine. Sous agitation, le mélange est chauffé au reflux pendant 16 heures. La solution brune obtenue est alors abandonnée à la température ambiante jusqu'à complet refroidissement ; on verse dans un mélange d'eau et de chlorure de méthylène, agite et sépare les 2 phases par décantation. La phase organique est lavée 2 fois à l'eau, séchée sur $Na_2SO_4$ puis évaporée sous vide pour donner 0,59 g de résidu solide brun.

Celui-ci est chromatographié sur colonne de silice avec élution au mélange $CH_2$ $Cl_2$ 99,5 / $CH_3$ OH 0,5.

On obtient, après réunion et évaporation des fractions pures en CCM, 0,46g du composé de l'exemple 199. Celui-ci est recristallisé dans l'éthanol pour donner 0,4 g de cristaux incolores.

Rendement = 77%

F (Tottoli) = 162°C

CCM ($CH_2$ $Cl_2$ 98,5 / $CH_3$ OH 1,5) : Rf = 0,35

R.M.N.$^1$H δ (ppm) : 1,7 - 1,85 (m, 8H) ; 3,3 (s, 4H) ; 5,3 (s, 2H) ; 7,25 - 7,5 (m, 5H) ; 8,0 (m, 1H) ; 8,15 (d, 1H) ; 8,4 (d, 1H)

Solvant : DMSO

Les composés (I) des exemples 200 à 214 (tableau 4) sont préparés selon le procédé de l'exemple 199.

## TABLEAU 4

| N° Composé | X1 | R | NR4R5 | Rdt (%) | PF (°C) | Méthode |
|---|---|---|---|---|---|---|
| 200 | H | CH3 | 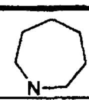 | 40 | 199 – 203 | A |
| 201 | H | C6H5CH2 | 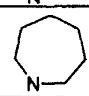 | 66 | 157 | A |
| 202 | 6-Cl | CH3 | 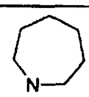 | 8,5 | > 275 | A |
| 203 | 7-Cl | CH3 | 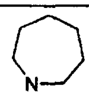 | 77 | 145 | A |

| N° Composé | X1 | R | NR4R5 | Rdt (%) | PF (°C) | Méthode |
|---|---|---|---|---|---|---|
| 204 | 7-Cl | CH3CH2 | 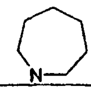 | 11 | 98-100 | A |
| 205 | 7-Cl | CH3 | 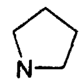 | 50 | 203-205 | A |
| 206 | 7-Cl | CH3 | 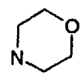 | 25 | 232 | A |
| 207 | 7-Cl | CH3 | 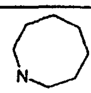 | 25 | 123-125 | A |
| 208 | 7-Cl | CH3 | 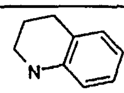 | 15 | 204 | A |
| 209 | 7-Cl | CH3 | 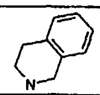 | 30 | 272 | A |
| 210 | 7-Cl | CH3 | 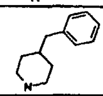 | 25 | 180 | A |
| 211 | 7-Cl | CH3 | 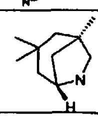 | 25 | 165 | A |
| 212 | 7-F | CH3 | 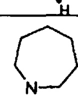 | 13 | 136 | A |
| 213 | 7-I | CH3 | 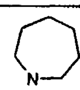 | 47 | 206 | A |
| 214 | 7-OCH3 | CH3 | 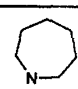 | 34 | 203 | A |

- **Composé 200 :**

R.M.N.1H δ (ppm) : 1,75-1,9 (m, 8H) ; 3,4 (m, 4H) ; 3,6 (s, 3H) ; 7,6 (t, 1H) ; 8 (t, 1H) ; 8,25 (d, 1H) ; 8,4 (d, 1H)

Solvant : DMSO

- **Composé 201 :**

R.M.N.1H δ (ppm) : 1,7 - 1,85 (m, 8H) ; 3,3 (m, 4H) ; 5,3 (s, 2H) ; 7,2 - 7,35 (m, 3H) ; 7,45 (d, 2H) ; 7,6 (t, 1H) ; 7,95 (t, 1H) ; 8,2 (d, 1H) ; 8,4 (d, 1H)

Solvant : DMSO

- **Composé 202** :

R.M.N.1H δ (ppm) : 1,5 - 1,8 (m, 8H) ; 3,4 (m, 4H) ; 3,5 (s,3H) ; 7,05 (d, 1H) ; 7,5 (t, 1H) ; 8,4 (d, 1H)

Solvant : DMSO

- **Composé 203** :

R.M.N.1H δ (ppm) : 1,7 - 1,85 (m, 8H) ; 3,3 (m, 4H) ; 3,5 (s, 3H) ; 7,95 (d, 1H) ; 8,1 (s, 1H) ; 8,35 (d, 1H)

Solvant : DMSO

- **Composé 204** :

R.M.N.1H δ (ppm) : 1,3 (t, 3H) ; 1,7 - 1,9 (m, 8H) ; 3,3 (m, 4H) ; 4,15 (q, 2H) ; 7,95 (d, 1H) ; 8,1 (s, 1H) ; 8,35 (d, 1H)

Solvant : DMSO

- **Composé 205** :

R.M.N.1H δ (ppm) : 2,0 (m, 4H) ; 3,35 (m, 4H) ; 3,75 (s, 3H) ; 7,65 (d, 1H) ; 8,15 (d, 1H) ; 8,3 (s, 1H)

Solvant : CDCl3

- **Composé 206** :

R.M.N.1H δ (ppm) : 3,1 - 3,35 (m, 4H) ; 3,65 (s, 3H) ; 3,85 (m, 2H) ; 4,0 (m, 2H) ; 7,75 (d, 1H) ; 8,35 (m, 2H)

Solvant : CDCl3

- **Composé 207** :

R.M.N.1H δ (ppm) : 1,8 (m, 10H) ; 3,4 (m, 4H) ; 3,75 (s, 3H) ; 7,75 (d, 1H) ; 8,35 (s, 1H) ; 8,4 (d, 1H)

Solvant : CDCl3

- **Composé 208** :

R.M.N.1H δ (ppm) : 2,1 (m, 2H) ; 2,8 - 3,1 (m, 2H) ; 3,65 (m,1H) ; 3,75 (s,3H) ; 3,9 (m,1H) ; 6,15 (d,1H) ; 6,75 (t, 1H) ; 6,85 (t,1H) ; 7,1 (d, 1H) ; 7,5 (d, 1H) ; 7,85 (d,1H) ; 8,3 (s, 1H)

Solvant : CDCl3

- **<u>Composé 209</u> :**

R.M.N.1H δ (ppm) : 2,9 (m, 1H) ; 3,2 (m, 1H) ; 3,4 (m, 1H) ; 3,6 (m, 1H) ; 3,7 (s, 3H) ; 4,3 (d, 1H) ; 4,45 (d, 1H) ; 7,05 (d, 1H) ; 7,2 (m, 3H) ; 7,6 (d, 1H) ; 8,2 (d, 1H) ; 8,3 (s, 1H)

Solvant : CDCl3

- **<u>Composé 210</u> :**

R.M.N.1H δ (ppm) : 1,4 (m, 2H) ; 1,7 (m, 3H) ; 2,6 (d, 2H) ; 2,9 - 3,15 (m, 2H) ; 3,3-3,5 (m, 2H) ; 3,65 (s, 3H) ; 7,0-7,35 (m, 5H) ; 7,7 (d, 1H) ; 8,3 (m, 2H)

Solvant : CDCl3

- **<u>Composé 211</u> :**

R.M.N.1H δ (ppm) : 1 (s, 3H) ; 1,1 (s, 3H) ; 1,25-1,4 (m, 5H) ; 1,45 (d, 1H) ; 1,6 (m, 2H) ; 1,9 (d,1H) ; 2,05 (m, 1H) ; 3,35 (d,1H) ; 3,45 (d, 1H) ; 3,7 (s, 3H) ; 4 (m, 1H) ; 7,65 (d, 1H) ; 8,3 (s, 1H) ; 8,6 (d, 1H)

Solvant : CDCl3

- **<u>Composé 212</u> :**

R.M.N.1H δ (ppm) : 1,7 – 1,8 (m, 8H) ; 3,3 (m, 4H (+H2O)) ; 3,5 (s, 3H) ; 7,8 (m, 1H) ; 7,9 (m, 1H) ; 8,4 (m,1H)

Solvant : DMSO

- **<u>Composé 213</u> :**

R.M.N.1H δ (ppm) : 1,7 - 1,9 (m, 8H) ; 3,3 (m, 4H) ; 3,7 (s, 3H) ; 8,0 (d, 1H) ; 8,1 (d, 1H) ; 8,65 (s,1H)

Solvant : CDCl3

- **<u>Composé 214</u> :**

R.M.N.1H δ (ppm) : 1,7 - 1,85 (m, 8H) ; 3,3 (s, 4H) ; 3,5 (s, 3H) ; 3,9 (s, 3H) ; 7,5 (d, 1H) ; 7,6 (s,1H) ; 8,3 (d, 1H)

Solvant : CDCl3

**Exemple 215** : 4-benzyl-7-bromo-1-(N-ethyl, N-methylamino)-4H-[1,2,4] triazolo [4,3-a] quinazolin-5-one.

0,3g (0,8 mmol) de 4-benzyl-7-bromo-1-(N-methylamino)-4H-[1,2,4] triazolo-[4,3-a]quinazolin-5-one (composé de l'exemple 188) est dissous dans 5ml de DMF. On additionne 0,135g (0,85 mmol) d'iodure de méthyle et 0,13g

(0,93 mmol) de carbonate de potassium. Le mélange obtenu est agité à température ambiante pendant une nuit puis chauffé à 100°C pendant 6 heures. Après refroidissement, le solvant est évaporé sous vide, le résidu repris par de l'eau et de l'acétate d'éthyle. La phase organique est séparée par décantation, lavée avec une solution saturée de chlorure de sodium, séchée sur Na2SO4 et évaporée sous vide. On obtient 0,3g de produit brut qui est purifié par chromatographie sur colonne de silice avec élution au mélange CH2C12 99 / CH3OH 1. Les fractions contenant le produit désiré sont réunies, concentrées sous vide puis le résidu est recristallisé dans du méthanol pour fournir 0,05g de composé de l'exemple 215 pur.
Rendement = 22%
F (Tottoli) = 148°C
CCM ($CH_2$ $Cl_2$ 98,5 / $CH_3$ OH 1,5) : Rf= 0,45
R.M.N.1H δ (ppm) : 1,25 (t, 3H) ; 2,9 (s, 3H) ; 3,2 - 3,4 (m, 2H) ; 5,45 (s, 2H) ; 7,2 - 7,35 (m, 3H) ; 7,7 (d, 2H) ; 7,9 (d, 1H) ; 8,3 (d, 1H) ; 8,5 (s, 1H)
Solvant : CDC13

**Exemple 216** : 4-benzyl-1-(N,N-diethyl)- 7-methyl-4H-[1,2,4] triazolo [4,3-a] quinazolin-5-one.

2,3g (5,87 mmol) de 4-benzyl-7-methyl-1-(thiamorpholin-4-yl)-4H-[1,2,4] triazolo [4,3-a] quinazolin-5-one (composé de l'exemple 193) sont mis en suspension dans 250ml d'éthanol. On additionne une quantité catalytique de Nickel de Raney et chauffe au reflux, sous agitation, pendant 24 heures. Le catalyseur est éliminé par filtration sur Célite et la solution alcoolique concentrée sous vide : on obtient 1,6g de produit brut qui est purifié par chromatographie sur colonne de silice avec élution au CH2C12 et gradient de méthanol à partir de 99,5/0,5 pour donner 0,9g de produit pur en CCM. Un échantillon est recristallisé dans l'éthanol pour la détermination des constantes physiques.
Rendement = 42%
F (Tottoli) = 154°C
CCM ($CH_2$ $Cl_2$ 99 / $CH_3$ OH 1) : Rf = 0,35
R.M.N.1H δ (ppm) : 1 - 1,3 (m, 6H) ; 2,4 (s, 3H) ; 2,9 - 3,45 (m, 4H) ; 5,4 (s, 2H) ; 7,1 - 7,3 (m, 3H) ; 7,45 (d, 1H); 7,6 (d, 2H) ; 8,15 (s, 1H); 8,3 (d, 1H)
Solvant : CDCl3

**Exemple 217** : 4-benzyl-7-bromo-1-(pyrrol-1-yl)-4H-[1,2,4] triazolo [4,3-a] quinazolin-5-one.

Dans un ballon de 25ml, on place 0,7g (1,8 mmol) de 1-amino-4-benzyl-7-bromo-4H-[1,2,4] triazolo [4,3-a] quinazolin-5-one (composé intermédiaire 10 de l'exemple 271) en suspension dans 5ml d'acide acétique. On ajoute 0,25g (1,9 mmol) de 2,5-dimethoxytetrahydrofuranne puis chauffe le mélange à reflux pendant 1 heure. Après refroidissement et évaporation de l'acide acétique sous vide, on obtient 0,8g de solide très coloré qui est purifié par chromatographie sur colonne de silice avec élution au mélange CH2C12 / CH3OH (99,4/0,6 puis 99/1). Le solide obtenu à partir des fractions pures est recristallisé dans l'éthanol pour donner 0,45g du composé de l'exemple 217.
Rendement = 55%
F (Tottoli) = 214°C
CCM ($CH_2$ $Cl_2$ 99 / $CH_3$ OH 1) : Rf= 0,5
R.M.N.1H δ (ppm) : 5,55 (s, 2H) ; 5,8 (d, 1H) ; 6,5 (s, 2H) ; 6,9 (s, 2H) ; 7,25 - 7,4 (m, 3H) ; 7,7 (d, 1H) ; 7,75 (d, 2H) ; 8,55 (s, 1H)
Solvant : CDCl3

**Exemple 218 :** 4-(4-aminobenzyl)-7-bromo-1-(pyrrolidin-1-yl)-4H-[1,2,4] triazolo [4,3-a] quinazolin-5-one.

Dans un ballon de 50ml, on charge 0,45g (0,96 mmol) de 7-bromo-4-(4-nitrobenzyl)-1-(pyrrol-1-yl)-4H-[1,2,4] triazolo [4,3-a] quinazolin-5-one (composé de l'exemple 48) dans 10ml d'éthanol. On ajoute 1.08g (24 mmol) de chlorure stanneux dihydrate puis chauffe à 70°C, sous agitation, pendant 30 minutes. Après refroidissement, le mélange est versé dans de l'eau glacée. On extrait plusieurs fois à l'acétate d'éthyle additionné d'un peu de CHCl3, lave la phase organique avec une solution saturée de chlorure de sodium, la sèche sur Na2SO4 puis concentre sous vide. Le résidu solide obtenu (0,35g) est lavé au méthanol (50ml) pour donner 0,25g de produit pur en CCM.
Rendement = 83%
F (Tottoli) = 263°C
CCM ($CH_2$ $Cl_2$ 98 / $CH_3$ OH 2) : Rf = 0,25
R.M.N.1H δ (ppm) : 1,9 - 2,05 (m, 4H) ; 3,3 - 3,4 (m, 4H) ; 5 (s, 2H) ; 5,1 (s, 2H) ; 6,5 (d, 2H) ; 7,2 (d, 2H) ; 8,1 (d, 1H) ; 8,2 (d, 1H) ; 8,3 (s, 1H)
Solvant : DMSO

**Exemple 219 :** 4-(benzyl)-7-hydroxy-1-(pyrrolidin-1-yl) -4H-[1,2,4] triazolo [4,3-a] quinazolin-5-one.

Exemple 219-1/ 4-benzyl-7-(4-tolylsulfonyloxy-4H-[1,2,4] triazolo [4,3-a] quinazolin-5-one.

Dans un réacteur équipé d'un système d' agitation, on charge 1,46g (5 mmol) de 4-benzyl-7-hydroxy-4H-[1,2,4] triazolo [4,3-a] quinazolin-5-one (intermédiaire obtenu par la méthode de l'exemple 255) dans 15ml de chlorure de méthylène sec. On ajoute 0,95g (5 mmol) de chlorure de tosyle puis coule, sous agitation, en 5 minutes, 1ml (7,5 mmol) de triéthylamine, la réaction étant légèrement exothermique. Après agitation supplémentaire à température ambiante pendant 2 heures, la solution organique obtenue est lavée à l'eau et séchée sur Na2SO4 pour donner, après évaporation du solvant, un résidu amorphe coloré qui est purifié par chromatographie sur colonne de silice avec élution à l'acétate d'éthyle. On obtient 1,9g de produit pur en CCM. Celui-ci sera utilisé tel quel dans l'étape suivante.
Rendement = 85%

2/ Exemple 219-2/ 4-benzyl-1-bromo-7-(4-tolylsulfonyloxy-4H-[1,2,4] triazolo [4,3-a] quinazolin-5-one.

0,4g de ce composé est obtenu à partir de 0,45g de 4-benzyl-7-(4-tolylsulfonyloxy-4H-[1,2,4] triazolo [4,3-a] quinazolin-5-one (exemple 219-1) par la méthode de bromation décrite dans l'exemple 256.
Rendement = 76%

3/ Exemple 219-3/ 4-benzyl-1-(pyrrolidin-1-yl)-7-(4-tolylsulfonyloxy)-4H-[1,2,4] triazolo [4,3-a] quinazolin-5-one et 4-benzyl-7-hydroxy-1-(pyrrolidin-1-yl)- 4H-[1,2,4] triazolo [4,3-a] quinazolin-5-one.

0,83g de dérivé bromé obtenu dans l'exemple 219-2 est traité par la pyrrolidine, dans les conditions de l'exemple 164. Après traitement, on obtient 1,0g de mélange brut de 2 composés majoritaires qui sont séparés par chromatographie sur colonne de silice avec élution au mélange CH2C12 98 / CH3OH 2. Les fractions contenant le premier produit pur sont réunies et concentrées pour donner 0,375g de 4-benzyl-1-(pyrrolidin-1-yl)-7-(4-tolylsulfonyloxy-4H-[1,2,4] triazolo [4,3-a] quinazolin-5-one.
Rendement = 45%
Les fractions contenant le deuxième produit pur sont réunies et évaporées sous vide pour donner 0,12g de 4-benzyl-7-hydroxy-1-(pyrrolidin-1-yl)-4H-[1,2,4] triazolo [4,3-a] quinazolin-5-one.
Rendement = 15%
F(Tottoli) = 287°C
R.M.N.1H $\delta$ (ppm) : 1,95 (m, 4H) ; 3,3 (m, 4H) ; 7,3 (s, 2H) ; 7,2- 7, 6 (m, 7H) ; 8,1 (d, 1H); 10,2 (s, 1H)
Solvant : DMSO

**Exemple 220** : 4-(4-cyanobenzyl)-7-hydroxy-1-(pyrrolidin-1-yl)-4H-[1,2,4] triazolo [4,3-a] quinazolin-5-one.

1/ Exemple 220-1/ 1-(pyrrolidin-1yl)-7-(4-tolylsulfonyloxy)4H-[1,2,4] triazolo [4,3-a] quinazolin-5-one.

1,3g de *ce* composé est obtenu à partir de 2,4g de 4-benzyl-1-(pyrrolidin-lyl)-7-(4-tolylsulfonyloxy-4H-[1,2,4] triazolo [4,3-a] quinazolin-5-one (exemple 219-3) par la méthode de débenzylation décrite dans l'exemple 263.
Rendement = 68%

2/ Exemple 220-2/ 4-(4-cyanobenzyl)-1-(pyrrolidin-1-yl)-7-(4-tolylsulfonyloxy-4H-[1,2,4] triazolo [4,3-a] quinazolin-5-one.

0,48g de ce composé est obtenu à partir de 0,66g de 1-(pyrrolidin-1yl)-7-(4-tolylsulfonyloxy-4H-[1,2,4] triazolo [4,3-a] quinazolin-5-one (exemple 220-1) par la méthode de N-alkylation décrite dans l'exemple 3.
Rendement = 52%

3/ Exemple 220-3/ 4-(4-cyanobenzyl)-7-hydroxy-1-(pyrrolidin-1-yl)-4H-[ 1,2,4] triazolo [4,3-a] quinazolin-5-one.

0,3g (0,55 mmol) de 4-(4-cyanobenzyl)-1-(pyrrolidin-1-yl)-7-(4-tolylsulfonyloxy-4H-[1,2,4] triazolo [4,3-a] quinazolin-5-one (décrit dans l'exemple 220-2) est dissous dans 1ml de DMF sec. On additionne 0,27ml de pyrrolidine (2,75 mmol) puis chauffe à 140°C, sous agitation, pendant 6 heures. Le solvant est évaporé sous vide et le résidu est repris par un mélange acétate d'éthyle / solution aqueuse N d'acide chlorhydrique. L'insoluble est séparé par filtration, lavé à l'eau jusqu'à pH neutre et séché sous vide ; on obtient 0,13g de produit brut, qui est cristallisé dans 5 ml d'éthanol, filtré et séché pour donner 0,085g de produit pur.

Rendement = 40%
F(Tottoli) = 305°C
R.M.N.1H δ (ppm) : 2 (m, 4H) ; 3,3 (m, 4H) ; 5,35 (s, 2H) ; 7,35 (d, 1H) ; 7,6 - 7,7 (m, 3H) ; 7,8 (d, 2H) ; 8,1 (d, 1H) ; 10,2 (s, 1H)
Solvant : DMSO

**Exemple 221:** 7-acetamido-4-benzyl-1-(pyrrolidin-1-yl) -4H-[1,2,4] triazolo [4,3-a] quinazolin-5-one.

1/ Exemple 221-1/ 7-acetamido-4-benzyl-1-bromo -4H-[1,2,4] triazolo [4,3-a] quinazolin-5-one.

0,45g de ce composé sont obtenus à partir de 0,5g de 7-acetamido-4-benzyl -4H-[1,2,4] triazolo [4,3-a] quinazolin-5-one par la méthode de bromation décrite dans l'exemple 256.
Rendement = 72 %

2/ Exemple 221-2/ 7-acetamido-4-benzyl-1-(pyrrolidin-1-yl)- 4H-[1,2,4] triazolo [4,3-a] quinazolin-5-one.

8,7g (21 mmol) de dérivé bromé obtenu dans l'exemple 221-1 sont traités par 3,7 ml (42 mmol) de pyrrolidine et 3,54g (42 mmol) de bicarbonate de sodium dans 80 ml de DMF, dans les conditions de l'exemple 164. Après traitement, on obtient 8,0g de produit brut qui est purifié par chromatographie sur colonne de silice avec élution au mélange CH2C12 98 / CH3OH 2. Les fractions contenant le produit pur sont réunies et concentrées puis le résidu est cristallisé dans l'éthanol pour donner 6,6g de 7-acetamido-4-benzyl-1-(pyrrolidin-1-yl) -4H-[1,2,4] triazolo [4,3-a] quinazolin-5-one.
Rendement = 78 %
F(Tottoli) = 265°C
R.M.N.1H δ (ppm) : 2 - 2,1 (m, 4H) ; 2,25 (s, 3H) ; 3,4 (m, 4H) ; 5,45 (s, 2H) ; 7,2 - 7,3 (m, 3H) ; 7,6 (d, 2H) ; 8,1 (s, 1H) ; 8,2 (m, 2H) ; 8,4 (d, 1H)
Solvant : CDCl3

**Exemple 222** : 7-acetamido-4-[(E)-3-phenylallyl]-1-(pyrrolidin-1-yl) -4H-[1,2,4] triazolo [4,3-a] quinazolin-5-one.

A partir de 1,2g (3,0 mmol) de 7-acetamido-4-benzyl-1-(pyrrolidin-lyl)-4H-[1,2,4] triazolo [4,3-a] quinazolin-5-one (décrite dans l'exemple 221) débenzylé en 7-acetamido-1-(pyrrolidin-lyl) -4H-[1,2,4] triazolo [4,3-a] quinazolin-5-one par la méthode au Palladium/C décrite dans l'exemple 257, puis directement traité par 0,59g de bromure de cinnamyle en présence de 0,98g de carbonate de césium, dans 15 ml de DMF, d'après la méthode décrite dans l'exemple 3, on obtient, après purification par chromatographie sur colonne de silice et recristallisation dans l'éthanol, 0,4g du composé pur de l'exemple 222.
Rendement = 31 %.
F (Tottoli) = 248° C
CCM ($CH_2$ $Cl_2$ 95 / $CH_3$ OH 5) : Rf= 0,30
RMN $^1H$ δ (ppm) CDCl3:
2,0-2,1 (m,4H) ; 2,25 (s, 3H) ; 3,45 (m, 4H) ; 5 (d, 2H) ; 6,35-6,4(dt, 1H) ; 6,8 (d, 1H) ; 7,15-7.35 (m, 5H) ; 8,1 (s, 1H) ; 8,2-8.3 (m, 2H) ; 8,4 (m, 1H)

**Exemple 223** : 7-amino-4-[(E)-3-phenylallyl]-1-(pyrrolidin-1-yl) -4H-[1,2,4] triazolo [4,3-a] quinazolin-5-one.

Dans un ballon de 20 ml, on place 0,2g (0,46 mmol) de 7-acetamido-4-[(E)-3-phenylallyl]-1-(pyrrolidin-1-yl) -4H-[1,2,4] triazolo [4,3-a] quinazolin-5-one (décrite dans l'exemple 222) dans 5 ml d'une solution 6N d'acide chlorhydrique et chauffe à reflux, sous agitation, pendant 15 minutes. Après refroidissement, la solution obtenue est alcalinisée par une solution de soude, extraite 3 fois au chlorure de méthylène. Les phases organiques jointes sont lavées avec une solution saturée de NaCl, séchées (Na2SO4) puis évaporées sous vide. Le produit brut (0,12g) est recristallisé dans l'éthanol pour donner 0,08g du composé pur de l'exemple 223.
Rendement = 44 %
F(Tottoli) = 199°C
RMN $^1H$ δ (ppm) CDCl3 :
2,1 (m, 4H) ; 3,4 (m, 4H) ; 4,0 (m, 2H) ; 5,1 (d, 2H) ; 6,5-6,6 (dt, 1H); 6,85 (d, 1H); 7,0-7,3 (m, 3H) ; 7,6 (m, 1H) ; 7,7 (m, 1H) ; 8,1 (m, 1H) ; 8,45 (s, 1H) ; 8,6 (s, 1H).
Les composés de formule générale (I) des exemples 224 à 233 du tableau 5 sont préparés par la méthode de l'exemple 223.

## TABLEAU 5

| N° Composé | X1 | R | NR4R5 | Rdt (%) | PF (°C) |
|---|---|---|---|---|---|
| 224 | 7-NH2 | C6H5CH2 | 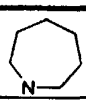 | 40 | 240 (dec) |
| 225 | 7-NH2 | C6H5CH2 | 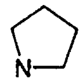 | 60 | 230 |
| 226 | 7-NH2 | 4-CNC6H4CH2 | 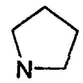 | 67 | 152 |
| 227 | 7-NH2 | (E) (3-pyridyl)-CH=CHCH2 | 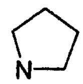 | 70 | 201 |
| 228 | 7-NH2 | 4-CNC6H4CH2 | 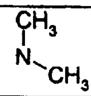 | 68 | 163 |
| 229 | 7-NH2 | (E) C6H5CH=CHCH2 | 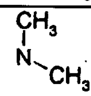 | 67 | 198 |
| 230 | 7-CH3NH | C6H5CH2 | 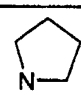 | 58 | 171 |
| 231 | 7-CH3NH | 4-CNC6H4CH2 | 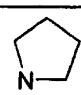 | 91 | 270 |
| 232 | 8-CH3NH | C6H5CH2 | 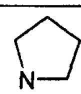 | 76 | - |
| 233 | 7-C2H5NH | C6H5CH2 | 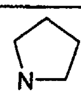 | 67 | 225 |

**- Composé 224 :**

R.M.N.1H δ (ppm) : 1,8 – 1,9 (m, 8H) ; 3,4 – 3,45 (m, 4H) ; 4 (s, 2H) ; 5,4 (s, 2H) ; 7 (m, 1H) ; 7,25 – 7,35 (m, 3H) ; 7,55 (s, 1H) ; 7,65 – 7,80 (m, 2H) ; 8,15 – 8,2 (m, 1H)

Solvant : CDCl3

**- Composé 225 :**

R.M.N.1H δ (ppm) : 2,1 (m, 4H) ; 3,4 (m, 4H) ; 4 (s, 2H) ; 5,45 (s, 2H) ; 7 (d, 1H) ; 7,2 – 7,35 (m, 3H) ; 7,6 (s, 1H) ; 7,7 – 7,8 (d, 2H) ; 8 – 8,1 (d, 1H)

Solvant : CDCl3

**- Composé 226 :**
R.M.N.1H δ (ppm) : 2 - 2,1 (m, 4H) ; 3,35 - 3,45 (m, 4H) ; 4,05 (s, 2H) ; 8,5 (s, 2H) ; 7,05 (m, 1H) ; 7,4 – 7,5 (m, 3H) ; 7,8 (s, 1H) ; 8,05 (d, 1H)
Solvant : CDCl3

**- Composé 227 :**
R.M.N.1H δ (ppm) : 2,1 (m, 4H) ; 3,4 (m, 4H) ; 4 (m, 2H) ; 5,1 (d, 2H) ; 6,4 – 6,5 (dt, 1H) ; 6,9 (d, 1H) ; 7,05 (m, 1H) ; 7,2 – 7,3 (m, 2H) ; 7,35 (d, 2H) ; 7,6 (s, 1H) ; 8,1 (d, 1H)
Solvant : CDCl3

**- Composé 228 :**
R.M.N.1H δ (ppm) : 2,8 (s, 6H) ; 5,4 (s, 2H) ; 5,7 (m, 2H) ; 7,10 – 7,15 (m, 1H) ; 7,4 (s, 1H) ; 7,6 (d, 2H) ; 7,8 (d, 2H) ; 8,05 (d, 1H)
Solvant : DMSO

**- Composé 229 :**
R.M.N.1H δ (ppm) : 2,9 (s, 6H) ; 4,95 (d, 2H) ; 5,75 (m, 2H) ; 6,45 – 6,5 (dt, 1H) ; 6,7 – 6,8 (d, 1H) ; 7,2 (m, 1H) ; 7,25 – 7,4 (m, 6H) ; 8,1 (d, 1H)
Solvant : DMSO

**- Composé 230 :**
R.M.N.1H δ (ppm) : 2,1 (m, 4H) ; 2,95 (s, 3H) ; 3,4 (m, 4H) ; 4,1 (m, 1H) ; 5,4 (s, 2H) ; 6,95 (d, 1H) ; 7,3 (m, 3H) ; 7,45 (s, 1H) ; 7,75 (dd, 2H) ; 8,1 (d, 1H)
Solvant : CDCl3

**- Composé 231 :**
R.M.N.1H δ (ppm) : 2,1 (m, 4H) ; 2,9 (s, 3H) ; 3,4 (m, 4H) ; 5,5 (s, 2H) ; 7 (m, 1H) ; 7,45 (s, 1H) ; 7,6 (m, 2H) ; 7,8 (m, 2H) ; 8,1 (d, 1H)
Solvant : CDCl3

**- Composé 232 :**
R.M.N.1H δ (ppm) : 1,9 - 2 (m, 4H) ; 2,85 (d, 3H) ; 3,3 (m, 4H) ; 5,3 (s, 2H) ; 6,7 (d, 1H) ; 7,2 (q, 1H) ; 7,25 – 7,45 (m, 6H) ; 7,9 (d, 1H)
Solvant : DMSO

**- Composé 233 :**

R.M.N.1H δ (ppm) : 1,3 (t, 3H) ; 2,1 (m, 4H) ; 3,25 (m, 2H) ; 3,4 (m, 4H) ; 3,9 (m, 1H) ; 5,45 (s, 2H) ; 7 (m, 1H) ; 7,2 – 7,3 (m, 3H) ; 7,45 (s, 1H) ; 7,7 (m, 2H) ; 8,1 (d, 1H)
Solvant : CDCl3

**Exemple 234**: 4-benzyl-7-(N-isopropylamino)-1-(pyrrolidin-1-yl) -4H-[1,2,4] triazolo [4,3-a] quinazolin-5-one

Dans un ballon de 20 ml, on place 0,31g (0,86 mmol) de 7-amino-4-benzyl-1-(pyrrolidin-1-yl) -4H-[1,2,4] triazolo [4,3-a] quinazolin-5-one (décrite dans l'exemple 225) en suspension dans 10 ml de chlorure de méthylène. On additionne 0,14 ml (1,9 mmol) d'acétone, 0,115 ml (1,9 mmol) d'acide acétique pur puis 0,546g (2,6 mmol) detriacétoxyborohydrure de sodium. Le mélange est agité à température ambiante, sous atmosphère d'azote, pendant 48 heures. Le solvant est évaporé sous vide et le résidu repris par de l'acétate d'éthyle. La phase organique est lavée par une solution de bicarbonate de sodium, puis une solution saturée de NaCl. Après séchage (Na2SO4) et élimination du solvant sous vide, on obtient 0,3g de produit brut qui est purifié par chromatographie sur colonne de silice, avec élution au mélange CH2C12 98 / CH3OH 2, pour donner 0,2g de composé de l'exemple 234 pur en CCM.
Rendement = 58%
F(Tottoli) = 208°C [EtOH]
R.M.N.1H δ (ppm) : 1,2 (m, 6H) ; 2,05 (m, 4H) ; 3,4 (m, 4H) ; 3,7 - 3,85 (m, 2H) ; 5,5 (s, 2H) ; 6,9 (m, 1H) ; 7,2 - 7,3 (m, 3H) ; 7,4 (s, 1H) ; 7,7 (m, 2H) ; 8,1 (m, 1H)
Solvant : CDCl3

**Exemple 235**: 4-benzyl-7-methylsulfonylamino-1-(pyrrolidin-1-yl) -4H-[1,2,4] triazolo [4,3-a] quinazolin-5-one et **exemple 247** : 4-benzyl-7-(N,N-dimethylsulfonylamino)-1-(pyrrolidin-1-yl) -4H-[1,2,4] triazolo [4,3-a] quinazolin-5-one.

Dans un ballon de 20 ml, on place 0,55g (1,5 mmol) de 7-amino-4-benzyl-1-(pyrrolidin-1-yl) -4H-[1,2,4] triazolo [4,3-a] quinazolin-5-one (décrite dans l'exemple 225) en suspension dans 10 ml de chlorure de méthylène. On additionne 0,42 ml (3,0 mmol) de triéthylamine puis 0,24 ml (3,0 mmol) de chlorure de méthanesulfonyle. La solution obtenue est agitée à température ambiante solution pendant 24 heures. Après refroidissement, la solution obtenue est lavée à l'eau, séchée (Na2SO4) puis évaporée sous vide. Le mélange brut des 2 composés obtenus (0,85g) est chromatographié sur colonne de silice avec élution au mélange CH2C12 99 / CH3OH 1 / NH4OH 0,1. Les fractions contenant le premier produit par ordre d'élution sont réunies et évaporées sous vide pour donner 0,65g de 4-benzyl-7-(N,N-dimethylsulfonylamino)-1-(pyrrolidin-1-yl) -4H-[1,2,4] triazolo [4,3-a] quinazolin-5-one.
F (Tottoli) = 221°C
R.M.N.1H δ (ppm) DMSO : 2,2 - 2,3 (m, 4H) ; 2,9 (s, 3H) ; 3,15 (m, 4H) ; 5,15 (s, 2H) ; 7,1 - 7,2 (m, 3H) ; 7,25 (m, 2H) ; 7,5 - 7,6 (d, 1H) ; 7,85 (s, 1H) ; 8,05 - 8,1 (d, 1H) ; 10,05 (s, 1H)

Les fractions contenant le deuxième produit par ordre d'élution sont traitées d'une façon similaire pour fournir 0,15g de 4-benzyl-7-methylsulfonylamino-1-(pyrrolidin-1-yl) -4H-[1,2,4] triazolo [4,3-a] quinazolin-5-one.
Rendement = 23%
F(Tottoli) = 283°C [EtOH]
R.M.N.$^1$H δ (ppm) DMSO : 2 (m, 4H) ; 3,45 (m, 4H) ; 3,5 (s, 3H) ; 5,45 (s, 2H) ; 7,3 (m, 3H) ; 7,7 (m, 3H) ; 6,35 (m, 2H)

**Exemple 236**: 7-(N,N-dimethylamino)-4-benzyl-1-(pyrrolidin-1-yl) -4H-[1,2,4] triazolo [4,3-a] quinazolin-5-one.

Dans un ballon, on place 0,75g (2,05 mmol) de 7-amino-4-benzyl-1-(pyrrolidin-1-yl) -4H-[1,2,4] triazolo [4,3-a] quinazolin-5-one (décrite dans l'exemple 225) en suspension dans 0,8 ml d'acide formique et 0,8 ml de formol. Sous agitation, le mélange est chauffé à 100°C pendant 1 heure. Après refroidissement, la solution obtenue est versée dans de l'eau glacée, la suspension extraite plusieurs fois à l'acétate d'éthyle ; les phases organiques jointes sont lavées avec une solution aqueuse saturée de chlorure de sodium, séchées sur Na2SO4 puis concentrées sous vide.
Le produit brut obtenu (0,8g) est purifié par chromatographie sur colonne de silice avec élution au mélange chlorure de méthylène 98 / méthanol 2. On obtient 0,23g de produit de l'exemple 236 pur en CCM.
Rendement = 29%
F (Tottoli) = 194°C [EtOH]
CCM ($CH_2$ $Cl_2$ 97 / $CH_3$ OH 3) : Rf = 0,65
R.M.N.1H δ (ppm) : 2,1 (m, 4H) ; 3,05 (s, 6H) ; 3,45 (m, 4H) ; 5,45 (s, 2H) ; 7,1 (m, 1H) ; 7,3 (m, 3H) ; 7,6 (d, 1H) ; 7,75 (m, 2H) ; 8,1 (d, 1H)

Solvant : CDC13

**Exemple 237**: 4-benzyl-7-cyano-1-(N,N-dimethylamino)-4H-[1,2,4] triazolo [4,3-a] quinazolin-5-one.

Dans un ballon de 500 ml muni d'un système d'agitation, d'un réfrigérant et d'une arrivée d'azote, on introduit 10,8g (27,1 mmol) de 4-benzyl-7-bromo-1-(pyrrolidin-1-yl)-4H-[1,2,4] triazolo [4,3-a] quinazolin-5-one (exemple 164) dans 100ml de N-methylpyrrolidinone (NMP). On ajoute 4,4g (49 mmol) de cyanure cuivreux puis chauffe le mélange, sous agitation et sous azote pendant 12 heures. Le solvant est éliminé par évaporation sous vide ; le résidu est agité dans 1 mélange de chlorure de méthylène et de solution d'ammoniaque 2N, l'insoluble éliminé par filtration puis les phases séparées par décantation. La phase organique estlavée avec une solution saturée en NaCl, séchée (Na2SO4) et évaporée pour donner 24,0g de produit brut. Celui-ci est purifié par chromatographie sur colonne de silice avec élution au mélange acétate d'éthyle 65 / cyclohexane 35. Les fractions pures en CCM sont réunies et évaporées sous vide : on obtient 8,4g du composé de l'exemple 237.
Rendement = 90%.
F (Tottoli) = 212-214°C
R.M.N.1H δ (ppm) : 2,9 (s, 6H) ; 5,3 (s, 2H) ; 7,3 (m, 3H) ; 7,5 (m, 2H) ; 8,4 (m, 1H) ; 8,5 (m, 1H) ; 8,6 (m, 1H)
Solvant : DMSO

**Exemple 238**: 4-benzyl-7-carboxy-1-(pyrrolidin-1-yl)-4H-[1,2,4] triazolo [4,3-a] quinazolin-5-one.

Dans un ballon de 250ml, on place 5,0 g (13,5 mmol) de 4-benzyl-7-cyano-1-(pyrrolidin-1-yl)-4H-[1,2,4] triazolo [4,3-a] quinazolin-5-one en suspension dans 100ml d'une solution 16 N d'acide chlorhydrique puis chauffe à reflux, sous agitation, pendant 3 heures.
Après refroidissement, le précipité est filtré, lavé plusieurs fois à l'eau, séché et purifié par chromatographie sur colonne de silice, avec élution au mélange CH2C12 97 / CH3OH 3, pour donner 2,3g de composé de l'exemple 238, pur en CCM.
Rendement = 44%
F(Tottoli) = 335-337°C
R.M.N.1H δ (ppm) : 1,9 (s, 4H) ; 3,4 (s, 4H) ; 5,3 (s, 2H) ; 7,3 (m, 3H) ; 7,4 (m, 2H) ; 8,2 (m, 1H) ; 8,4 (m, 1H) ; 8,7 (s, 1H)
Solvant : DMSO

**Exemple 239**: 7-bromo-4-[(4-methoxycarbonylmethyl)benzyl]-1-(pyrrolidin-1-yl) -4H-[1,2,4] triazolo [4,3-a] quinazolin-5-one.

0,8g (1,65 mmol) de 7-bromo-4-[(4-carboxymethyl)benzyl]-1-(pyrrolidin-1-yl) -4H-[1,2,4] triazolo [4,3-a] quinazolin-5-one (exemple 56) et 0,25g de carbonate de potassium sont mis en suspension dans 10 ml de DMF. On ajoute 0,26g (1,82 mmol) d'iodure de méthyle puis chauffe à 80°C, sous agitation, pendant 2 heures. Le solvant est évaporé sous vide, le résidu est repris par de l'eau, laquelle est extraite 3 fois à l'acétate d'éthyle ; les phases organiques jointes sont lavées avec une solution saturée de chlorure de sodium, séchées sur Na2SO4 puis le solvant est évaporé sous vide pour fournir 0,7g de produit brut.
Celui-ci est purifié par chromatographie sur colonne de silice avec élution au mélange CH2C12 99/ $CH_3OH$ 1. On obtient 0,5g de produit pur en CCM.
Rendement = 61 %
F(Tottoli) = 161-162°C [C2H5OH]
R.M.N.1H δ (ppm) : 2 - 2,1 (m, 4H) ; 3,35 - 3,45 (m, 4H) ; 3,6 (s, 2H) ; 3,7 (s, 3H) ; 5,45 (s, 2H) ; 7,2 (d, 2H) ; 7,65 (d, 2H) ; 7,85 (d, 1H) ; 8,15 (d, 1H); 8,5 (s, 1H)
Solvant : CDCI3

**Exemple 240** : 7-bromo-4-[(4-(N-methylcarbamoyl)methyl)benzyl]-1-(pyrrolidin-1-yl) -4H-[1,2,4] triazolo [4,3-a] quinazolin-5-one.

240-1/ 7-bromo-4-[(4-chloroformylmethyl)benzyl]-1-(pyrrolidin-1-yl) -4H-[1,2,4] triazolo [4,3-a] quinazolin-5-one.

0,85g (1,76 mmol) de 7-bromo-4-[(4-carboxymethyl)benzyl]-1-(pyrrolidin-1-yl) -4H-[1,2,4] triazolo [4,3-a] quinazolin-5-one (exemple XX) est placé dans 85 ml de chloroforme sec. Sous courant d'azote, on agite puis additionne 0,42g (3,52 mmol) de chlorure de thionyle en maintenant la température inférieure à +5°C. Après 1h30, la réaction est pratiquement complète et le chlorure d'acide a tendance à précipiter sous forme de cristaux. Cette solution sera utilisée telle quelle pour l'étape suivante.

240-2/ 7-bromo-4-[(4-(N-methylcarbamoyl)methyl)benzyl]-1-(pyrrolidin-1-yl) -4H-[1,2,4] triazolo [4,3-a] quinazolin-5-one.

A une solution refroidie à 0°C de 0,6g (8,8 mmol) de chlorhydrate de methylamine et 1,06g de triethylamine dans 85 ml d'acétone, on additionne lentement la solution obtenue dans l'exemple 240-1 en maintenant la température < +5°C. L'agitation est ensuite maintenue à 0°C pendant 15 minutes puis la solution obtenue est concentrée sous vide. On dissout le résidu dans du chlorure de méthylène, lave la phase organique 2 fois à l'eau, sèche sur Na2SO4, évapore le solvant sous vide et récupère ainsi 1,0g de produit brut. Celui-ci est chromatographié sur colonne de silice avec élution au mélange CH2C12 96 / CH3OH 4 pour donner 0,4g qui est recristallisé dans l'éthanol . On obtient, après séchage, 0,27g de composé pur.
Rendement = 31 %
F (Tottoli) = 240°C
CCM ($CH_2$ $Cl_2$ 92 / $CH_3$ OH 8) : Rf= 0,5
R.M.N.1H δ (ppm) : 1,95 - 2,1 (m, 4H) ; 2,7 (d, 3H) ; 3,35 - 3,45 (m, 4H) ; 3,5 (s, 2H) ; 5,3 - 5,5 (m, 3H) ; 7,15 (d, 2H) ; 7,65 (d, 2H) ; 7,9 (d, 1H) ; 8,2 (d, 1H) ; 8,5 (s, 1H)
Solvant : CDCl3

Les composés (I) des exemples 241 à 243 (tableau 6) sont préparés selon le procédé de l'exemple 240.

## TABLEAU 6

| N° Composé | R | NR4R5 | Rdt (%) | PF (°C) |
|---|---|---|---|---|
| 241 | 4-(NH2COCH2)C6H4CH2 | 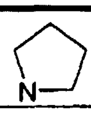 | 38 | 268 |
| 242 | 4-(Me2NCOCH2)C6H4CH2 | 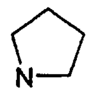 | 74 | 202 |
| 243 | 4-(HONHCOCH2)C6H4CH2 | 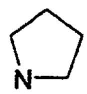 | 47 | 229 |

**- Composé 241 :**

R.M.N.1H δ (ppm) : 2,7 (s, 6H) ; 3,2 (s, 2H) ; 5,1 (s, 2H) ; 6,7 (s, 1H) ; 7,05 (d, 2H) ; 7,2 (m, 3H) ; 7,95 (m, 1H) ; 8,05 (d, 1H) ; 8,15 (s, 1H)

Solvant : DMSO

**- Composé 242 :**

R.M.N.1H δ (ppm) : 2 - 2,15 (m, 4H) ; 2,9 (s, 3H) ; 2,95 (s, 3H) ; 3,35 – 3,45 (m, 4H) ; 3,7 (s, 2H) ; 5,45 (s, 2H) ; 7,15 (d, 2H) ; 7,65 (d, 2H) ; 7,85 (d, 1H) ; 8,15 (d, 1H) ; 8,5 (s, 1H)
Solvant : CDCl3

**- Composé 243 :**

R.M.N.1H δ (ppm) : 1,95 - 2,1 (m, 4H) ; 3,3 (s, 2H) ; 3,3 – 3,4 (m, 4H) ; 5,3 (s, 2H) ; 7,25 (d, 2H) ; 7,45 (d, 2H) ; 8,15 (d, 1H) ; 8,25 (d, 1H) ; 8,35 (s, 1H) ; 8,8 (s, 1H) ; 10,7 (s, 1H)
Solvant : DMSO

**Exemple 244 :** 7-methyl-4-(4-cyanobenzyl-1-(N,N-dimethylamino)-4H-[1,2,4] triazolo [4,3-a] quinazolin-5-thione.

244-1/ 7-methyl-1-(N,N-dimethylamino)-4H-[1,2,4] triazolo [4,3-a] quinazolin-5-thione.

Dans un ballon tricol équipé d'un système d'agitation, d'un réfrigérant et d'un système d'introduction d'azote, on place 1,0g (4,1 mmol) de 7-methyl-1-(N,N-dimethylamino)-4H-[1,2,4] triazolo [4,3-a] quinazolin-5-one dans 70 ml de toluène et ajoute en une fois 3,3g (8,2 mmol) de réactif de Lawesson. Sous agitation, le mélange est chauffé à reflux pendant 24 heures. Après refroidissement, on additionne 30 ml d'une solution 5% d'acide chlorhydrique puis verse dans 250 ml de méthanol en agitant. On ajoute 250 ml de cyclohexane et élimine l'insoluble par filtration. La phase méthanolique acide est séparée par décantation, concentrée sous vide et le résidu est repris par de la glace et y est trituré plusieurs fois. L'insoluble récupéré sous forme de laque est dissous dans 10 ml d'isopropanol ; à partir de la solution obtenue, agitée pendant 30 minutes, les cristaux jaune qui ont précipité sont filtrés, lavés à l'isopropanol puis à l'éther et séchés sous vide.
On obtient 0,98g de produit qui sera utilisé tel quel pour l'étape suivante.
Rendement = 80 %

244-2/ 4-(4-cyanobenzyl)-1-(N,N-dimethylamino)-7-methyl-4H-[1,2,4] triazolo [4,3-a] quinazolin-5-thione.

A partir de 0,5g (1,93 mmol) de 7-methyl-1-(N,N-dimethylamino)-4H-[1,2,4] triazolo [4,3-a] quinazolin-5-thione (exemple 244-2), en utilisant la méthode B décrite à l'exemple 3, on obtient, après recristallisation dans l'éthanol, 0,29g de composé de l'exemple 244.
Rendement = 40 %
F(Tottoli) = 236°C
R.M.N.1H δ (ppm) : 2.9(s,6H) ; 3.7(s,2H) ; 5.45(s,2H) ; 7.25(m,2H) ; 7.7(m,2H) ; 7.85(m,1H); 8.2(d,1H); 8.5(s,1H)
Solvant : $CDCl_3$
Les composés (I) des exemples 245 à 246 (tableau 7) sont préparés selon le procédé de l'exemple 244.

## TABLEAU 7

| N° Composé | X1 | R | NR4R5 | Rdt (%) | PF (°C) |
|---|---|---|---|---|---|
| 245 | 7-Br | 4-CNC6H4CH2 | 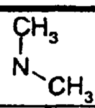 | 13 | 276 |
| 246 | 7-CH3 | (E) (pyridin-3-yl)-CH=CHCH2 | 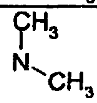 | 26 | 133 |

- <u>Composé 245</u> :

R.M.N.1H δ (ppm) : 2,9 (s, 6H) ; 4,7 (s, 2H) ; 7,65 (d, 2H) ; 7,75 (d, 2H) ; 8,1 (m, 2H) ; 8,4 (d, 1H)

Solvant : DMSO

- <u>Composé 246</u> :

R.M.N.1H δ (ppm) : 2,5 (s, 3H) ; 3,0 (s, 6H) ; 4,25 (d, 2H) ; 6,45 (dt, 1H) ; 6,75 (d, 1H) ; 7,2 (m, 1H) ; 7,6 (d, 1H) ; 7,7 (d, 1H) ; 7,9 (s, 1H) ; 8,4 (m, 2H) ; 8,6 (bs, 1H)

Solvant : CDCl3

**<u>B. Composés intermédiaires</u>**

Des réalisations particulièrement préférées des composés intermédiaires de la présente invention peuvent être préparées selon les exemples qui suivent. La personne versée dans l'art pourra cependant facilement modifier les modes opératoires décrits ci-dessous en fonction de l'intermédiaire désiré.

<u>Exemple 250</u>

**<u>Intermédiaire 1</u>** :

1,2,3,4-tétrahydro-3-benzyl-6-bromo-4-oxo-2-thia-quinazoline à partir de l'acide 5-bromo anthranilique.

Dans un réacteur muni d'une agitation, d'un réfrigérant et d'une ampoule à brome sont placés 150 g (694 mmol) d'acide 5-bromo-2-amino-benzoïque en suspension dans 1,5 1 d'acide acétique.
Sous agitation, le mélange est chauffé à reflux, puis 92 ml (103 g ; 694 mmol) d'isothiocyanate de benzyle sont additionnés lentement et régulièrement par l'ampoule à brome.
Après fin de l'addition, l'agitation et le chauffage à reflux sont maintenus pendant 6 heures ; durant cette période, la solubilisation se fait progressivement.
Après refroidissement, jusqu'à température ambiante le solide qui a précipité est filtré et lavé à l'acide acétique.
Le produit obtenu est séché sous vide, à 60° C, pour donner 125,2 g du composé attendu, pur en CCM (solvant d'élution : CH2C12 99,2 / CH3OH 0,8 ; Rf = 0,9)
Rendement = 52 %

Les spectres RMN $^1H$ et $^{13}C$ sont compatibles avec la structure attendue.

Exemple 251

**Intermédiaire 2 :**

3, 4-Dihydro-3-benzyl-6-bromo-2-hydrazino-quinazolin-4-one.

Dans un réacteur muni d'une agitation et d'un réfrigérant sont placés 125,2 g (360 mmol) de 1,2,3,4-tétrahydro-3-benzyl-6-bromo-4-oxo-2-thia-quinazoline (Intermédiaire 1) en suspension dans 3,5 l d'éthanol.
Sous agitation, on additionne 167,6 g (3.348 mmol) d'hydrate d'hydrazine.
La suspension obtenue est chauffée à reflux pendant 18 heures, pendant lesquelles le passage en solution se fait progressivement.
Après refroidissement à la température ambiante, la moitié environ du solvant est évaporée sous vide et la solution résiduelle obtenue est abandonnée dans un bain de glace pendant 1 heure.

Après filtration du précipité, lavage à l'éthanol froid puis séchage sous vide à 60° C, on obtient 89,7 g du composé attendu, pur en CCM (solvant d'élution : CH2C12 99 / CH3OH 1 ; Rf=0.1)
Rendement = 72 %
les spectres RMN $^{1}H$ et $^{13}C$ sont compatibles avec la structure attendue.

Exemple 252

**Intermédiaire 3 :**

4-benzyl-7-chlore-1-mercapto-4H-[1,2,4J triazolo [4, 3-a] quinazoline -5-one

Dans un réacteur muni d'une agitation et d'un réfrigérant, on met 47,7 g (158 mmol) de 3,4-dihydro-3-benzyl-6-chloro-2-hydrazino-quinazolin-4-one (préparée de façon similaire à l'intermédiaire 2) en solution dans 600 ml de pyridine.
On additionne alors 25,3 g (158 mmol) de xanthogenate de potassium par fractions, la solution obtenue est chauffée à reflux pendant 7 heures, sous agitation, au cours desquelles un solide précipite progressivement.
Après repos à température ambiante pendant une nuit, le précipité est séparé par filtration puis redissous dans 1,5 litre d'eau.
La solution obtenue est neutralisée par de i'acide acétique, puis le précipité formé est filtré, lavé à l'eau jusqu'à pH neutre et séché.
On obtient 54,0 g de produit brut qui sera utilisé tel quel pour l'étape suivante.
Rendement ≈ 100 %

Exemple 253

**Intermédiaire 4 :**

4-benzyl-7-chloro-1-méthylthio-4H-[1,2,4]triazolo [4, 3-a] quinazolin-5-one.

Dans un réacteur muni d'une agitation et d'une ampoule à brome, on place une solution de 6,72g de soude dans 1200 ml d'eau puis additionne 57,0 g (166 mmol) de 4-benzyl-7-chloro-1-mercapto-triazolo [4, 3-a] quinazolin-5-one (Intermédiaire 3).
Sous agitation, on additionne 15,74 ml (166 mmol) de sulfate de diméthyle, à température ambiante, sur une période de 30 minutes. L'agitation est maintenue pendant 7 heures.
Après abandon à température ambiante pendant une nuit, le précipité est filtré, lavé à l'eau puis séché sous vide.
On obtient 51,2 g de solide brut qui est utilisé tel quel pour l'étape suivante.
Rendement = 100%

Exemple 254

**Intermédiaire 5 :** 4-benzyl-1,7-dichloro-4H-[1,2,4] triazolo [4, 3-a] quinazolin-5-one.

Dans un réacteur muni d'une agitation, d'un tube plongeant et d'un réfrigérant, on place 51,0 g (143 mmol) de 4-benzyl-7-chloro-1-méthylthio-triazolo [4, 3-a] quinazolin-5-one (Intermédiaire 4) dans un mélange de 1,5 l de chloroforme et 0,9 l d'eau.

Sous agitation, on refroidit à 0° C, puis fait passer un courant de chlore, en maintenant la température inférieure à 10° C, pendant 2 heures.
On interrompt alors l'arrivée de chlore, laisse le mélange revenir à la température ambiante puis maintient l'agitation pendant 2 heures.
Les 2 phases sont séparées par décantation, la phase chloroformique est séchée sur Na2SO4 et concentrée sous vide.
On obtient 50,9 g de résidu solide brut. Celui-ci est mis en suspension dans 400 ml d'éthanol et le mélange hétérogène est agité pendant 30 minutes. L'insoluble est filtré, lavé à l'éthanol et séché à 50° C sous vide pour donner 46,5 g du composé attendu, pur en CCM (solvant d'élution : CH2C12 99 / CH3OH 1 ; Rf = 0,50)
Rendement = 94 %
Les spectres RMN du proton et du $^{13}C$ sont compatibles avec la structure attendue.

Exemple 255

**Intermédiaire 6 :** 4-benzyl-7-bromo-4H-[1,2,4] triazolo [4, 3-a] quinazolin-5-one.

Dans un réacteur de 6 litres, muni d'une agitation, on place 89,7 g (260 mmol) de 3,4-dihydro-3-benzyl-6-bromo-2-hydrazino-quinazolin-4-one (Intermédiaire 2) en suspension dans 2,91 de chloroforme sec.

On agite, refroidit la suspension à 0° C au moyen d'un bain de glace, puis additionne 216 ml (192,5 g; 1.299 mmol) d'orthoformiate de triéthyle, ce qui entraîne une légère augmentation de température (jusqu'à 6° C).
En maintenant la température en dessous de 5° C, on ajoute en une coulée 8,2 ml d'acide sulfurique concentré. On agite ensuite pendant 15 mn à température < 5° C, puis ôte le bain de glace ; l'agitation est maintenue pendant 4 heures supplémentaires pendant lesquelles un solide précipite progressivement.
On additionne 1,5 l d'eau et 0,7 l de chloroforme, agite jusqu'à complète répartition entre les 2 phases puis neutralise la phase aqueuse à pH7 par du bicarbonate de sodium.
La phase organique est décantée, lavée avec une solution saturée en NaCl, séchée ($Na_2SO_4$) et évaporée sous vide pour donner 91,3 g du composé attendu, pur en CCM (solvant d'élution : CH2C12 97 / CH3OH 3 / NH4OH 0,3 ; Rf = 0,5).
Rendement = 99 %
PF (Tottoli) = 237° C
Les spectres RMN $^1H$ et $^{13}C$ sont compatibles avec la structure attendue.

Exemple 256

**Intermédiaire 7 :**

4-benzyl-1, 7-dibromo-4H-[1,2,4] triazolo [4, 3-a] quinazolin-5-one.

Dans un réacteur de 3 litres équipé d'une agitation, d'un réfrigérant et d'une ampoule à brome, on place 35 g (98,5 mmol) de 4-benzyl-7-bromo-4H-[1,2,4] triazolo [4,3-a]quinazoline-5-one (Intermédiaire 6) en suspension dans 630 ml de chloroforme et 11 ml de pyridine.
Sous agitation, 16,4 ml (320 mmol) de brome sont ensuite additionnés à température ambiante, sur une période de 30 minutes.
Après fin de l'addition, l'agitation à température ambiante est maintenue pendant 1 heure ; le milieu réactionnel est alors partagé entre 1 1 d'eau et 1,5 1 de chloroforme et le mélange hétérogène agité pendant 15 mn.
L'insoluble est essoré, lavé à l'eau jusqu'à pH neutre puis trituré dans l'éthanol.
Après séchage sous vide, à 50 ° C, on obtient une première fraction de 8,2 g du composé attendu pur en CCM (solvant d'élution : CH2C12 99/ CH3OH 1 ; Rf= 0,6).
Après séparation de la phase chloroformique, lavage avec une solution de bicarbonate de sodium puis avec de l'eau, séchage ($Na_2SO_4$), évaporation du solvat sous vide puis trituration du résidu dans de l'éthanol, filtration et séchage du solide, à 50° C, on obtient 33,1 g d'une seconde fraction du composé attendu, équivalent en CCM à la fraction précédente.
Rendement total (des 2 fractions) = 96%
Le spectre RMN $^1H$ est compatible avec la structure attendue.

Exemple 257

**Intermédiaire 8 :** 1-Axepanyl-4H-[1,2,4] triazolo[4,3-a] quinazolin-5-one

Dans un ballon de 150 ml muni d'une agitation et d'un réfrigérant, on dissout 1,0 g (2,68 mmol) de 1-Azepanyl-4-benzyl-4H-[1,2,4] triazolo[4,3-a] quinazolin-5-one dans 60 ml de tétrahydrofuranne.
On additionne 2,0 g de formiate d'ammonium puis 1,5 g de palladium activé à 10 % sur charbon.
Le mélange est agité et chauffé au reflux du solvant pendant 5 heures.
Après refroidissement, la suspension est filtrée puis le solvant évaporé sous vide pour donner 0,55 g de solide résiduel.
Celui-ci est chromatographié sur colonne de silice avec élution au mélange $CH_2$ $Cl_2$ 97 / $CH_3$ OH 3 ; les fractions pures en CCM sont regroupées et concentrées sous vide pour fournir 0,42 g de résidu solide.
Rendement = 55%
F (Tottoli) = 222 - 224°C
CCM ($CH_2$ $Cl_2$ 95 / $CH_3$ OH 5) : Rf = 0,4
R.M.N.$^1$H δ (ppm) : 1,65-1,85 (m, 8H) ; 3,25 (m, 4H) ; 7,5 (t,1H) ; 7,9 (t, 1H); 8,15 (d, 1H) ; 8,3 (d,1H) ; 12,6 (m, 1H)
Solvant : DMSO

Les composés (I ; R = H) des exemples 258 à 262 (tableau 8) sont préparés selon le procédé de l'exemple 257.

## TABLEAU 8

| N° Composé | X1 | NR4R5 | Rdt (%) | PF (°C) |
|---|---|---|---|---|
| 258 | 7-Br | 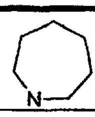 | 96 | >290 |
| 259 | 8-CH3 | 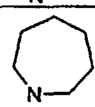 | 64 | - |
| 260 | 8- 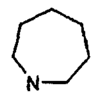 | 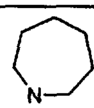 | 75 | - |
| 261 | 7-Br | 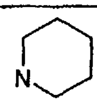 | 89 | >300 |
| 262 | 7-Br | 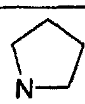 | 90,5 | >300 |

Exemple 263

**<u>Intermédiaire 9</u> :** 1-Azepanyl-7-chloro-4H-[1,2,4] triazolo[4,3-a] quinazolin-5-one

10,0 g de 1-Azepanyl-4-benzyl-7-chloro-4H-[1,2,4]triazolo[4,3-a]quinazolin-5-one (24,5mmol) puis 19,6 g (147 mmol) de chlorure d'aluminium sec sont placés en suspension dans 200 ml de benzène anhydre.
La suspension est agitée et chauffée à 50° C, à l'abri de l'humidité.
Après 1 heure 30, on laisse refroidir, additionne de la glace au mélange réactionnel puis agite vigoureusement le mélange pendant 30 minutes.
Le précipité obtenu est essoré, lavé à l'eau jusqu'à neutralité et séché à 50°C pour donner 7,5 g de solide pur en CCM.
Rendement = 96 %
F (Tottoli) : >300°C
CCM ($CH_2$ $Cl_2$ 95 / $CH_3$ OH 5) : Rf = 0,35
R.M.N.$^1$H δ (ppm) : 1,65-1,9 (m, 8H); 3,3 (m, 4H); 7,95 (d, 1H); 8,05 (s, 1H); 8,3 (d, 1H); 12,8 (m,1H)

## TABLEAU 9

| N° Composé | X1 | NR4R5 | PF (°C) |
|---|---|---|---|
| 264 | H | 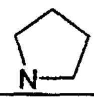 | 283 |
| 265 | 7-CH3 | 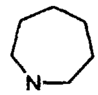 | 298 |
| 266 | 7-CH3 | 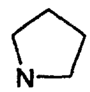 | >300 |
| 267 | 7-CH3 | 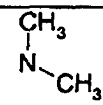 | - |
| 268 | 7-OH | 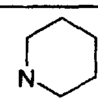 | 295 |
| 269 | 7-CN | 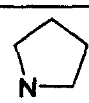 | >300 |
| 270 | 7-CN | 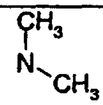 | - |

Exemple 271

**<u>Intermédiaire 10</u>:** 1-amino-4-benzyl-7-bromo-4H-[1,2,4] triazolo[4,3-a] quinazolin-5-one

Dans un réacteur de 500ml muni d'un système d'agitation, d'un réfrigérant équipé d'une garde à potasse, d'un thermomètre plongeant et d'une arrivée d'azote, on place 5,0g (14,5 mmol) de 3, 4-dihydro-3-benzyl-6-bromo-2-hydrazino-quinazolin-4-one (préparé suivant l'exemple XX) en suspension dans 150 ml de méthanol sec. On additionne 1,62g (15,3 mmol) de bromure de cyanogène et agite le mélange hétérogène pendant 1 heure à température ambiante, puis à reflux pendant 5 heures. Après refroidissement, on ajoute goutte à goutte, sous bonne agitation, une solution aqueuse saturée de bicarbonate de sodium jusqu'à pH 8. Le solide insoluble est filtré, lavé plusieurs fois avec de l'eau et séché sous vide pour donner 4,9g de produit brut.
Celui-ci est trituré dans 100 ml de méthanol, la fraction insoluble est séparée par filtration, lavée au méthanol et séchée sous vide. On obtient 4,6g de produit pur en CCM. Les spectres RMN $^1H$ et $^{13}C$ sont compatibles avec la structure attendue.
Rendement = 86,5 %
F (Tottoli) = 287°C
CCM($CH_2Cl_2$ 95 / $CH_3$ OH 5) : Rf= 0,5

**<u>Evaluation de l'activité *in vitro* des composés préférés de l'invention</u>**

**Inhibition de la phosphodiestérase**

La capacité des composés de formule (I) de l'invention à inhiber les phosphodiestérases des nucléotides cycliques est évaluée par la mesure de leur $CI_{50}$ (concentration nécessaire pour inhiber 50 % de l'activité enzymatique).
Les phosphodiestérases de type 4 sont obtenus à partir d'une préparation cytosolique extraite d'une lignée cellulaire d'origine humaine U937 selon la méthode adaptée de T.J. Torphy et al., 1992, J.Pharm.Exp. Ther. <u>263</u> : 1195-1205
Les autres types de phosphodiestérases sont obtenus lors d'une purification partielle par FPLC sur colonne Mono Q

(anion exchange column ) selon une méthode adaptée de **Lavan B. E., Lakey T., Houslay M. D.** Biochemical Pharmacology, 1989, 38(22), 4123-4136.,et de **Silver P.J** et al., 1988, Eur.J. Pharmacol. 150 : 85-94, soit, à partir de lignées cellulaires d'origine humaine pour PDE 1 (lignée monocytaire TPH1) et PDE5 (lignée issue d'un adénocarcinome MCF7), soit à partir d'aorte de chien pour PDE 3, soit, pour la PDE3A humaine, à partir d'un clonage de gènes dans des cellules d'insectes SF21 dans baculovirus, selon la méthode adaptée de Luckow, V. A. and al., 1991 in Recombinant DNA Technology&Applications.,eds. Prokop, Bajpai,R.K.&Ho,C.S., pp97-152.

La mesure de l'activité enzymatique des différents types de PDE, et en particulier des PDE 4, est faite selon une méthode adaptée de W.J. Thompson et al. 1979, Advances in Cyclic Nucleotide Research, Vol. 10 : 69-92, ed. G. Brooker et al. Raven Press, NY.

Pour la détermination de la $CI_{50}$, factivité enzymatique est mesurée en présence de l'inhibiteur dans une gamme de concentrations de 0,1 à 100µM.

Le tableau suivant illustre l'activité inhibitrice de PDE4 sur une préparation d'enzyme obtenue à partir de la lignée U937.

| N° Composé | $IC_{50}$ (µM) | N° composé | $IC_{50}$ (µM) | N° composé | $IC_{50}$ (µM) |
|---|---|---|---|---|---|
| 1 | 0,054 | 59 | 0,090 | 190 | 0,19 |
| 3 | 0,079 | 60 | 0,050 | 218 | 0,048 |
| 11 | 0,080 | 61 | 0,011 | 223 | 0,012 |
| 13 | 0,060 | 62 | 0,053 | 224 | 0,075 |
| 20 | 0,04 | 75 | 0,078 | 227 | 0,028 |
| 22 | 0,41 | 76 | 0,070 | 229 | 0,080 |
| 32 | 0,053 | 78 | 0,038 | 230 | 0,002 |
| 34 | 0,056 | 79 | 0,14 | 231 | 0,00027 |
| 35 | 0,020 | 80 | 0,073 | 233 | 0,18 |
| 37 | 0,015 | 81 | 0,016 | 234 | 2,69 |
| 40 | 0,014 | 83 | 0,012 | 239 | 0,005 |
| 41 | 0,018 | 85 | 0,041 | 240 | 0,013 |
| 42 | 0,024 | 89 | 0,027 | 242 | 0,011 |
| 43 | 0,030 | 92 | 0,030 | 243 | 0,028 |
| 44 | 0,090 | 94 | 0,029 | 246 | 0,041 |
| 46 | 0,090 | 96 | 0,058 | | |
| 47 | 0,050 | 98 | 0,029 | | |

(suite)

| N° Composé | $IC_{50}$ (µM) | N° composé | $IC_{50}$ (µM) | N° composé | $IC_{50}$ (µM) |
|---|---|---|---|---|---|
| 48 | 0,025 | 102 | 0,060 | | |
| 49 | 0,080 | 103 | 0,039 | | |
| 50 | 0,035 | 104 | 0,077 | | |
| 51 | 0,027 | 164 | 0,090 | | |
| 52 | 0,030 | 186 | 0,090 | | |
| 57 | 0,014 | 189 | 0,078 | | |

L'examen des résultats du tableau ci-dessus montre que les produits préférés de l'invention testés dans l'essai inhibent l'enzyme PDE4 in vitro de manière efficace.

Inhibition de la production du TNFα par des leucocytes humains stimulés par du lipopolysaccharide

Ce test a pour but d'évaluer la capacité des composés de l'invention à inhiber la production de TNFα (tumor necrosis-α) par des leucocytes humains en présence d'une haute concentration de sérum humain (75%). En effet, il est apparu que nombre de composés ayant la capacité d'inhiber la phosphodiestérase 4 dans des tests enzymatiques ou cellulaires ne présentent plus cette capacité lorsque le test est effectué dans du sang humain. Le test décrit ici est basé sur l'utilisation de leucocytes humains cultivés dans 75% de sérum humain. Il a été préalablement documenté que ces conditions miment la situation observée lorsque le dosage du TNFα est effectué dans du sang humain.

Les composés à tester sont dissous à 20 mM (parfois 6 mM) dans du DMSO. 100 µl de DMSO sont distribués dans 7 puits d'une microplaque à 96 puits (puits B à H). 150 µl de la solution de composés sont distribués dans les puits de la ligne A. 50 µl sont alors séquentiellement transférés 7 fois. 20 µl de ces dilutions sérielles de composés sont transférés séquentiellement deux fois dans des puits contenant 180 µl de RPMI 1640 (Gibco). 50 µl de ces dilutions sont alors transférés dans des puits où seront ajoutées les cellules.

Chaque test comprend une série de huit puits sans LPS (100% d'inhibition), huit puits avec LPS (0% d'inhibition) et une série de dilutions de Rolipram afin de pouvoir comparer les tests entre eux et ainsi évaluer leur variabilité.

Une ampoule de leucocytes est décongelée au bain-marie (37°C), son contenu est transféré dans un tube de 15 ml contenant 10 ml de RPMI additionné de 5% de sérum humain (RPMI-5% HS). Les cellules sont sédimentées (800 g, 6 minutes, 4°C), reprises dans 10 ml du même milieu et comptées par dilution dans une solution de Bleu de Trypan. Après centrifugation (800 g, 6 minutes, 4°C), les cellules sont reprises à 2 x $10^6$/ml dans du sérum humain.

A 50 µl des différentes dilutions de composés, 100 µl de cellules sont ajoutés. Les plaques sont alors incubées 30 minutes à 37°C, puis 50 µl d'une solution 4 µg/ml de LPS préparée dans du sérum humain sont ajoutés. Les plaques sont incubées pendant la nuit à 37°C.

Après incubation de 15-18 heures, 90 µl de surnageant de culture sont prélevés et transférés dans des puits de microplaque à fonds ronds. La présence de TNFα est alors évaluée par ELISA (Pharmingen) en utilisant 50 µl de surnageant. Le protocole décrit par le fabricant est strictement appliqué.

Les résultats obtenus pour certains des composés préférés de la présente invention sont illustrés dans le tableau suivant.

| Composé | Inhibition (leucocytes humains) $IC_{50}$ µM |
|---|---|
| 3 | 3,4 |
| 104 | 8,1 |
| 94 | 6,3 |
| 101 | 8,6 |
| 85 | 6,8 |
| 98 | - |
| 79 | 5,2 |
| 91 | - |
| 93 | 4,3 |

(suite)

| Composé | Inhibition (leucocytes humains) $IC_{50}$ μM |
|---|---|
| 103 | 10,7 |
| 46 | - |
| 35 | - |

## Evaluation de l'activité *in vivo* des composés de l'invention

### Modèle TNFα *in vivo* chez le rat Wistar

Le TNFα est une cytokine jouant un rôle central dans les mécanismes de l'inflammation.

Sa production peut être induite par une injection de lipopolysaccharide (LPS). Il a été montré que l'augmentation d'AMPc intracellulaire, produite notamment par les inhibiteurs de PDE4, dimininue la production de TNFα dans des modèles *in vitro* et *in vivo.* Il s'agit donc ici, de quantifier *in vivo*, le potentiel anti-inflammatoire des composés de l'invention, administrés par voie orale (p.o.) en mesurant l'inhibition de la production de TNFα dans le plasma de rats, ceux-ci ayant reçu une injection intrapéritonéale (i.p.) de lipopolysaccharide (LPS). Le traitement par les composés de l'invention ou le véhicule sont administrés par voie orale à des rats mâles Wistar, 30 min. avant l'injection de LPS. Les rats sont sacrifiés 90 min. après la stimulation par le LPS, le sang est recueilli sur EDTA et la concentration en TNFα est mesurée dans chaque échantillon de plasma. Les résultats obtenus sur certains des composés de la présente invention sont présentés dans le tableau ci-dessous.

| Composé | % Inhibition à 10 mg/kg |
|---|---|
| 3 | - 98 % |
| 104 | - 94 % |
| 94 | - 87 % |
| 101 | - 80 % |
| 85 | - 77 % |
| 98 | - 75 % |
| 79 | - 72 % |
| 91 | - 70 % |
| 93 | - 67 % |
| 103 | - 64 % |
| 46 | - 58 % |
| 35 | - 51 % |

Références

Chen, Y. L., Le Vraux, V., Giroud, J. P. and Chauvelot-Moachon L. (1994). Anti-tumor necrosis factor properties of non-peptide drugs in acute-phase responses. Eur. J. Pharmacol., 271 (2-3), 319-27.

Prabhakar, U., Lipshutz, D., O'Leary Barthus, J., Slivjak, J., Smith III E. F., Lee, J. C. and Esser K. M. (1994). Characterization of cAMP-dependent inhibition of LPS-induced TNFa production by rolipram, a specific phosphodiesterase IV (PDE IV) inhibitor. Int. J. Immunopharmacol., 16 (10), 805-816.

### Modèle d'éosinophilie chez le rat

Les études conduites à partir de ce modèle expérimental ont pour but d'évaluer l'action inhibitrice des composés de l'invention sur l'afflux de cellules inflammatoires et en particulier d'éosinophiles dans la lumière de l'arbre trachéo-bronchique de rat. Les éosinophiles jouent un rôle majeur dans la physiopathologie de l'asthme chez l'homme en libérant au niveau du parenchyme pulmonaire des médiateurs proinflammatoires comme les leucotriènes, des protéines et enzymes spécifiques (ECP, EPO, MBP) et des cytokines. Le recrutement massif de ce type cellulaire au niveau des voies aériennes de l'asthmatique conduit à une dégradation progressive du tissu pulmonaire expliquant l'hyperreactivité bronchique, l'aspect chronique de la maladie et les exacerbations en l'absence de traitement. Ce

modèle utilise des rats Brown Norway, dont la particularité est de produire, comme chez les patients atopiques, des taux d'immunoglobuline E (IgE) en réponse à une sensibilisation par un antigène. Le protocole utilisé fait intervenir deux sensibilisations à l'ovalbumine à quatorze jours d'intervalle puis un challenge sept jours plus tard avec un aérosol d'ovalbumine. 48 heures après le challenge antigénique, les animaux subissent un lavage bronchoalvéolaire sous anesthésie afin de recueillir l'infiltrat de cellules inflammatoires au niveau des poumons. Ces cellules sont ensuite comptées et différenciées selon des critères morphologiques. Les produits de l'invention sont administrés par voie orale, 1 heure avant le challenge antigénique. La plupart des composés préférés de la présente invention testés dans ce modèle ont également démontré une excellente activité.

Références

Corrigan et al. (1992) Immunology today 13 : 501-507
Elwood et al. (1995) Inflamm Res 44 : 83-86

**Modèle de neutrophilie chez la souris**

Les études conduites à partir de ce protocole expérimental ont pour but d'évaluer l'action modulatrice des composés de l'invention sur l'afflux de cellules pro-inflammatoires (phase précoce) dans la lumière de l'arbre trachéo-bronchique de souris. Cet afflux cellulaire est consécutif à une stimulation mimant une infection bactérienne (Lipopolysaccharide bactérien ou LPS). Ce stade précoce inflammatoire est le résultat d'une combinaison d'événements dont les principaux sont la synthèse et libération de facteurs stimulants (TNFαi) et chimiotactiques (IL-8ii), l'accroissement de la perméabilité vasculaire au niveau de la micro-circulation trachéo-bronchique et l'infiltration de polynucléaires neutrophilesiii concomitante à l'exsudation des protéines plasmatiques dans les tissus pulmonaires.
Ce processus pathologique est retrouvé dans la broncho-pneumopathie chronique obstructive (ou COPD en anglais) où le neutrophile, de concert avec le macrophage, joue un rôle clé dans la mise en place de l'amplification du recrutement des neutrophiles, eux-mêmes, mais aussi dans la déstructuration des tissus pulmonaires (déclin des fonctions pulmonaires), l'hypersécrétion de mucus trachéo-bronchique (engorgement des voies aériennes), l'inflammation tissulaire (libération de médiateurs inflammatoires et de radicaux libres) et accroissement du tonus basal des fibres musculaires lisses pulmonaires (gène respiratoire chronique). Certains de composés des exemples ont démontré une activité dans ce modèle.

Références

i SUTER P.M., SUTER S., GIRARDIN E., ROUX-LOMBARD P., GRAU G.E. and DAYER J.-M. 1992. High bronchoalveolar levels of tumor necrosis factor and its inhibitors, interleukin-1, interferon and elastase, in patients with adult respiratory distress syndrome after trauma, shock or sepsis. Am. Rev. Respir. Dis. 145: 1016-1022.
ii MARTIN T.R. and GOODMAN R.B. 1999. The role of chemokines in the pathology of the acute respiratory distress syndrome. Chapter 6 in Chemokines in disease: Biology and clinical research edited by: C.A. Hébert, Humana Press Inc., Totowa, NJ.
iii REPINE J.E. and BEEHLER C.J. 1991. Neutrophils and the adult respiratory distress syndrome: two interlocking perspectives. Am. Rev. Respir. Dis. 144: 251-252.

**Références**

Barad, M. et al., PNAS, 1998, Vol. 95(25), p. 15020-15025
Belayev, L. et al., Brain Res., 1998 March 23, Vol. 787(2), p. 277-285
Block, F. et al., Neuroreport, 1997 Decemberl, Vol. 8(17), p. 3829-3832
Egawa, T. et al., Jon J. Pharmacol., 1997 November, Vol. 75(3), p. 275-281
Gonccalves de Moraes, V.-L. et al., Br. J. Pharmacol., 1998 February, Vol. 123(4), p. 631-636
Hasko, G. et al., Eur. J. Immunol., 1998 February, Vol. 28(2), p. 468-472
Herzer, W.-A. et al., J. Cardiovasc. Pharmacol., 1998, Vol. 32(5), p. 769-776
Itoh, A. et al., Methods and Findings in Exp. and Clin. Pharm., 1998, Vol. 20(7), p. 619-625
Kim, O. H., Lerner A., Blood, 1998 October 1, Vol. 92(7), p. 2484-2494
Lelkas, Z. et al., Pharmacol. Biochem. Behav., 1998 August, Vol. 60(4), p. 835-839
Liang, L. et al., Diabetes, 1998 April, Vol. 47(4), p. 570-575
Merz, K.-H. et al., J. Med. Chem., 1998 November 19, Vol. 41(24), p. 4733-4743

Miotta, J.-M. et al., Am. J. Respir. Cell. Mol. Biol., 1998 March, Vol. 18(3), p. 411-420

## Revendications

**1.** Triazolo [4,3-a] quinazoline-5-ones et/ou -5-thiones de formule I ou II :

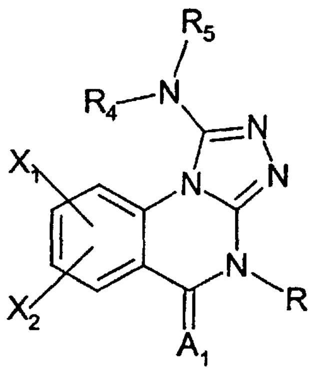

I

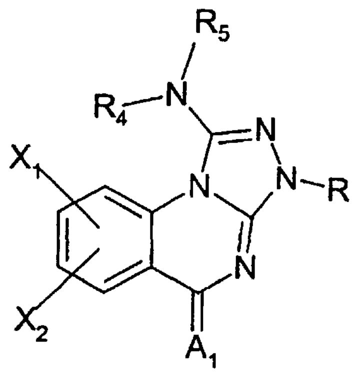

II

I et II étant des isomères de position du groupe R sur les azotes 3 ou 4, dans lesquelles :

- $A_1$ est O ou S ;
- $X_1$ et $X_2$, semblables ou différents, représentent :
    - hydrogène, hydroxy, halogène, amino, nitro, mercapto, cyano, carboxy,
    - alkyle inférieur, alcoxy inférieur ou $-S(O)_mR_8$ dans lequel m est 0, 1 ou 2 et $R_8$ est un alkyle inférieur, éventuellement substitués par un ou plusieurs atomes halogènes,
    - $-CO-Q_1-Q_2-Q_3$ dans lequel :
        - $Q_1$- est : une liaison de valence simple, -O-,

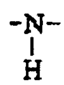 , 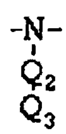 , ou 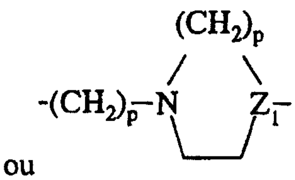 ,

où p est un nombre entier pouvant varier de 0 à 3, et $Z_1$ est CH, N, O ou S,

- $Q_2$- est :

    a) $-(CH_2)_q-$, q étant égal à 0, 1, 2, 3, ou 4, ou
    b) $-(CH_2-CH_2-O)_r-$, r étant égal à 2, 3, ou 4, et

- $Q_3$ est : -H, -OH, alkoxy inférieur, -O-CO- $X_3$ -$NHX_3$ ou

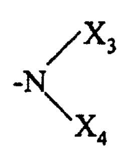

dans lequel $X_3$ et $X_4$, semblables ou différents, représentent un groupement alkyle inférieur, $X_3$ et $X_4$ pouvant être liés pour former un cycle, comprenant un ou plusieurs hétéroatomes choisis parmi O, S ou N,

- $-NH-R_1$ dans lequel $R_1$ représente un groupement alkyle inférieur, éventuellement substitué par un ou plusieurs groupements choisis parmi halogène, hydroxy, cyano, alcoxy inférieur ou $-CO-Q_1-Q_2-Q_3$, ou
- $-NR_2R_3$ dans lequel $R_2$ et $R_3$, semblables ou différents, représentent un alkyle inférieur, éventuellement substitué par un ou plusieurs groupements hydroxy, halogène, cyano, alcoxy inférieur ou $-CO-Q_1-Q_2-Q_3$, $R_2$ et $R_3$ pouvant être liés pour former un cycle, comprenant un ou plusieurs hétéroatomes choisis parmi O, S ou N et éventuellement ponté par un alkyle inférieur, gem dialkylé ou substitué par un ou plusieurs groupements choisis parmi hydroxy, kéto, alkyle inférieur, alcoxy inférieur ou $-CO-Q_1-Q_2-Q_3$ ;

- R représente :

  - alkyle inférieur, alcényle inférieur, alcynyle inférieur, aryl alcynyle, 2-, 3- ou 4-pyridylalkyle éventuellement substitué par un alkyle inférieur, un alcoxy inférieur, un

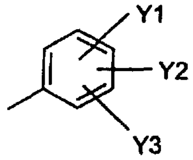
,
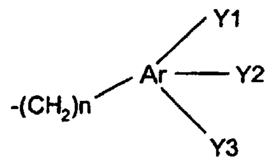
,
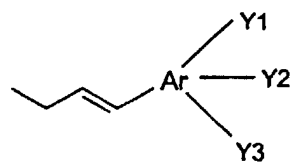

ou

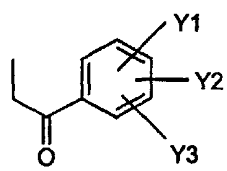

groupement hydroxy, halogène ou amino,

dans lesquels :

  - n est un nombre entier de 1 à 5,
  - Ar est un cycle aromatique comprenant 5 ou 6 atomes incluant de 0 à 3 hétéroatomes choisis parmi O, S ou N.
  - Y1, Y2 et Y3, semblables ou différents représentent :
  - hydrogène, hydroxy, mercapto, amino, nitro, halogène, $NHR_1$, $NR_2R_3$, $-(CH_2)_S-CN$, $-(CH_2)_SCO-Q_1-Q_2-Q_3$ dans lesquels s est un nombre entier de 0 à 6 ;
  - alkyle inférieur, alcoxy inférieur ou $-S(O)_mR_8$ dans lequel m est 0, 1 ou 2 et $R_8$ est un alkyle inférieur, chacun pouvant être éventuellement substitué par un ou plusieurs atomes halogènes ; et

- $R_4$ et $R_5$, représentent :

  - alkyle inférieur lorsque R4 et R5 sont semblables, aralkyle, cycloalkyle ou cycloalkyl alkyle, lorsque $R_4$ et $R_5$ sont différents,
  - alkyle inférieur, $R_4$ et $R_5$ pouvant être liés pour former un cycle saturé ou comportant une ou plusieurs doubles liaisons comprenant un ou plusieurs hétéroatomes choisis parmi O, S ou N et éventuellement substitué par un alkyle inférieur, un hydroxy ou un alkoxy inférieur ou ponté par un alkyle inférieur, gem dialkylé ou substitué par un ou plusieurs groupements choisis parmi hydroxy, kéto, alkyle inférieur, alcoxy inférieur, phényle alkyle ou $CO-Q_1-Q_2-Q_3$, deux des atomes du cycle ainsi formé pouvant également faire partie d'un autre cycle choisi parmi phényle ou hétéroaryle comportant de 4 à 8 atomes incluant 1 à 4

hétéroatomes ;

éventuellement leurs formes racémiques et leurs isomères, ainsi que leurs sels pharmaceutiquement acceptables.

**2.** Triazolo [4,3-a] quinazoline-5-ones et/ou -5-thiones selon la revendication 1, dans lesquelles :

$A_1$ représente un atome d'oxygène ;
$X_1$ représente un atome d'hydrogène et $X_2$ est un groupement halogène, amino, alkyle inférieur, hydroxy ou $-NHR_2$, $R_1$ étant tel que défini précédemment.
R représente :

- un groupement alkyle inférieur, alcényle inférieur, aryl alcynyle, 2-, 3- ou 4-pyridylalkyl éventuellement substitué sur le noyau pyridine par un alkyle inférieur, un halogène ou un hydroxy ;

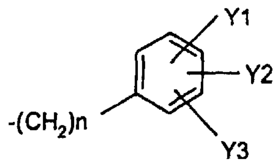

dans lequel :

- n est un nombre entier de 1 à 3,
- Y1, Y2 et Y3 représentent chacun un atome d'hydrogène ou un groupement alcoxy inférieur, plus particulièrement méthoxy,
- Y1 et Y2 représentent chacun un atome d'hydrogène et Y3 représente un groupement alcoxy inférieur, un groupement amino, $NHR_1$, $NR_2R_3$, nitro, hydroxy, un groupement $-(CH_2)_SCO-Q_1-Q_2-Q_3$, un groupement $(CH_2)_S-CN$

dans lesquels s, $Q_1$, $Q_2$, $Q_3$ sont tels que définis précédemment, ou un groupement alkyle inférieur éventuellement substitué par un ou plusieurs atomes d'halogène, la position particulièrement préférée du substituant Y3 étant la position 4, ou,

- Y1 représente un atome d'hydrogène et Y2 et Y3, semblables ou différents, représentent un groupement hydroxy, halogène ou alcoxy inférieur, ou

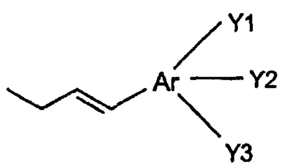

dans lequel :

- Ar est tel que défini précédemment ;
- Y1, Y2 et Y3 représentent chacun un atome d'hydrogène, ou
- Y1 et Y2 représentent chacun un atome d'hydrogène et Y3 est alcoxy inférieur ou halogène ;

$R_4$ et $R_5$, représentent :

- alkyle inférieur lorsque R4 et R5 sont semblables, aralkyle, cycloalkyle ou cycloalkyl alkyle, lorsque $R_4$ et $R_5$ sont différents,

alkyle inférieur, $R_4$ et $R_5$ pouvant être liés pour former un cycle saturé ou comportant une ou plusieurs doubles liaisons comprenant un ou plusieurs hétéroatomes choisis parmi O, S ou N et éventuellement substitué par un alkyle inférieur, un hydroxy ou un alkoxy inférieur ou ponté par un alkyle inférieur, gem dialkylé ou substitué par un ou plusieurs groupements choisis parmi hydroxy, kéto, alkyle inférieur, alcoxy inférieur, phényle alkyle ou $CO-Q_1-Q_2-Q_3$, deux des atomes du cycle ainsi formé pouvant également faire partie d'un autre cycle choisi parmi phényle ou hétéroaryle comportant de 4 à 8 atomes incluant 1 à 4 hétéroatomes .

**3.** Triazolo [4,3-a] quinazoline-5-ones et/ou -5-thiones selon la revendication 1 ou 2, dans lesquelles:

$X_1$ représente un atome d'hydrogène,
$X_2$ représente un groupement halogène, amino, alkyle inférieur, hydroxy ou $--NHR_1$;
R représente :

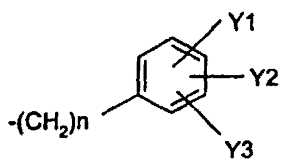

dans lequel :

- n est un nombre entier de 1 à 3,
- Y1, Y2 et Y3 représentent chacun un atome d'hydrogène ou un groupement alcoxy inférieur, plus particulièrement méthoxy et en particulier le 3, 4, 5-triméthoxy,
- Y1 et Y2 représentent chacun un atome d'hydrogène et Y3 représente un groupement alcoxy inférieur, amino, $NHR_1$, $NR_2R_3$, nitro, ou hydroxy, un groupement alkyle inférieur éventuellement substitué par un ou plusieurs atomes d'halogène, un groupement $-(CH_2)_SCO-Q_1-Q_2-Q_3$ dans lequel s est 0 ou 1, $Q_1$ est O, -NH- ou une liaison de valence, $Q_2$ est $-(CH_2)_q-$, q étant égal à 0, 1, 2, 3 ou 4 et $Q_3$ est H, OH ou $-NX_3X_4$ dans lequel $X_3$ et $X_4$ sont tels que définis précédemment, un groupement $(CH_2)_S-CN$ dans lequel s est 0 ou 1, la position particulièrement préférée du substituant Y3 étant la position 4, ou
- Y1 représente un atome d'hydrogène et Y2 et Y3, semblables ou différents, représentent un groupement hydroxy, halogène ou alcoxy inférieur; ou

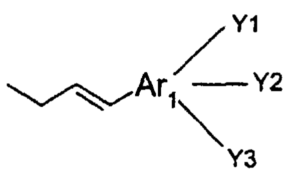

dans lequel :

- $Ar_1$ est un cycle aromatique comprenant 6 atomes pouvant inclure un atome d'azote en position 2, 3 ou 4 et de préférence en position 3 ;
- Y1, Y2 et Y3 représentent chacun un atome d'hydrogène, ou
- Y1 et Y2 représentent chacun un atome d'hydrogène et Y3 est un groupement alcoxy inférieur ou n groupement halogène lorsque $Ar_1$ ne comprend pas d'atome d'azote ; et

$R_4$ et $R_5$, représentent :

- alkyle inférieur lorsque R4 et R5 sont semblables, aralkyle, cycloalkyle ou cycloalkyl alkyle, lorsque $R_4$ et $R_5$ sont différents,
- alkyle inférieur, $R_4$ et $R_5$ pouvant être liés pour former un cycle saturé ou comportant une ou plusieurs doubles liaisons comprenant un ou plusieurs hétéroatomes choisis parmi O, S ou N et éventuellement substitué par un alkyle inférieur, un hydroxy ou un alkoxy inférieur ou ponté par un alkyle inférieur, gem

dialkylé ou substitué par un ou plusieurs groupements choisis parmi hydroxy, kéto, alkyle inférieur, alcoxy inférieur, phényle alkyle ou $CO-Q_1-Q_2-Q_3$, deux des atomes du cycle ainsi formé pouvant également faire partie d'un autre cycle choisi parmi phényle ou hétéroaryle comportant de 4 à 8 atomes incluant I à 4 hétéroatomes .

**4.** Triazolo [4,3-a] quinazoline-5-ones et/ou -5-thiones selon l'une quelconque des revendications 1 à 3, dans lesquelles :

- le groupement halogène est choisi parmi F, Cl, Br ou I,
- le groupement alkyle inférieur est un groupement linéaire ou ramifié comportant de 1 à 6 atomes de carbone,
- le groupement alcoxy inférieur est un groupement linéaire ou ramifié comportant de 1 à 5 atomes de carbone,
- le groupement alkylthio inférieur est un groupement linéaire ou ramifié comportant de I à 5 atomes de carbone,
- le groupement alcényle inférieur comporte de 3 à 6 atomes de carbone,
- le groupement alcynyle inférieur comporte de 3 à 6 atomes de carbone,
- le groupement 2-, 3- ou 4-pyridylalkyle comprend un alkyle comprenant 1 à 5 atomes de carbone,
- le groupement aryle comporte de 5 à 8 atomes,
- le groupement aralkyle comprend un alkyle comprenant de 1 à 6 atomes de carbone,
- le groupement cycloalkyle comporte de 3 à 8 atomes de carbone,
- le groupement cycloalkyl alkyle comprend un alkyle comprenant de 1 à 6 atomes de carbone,
- les groupements alkyle inférieur, alcoxy inférieur ou alkylthio inférieur substitués par un ou plusieurs atomes halogènes sont choisis parmi les groupements $-(CH_2)_p-CF_3$, $-O-(CH_2)_p-CF_3$ ou $-S-(CH_2)_p-CF_3$, dans lesquels p est un nombre entier de 0 à 3.

**5.** Triazolo [4,3-a] quinazoline-5-ones et/ou -5-thiones selon la revendication 1, dans lesquelles :

- $A_1$ est O ou S ;
- $X_1$ et $X_2$, semblables ou différents, représentent :
  - hydrogène, hydroxy, halogène, amino, nitro, mercapto, cyano, carboxy,
  - alkyle inférieur, alcoxy inférieur ou $-S(O)_mR_8$ dans lequel m est 0, 1 ou 2 et $R_8$ est un alkyle inférieur, éventuellement substitués par un ou plusieurs atomes halogènes ;
- R représente:

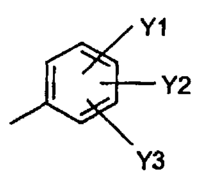

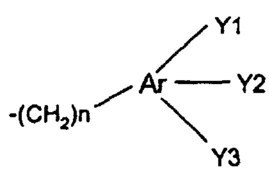

ou

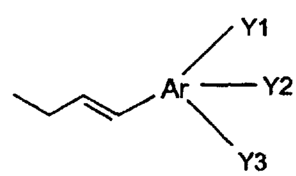

dans lesquels:

- n est un nombre entier de 1 à 5,
- Ar est un cycle aromatique comprenant 5 ou 6 atomes incluant de 0 à 3 hétéroatomes choisis parmi O, S ou N,
- Y1, Y2 et Y3, semblables ou différents représentent :
  - hydrogène, hydroxy, mercapto, amino, $NHR_1$, $NR_2R_3$,nitro, halogène, $-(CH_2)_sCO-Q_1-Q_2-Q_3$, $-(CH_2)_S-CN$ dans lesquels s est un nombre entier de 0 à 6 ;
  - alkyle inférieur, alcoxy inférieur ou $-S(O)_mR_8$ dans lequel m est 0, 1 ou 2 et $R_8$ est un alkyle inférieur, chacun pouvant être éventuellement substitué par un ou plusieurs atomes halogènes ;
- $R_4$ et $R_5$, semblables ou différents, représentent :

  alkyle inférieur, $R_4$ et $R_5$ pouvant être liés pour former un cycle saturé ou comportant une ou plusieurs

doubles liaisons comprenant un ou plusieurs hétéroatomes choisis parmi O, S ou N et éventuellement ponté par un alkyle inférieur, gem dialkylé ou substitué par un ou plusieurs groupements choisis parmi hydroxy, kéto, alkyle inférieur, alcoxy inférieur, phényle alkyle ou CO-$Q_1$-$Q_2$-$Q_3$.

**6.** Triazolo [4,3-a] quinazoline-5-ones et/ou -5-thiones selon la revendication 5, choisies parmi le groupe comprenant :

7-Bromo-1-dimethylamino-4-((E)-3-pyridin-3-yl-allyl)-4H-[1,2,4]triazolo[4,3-a]quinazolin-5-one 1-Dimethylamino-7-methyl-4-(3-pyridin-3-yl-allyl)-4H-[1,2,4]triazolo[4,3-a]quinazolin-5-one

4-(3,4-Dimethoxy-benzyl)-7-methyl-1-pyrrolidin-1-yl-4H-[1,2,4]triazolo[4,3-a]quinazolin-5-one

4-(1-Dimethylamino-7-methyl-5-oxo-5H-[1,2,4]triazolo[4,3-a]quinazolin-4-ylmethyl)-benzonitrile 7-Bromo-1-dimethylamino-4-(3-phenyl-allyl)-4H-[1,2,4]triazolo[4,3-a]quinazolin-5-one

7-Methyl-4-(3-phenyl-allyl)-1-pyrrolidin-1-yl-4H-[1,2,4]triazolo[4,3-a]quinazolin-5-one

4-(7-Bromo-1-dimethylamino-5-oxo-5H-[1,2,4]triazolo[4,3-a]quinazolin-4-ylmethyl)-benzonitrile 1-Azepan-1-yl-7-methyl-4-pyridin-3-ylmethyl-4H-[1,2,4]triazolo[4,3-a]quinazolin-5-one

4-(7-Methyl-5-oxo-1-pyrrolidin-1-yl-5H-[1,2,4]triazolo[4,3-a]quinazolin-4-ylmethyl)-benzonitrile 1-Dimethylamino-7-methyl-4-((E)-3-phenyl-allyl)-4H-[1,2,4]triazolo[4,3-a]quinazolin-5-one

4-(7-Bromo-5-oxo-1-pyrralidin-1-yl-5H-[1,2,4]triazolo[4,3-a]quinazolin-4-ylmethyl)-benzonitrile 1-Azepan-1-yl-7-bromo-4-(3,4-dimethoxy-benzyl)-4H-[1,2,4]triazolo[4,3-a)quinazolin-5-one

**7.** Triazolo [4,3-a] quinazoline-5-ones et/ou -5-thiones selon la revendication 1, 2, 3, ou 4, choisies parmi le groupe comprenant :

1-(Azepan-1-yl)-7-chloro-4-(3-phenylallyl)-4H-[1,2,4]triazolo[4,3-a]quinazolin-5-one

1-(azepan-1-yl)-7-chloro-3-(3-phenylallyl)-3H-[1,2,4]triazolo[4,3-a]quinazolin-5-one

7-Bromo-1-dimethylamino-4-((E)-3-pyridin-3-yl-allyl)-4H-[1,2,4]triazolo[4,3-a]quinazolin-5-one

7-Bromo-4-pyridin-3-ylmethyl-1-pyrrolidin-1-yl-4H-[1,2,4]triazolo[4,3-a]quinazolin-5-one

7-Bromo-3-pyridin-3-ylmethyl-1-pyrrolidin-1-yl-3H-[1,2,4]triazolo[4,3-a]quinazolin-5-one

1-Azepan-1-yl-4-(3-phenyl-allyl)-4H-[1,2,4]triazolo[4,3-a]quinazolin-5-one

1-(azepan-1-yl)-7-chloro-4-allyl-4H-[1,2,4]triazolo[4,3-a]- quinazolin-5-one

1-(azepan-1-yl)-7-chloro-4-(4-methylbenzyl)-4H-[1,2,4]triazolo[4,3-a]quinazolin-5-one

1-(azepan-1-yl)-7-chloro-4-(2-chlorobenzyl)-4H-[1,2,4]triazolo[4,3-a]quinazolin-5-one

1-(azepan-1-yl)-7-chloro-4-(3-chlorobenzyl)-4H-[1,2,4]triazolo[4,3-a]quinazolin-5-one

1-(azepan-1-yl)-7-chloro-4-(4-chlorobenzyl)-4H-[1,2,4]triazolo[4,3-a]quinazolin-5-one

1-(azepan-1-yl)-7-chloro-4-(4-bromobenzyl)-4H-[1,2,4]triazolo[4,3-a]quinazolin-5-one

1-(azepan-1-yl)-7-chloro-4-(4-fluorobenzyl)-4H-[1,2,4]triazolo[4,3-a]quinazolin-5-one

1-(azepan-1-y1)-7-chloro-4-(4-(trifluoromethyl)benzyl)-4H-[1,2,4]triazolo[4,3-a]quinazolin-5-one

1-(azepan-1-yl)-7-chloro-4-(4-cyanobenzyl)-4H-[1,2,4]triazolo[4,3-a]quinazolin-5-one

1-(azepan-1-yl)-7-chloro-4-(2-methoxybenzyl)-4H-[1,2,4]triazolo[4,3-a]quinazolin-5-one

1-(azepan-1-yl)-7-chloro-4-(3-methoxybenzyl)-4H-[1,2,4]triazolo[4,3-a]quinazolin-5-one

1-(azepan-1-yl)-7-chloro-4-(4-methoxybenzyl)-4H-[1,2,4]triazolo[4,3-a]quinazolin-5-one

1-(azepan-1-yl)-7-chloro-4-(3,4-dichlorobenzyl)-4H-[1,2,4]triazolo[4,3-a]quinazolin-5-one

1-(azepan-1-yl)-7-chloro-4-(3,4-dimethoxybenzyl)-4H-[1,2,4]triazolo[4,3-a]quinazolin-5-one

1-(azepan-1-yl)-7-chloro-4-(2-pyridylmethyl)-4H-[1,2,4]triazolo[4,3-a]quinazolin-5-one

1-(azepan-1-yl)-7-chloro-4-(3-pyridylmethyl)-4H-[1,2,4]triazolo[4,3-a]quinazolin-5-one

1-(azepan-1-yl)-7-chloro-4-(4-pyridylmethyl)-4H-[1,2,4]triazolo[4,3-a]quinazolin-5-one

1-(azepan-1-yl)-7-chloro-4-(2-phenylethyl)-4H-[1,2,4]triazolo[4,3-a]quinazolin-5-one

1-(azepan-1-yl)-7-chloro-4-[2-(4-methoxyphenyl)ethyl]-4H-[1,2,4]triazolo[4,3-a]quinazolin-5-one

1-(azepan-1-yl)-7-chloro-4-(3-phenylpropyl)-4H-[1,2,4]triazolo[4,3-a]quinazolin-5-one

1-Azepan-1-yl-7-chloro-4-(2-oxo-2-phenyl-ethyl)-4H-[1,2,4]triazolo[4,3-a]quinazolin-5-one

1-(azepan-1-yl)-7-chloro-4-[2-(4-methoxyphenyl)-2-oxoethyl]-4H-[1,2,4]triazolo[4,3-a]quinazolin-5-one

1-(azepan-1-yl)-7-chloro-4-[2-(4-chlorophenyl)-2-oxoethyl]-4H-[1,2,4]triazolo[4,3-a]quinazolin-5-one

5-[(1-(azepan-1-yl)-7-chloro-5-oxo-5H-[1,2,4]triazolo[4,3-a]- quinazolin-4-yl)acetyl]-2-methoxybenzoic acid methyl ester

7-Chloro-4-pyridin-3-ylmethyl-1-pyrrolidin-1-yl-4H-[1,2,4]triazolo[4,3-a]quinazolin-5-one

1-(azepan-1-yl)-7-bromo-4-(4-chlorophenylmethyl)-4H-[1,2,4]triazolo[4,3-a]quinazolin-5-one

1-Azepan-1-yl-7-bromo-4-(4-fluoro-benzyl)-4H-[1,2,4]triazolo[4,3-a]quinazolin-5-one

4-(1-Azepan-1-yl-7-bromo-5-oxo-5H-[1,2,4]triazolo[4,3-a]quinazolin-4-ylmethyl)-benzonitrile

1-Azepan-1-yl-7-bromo-4-(3,4-dimethoxy-benzyl)-4H-[1,2,4]triazolo[4,3-a]quinazolin-5-one

1-(azepan-1-yl)-7-bromo-4-(3-pyridinylmethyl)-4H-[1,2,4]triazolo[4,3-a]quinazolin-5-one

1-(azepan-1-yl)-7-bromo-4-(3-phenylallyl)-4H-[1,2,4]triazolo[4,3-a]quinazolin-5-one

1-Azepan-1-yl-7-bromo-4-[3-(4-chloro-phenyl)-allyl]-4H-[1,2,4]triazolo[4,3-a]quinazolin-5-one

1-Azepan-1-yl-7-bromo-4-[3-(4-methoxy-phenyl)-allyl]-4H-[1,2,4]triazolo[4,3-a]quinazolin-5-one

1-Azepan-1-yl-7-bromo-4-((E)-3-pyridin-3-yl-allyl)-4H-[1,2,4]triazolo[4,3-a]quinazolin-5-one

1-Azepan-1-yl-7-bromo-4-(3-pyridin-4-yl-allyl)-4H-[1,2,4]triazolo[4,3-a]quinazolin-5-one

7-Bromo-4-(4-methyl-benzyl)-1-pyrrolidin-1-yl-4H-[1,2,4]triazolo[4,3-a]quinazolin-5-one

7-Bromo-4-(4-chloro-benzyl)-1-pyrrolidin-1-yl-4H-[1,2,4]triazolo[4,3-a]quinazolin-5-one

7-Bromo-4-(4-fluoro-benzyl)-1-pyrrolidin-1-yl-4H-[1,2,4]triazolo[4,3-a]quinazolin-5-one

3-(7-Bromo-5-oxo-1-pyrrolidin-1-yl-5H-[1,2,4]triazolo[4,3-a]quinazolin-4-ylmethyl)-benzonitrile

4-(7-Bromo-5-oxo-1-pyrrolidin-1-yl-5H-[1,2,4]triazolo[4,3-a]quinazolin-4-ylmethyl)-benzonitrile

4-(7-Bromo-5-oxo-1-pyrrolidin-1-yl-5H-[1,2,4]triazolo[4,3-a]quinazolin-4-ylmethyl)-benzoic acid methyl ester

7-Bromo-4-(4-nitro-benzyl)-1-pyrrolidin-1-yl-4H-[1,2,4]triazolo[4,3-a]quinazolin-5-one

7-Bromo-4-(4-methoxy-benzyl)-1-pyrrolidin-1-yl-4H-[1,2,4]triazolo[4,3-a]quinazolin-5-one

Acetic acid 4-(7-bromo-5-oxo-1-pyrrolidin-1-yl-5H-[1,2,4]triazolo[4,3-a]quinazolin-4-ylmethyl)-phenyl ester

7-Bromo-4-(4-hydroxy-benzyl)-1-pyrrolidin-1-yl-4H-[1,2,4]triazolo[4,3-a]quinazolin-5-one

7-Bromo-4-(3,4-dimethoxy-benzyl)-1-pyrrolidin-1-yl-4H-[1,2,4]triazolo[4,3-a]quinazolin-5-one

4-Benzo[1,3]dioxol-5-ylmethyl-7-bromo-1-pyrrolidin-1-yl-4H-[1,2,4]triazolo[4,3-a]quinazolin-5-one

7-Bromo-4-(3,5-dimethoxy-benzyl)-1-pyrrolidin-1-yl-4H-[1,2,4]triazolo[4,3-a]quinazolin-5-one

7-Bromo-1-pyrrolidin-1-yl-4-(3,4,5-trimethoxy-benzyl)-4H-[1,2,4]triazolo[4,3-a]quinazolin-5-one

[4-(7-Bromo-5-oxo-1-pyrrolidin-1-yl-5H-[1,2,4]triazolo[4,3-a]quinazolin-4-ylmethyl)-phenyl]-acetic acid

1-(pyrrolidin-1-yl)-7-bromo-4-(3-phenylallyl)-4H-[1,2,4]triazolo[4,3-a]quinazolin-5-one

7-Bromo-4-(3-phenyl-allyl)-1-pyrrolidin-1-yl-4H-[1,2,4]triazolo[4,3-a]quinazolin-5-one

7-Bromo-4-[(E)-3-(4-chloro-phenyl)-allyl]-1-pyrrolidin-1-yl-4H-[1,2,4]triazolo[4,3-a]quinazolin-5-one

7-Bromo-4-[3-(4-methoxy-phenyl)-allyl]-1-pyrrolidin-1-yl-4H-[1,2,4]triazolo[4,3-a]quinazolin-5-one

7-Bromo-4-(3-pyridin-3-yl-allyl)-1-pyrrolidin-1-yl-4H-[1,2,4]triazolo[4,3-a]quinazolin-5-one

7-Bromo-4-((E)-3-pyridin-4-yl-allyl)-1-pyrrolidin-1-yl-4H-[1,2,4]triazolo[4,3-a]quinazolin-5-one

7-Bromo-4-(1H-imidazol-4-ylmethyl)-1-pyrrolidin-1-yl-4H-[1,2,4]triazolo[4,3-a]quinazolin-5-one

7-Bromo-4-(3,5-dimethyl-isoxazol-4-ylmethyl)-1-pyrrolidin-1-yl-4H-[1,2,4]triazolo[4,3-a]quinazolin-5-one

7-Bromo-4-cyclopentylmethyl-1-pyrrolidin-1-yl-4H-[1,2,4]triazolo[4,3-a]quinazolin-5-one

7-Bromo-4-butyl-1-pyrrolidin-1-yl-4H-[1,2,4]triazolo[4,3-a]quinazolin-5-one

7-Bromo-1-pyrrolidin-1-yl-4-(2,2,2-trifluoro-ethyl)-4H-[1,2,4]triazolo[4,3-a]quinazolin-5-one

7-Bromo-4-(2-hydroxy-ethyl)-1-pyrrolidin-1-yl-4H-[1,2,4]triazolo[4,3-a]quinazolin-5-one

7-Bromo-4-(2-diethylamino-ethyl)-1-pyrrolidin-1-yl-4H-[1,2,4]triazolo[4,3-a]quinazolin-5-one

7-Bromo-4-prop-2-ynyl-1-pyrrolidin-1-yl-4H-[1,2,4]triazolo[4,3-a]quinazolin-5-one

7-Bromo-4-(2-phenoxy-ethyl)-1-pyrrolidin-1-yl-4H-[1,2,4]triazolo[4,3-a]quinazolin-5-one

7-Bromo-4-(2-phenylsulfényl-ethyl)-1-pyrrolidin-1-yl-4H-[1,2,4]triazolo[4,3-a]quinazolin-5-one

(7-Bromo-5-oxo-1-pyrrolidin-1-yl-5H-[1,2,4]triazolo[4,3-a]quinazolin-4-yl)-phenylacetic acid methyl ester

4-(7-Bromo-5-oxo-1-piperidin-1-yl-5H-[1,2,4]triazolo[4,3-a]quinazolin-4-ylmethyl)-benzonitrile

7-Bromo-4-(3,4-dimethoxy-benzyl)-1-piperidin-1-yl-4H-[1,2,4]triazolo[4,3-a]quinazolin-5-one

1-(piperidin-1-yl)-7-bromo-4-(3-phenylallyl)-4H-[1,2,4]triazolo[4,3-a]quinazolin-5-one

7-Bromo-4-(3-pyridin-3-yl-allyl)-1-thiomorpholin-4-yl-4H-[1,2,4]triazolo[4,3-a]quinazolin-5-one

7-Bromo-1-dimethylamino-4-(4-methyl-benzyl)-4H-[1,2,4]triazolo[4,3-a]quinazolin-5-one

4-(7-Bromo-1-dimethylamino-5-oxo-5H-[1,2,4]triazolo[4,3-a]quinazolin-4-ylmethyl)-benzonitrile

7-Bromo-1-dimethylamino-4-(4-hydroxy-benzyl)-4H-[1,2,4]triazolo[4,3-a]quinazolin-5-one

4-(7-Bromo-1-dimethylamino-5-oxo-5H-[1,2,4]triazolo[4,3-a]quinazolin-4-ylmethyl)-benzoic acid methyl ester

[4-(7-Bromo-1-dimethylamino-5-oxo-5H-[1,2,4]triazolo[4,3-a]quinazolin-4-ylmethyl)-phenyl]-acetic acid

[4-(7-Bromo-1-dimethylamino-5-oxo-5H-[1,2,4]triazolo[4,3-a]quinazolin-4-ylmethyl)-phenyl]-acetonitrile

7-Bromo-1-dimethylamino-4-(pyridin-3-ylmethyl)-4H-[1,2,4]triazolo[4,3-a]quinazolin-5-one

7-Bromo-1-dimethylamino-4-(3-phenyl-allyl)-4H-[1,2,4]triazolo[4,3-a]quinazolin-5-one

7-Bromo-1-dimethylamino-4-(3-phenyl-allyl)-4H-[1,2,4]triazolo[4,3-a]quinazolin-5-one

7-Bromo-1-dimethylamino-4-(3-pyridin-4-yl-allyl)-4H-[1,2,4]triazolo[4,3-a]quinazolin-5-one

7-Bromo-1-dimethylamino-4-prop-2-ynyl-4H-[1,2,4]triazolo[4,3-a]quinazolin-5-one

7-Bromo-1-dimethylamino-4-(3-phenyl-prop-2-ynyl)-4H-[1,2,4]triazolo[4,3-a]quinazolin-5-one

4-(7-Bromo-1-dimethylamino-5-oxo-5H-[1,2,4]triazolo[4,3-a]quinazolin-4-yl)-phenyl-acetic acid methyl ester

1-Azepan-1-yl-7-methyl-4-pyridin-3-ylmethyl-4H-[1,2,4]triazolo[4,3-a]quinazolin-5-one

1-Azepan-1-yl-7-methyl-4-(3-phenyl-allyl)-4H-[1,2,4]triazolo[4,3-a]quinazolin-5-one

4-(7-Methyl-5-oxo-1-pyrrolidin-1-yl-5H-[1,2,4]triazolo[4,3-a]quinazolin-4-ylmethyl)-benzonitrile

4-(3,4-Dimethoxy-benzyl)-7-methyl-1-pyrrolidin-1-yl-4H-[1,2,4]triazolo[4,3-a]quinazolin-5-one

4-(7-Methyl-5-oxo-1-pyrrolidin-1-yl-5H-[1,2,4]triazolo[4,3-a]quinazolin-4-ylmethyl)-benzoic acid methyl ester

[4-(7-Methyl-5-oxo-1-pyrrolidin-1-yl-5H-[1,2,4]triazolo[4,3-a]quinazolin-4-ylmethyl)-phenyl]-acetic acid

7-Methyl-4-pyridin-3-ylmethyl-1-pyrrolidin-1-yl-4H-[1,2,4]triazolo[4,3-a]quinazolin-5-one

7-Methyl-4-(3-phenyl-allyl)-1-pyrrolidin-1-yl-4H-[1,2,4]triazolo[4,3-a]quinazolin-5-one

[4-(7-Methyl-5-oxo-1-thiomorpholin-4-yl-5H-[1,2,4]triazolo[4,3-a]quinazolin-4-ylmethyl)-phenyl]-acetic acid

7-Methyl-4-(3-pyridin-3-y1-allyl)-1-thiomorpholin-4-yl-4H-[1,2,4]triazolo[4,3-a]quinazolin-5-one

4-(1-Dimethylamino-7-methyl-5-oxo-5H-[1,2,4]triazolo[4,3-a]quinazolin-4-ylmethyl)-benzonitrile

[4-(Dimethylamino-methyl-oxo-5H-[1,2,4]triazolo[4,3-a]quinazolin-4-ylmethyl)-phenyl]-acetic acid

1-Dimethylamino-7-methyl-4-((E)-3-phenyl-allyl)-4H-[1,2,4]triazolo[4,3-a]quinazolin-5-one

1-Dimethylamino-7-methyl-4-(3-pyridin-3-yl-allyl)-4H-[1,2,4]triazolo[4,3-a]quinazolin-5-one

1-Dimethylamino-7-methyl-4-(3-pyridin-4-yl-allyl)-4H-[1,2,4]triazolo[4,3-a]quinazolin-5-one

1-(azepan-1-yl)-8-methyl-4-(3-phenylallyl)-4H-[1,2,4]triazolo[4,3-a]quinazolin-5-one

(4-Cyano-benzyl)-dimethylamino-oxo-4,5-dihydro-[1,2,4]triazolo[4,3-a]quinazoline-7-carbonitrile

7-Hydroxy-4-((E)-3-phenyl-allyl)-1-pyrrolidin-1-yl-4H-[1,2,4]triazolo[4,3-a]quinazolin-5-one

1-(azepan-1-yl)-3-(3-phenylallyl)- 3H-[1,2,4]triazolo[4,3-a]- quinazolin-5-one

3-Allyl-1-azepan-1-yl-7-chloro-3H-[1,2,4]triazolo[4,3-a]quinazolin-5-one

1-(azepan-1-yl)-7-chloro-3-benzyl-3H-[1,2,4]triazolo[4,3-a]-quinazolin-5-one

1-Azepan-1-yl-7-chloro-3-(4-methyl-benzyl)-3H-[1,2,4]triazolo[4,3-a]quinazolin-5-one

1-(azepan-1-yl)-7-chloro-3-(2-chlorobenzyl)-3H-[1,2,4]triazolo[4,3-a]quinazolin-5-one

1-(azepan-1-yl)-7-chloro-3-(3-chlorobenzyl)-3H-[1,2,4]triazolo[4,3-a]quinazolin-5-one

1-(azepan-1-yl)-7-chloro-3-(4-chlorobenzyl)-3H-[1,2,4]triazolo[4,3-a]quinazolin-5-one

1-(azepan-1-yl)-7-chloro-3-(4-bromobenzyl)-3H-[1,2,4]triazolo[4,3-a]quinazolin-5-one

1-(azepan-1-yl)-7-chloro-3-(4-fluorobenzyl)-3H-[1,2,4]triazolo[4,3-a]quinazolin-5-one

1-(azepan-1-yl)-7-chloro-3-(4-(trifluoromethyl)benzyl)-3H-[1,2,4]triazolo[4,3-a]quinazolin-5-one

1-(azepan-1-yl)-7-chloro-3-(4-cyanobenzyl)-3H-[1,2,4]triazolo[4,3-a]quinazolin-5-one

1-(azepan-1-yl)-7-chlore-3-(2-methoxybenzyl)-3H-[1,2,4]triazolo[4,3-a]quinazolin-5-one

1-(azepan-1-yl)-7-chloro-3-(3-methoxybenzyl)-3H-[1,2,4]triazolo[4,3-a]quinazolin-5-one

1-(azepan-1-yl)-7-chloro-3-(4-methoxybenzyl)-3H-[1,2,4]triazolo[4,3-a]quinazolin-5-one

1-(azepan-1-yl)-7-chloro-3-(3,4-dichlorobenzyl)-3H-[1,2,4]triazolo[4,3-a]quinazolin-5-one

1-(azepan-1-yl)-7-chloro-3-(3,4-dimethoxybenzyl)-3H-[1,2,4]triazolo[4,3-a]quinazolin-5-one

1-(azepan-1-yl)-7-chloro-3-(2-pyridylmethyl)-3H-[1,2,4]triazolo[4,3-a]quinazolin-5-one

1-(azepan-1-yl)-7-chloro-3-(3-pyridylmethyl)-3H-[1,2,4]triazolo[4,3-a]quinazolin-5-one

1-(azepan-1-yl)-7-chloro-3-(2-phenylethyl)-3H-[1,2,4]triazolo[4,3-a]quinazolin-5-one

1-(azepan-1-yl)-7-chloro-3-[2-(4-methoxyphenyl)ethyl]-3H-[1,2,4]triazolo[4,3-a]quinazolin-5-one

1-(azepan-1-yl)-7-chloro-3-(3-phenylpropyl)-3H-[1,2,4]triazolo[4,3-a]quinazolin-5-one

1-Azepan-1-yl-7-chloro-3-(2-oxo-2-phenyl-ethyl)-3H-[1,2,4]triazolo[4,3-a]quinazolin-5-one

1-(azepan-1-yl)-7-chloro-3-[2-(4-methoxyphenyl)-2-oxoethyl]-3H-[1,2,4]triazolo[4,3-a]quinazolin-5-one

1-(azepan-1-yl)-7-chloro-3-[2-(4-chlorophenyl)-2-oxoethyl]-3H-[1,2,4]triazolo[4,3-a]quinazolin-5-one

5-[(1-(azepan-1-yl)-7-chloro-5-oxo-5H-[1,2,4]triazolo[4,3-a]- quinazolin-3-yl)acetyl]-2-methoxybenzoic acid methyl ester

1-(azepan-1-yl)-7-brome-3-(4-chlorobenzyl)-3H-[1,2,4]triazolo[4,3-a]quinazolin-5-one

1-(azepan-1-yl)-7-brome-3-(4-fluorobenzyl)-3H-[1,2,4]triazolo[4,3-a]quinazolin-5-one

4-(1-(azepan-1-yl)-7-bromo-5-oxo-5H-[1,2,4]triazolo[4,3-a]- quinazolin-3-ylmethyl)- benzonitrile

1-(azepan-1-yl)-7-bromo-3-(3,4-dimethoxybenzyl)-3H-[1,2,4]triazolo[4,3-a]quinazolin-5-one

[4-(7-Bromo-5-oxo-1-perhydro-azepin-1-yl-5H-[1,2,4]triazolo[4,3-a]quinazolin-3-ylmethyl)-phenyl]-acetic acid

1-(azepan-1-yl)-7-bromo-3-(pyridin-3-ylmethyl)-3H-[1,2,4]triazolo[4,3-a]quinazolin-5-one

1-Azepan-1-yl-7-bromo-3-((E)-3-phenyl-allyl)-3H-[1,2,4]triazolo[4,3-a]quinazolin-5-one

7-Bromo-3-((E)-3-phenyl-allyl)-1-piperidin-1-yl-3H-[1,2,4]triazolo[4,3-a]quinazolin-5-one

7-bromo-3-(4-chlorobenzyl)-1-(pyrrolidin-1-yl)-3H-[1,2,4]triazolo[4,3-a]quinazolin-5-one

7-bromo-3-(4-fluorobenzyl)-1-(pyrrolidin-1-yl)-3H-[1,2,4]triazolo[4,3-a]quinazolin-5-one

4-(7-bromo-5-oxo-1-(pyrrolidin-1-yl)-5H-[1,2,4]triazolo[4,3-a]- quinazolin-3-ylmethyl)- benzonitrile

4-(7-bromo-5-oxo-1-(pyrrolidin-1-yl)-5H-[1,2,4]triazolo[4,3-a]- quinazolin-3-ylmethyl)benzoic acid methyl ester

7-Bromo-3-(4-methoxy-benzyl)-1-pyrrolidin-1-yl-3H-[1,2,4]triazolo[4,3-a]quinazolin-5-one

Acetic acid 4-(7-bromo-5-oxo-1-pyrrolidin-1-yl-5H-[1,2,4]triazolo[4,3-a]quinazolin-3-ylmethyl)-phenyl ester

7-Bromo-1-dimethylamino-3-(4-hydroxy-benzyl)-3H-[1,2,4]triazolo[4,3-a]quinazolin-5-one

3-(benzo[1,3]dioxol-5-ylmethyl)-7-brome-1-(pyrrolidin-1-yl)-3H-[ 1,2,4]triazolo[4,3-a]quinazolin-5-one

7-bromo-3-(3,5-dimethoxy-benzyl)-1-(pyrrolidin-1-yl)-3H-[1,2,4]triazolo- [4,3-a]quinazolin-5-one

7-bromo-1-(pyrrolidin-1-yl)-3-(3,4,5-trimethoxybenzyl)-3H-[1,2,4]triazolo[4,3-a]quinazolin-5-one

7-Bromo-3-(1H-imidazol-4-ylmethyl)-1-pyrrolidin-1-yl-3H-[1,2,4]triazolo[4,3-a]quinazolin-5-one

7-bromo-3-(n-butyl)-1-(pyrrolidin-1-yl)-3H-[1,2,4]triazolo[4,3-a]- quinazolin-5-one

(7-Bromo-5-oxo-1-pyrrolidin-1-yl-5H-[1,2,4]triazolo[4,3-a]quinazolin-3-yl)-phenylacetic acid methyl ester

7-Bromo-1-dimethylamino-3-(3-phenyl-allyl)-3H-[1,2,4]triazolo[4,3-a]quinazolin-5-one

4-(7-Bromo-1-dimethylamino-5-oxo-5H-[1,2,4]triazolo[4,3-a]quinazolin-3-yl)-phenyl-acetic acid methyl ester

1-(azepan-1-yl)-7-methyl-3-(3-phenylallyl)-3H-[1,2,4]triazolo[4,3-a]quinazolin-5-one

7-methyl-3-(3-phenylallyl)-1-(pyrrolidin-1-yl)-3H-[1,2,4]triazolo[4,3-a]quinazolin-5-one

1-(azepan-1-yl)-3,8-dimethyl-3H-[1,2,4]triazolo[4,3-a]- quinazolin-5-one

1-Azepan-1-yl-8-methyl-3-((E)-3-phenyl-allyl)-3H-[1,2,4]triazolo[4,3-a]quinazolin-5-one

7-hydroxy-3-(3-phenylallyl)-1-(pyrrolidin-1-yl)-3H-[1,2,4]triazolo[4,3-a]quinazolin-5-one

1,8-bis(azepan-1-yl)-3-(3-phenylallyl)-3H-[1,2,4]triazolo[4,3-a]quinazolin-5-one

1-(azepan-1-yl)-4-benzyl-7-bromo-4H-[1,2,4]triazolo[4,3-a]- quinazolin-5-one

4-benzyl-7-bromo-1-(pyrrolidin-1-yl)-4H-[1,2,4]triazolo[4,3-a]- quinazolin-S-one

4-Benzyl-7-bromo-1-(butyl-methyl-amino)-4H-[1,2,4]triazolo[4,3-a]quinazolin-5-one

4-benzyl-1-(pyrrolidin-1-yl )-4H-[1,2,4]triazolo[4,3-a]quinazolin-5-one

7-chloro-1-dibutylamino-4-methyl-4H-[1,2,4]triazolo[4,3-a]- quinazolin-5-one

7-chloro-4-methyl-1-(piperidin-1-yl)-4H-[1,2,4]triazolo[4,3-a]- quinazolin-5-one

7-Chloro-4-methyl-1-(4-methyl-piperazin-1-yl)-4H-[1,2,4]triazolo[4,3-a]quinazolin-5-one

7-Chloro-4-methyl-1-(1,8,8-trimethyl-3-aza-bicyclo[3.2.1]oct-3-yl)-4H-[1,2,4]triazolo[4,3-a]quinazolin-5-one

1-(azepan-1-yl)-7-chloro-4-phenyl-4H-[1,2,4]triazolo[4,3-a]- quinazolin-5-one

1-(azepan-1-yl)-4-benzyl-7-chloro-4H-[1,2,4]triazolo[4,3-a]- quinazolin-5-one

4-benzyl-7-chloro-1-(pyrrolidin-1-yl)-4H-[1,2,4]triazolo[4,3-a]- quinazolin-5-one

4-benzyl-7-chloro-1-(piperidin-1-yl)-4H-[1,2,4]triazolo[4,3-a]- quinazolin-5-one

1-(azepan-1-yl)-8-chloro-4-methyl-4H-[1,2,4]triazolo[4,3-a]- quinazolin-5-one

1-(azepan-1-yl)-4-benzyl-8-chloro-4H-[1,2,4]triazolo[4,3-a]- quinazolin-5-one

1-(azepan-1-yl)-7-bromo-4-methyl-4H-[1,2,4]triazolo[4,3-a]- quinazolin-5-one

4-benzyl-7-bromo-1-(piperidin-1-yl)-4H-[1,2,4]triazolo[4,3-a]- quinazolin-5-one

4-Benzyl-7-bromo-1-dimethylamino-4H-[1,2,4]triazolo[4,3-a]quinazolin-5-one

4-Benzyl-7-bromo-1-morpholin-4-yl-4H-[1,2,4]triazolo[4,3-a]quinazolin-5-one

4-Benzyl-7-bromo-1-thiomorpholin-4-yl-4H-[1,2,4]triazolo[4,3-a]quinazolin-5-one

4-Benzyl-7-brome-1-(4-methyl-piperazin-1-yl)-4H-[1,2,4]triazolo[4,3-a]quinazolin-5-one

4-Benzyl-7-brome-1-(4-phenyl-piperazin-1-yl)-4H-[1,2,4]triazolo[4,3-a]quinazolin-5-one

4-Benzyl-1-(4-benzyl-piperazin-1-yl)-7-bromo-4H-[1,2,4]triazolo[4,3-a]quinazolin-5-one

4-Benzyl-7-bromo-1-(3,6-dihydro-2H-pyridin-1-yl)-4H-[1,2,4]triazolo[4,3-a]quinazolin-5-one

4-Benzyl-7-bromo-1-(2,5-dihydro-pyrrol-1-yl)-4H-[1,2,4]triazolo[4,3-a]quinazolin-5-one

4-Benzyl-7-brome-1-(3-hydroxy-pyrrolidin-1-yl)-4H-[1,2,4]triazolo[4,3-a]quinazolin-5-one

4-Benzyl-7-bromo-1-methylamino-4H-[1,2,4]triazolo[4,3-a]quinazolin-5-one

4-Benzyl-7-iodo-1-pyrrolidin-1-yl-4H-[1,2,4]triazolo[4,3-a]quinazolin-5-one

1-Azepan-1-yl-4-benzyl-7-methyl-4H-[1,2,4]triazolo[4,3-a]quinazolin-5-one

4-Benzyl-7-methyl-1-pyrrolidin-1-yl-4H-[1,2,4]triazolo[4,3-a]quinazolin-5-one

4-Benzyl-1-dimethylamino-7-methyl-4H-[1,2,4]triazolo[4,3-a]quinazolin-5-one

4-Benzyl-7-methyl-1-thiomorpholin-4-yl-4H-[1,2,4]triazolo[4,3-a]quinazolin-5-one

1-Azepan-1-yl-4-benzyl-8-methyl-4H-[1,2,4]triazolo[4,3-a]quinazolin-5-one

1-Azepan-1-yl-4-benzyl-7-methoxy-4H-[1,2,4]triazolo[4,3-a]quinazolin-5-one

4-Benzyl-7-methoxy-1-pyrrolidin-1-yl-4H-[1,2,4]triazolo[4,3-a]quinazolin-5-one

4-Benzyl-5-oxo-1-pyrrolidin-1-yl-4,5-dihydro-[1,2,4]triazolo[4,3-a]quinazoline-7-carbonitrile

1-Azepan-1-yl-4-benzyl-7-nitro-4H-[1,2,4]triazolo[4,3-a]quinazolin-5-one

1-(azepan-1-yl)-4-benzyl-7-chloro-4H-[1,2,4]triazolo[4,3-a]- quinazolin-5-one

1-(azepan-1-yl)-4-methyl-4H-[1,2,4]triazolo[4,3-a]quinazolin-5-one

1-(azepan-1-yl)-4-benzyl-4H-[1,2,4]triazolo[4,3-a]quinazolin-5-one

1-(azepan-1-yl)-6-chloro-4-methyl-4H-[1,2,4]triazolo[4,3-a]- quinazolin-5-one

1-(azepan-1-yl)-7-chloro-4-methyl-4H-[1,2,4]triazolo[4,3-a]- quinazolin-5-one

1-(azepan-1-yl)-7-chloro-4-ethyl-4H-[1,2,4]triazolo[4,3-a]- quinazolin-5-one

7-chloro-4-methyl-1-(pyrrolidin-1-yl)-4H-[1,2,4]triazolo[4,3-a]- quinazolin-5-one

7-chloro-4-methyl-1-(morpholin-4-yl)-4H-[1,2,4]triazolo[4,3-a]- quinazolin-5-one

1-(azocan-1-yl)-7-chloro-4-methyl-4H-[1,2,4]triazolo[4,3-a]- quinazolin-5-one

7-chloro-1-(3,4-dihydro-2H-quinolin-1-yl)-4-methyl-4H-[1,2,4]triazolo[4,3-a]quinazolin-5-one

7-chloro-1-(3,4-dihydro-1H-isoquinolin-2-yl)-4-methyl-4H-[1,2,4]triazolo[4,3-a]quinazolin-5-one

1-(4-benzylpiperidin-1-yl)-7-chloro-4-methyl-4H-[1,2,4]triazolo[4,3-a]quinazolin-5-one

7-chloro-4-methyl-1-(1,3,3-trimethyl-6-azabicyclo[3,2,1]oct-6-yl)-4H-[1,2,4]triazolo[4,3-a]- quinazolin-5-one

1-(azepan-1-yl)-7-fluoro-4-methyl-4H-[1,2,4]triazolo[4,3-a]- quinazolin-5-one

1-(azepan-1-yl)-7-iodo-4-methyl-4H-[1,2,4]triazolo[4,3-a]- quinazolin-5-one

1-(azepan-1-yl)-7-methoxy-4-methyl-4H-[1,2,4]triazolo[4,3-a]- quinazolin-5-one

4-Benzyl-7-bromo-1-(ethyl-methyl-amino)-4H-[1,2,4]triazolo[4,3-a]quinazolin-5-one

4-Benzyl-1-diethylamino-7-methyl-4H-[1,2,4]triazolo[4,3-a]quinazolin-5-one

4-Benzyl-7-bromo-1-pyrrol-1-yl-4H-[1,2,4]triazolo[4,3-a]quinazolin-5-one

4-(4-Amino-benzyl)-7-bromo-1-pyrrolidin-1-yl-4H-[1,2,4]triazolo[4,3-a]quinazolin-5-one

4-Benzyl-7-hydroxy-1-pyrrolidin-1-yl-4H-[1,2,4]triazolo[4,3-a]quinazolin-5-one

4-(7-Hydroxy-5-oxo-1-pyrrolidin-1-yl-5H-[1,2,4]triazolo[4,3-a]quinazolin-4-ylmethyl)-benzonitrile

N-(4-Benzyl-5-oxo-1-pyrrolidin-1-yl-4,5-dihydro-[1,2,4]triazolo[4,3-a]quinazolin-7-yl)-acetamide

N-[5-Oxo-4-(3-phenyl-allyl)-1-pyrrolidin-1-yl-4,5-dihydro-[1,2,4]triazolo[4,3-a]quinazolin-7-yl]-acetamide

7-Amino-4-((E)-3-phenyl-allyl)-1-pyrrolidin-1-yl-4H-[1,2,4]triazolo[4,3-a]quinazolin-5-one

7-Amino-1-azepan-1-yl-4-benzyl-4H-[1,2,4]triazolo[4,3-a]quinazolin-5-one

7-Amino-4-benzyl-1-pyrrolidin-1-yl-4H-[1,2,4]triazolo[4,3-a]quinazolin-5-one

4-(7-Amino-5-oxo-1-pyrrolidin-1-yl-5H-[1,2,4]triazolo[4,3-a]quinazolin-4-ylmethyl)-benzonitrile

7-Amino-4-((E)-3-pyridin-3-yl-allyl)-1-pyrrolidin-1-yl-4H-[1,2,4]triazolo[4,3-a]quinazolin-5-one

4-(Amino-dimethylamino-oxo-5H-[1,2,4]triazolo[4,3-a]quinazolin-4-ylmethyl)-benzonitrile

7-Amino-1-dimethylamino-4-((E)-3-phenyl-allyl)-4H-[1,2,4]triazolo[4,3-a]quinazolin-5-one

4-Benzyl-7-methylamino-1-pyrrolidin-1-yl-4H-[1,2,4]triazolo[4,3-a]quinazolin-5-one

4-(7-Methylamino-5-oxo-1-pyrrolidin-1-yl-5H-[1,2,4]triazolo[4,3-a]quinazolin-4-ylmethyl)-benzonitrile

4-Benzyl-8-methylamino-1-pyrrolidin-1-yl-4H-[1,2,4]triazolo[4,3-a]quinazolin-5-one

4-Benzyl-7-ethylamino-1-pyrrolidin-1-yl-4H-[1,2,4]triazolo[4,3-a]quinazolin-5-one

4-Benzyl-7-isopropylamino-1-pyrrolidin-1-yl-4H-[1,2,4]triazolo[4,3-a]quinazolin-5-one

N-(4-Benzyl-5-oxo-1-pyrrolidin-1-yl-4,5-dihydro-[1,2,4]triazolo[4,3-a]quinazolin-7-yl)-methanesulfonamide

4-Benzyl-7-dimethylamino-1-pyrrolidin-1-yl-4H-[1,2,4]triazolo[4,3-a]quinazolin-5-one

4-Benzyl-1-dimethylamino-5-oxo-4,5-dihydro-[1,2,4]triazolo[4,3-a]quinazoline-7-carbonitrile

4-Benzyl-5-oxo-1-pyrrolidin-1-yl-4,5-dihydro-[1,2,4]triazolo[4,3-a]quinazoline-7-carboxylic acid

[4-(7-Bromo-5-oxo-1-pyrrolidin-1-yl-5H-[1,2,4]triazolo[4,3-a]quinazolin-4-ylmethyl)-phenyl]-acetic acid methyl ester

2-[4-(7-Bromo-5-oxo-1-pyrrolidin-1-yl-5H-[1,2,4]triazolo[4,3-a]quinazolin-4-ylmethyl)-phenyl]-N-methyl-acetamide

2-[4-(7-Bromo-1-dimethylamino-5-oxo-5H-[1,2,4]triazolo[4,3-a]quinazolin-4-ylmethyl)-phenyl]-acetamide

2-[4-(7-Bromo-5-oxo-1-pyrrolidin-1-yl-5H-[1,2,4]triazolo[4,3-a]quinazolin-4-ylmethyl)-phenyl]-N,N-dimethyl-acetamide

2-[4-(7-Bromo-5-oxo-1-pyrrolidin-1-yl-5H-[1,2,4]triazolo[4,3-a]quinazolin-4-ylmethyl)-phenyl]-N-hydroxy-acetamide

4-(1-Dimethylamino-7-methyl-5-thioxo-5H-[1,2,4]triazolo[4,3-a]quinazolin-4-ylmethyl)-benzonitrile

4-(7-Bromo-1-dimethylamino-5-thioxo-5H-[1,2,4]triazolo[4,3-a]quinazolin-4-ylmethyl)-benzonitrile

1-Dimethylamino-7-methyl-4-(3-pyridin-3-yl-allyl)-4H-[1,2,4]triazolo[4,3-a]quinazoline-5-thione

4-benzyl-7-(N,N-dimethylsulfonylamino)-1-(pyrrolidin-1-yl)-4H-[1,2,4] triazolo [4,3-a] quinazolin-5-one

**8.** Triazolo [4,3-a] quinazoline-5-ones et/ou -5-thiones selon l'une quelconque des revendication 1 à 4, choisies parmi le groupe comprenant :

1-(Azepan-1-yl)-7-chloro-4-(3-phenylallyl)-4H-[1,2,4]triazolo[4,3-a]quinazolin-5-one

7-Bromo-1-dimethylamino-4-((E)-3-pyridin-3-yl-allyl)-4H-[1,2,4]triazolo[4,3-a]quinazolin-5-one

1-(azepan-1-yl)-7-chloro-4-(4-chlorobenzyl)-4H-[1,2,4]triazolo[4,3-a]quinazolin-5-one

1-(azepan-1-yl)-7-chloro-4-(4-fluorobenzyl)-4H-[1,2,4]triazolo[4,3-a]quinazolin-5-one

1-(azepan-1-yl)-7-chloro-4-(3,4-dimethoxybenzyl)-4H-[1,2,4]triazolo[4,3-a]quinazolin-5-one

1-(azepan-1-yl)-7-chloro-4-(3-pyridylmethyl)-4H-[1,2,4]triazolo[4,3-a]quinazolin-5-one

1-(azepan-1-yl)-7-bromo-4-(4-chlorophenylmethyl)-4H-[1,2,4]triazolo[4,3-a]quinazolin-5-one

4-(1-Azepan-1-yl-7-bromo-5-oxo-5H-[1,2,4]triazolo[4,3-a]quinazolin-4-ylmethyl)-benzonitrile

1-Azepan-1-yl-7-bromo-4-(3,4-dimethoxy-benzyl)-4H-[1,2,4]triazolo[4,3-a]quinazolin-5-one

1-(azepan-1-yl)-7-bromo-4-(3-phenylallyl)-4H-[1,2,4]triazolo[4,3-a]quinazolin-5-one

1-Azepan-1-yl-7-bromo-4-((E)-3-pyridin-3-yl-allyl)-4H-[1,2,4]triazolo[4,3-a]quinazolin-5-one

1-Azepan-1-yl-7-bromo-4-(3-pyridin-4-yl-allyl)-4H-[1,2,4]triazolo[4,3-a]quinazolin-5-one

7-Bromo-4-(4-methyl-benzyl)-1-pyrrolidin-1-yl-4H-[1,2,4]triazolo[4,3-a]quinazolin-5-one

7-Bromo-4-(4-chloro-benzyl)-1-pyrrolidin-1-yl-4H-[1,2,4]triazolo[4,3-a]quinazolin-5-one

7-Bromo-4-(4-fluoro-benzyl)-1-pyrrolidin-1-yl-4H-[1,2,4]triazolo[4,3-a]quinazolin-5-one

4-(7-Bromo-5-oxo-1-pyrrolidin-1-yl-5H-[1,2,4]triazolo[4,3-a]quinazolin-4-ylmethyl)-benzonitrile

4-(7-Bromo-5-oxo-1-pyrrolidin-1-yl-5H-[1,2,4]triazolo[4,3-a]quinazolin-4-ylmethyl)-benzoic acid methyl ester

7-Bromo-4-(4-nitro-benzyl)-1-pyrrolidin-1-yl-4H-[1,2,4]triazolo[4,3-a]quinazolin-5-one

7-Bromo-4-(4-methoxy-benzyl)-1-pyrrolidin-1-yl-4H-[1,2,4]triazolo[4,3-a]quinazolin-5-one

Acetic acid 4-(7-bromo-5-oxo-1-pyrrolidin-1-yl-5H-[1,2,4]triazolo[4,3-a]quinazolin-4-ylmethyl)-phenyl ester

7-Bromo-4-(4-hydroxy-benzyl)-1-pyrrolidin-1-yl-4H-[1,2,4]triazolo[4,3-a]quinazolin-5-one

7-Bromo-4-(3,4-dimethoxy-benzyl)-1-pyrrolidin-1-yl-4H-[1,2,4]triazolo[4,3-a]quinazolin-5-one

1-(pyrrolidin-1-yl)-7-brome-4-(3-phenylallyl)-4H-[1,2,4]triazolo[4,3-a]quinazolin-5-one

7-Bromo-4-[(E)-3-(4-chloro-phenyl)-allyl]-1-pyrrolidin-1-yl-4H-[1,2,4]triazolo[4,3-a]quinazolin-5-one

7-Bromo-4-[3-(4-methoxy-phenyl)-allyl]-1-pyrrolidin-1-yl-4H-[1,2,4]triazolo[4,3-a]quinazolin-5-one

7-Bromo-4-(3-pyridin-3-yl-allyl)-1-pyrrolidin-1-yl-4H-[1,2,4]triazolo[4,3-a]quinazolin-5-one

7-Bromo-4-((E)-3-pyridin-4-yl-allyl)-1-pyrrolidin-1-yl-4H-[1,2,4]triazolo[4,3-a]quinazolin-5-one

7-Bromo-4-(3,4-dimethoxy-benzyl)-1-piperidin-1-yl-4H-[1,2,4]triazolo[4,3-a]quinazolin-5-one

1-(piperidin-1-yl)-7-bromo-4-(3-phenylallyl)-4H-[1,2,4]triazolo[4,3-a]quinazolin-5-one

7-Bromo-1-dimethylamino-4-(4-methyl-benzyl)-4H-[1,2,4]triazolo[4,3-a]quinazolin-5-one

4-(7-Bromo-1-dimethylamino-5-oxo-5H-[1,2,4]triazolo[4,3-a]quinazolin-4-ylmethyl)-benzonitrile

7-Bromo-1-dimethylamino-4-(4-hydroxy-benzyl)-4H-[1,2,4]triazolo[4,3-a]quinazolin-5-one

4-(7-Bromo-1-dimethylamino-5-oxo-5H-[1,2,4]triazolo[4,3-a]quinazolin-4-ylmethyl)-benzoic acid methyl ester

[4-(7-Bromo-1-dimethylamino-5-oxo-5H-[1,2,4]triazolo[4,3-a]quinazolin-4-ylmethyl)-phenyl]-acetonitrile

7-Bromo-1-dimethylamino-4-(3-phenyl-allyl)-4H-[1,2,4]triazolo[4,3-a]quinazolin-5-one

7-Bromo-1-dimethylamino-4-(3-phenyl-prop-2-ynyl)-4H-[1,2,4]triazolo[4,3-a]quinazolin-5-one

1-Azepan-1-yl-7-methyl-4-(3-phenyl-allyl)-4H-[1,2,4]triazolo[4,3-a]quinazolin-5-one

4-(3,4-Dimethoxy-benzyl)-7-methyl-1-pyrralidin-1-yl-4H-[1,2,4]triazolo[4,3-a]quinazolin-5-one

[4-(7-Methyl-5-oxo-1-pyrrolidin-1-yl-5H-[1,2,4]triazolo[4,3-a]quinazolin-4-ylmethyl)-phenyl]-acetic acid

7-Methyl-4-(3-phenyl-allyl)-1-pyrrolidin-1-yl-4H-[1,2,4]triazolo[4,3-a]quinazolin-5-one

[4-(Dimethylamino-methyl-oxo-5H-[1,2,4]triazolo[4,3-a]quinazolin-4-ylmethyl)-phenyl]-acetic acid

1-Dimethylamino-7-methyl-4-((E)-3 -phenyl-allyl)-4H-[1,2,4]triazolo[4,3-a]quinazolin-5-one

1-Dimethylamino-7-methyl-4-(3-pyridin-3-yl-allyl)-4H-[1,2,4]triazolo[4,3-a]quinazolin-5-one

[4-(7-Bromo-5-oxo-1-perhydro-azepin-1-yl-5H-[1,2,4]triazolo[4,3-a]quinazolin-3-ylmethyl)-phenyl]-acetic acid

4-benzyl-7-bromo-1-(pyrrolidin-1-yl)-4H-[1,2,4]triazolo[4,3-a]-quinazolin-5-one

4-Benzyl-7-bromo-1-(2,5-dihydro-pyrrol-1-yl)-4H-[1,2,4]triazolo[4,3-a]quinazolin-5-one

4-Benzyl-7-iodo-1-pyrrolidin-1-yl-4H-[1,2,4]triazolo[4,3-a]quinazolin-5-one

1-Azepan-1-yl-4-benzyl-7-methyl-4H-[1,2,4]triazolo[4,3-a]quinazolin-5-one

4-(4-Amino-benzyl)-7-bromo-1-pyrrolidin-1-yl-4H-[1,2,4]triazolo[4,3-a]quinazolin-5-one

7-Amino-4-((E)-3-phenyl-allyl)-1-pyrrolidin-1-yl-4H-[1,2,4]triazolo[4,3-a]quinazolin-5-one

7-Amino-1-azepan-1-yl-4-benzyl-4H-[1,2,4]triazolo[4,3-a]quinazolin-5-one

7-Amino-4-((E)-3-pyridin-3-yl-allyl)-1-pyrrolidin-1-yl-4H-[1,2,4]triazolo[4,3-a]quinazolin-5-one

7-Amino-1-dimethylamino-4-((E)-3-phenyl-allyl)-4H-[1,2,4]triazolo[4,3-a]quinazolin-5-one

4-Benzyl-7-methylamino-1-pyrrolidin-1-yl-4H-[1,2,4]triazolo[4,3-a]quinazolin-5-one

4-(7-Methylamino-5-oxo-1-pyrrolidin-1-yl-5H-[1,2,4]triazolo[4,3-a]quinazolin-4-ylmethyl)-benzonitrile

4-Benzyl-8-methylamino-1-pyrrolidin-1-yl-4H-[1,2,4]triazolo[4,3-a]quinazolin-5-one

4-Benzyl-7-ethylamino-1-pyrrolidin-1-yl-4H-[1,2,4]triazolo[4,3-a]quinazolin-5-one

4-Benzyl-7-isopropylamino-1-pyrrolidin-1-yl-4H-[1,2,4]triazolo[4,3-a]quinazolin-5-one

[4-(7-Bromo-5-oxo-1-pyrrolidin-1-yl-5H-[1,2,4]triazolo[4,3-a]quinazolin-4-ylmethyl)-phenyl]-acetic acid methyl ester

2-[4-(7-Bromo-5-oxo-1-pyrrolidin-1-yl-5H-[1,2,4]triazolo[4,3-a]quinazolin-4-ylmethyl)-phenyl]-N-methyl-acetamide

2-[4-(7-Bromo-1-dimethylamino-5-oxo-5H-[1,2,4]triazolo[4,3-a]quinazolin-4-ylmethyl)-phenyl]-acetamide

2-[4-(7-Bromo-5-oxo-1-pyrrolidin-1-yl-5H-[1,2,4]triazolo[4,3-a]quinazolin-4-ylmethyl)-phenyl]-N,N-dimethyl-acetamide

2-[4-(7-Bromo-5-oxo-1-pyrrolidin-1-yl-5H-[1,2,4]triaxolo[4,3-a]quinaxolin-4-ylmethyl)-phenyl]-N-hydroxy-acetamide

1-Dimethylamino-7-methyl-4-(3-pyridin-3-yl-allyl)-4H-[1,2,4]triazolo[4,3-a]quinaztrline-5-thione

**9.** Triazolo [4,3-a] quinazoline-5-ones et/ou -5-thiones selon l'une quelconque des revendications 1 à 4, choisies parmi le groupe comprenant :

7-Bromo-1-dimethylamino-4((E)-3-pyridin-3-yl-allyl)-4H-[1,2,4]triazolo[4,3-a]quinazolin-5-one

1-(azepan-1-yl)-7-chloro-4-(3,4-dimethoxybenzyl)-4H-[1,2,4]triazolo[4,3-a]quinazolin-5-one

1-(azepan-1-yl)-7-chloro-4-(3-pyridylmethyl)-4H-[1,2,4]triazolo[4,3-a]quinazolin-5-one

4-(1-Azepan-1-yl-7-bromo-5-oxo-5H-[1,2,4]triazolo[4,3-a]quinazolin-4-ylmethyl)-benzonitrile

1-Azepan-1-yl-7-bromo-4-(3,4-dimethoxy-benzyl)-4H-[1,2,4]triazolo[4,3-a]quinazolin-5-one

1-(azepan-1-yl)-7-brome-4-(3-phenylallyl)-4H-[1,2,4]triazolo[4,3-a]quinazolin-5-one

1-Azepan-1-yl-7-bromo-4-((E)-3-pyridin-3-yl-allyl)-4H-[1,2,4]triazolo[4,3-a]quinazolin-5-one

1-Azepan-1-yl-7-bromo-4-(3-pyridin-4-yl-allyl)-4H-[1,2,4]triazolo[4,3-a]quinazolin-5-one

7-Bromo-4-(4-methyl-benzyl)-1-pyrrolidin-1-yl-4H-[1,2,4]triazolo[4,3-a]quinazolin-5-one

7-Bromo-4-(4-chloro-benzyl)-1-pyrrolidin-1-yl-4H-[1,2,4]triazolo[4,3-a]quinazolin-5-one

7-Bromo-4-(3,4-dimethoxy-benzyl)-1-pyrrolidin-1-yl-4H-[1,2,4]triazolo[4,3-a]quinazolin-5-one

1-(pyrrolidin-1-yl)-7-brome-4-(3-phenylallyl)-4H-[1,2,4]triazolo[4,3-a]quinazolin-5-one

7-Bromo-4-(3-pyridin-3-yl-allyl)-1-pyrrolidin-1-yl-4H-[1,2,4]triazolo[4,3-a]quinazolin-5-one

1-(piperidin-1-yl)-7-brome-4-(3-phenylallyl)-4H-[1,2,4]triazolo[4,3-a]quinazolin-5-one

4-(7-Bromo-1-dimethylamino-5-oxo-5H-[1,2,4]triazolo[4,3-a]quinazolin-4-ylmethyl)-benzonitrile

4-(Bromo-dimethylamino-oxo-5H-[1,2,4]triazolo[4,3-a]quinazolin-4-ylmethyl)-benzoic acid methyl ester

7-Bromo-1-dimethylamino-4-(3-phenyl-allyl)-4H-[1,2,4]triazolo[4,3-a]quinazolin-5-one

7-Bromo-1-dimethylamino-4-(3-phenyl-prop-2-ynyl)-4H-[1,2,4]triazolo[4,3-a]quinazolin-5-one

1-Azepan-1-yl-7-methyl-4-(3-phenyl-allyl)-4H-[1,2,4]triazolo[4,3-a]quinazolin-5-one

4-(3,4-Dimethoxy-benzyl)-7-methyl-1-pyrrolidin-1-yl-4H-[1,2,4]triazolo[4,3-a]quinazolin-5-one

7-Methyl-4-(3-phenyl-allyl)-1-pyrrolidin-1-yl-4H-[1,2,4]triazolo[4,3-a]quinazolin-5-one

7-Amino-4-((E)-3-phenyl-allyl)-1-pyrrolidin-1-yl-4H-[1,2,4]triazolo[4,3-a]quinazolin-5-one

7-Amino-4-((E)-3-pyridin-3-yl-allyl)-1-pyrrolidin-1-yl-4H-[1,2,4]triazolo[4,3-a]quinazolin-5-one

4-Benzyl-7-methylamino-1-pyrrolidin-1-yl-4H-[1,2,4]triazolo[4,3-a]quinazolin-5-one

4-(7-Methylamino-5-oxo-1-pyrrolidin-1-yl-5H-[1,2,4]triazolo[4,3-a]quinazolin-4-ylmethyl)-benzonitrile

[4-(7-Bromo-5-oxo-1-pyrrolidin-1-yl-5H-[1,2,4]triazolo[4,3-a]quinazolin-4-ylmethyl)-phenyl]-acetic acid methyl ester

2-[4-(7-Bromo-5-oxo-1-pyrrolidin-1-yl-5H-[1,2,4]triazolo[4,3-a]quinazolin-4-ylmethyl)-phenyl]-N-methyl-acetamide

2-[4-(7-Bromo-5-oxo-1-pyrrolidin-1-yl-5H-[1,2,4]triazolo[4,3-a]quinazolin-4-ylmethyl)-phenyl]-N,N-dimethylacetamide

1-Dimethylamino-7-methyl-4-(3-pyridin-3-yl-allyl)-4H-[1,2,4]triazolo[4,3-a]quinazoline-5-thione

**10.** Composés intermédiaires de formule générale III :

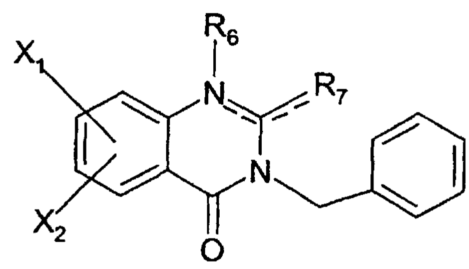

**III**

dans laquelle :
dans laquelle :

- $X_1$, $X_2$ et $A_1$ sont tels que définis à la revendicationl ;
- les traits pointillés représentent des doubles liaisons optionnelles ;
- $R_6$ est hydrogène ; et
- $R_7$ est S ou hydrazino ;

$R_7$ pouvant être lié à l'azote en $R_6$ pour former un cycle, particulièrement un triazole, éventuellement substitué par un groupement thioalkyle inférieur, mercapto ou halogène,
à la condition que lorsque $R_7$ est S ou hydrazino et que $X_1$ est hydrogène, halogène, radical akyle contenant de 1 à 3 atomes de carbone, radical alcoxy contenant de 1 à 3 atomes de carbone ou radical nitro, alors $X_2$ n'est pas hydrogène, halogène, radical akyle contenant de 1 à 3 atomes de carbone, radical alcoxy contenant de 1 à 3 atomes de carbone ou radical nitro.

**11.** Composés intermédiaires de formule générale IV :

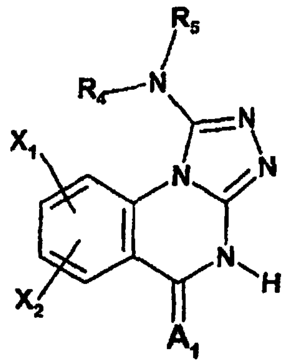

**IV**

dans laquelle $X_1$, $X_2$, $A_1$, $R_4$ et $R_5$ sont tels que définis à la revendication 1.

**12.** Composés intermédiaires de formule générale V:

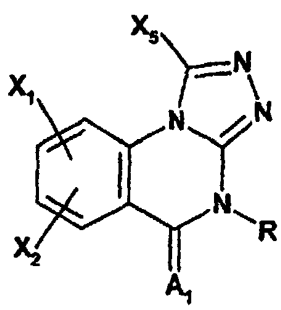

**(V)**

dans laquelle $X_1$, $X_2$, $A_1$ et R sont tels que définis à la revendication 1 et $X_5$ est un groupement halogène, particulièrement F, Br ou Cl, $-OCOX_7$, $-OSO_2X_7$ ou $-SO_2X_7$ dans lesquels $X_7$ est un groupement allyle inférieur ou aryle.

**13.** Procédé de fabrication des composés de formule générale I et II :

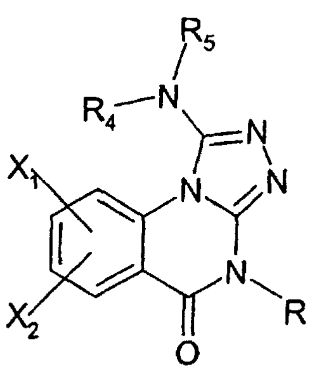

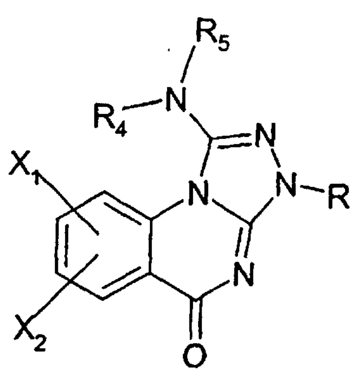

**I** **II**

dans lesquels $X_1$, $X_2$, R, $R_4$ et $R_5$ sont tels que définis à la revendication 1,
ledit procédé étant **caractérisé en ce qu'**il comprend la réaction d'un composé de formule générale IV :

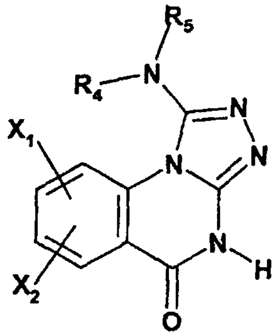

IV

dans laquelle $X_1$, $X_2$, $R_4$ et $R_5$ sont tels que définis à la revendication 1, avec un composé de formule générale

R-X'

dans laquelle R est tel que défini précédemment et X' est un groupement halogène, particulièrement F, Br ou Cl, $-OCOX_7$ ou $-OSO_2X_7$ dans lesquels $X_7$ est un groupement alkyle inférieur ou aryle ; pour obtenir un mélange des composés de formule générale I et II qui sont ensuite éventuellement séparés.

**14.** Procédé de fabrication des composés de formule générale I :

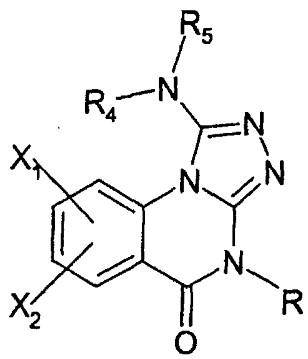

I

dans lesquels $X_1$, $X_2$, R, $R_4$ et $R_5$ sont tels que définis à la revendication 1, ledit procédé étant **caractérisé en ce qu'**il comprend la réaction d'un composé de formule générale V:

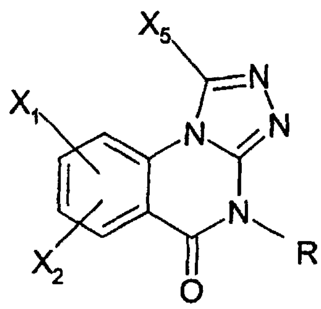

V

dans laquelle $X_1$, $X_2$ et R sont tels que définis à la revendication 1 et $X_5$ est un groupement halogène, particulièrement F, Br ou Cl, $-OCOX_7$, $-OSO_2X_7$ ou $-SO_2X_7$ dans lesquels $X_7$ est un groupement alkyle inférieur ou aryle; avec un composé de formule générale :

$$HNR_4R_5$$

dans laquelle $R_4$ et $R_5$ sont tels que définis à la revendication 1,
pour obtenir un composé de formule générale I.

**15.** Procédé selon la revendication 14, **caractérisé en ce que** lorsque $X_1$ est $-NR_2R_3$ et $-NR_2R_3$ et $-NR_4R_5$ sont identiques, les composés de formule I sont obtenus en faisant réagir un composé de formule générale VI:

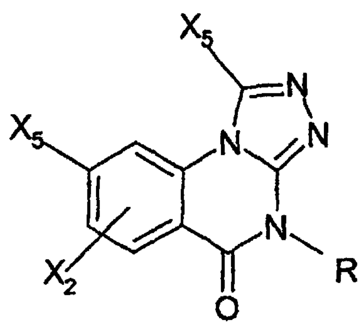

VI

dans laquelle $X_2$ et R sont tels que définis à la revendication 1 et $X_5$ tel que défini à la revendication 12, avec un composé de formule générale :

$$HNR_2R_3$$

dans laquelle $R_2$ et $R_3$ sont tels que définis à la revendication 1,
pour obtenir un composé de formule générale (I):

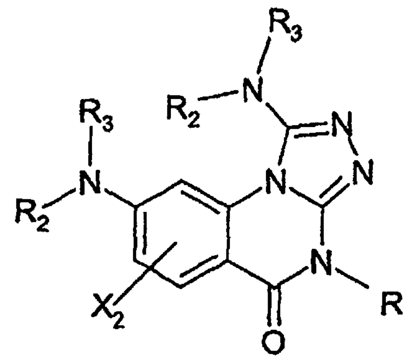

I

**16.** Procédé selon la revendication 14, **caractérisé en ce que** lorsque $X_1$ est $-NR_2R_3$ et $-NR_2R_3$ et $-NR_4R_5$ sont différents, les composés de formule I sont obtenus en faisant réagir un composé de formule générale VII :

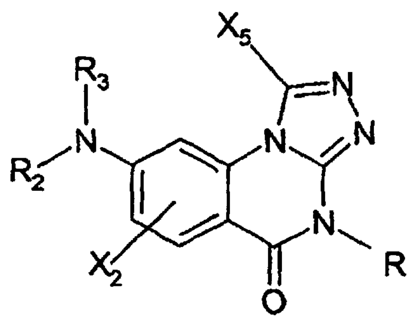

dans laquelle $X_2$, R, $R_2$ et $R_3$ sont tels que définis à la revendication 1 et $X_5$ tel que défini à la revendication 12, avec un composé de formule générale :

$$HNR_4R_5$$

dans laquelle $R_4$ et $R_5$ sont tels que définis à la revendication 1,
pour obtenir un composé de formule générale (I):

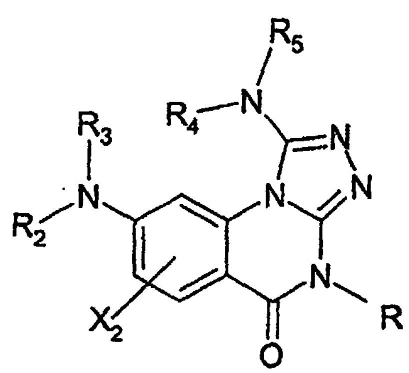

I

**17.** Composition pharmaceutique comprenant un composé selon l'une quelconque des revendications 1 à 9 et un

excipient acceptable en pharmacie.

**18.** Utilisation d'un composé selon l'une quelconque des revendications 1 à 9 pour la préparation d'un médicament destiné au traitement d'une maladie ou d'une affection relevant d'une thérapie par l'inhibition de phosphodiestérases, et plus particuliérement de la PDE4.

**19.** Utilisation selon la revendication 18, **caractérisée en ce que** la maladie est l'asthme.

**20.** Utilisation selon la revendication 18, **caractérisée en ce que** la maladie est la bronchite chronique ou l'atteinte pulmonaire aigüe.

**21.** Utilisation selon la revendication 18, **caractérisée en ce que** la maladie est la dermatite atopique.

**22.** Utilisation selon la revendication 18, **caractérisée en ce que** la maladie est l'hypertension pulmonaire.

**23.** Utilisation selon la revendication 18, **caractérisée en ce que** la maladie est l'insuffisance cardiaque ou pulmonaire.

**24.** Utilisation selon la revendication 18, **caractérisée en ce que** la maladie est le psoriasis.

**25.** Utilisation selon la revendication 18, **caractérisée en ce que** la maladie est une maladie inflammatoire du système digestif telle que la rectocolite hémorragique ou la maladie de Crohn.

**26.** Utilisation selon la revendication 18, **caractérisée en ce que** la maladie est le diabète ou une maladie liée à un taux élevé de TNF-$\alpha$ telles que le syndrome de détresse respiratoire aiguë et la pancréatite aiguë.

**27.** Utilisation selon la revendication 18, **caractérisée en ce que** la maladie est l'hypertrophie bénigne de la prostate.

**28.** Utilisation selon la revendication 18, **caractérisée en ce que** la maladie est choisie parmi l'arthrite rhumatoïde et la sclérose en plaques.

**29.** Utilisation selon la revendication 18, **caractérisée en ce que** la maladie est choisie parmi la dépression, l'atteinte neuronale causée par ischémie et l'ischémie cérébrale partielle.

**30.** Utilisation selon la revendication 18, **caractérisée en ce que** la maladie est le cancer, plus particulièrement les tumeurs malignes ou la leucémie lymphoïde chronique.

**31.** Utilisation d'un composé selon l'une quelconque des revendications 1 à 9 pour atténuer le développement de phénomènes de tolérance ou de dépendance à la morphine.

**32.** Utilisation d'un composé selon l'une quelconque des revendications 1 à 9 pour diminuer les pertes de mémoire du comportement.

**33.** Utilisation d'un composé selon l'une quelconque des revendications 1 à 9 pour la prévention d'accouchements prématurés.

**34.** Utilisation selon la revendication 18, **caractérisée en ce que** la maladie est la septicémie ou la défaillance polyviscérale.

**35.** Utilisation selon la revendication 18, **caractérisée en ce que** la maladie est la pneumopathie obstructive chronique (ou COPD).

**36.** Utilisation selon la revendication 18, **caractérisée en ce que** la maladie est l'emphysème.

**37.** Utilisation selon la revendication 18, **caractérisée en ce que** la maladie est la rhinite allergique.

**38.** Utilisation selon la revendication 18, **caractérisée en ce que** la maladie est l'insuffisance cardiaque congestive.

**39.** Utilisation selon la revendication 18, **caractérisée en ce que** la maladie est l'ostéoporose.

## Patentansprüche

**1.** Triazol[4,3-a]chinazolin-5-one und/oder -5-thione der Formel I oder II:

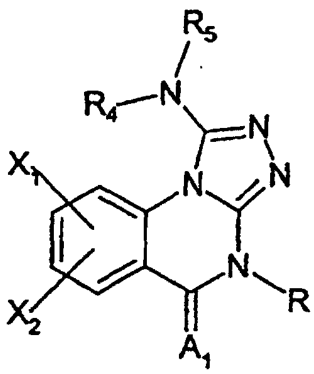

I

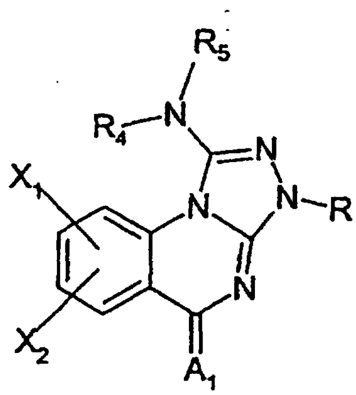

II

wobei I und II Positionsisomere der R-Gruppe an den Stickstoffen 3 oder 4 sind; in diesen:

- ist $A_1$ O oder S;
- stehen $X_1$ und $X_2$, die gleich oder verschieden sind, für:
    - Wasserstoff, Hydroxy, Halogen, Amino, Nitro, Mercapto, Cyan,. Carboxy,
    - niederes Alkyl, niederes Alkoxy oder $-S(O)_mR_8$, wobei m 0, 1 oder 2 ist, und $R_8$ ein niederes Alkyl ist, das gegebenenfalls mit einem oder mehreren Halogenatomen substituiert ist,
    - $-CO-Q_1-Q_2-Q_3$, wobei:
        - $Q_1$: eine einwertige Bindung, -O-,

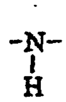
,
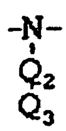
; oder
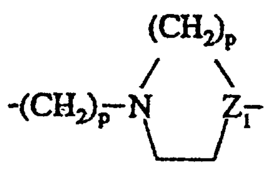

ist, wobei p eine ganze Zahl ist, die von 0 bis 3 variieren kann, und $Z_1$ CH, N, O oder S ist,
        - $Q_2$:

a) $-(CH_2)_q-$ ist, wobei q gleich 0, 1, 2, 3, oder 4 ist, oder
b) $-(CH_2-CH_2-O)_r-$ ist, wobei r gleich 2, 3, oder 4 ist, und

        - $Q_3$: -H, -OH, niederes Alkoxy, $-O-CO-X_3$ $-NHX_3$ oder

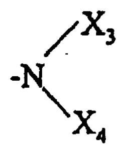

ist, wobei $X_3$ und $X_4$, die gleich oder verschieden sind, für eine niedere Alkylgruppe stehen, und $X_3$ und $X_4$ gebunden sein können, um einen Ring mit einem oder mehreren unter O, S oder N ausgewählten Heteroatomen zu bilden,

- $-NH-R_1$, wobei $R_1$ für eine niedere Alkylgruppe steht, die gegebenenfalls mit einer oder mehreren, unter Halogen, Hydroxy, Cyan, niederem Alkoxy oiler $-CO-Q_1-Q_2-Q_3$ ausgewählten Gruppen substituiert ist, oder
- $-NR_2R_3$, wobei $R_2$ und $R_3$, die gleich oder verschieden sind, für ein niederes Alkyl stehen, das gegebenenfalls mit einer oder mehreren Hydroxy-, Halogen-, Cyan-, niederen Alkoxy- oder $-CO-Q_1-Q_2-Q_3$-Gruppen substituiert ist, wobei $R_2$ und $R_3$ gebunden sein können, um einen Ring mit mehreren unter O, S oder N ausgewählten Heteroatomen zu bilden, und gegebenenfalls durch ein geminales dialkyliertes niederes Alkyl verbrückt sind oder mit einer oder mehreren Gruppen substituiert sind, die unter Hydroxy, Keto, niederem Alkyl, niederem Alkoxy oder $-CO-Q_1-Q_2-Q_3$ ausgewählt sind;

- steht R für

  - niederes Alkyl, niederes Alkenyl, niederes Alkynyl, Arylalkynyl, 2-, 3-oder 4-Pyridylalkyl gegebenenfalls substituiert mit einem niederen Alkyl, einem niederen Alkoxy, einer

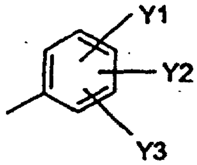
,
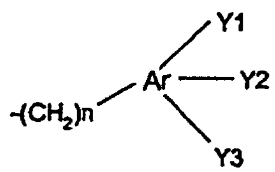
,
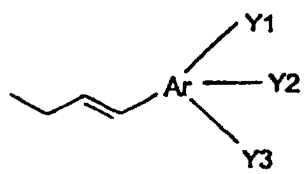

oder

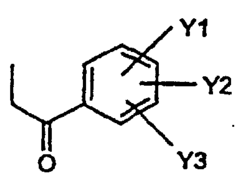

Hydroxy-, Halogen- oder Aminogruppe,
in denen
- n eine game Zahl von 1 bis 5 ist,
- Ar ein aromatischer Ring mit 5 oder 6 Atomen einschließlich 0 bis 3 Heteroatomen ist, die unter O, S oder N ausgewählt sind,
- Y1, Y2 und Y3, die gleich oder verschieden sind, für

  - Wasserstoff, Hydroxy, Mercapto, Amino, Nitro, Halogen, $NHR_1$, $NR_2R_3$, $-(CH_2)_s-CN$, $-(CH_2)_sCO-Q_1-Q_2-Q_3$ stehen, wobei s eine ganze Zahl von 0 bis 6 ist;
  - niederes Alkyl, niederes Alkoxy oder $-S(O)_mR_8$ stehen, wobei m 0, 1 oder 2 und $R_8$ ein niederes Alkyl

ist, die gegebenenfalls jeweils mit einem oder mehreren Halogenatomen substituiert sein können; und

- stehen $R_4$ und $R_5$ für:
  - niederes Alkyl, wenn $R_4$ und $R_5$ gleich sind; Aralkyl, Cycloalkyl oder Cycloalkylalkyl, wenn $R_4$ und $R_5$ verschieden sind,
  - niederes Alkyl, wobei $R_4$ und $R_5$ gebunden sein können, um einen Ring zu bilden, der gesättigt ist oder eine oder mehrere Doppelbindungen mit einem oder mehreren Heteroatomen aufweist, welche unter O, S oder N ausgewählt sind, und gegebenenfalls mit einem niederem Alkyl, einem Hydroxy oder einem niederen Alkoxy substituiert oder durch ein geminales dialkyliertes niederes Alkyl verbrückt sind oder mit einer oder mehreren Gruppen substituiert sind, die unter Hydroxy, Keto, niederem Alkyl, niederem Alkoxy, Phenylalkyl oder CO-$Q_1$-$Q_2$-$Q_3$ ausgewählt sind, wobei zwei der Atome des derart gebildeten Rings auch Teil eines weiteren Rings sein können, der unter Phenyl oder Heteroaryl mit 4 bis 8 Atomen einschließlich 1 bis 4 Heteroatomen ausgewählt ist;

gegebenenfalls deren racemische Formen und deren Isomere sowie deren pharmazeutisch akzeptable Salze.

**2.** Triazol[4,3-a]chinazolin-5-one und/oder -5-thione nach Anspruch 1, in denen $A_1$ für ein Sauerstoffatom steht; $X_1$ für ein Wasserstoffatom steht und $X_2$ eine Halogen-, Amino-, niedere Alkyl-, Hydroxy- oder -NH$R_1$-Gruppe ist, wobei $R_1$ wie obenstehend definiert ist.
R steht für:

- eine niedere Alkyl-, niedere Alkenyl-, Arylalkynyl-, 2-, 3- oder 4-Pyridylalkylgruppe, die gegebenenfalls am Pyridinring mit einem niederen Alkyl, einem Halogen oder einem Hydroxy substituiert ist;

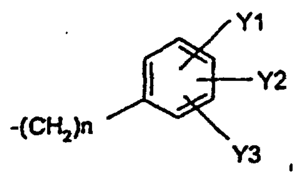

wobei:

  - n eine ganze Zahl von 1 bis 3 ist,
  - Y1, Y2 und Y3 jeweils für ein Wasserstoffatom oder eine niedere Alkoxygruppe, insbesondere Methoxygruppe stehen,
  - Y1 und Y2 jeweils für ein Wasserstoffatom stehen und Y3 für eine niedere Alkoxygruppe, eine Aminogruppe, $NHR_1$-, $NR_2R_3$-, Nitro, Hydroxy, eine -$(CH_2)_s$CO-$Q_1$-$Q_2$-$Q_3$-Gruppe, eine $(CH_2)_s$-CN-Gruppe steht, in denen s, $Q_1$, $Q_2$, $Q_3$ wie vorausgehend definiert sind, oder für eine niedere Alkylgruppe, die gegebenenfalls mit einem oder mehreren Halogenatomen substituiert ist, wobei die insbesondere bevorzugte Position des Substituenten Y3 die Position 4 ist, oder
  - Y1 für ein Wasserstoffatom steht und Y2 und Y3, die gleich oder verschieden sind, für eine Hydroxy-, Halogen- oder niedere Alkoxygruppe stehen, oder

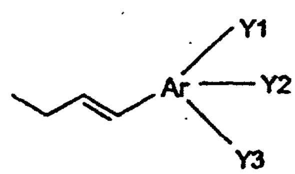

wobei:

  - Ar wie vorstehend definiert ist;

- Yl, Y2 und Y3 jeweils für ein Wasserstoffatom stehen, oder
- Y1 und Y2 jeweils für ein Wasserstoffatom stehen und Y3 niederes Alkoxy oder Halogen ist;

$R_4$ und $R_5$, stehen für:

- niederes Alkyl, wean $R_4$ und $R_5$ gleich sind, Aralkyl, Cycloalkyle oder Cycloalkylalkyl, wenn $R_4$ und $R_5$ verschieden sind,

niederes Alkyl, wobei $R_4$ und $R_5$ gebunden sein können, um einen Ring zu bilden, der gesättigt ist oder eine oder mehrere Doppelbindungen mit einem oder mehreren Heteroatomen aufweist, welche unter O, S oder N ausgewählt sind, und gegebenenfalls mit einem niederem Alkyl, einem Hydroxy oder einem niederen Alkoxy substituiert oder durch ein geminales dialkyliertes niederes Alkyl verbrückt ist oder mit einer oder mehreren Gruppen substituiert ist, die unter Hydroxy, Keto, niederem Alkyl, niederem Alkoxy, Phenylalkyl oder CO-$Q_1$-$Q_2$-$Q_3$ ausgewählt sind, wobei zwei der Atome des derart gebildeten Rings ebenfalls Teil eines weiteren Rings sein können, der unter Phenyl oder Heteroaryl mit 4 bis 8 Atomen einschließlich 1 bis 4 Heteroatomen ausgewählt ist;

**3.** Triazol[4,3-a]chinazolin-5-one und/oder -5-thione nach Anspruch 1 oder 2, in denen:

$X_1$ für ein Wasserstoffatom steht,
$X_2$ für eine Halogen-, Amino-, niedere Alkyl-, Hydroxy- oder -NHR$_1$-Gruppe steht;
R steht für:

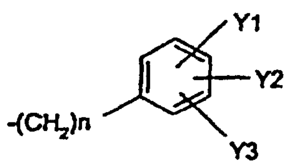

wobei:

- n eine ganze Zahl von 1 bis 3 ist,
- Y1, Y2 und Y3 jeweils für ein Wasserstoffatom oder eine niedere Alkoxygruppe, insbesondere Methoxy und insbesondere(?) 3-, 4-, 5-Trimethoxy stehen,
- Y1 und Y2 jeweils für ein Wasserstoffatom stehen und Y3 steht für: eine niedere Alkoxygruppe, eine Amino-, NHR$_1$-, NR$_2$R$_3$-, Nitro- oder Hydroxygruppe, eine niedere Alkylgruppe, die gegebenenfalls mit einem oder mehreren Halogenatomen substituiert ist, eine -$(CH_2)_s$CO-$Q_1$-$Q_2$-$Q_3$-Gruppe, in der s 0 oder 1 ist, $Q_1$ O, -NH- oder eine Valenzbindung ist, $Q_2$ -$(CH_2)_q$- ist, wobei q gleich 0, 1, 2, 3 oder 4 ist, und $Q_3$ H, OH oder -NX$_3$X$_4$ ist, wobei $X_3$ und $X_4$ wie vorausgehend definiert sind, eine $(CH_2)$s-CN-Gruppe, in der s 0 oder 1 ist, wobei die insbesondere bevorzugte Position des Substituenten Y3 die Position 4 ist, oder
- Y1 für ein Wasserstoffatom steht und Y2 und Y3, die gleich oder verschieden sind, für eine Hydroxy-, Halogen- oder niedere Alkoxygruppe stehen; oder

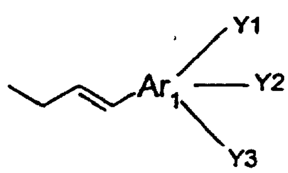

worin:

- $Ar_1$ ein aromatischer Ring mit 6 Atomen ist, die in Position 2, 3 oder 4 und bevorzugt in Position 3 ein Stickstoffatom aufweisen können;

- Y1, Y2 und Y3 jeweils für ein Wasserstoffatom stehen, oder
- Y1 und Y2 jeweils für ein Wasserstoffatom stehen und Y3 eine niedere Alkoxygruppe oder eine Hatogengruppe ist, wenn $Ar_1$ kein Stickstoffatom aufweist; und

$R_4$ und $R_5$ stehen für:

- niederes Alkyl, wenn $R_4$ und $R_5$ gleich sind; Aralkyl, Cycloalkyl oder Cycloalkylalkyl, wenn $R_4$ und $R_5$ verschieden sind,
- niederes Alkyl, wobei $R_4$ und $R_5$ gebunden sein können, um einen Ring zu bilden, der gesättigt ist oder eine oder mehrere Doppelbindungen mit einem oder mehreren Heteroatomen aufweist, welche unter O, S oder N ausgewählt sind, und gegehenenfalls mit einem niederem Alkyl, einem Hydroxy oder einem niederen Alkoxy substituiert oder durch ein geminales dialkyliertes niederes Alkyl verbrückt sind oder mit einer oder mehreren Gruppen substituiert sind, die unter Hydroxy, Keto, niederem Alkyl, niederem Alkoxy, Phenylalkyl oder $CO\text{-}Q_1\text{-}Q_2\text{-}Q_3$ ausgewählt sind, wobei zwei der Atome des derart gebildeten Rings auch Teil eines weiteren Rings sein können, der unter Phenyl oder Heteroaryl mit 4 bis 8 Atomen einschließlich 1 bis 4 Heteroatomen ausgewählt ist.

**4.** Triazol[4,3-a]chinazolin-5-one und/oder -5-thione nach einem der Ansprüche 1 bis 3, bei denen:

- die Halogengruppe unter F, Cl, Br oder I ausgewählt ist,
- die niedere Alkylgruppe eine geradkettige oder verzweigte Gruppe mit von 1 bis 6 Kohlenstoffatomen ist,
- die niedere Alkoxygruppe eine geradkettige oder verzweigte Gruppe mit von 1 bis 5 Kohlenstoffatomen ist,
- die niedere Alkylthiogruppe eine geradkettige oder verzweigte Gruppe mit von 1 bis 5 Kohlenstoffatomen ist,
- die niedere Alkenylgruppe 3 bis 6 Kohlenstoffatome aufweist,
- die niedere Alkynylgruppe 3 bis 6 Kohlenstoffatome aufweist,
- die 2-, 3- oder 4-Pyridylalkylgrappe ein Alkyl mit von 1 bis 5 Kohlenstoffatomen aufweist,
- die Arylgruppe 5 bis 8 Atome aufweist,
- die Aralkylgruppe ein Alkyl mit von 1 bis 6 Kohlenstoffatomen aufweist,
- die Cycloalkylgruppe 3 bis 8 Kohlenstoffatome aufweist,
- die Cycloalkylalkylgruppe ein Alkyl mit von 1 bis 6 Kohlenstoffatomen aufweist,
- die niedere Alkyl-, niedere Alkoxy- oder niedere Alkylthiogruppe mit einem oder mehreren Halogenatomen substituiert ist, die unter den Gruppen $-(CH_2)_p\text{-}CF_3$, $-O\text{-}(CH_2)_p\text{-}CF_3$ oder $-S\text{-}(CH_2)_p\text{-}CF_3$, bei denen p eine ganze Zahl von 0 bis 3 ist.

**5.** Triazol[4,3-a]chinazolin-5-one und/oder -5-thione nach Anspruch 1, in denen:

- $A_1$ O oder S ist
- $X_1$ und $X_2$, die gleich oder verschieden sind, stehen für:
    - Wasserstoff, Hydroxy, Halogen, Amin, Nitro, Mercapto, Cyan, Carboxy,
    - niederes Alkyl, niederes Alkoxy oder $-S(O)_mR_8$, wobei m 0, 1 oder 2 ist und $R_8$ ein niederes Alkyl ist, das gegebenenfalls mit einem oder mehreren Halogenatomen substituiert ist;
- R für

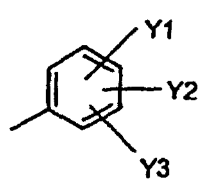

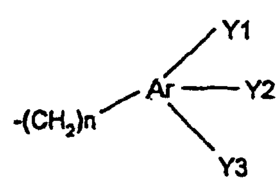

oder

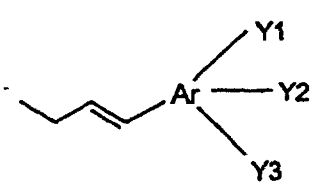

steht, in denen:

- n eine gauze Zahl von 1 bis 5 ist,

- Ar ein aromatischer Ring mit 5 oder 6 Atomen einschließlich 0 bis 3 unter O, S oder N ausgewählten Heteroatomen ist,
- Y1, Y2 und Y3, die gleich oder verschieden sind, stehen für:
- Wasserstoff, Hydroxy, Mercapto, Amino, $NHR_1$, $NR_2R_3$, Nitro, Halogen, $-(CH_2)_sCO-Q_1-Q_2-Q_3$, $-(CH_2)$, -CN, in denen s eine ganze Zahl von 0 bis 6 ist;
- niederes Alkyl, niederes Alkoxy oder $-S(O)_mR_8$, bei denen m 0, 1 oder 2 ist und $R_8$ ein niederes Alkyl ist, die jeweils mit einem oder mehreren Halogenatomen substituiert sein können,

$R_4$ und $R_5$, die gleich oder verschieden sind, stehen für
niederes Alkyl, wobei $R_4$ und $R_5$ gebunden sein können, um einen Ring zu bilden, der gesättigt ist oder eine oder mehrere Doppelbindungen mit einem oder mehreren Heteroatomen aufweist, welche unter O, S oder N ausgewählt sind, und gegebenenfalls mit einem niederem Alkyl, einem Hydroxy oder einem niederen Alkoxy substituiert oder durch ein geminales dialkyliertes niederes Alkyl verbrückt sind oder mit einer oder mehreren Gruppen substituiert sind, die unter Hydroxy, Keto, niederem Alkyl, niederem Alkoxy, Phenylalkyl oder $CO-Q_1-Q_2-Q_3$ ausgewählt sind.

**6.** Triazol[4,3-a]chinazolin-5-one und/oder -5-thione nach Anspruch 5, ausgewählt aus der Gruppe, welche umfaßt:

7-Brom-1-dimethylamino-4-((E)-3-pyridin-3-yl-allyl)-4H-[1,2,4]triazol[4,3-a]chinazolin-5-on

1-Dimethylamino-7-methyl-4-(3-pyridin-3-yl-allyl)-4H-[1,2,4]triazol[4,3-a]chinazolin-5-on

4-(3,4-Dimethoxy-benzyl)-7-methyl-1-pyrrolidin-1-yl-4H-[1,2,4]triazol[4,3-a]chinazolin-5-on

4-(1-Dimethylamino-7-methyl-5-oxo-5H-[1,2,4]triazol[4,3-a]chinazolin-4-ylmethyl)-benzonitril

7-Brom-1-dimethylamino-4-(3-phenyl-allyl)-4H-[1,2,4]triazol[4,3-a]chinazolin-5-on

7-Methyl-4-(3-phenyl-allyl)-1-pyrrolidin-1-yl-4H-[1,2,4]triazol[4,3-a]chinazolin-5-on

4-(7-Brom-1-dimethylamino-5-oxo-5H-[1,2,4]triazol[4,3-a]chinazolin-4-ylmethyl)-benzonitril

1-Azepan-1-yl-7-methyl-4-pyridin-3-ylmethyl-4H-[1,2,4]triazol[4,3-a]chinazolin-5-on

4-(7-Methyl-5-oxo-1-pyrrolidin-1-yl-5H-[1,2,4]triazol[4,3-a]chinazolin-4-ylmethyl)-benzonitril

1-Dimethylamino-7-methyl-4-((E)-3-phenyl-allyl)-4H-[1,2,4]triazol[4,3-a]chinazolin-5-on

4-(7-Brom-5-oxo-1-pyrrolidin-1-yl-5H-[1,2,4]triazol[4,3-a]chinazolin-4-ylmethyl)-benzonitril

1-Azepan-1-yl-7-brom-4-(3,4-dimethoxy-benzyl)-4H-[1,2,4]triazol[4,3-a]chinazolin-5-on

**7.** Triazol[4,3-a]chinazolin-5-one und/oder -5-thione nach Anspruch 1, 2, 3 oder 4, ausgewählt aus der Gruppe umfassend:

1-(Azepan-1-yl)-7-chlor-4-(3-phenylallyl)-4H-[1,2,4]triazol[4,3-a]chinazolin-5-on

1-(Azepan-1-yl)-7-chlor-3-(3-phenylallyl)-3H-[1,2,4]triazol[4,3-a]chinazolin-5-on

7-Brom-1-dimethylamino-4-((E)-3-pyridin-3-yl-allyl)-4H-[1,2,4]triazol[4,3-a]chinazolin-5-on

7-Brom-4-pyridin-3-ylmethyl-1-pyrrolidin-1-yl-4H-[1,2,4]triazol[4,3-a]chinazolin-5-on

7-Brom-3-pyridin-3-ylmethyl-1-pyrrolidin-1-yl-3H-[1,2,4]triazol[4,3-a]chinazolin-5-on

1-Azepan-1-yl-4-(3-phenyl-allyl)-4H-[1,2,4]triazol[4,3-a]chinazolin-5-on

1-(Azepan-1-yl)-7-chlor-4-allyl-4H-[1,2,4]triazol[4,3-a]-chinazolin-5-on

1-(Azepan-1-yl)-7-chlor-4-(4-methylbenzyl)-4H-[1,2,4]triazol[4,3-a]chinazolin-5-on

1-(Azepan-1-yl)-7-chlor-4-(2-chlorbenzyl)-4H-[1,2,4]triazol[4,3-a]chinazolin-5-on

1-(Azepan-1-yl)-7-chlor-4-(3-chlorbenzyl)-4H-[1,2,4]triazol[4,3-a]chinazolin-5-on

1-(Azepan-1-yl)-7-chlor-4-(4-chlorbenzyl)-4H-[1,2,4]triazol[4,3-a]chinazolin-5-on

1-(Azepan-1-yl)-7-chlor-4-(4-brombenzyl)-4H-[1,2,4]triazol[4,3-a]chinazolin-5-on

1-(Azepan-1-yl)-7-chlor-4-(4-fluorbenzyl)-4H-[1,2,4]triazol[4,3-a]chinazolin-5-on

1-(Azepan-1-yl)-7-chlor-4-(4-(trifluormethyl)benzyl)-4H-[1,2,4]triazol[4,3-a]chinazolin-5-on

1-(Azepan-1-yl)-7-chlor-4-(4-cyanbenzyl)-4H-[1,2,4]triazol[4,3-a]chinazolin-5-on

1-(Azepan-1-yl)-7-chlor-4-(2-methoxybenzyl)-4H-[1,2,4]triazol[4,3-a]chinazolin-5-on

1-(Azepan-1-yl)-7-chlor-4-(3-methoxybenzyl)-4H-[1,2,4]triazol[4,3-a]chinazolin-5-on

1-(Azepan-1-yl)-7-chlor-4-(4-methoxybenzyl)-4H-[1,2,4]triazol[4,3-a]chinazolin-5-on

1-(Azepan-1-y1)-7-chlor-4-(3,4-dichlorbenzyl)-4H-[1,2,4]triazol[4,3-a]chinazolin-5-on

1-(Azepan-1-yl)-7-chlor-4-(3,4-dimethoxybenzyl)-4H-[1,2,4]triazol[4,3-a]chinazolin-5-on

1-(Azepan-1-yl)-7-chlor-4-(2-pyridylmethyl)-4H-[1,2,4]triazol[4,3-a]chinazolin-5-on

1-(Azepan-1-yl)-7-chlor-4-(3-pyridylmethyl)-4H-[1,2,4]triazol[4,3-a]chinazolin-5-on

1-(Azepan-1-yl)-7-chlor-4-(4-pyridylmethyl)-4H-[1,2,4]triazol[4,3-a]chinazolin-5-on

1-(Azepan-1-yl)-7-chlor-4-(2-phenylethyl)-4H-[1,2,4]triazol[4,3-a]chinazolin-5-on

1-(Azepan-1-yl)-7-chlor-4-[2-(4-methoxyphenyl)ethyl]-4H-[1,2,4]triazol[4,3-a]chinazolin-5-on

1-(Azepan-1-yl)-7-chlor-4-(3-phenylpropyl)-4H-[1,2,4]triazol[4,3-a]chinazolin-5-on

1-Azepan-1-yl-7-chlor-4-(2-oxo-2-phenyl-ethyl)-4H-[1,2,4]triazol[4,3-a]chinazolin-5-on

1-(Azepan-1-yl)-7-chlor-4-[2-(4-methoxyphenyl)-2-oxoethyl]-4H-[1,2,4]triazol[4,3-a]chinazolin-5-on

1-(Azepan-1-yl)-7-chlor-4-[2-(4-chlorphenyl)-2-oxoethyl]-4H-[1,2,4]triazol[4,3-a]chinazolin-5-on

5-[(1-(Azepan-1-yl)-7-chlor-5-oxo-5H-[1,2,4]triazol[4,3-a]-chinazolin-4-yl)acetyl]-2-methoxybenzoesäuremethylester

7-Chlor-4-pyridin-3-ylmethyl-1-pyrrolidin-1-yl-4H-[1,2,4]triazol[4,3-a]chinazolin-5-on

1-(Azepan-1-yl)-7-brom-4-(4-chlorphenylmethyl)-4H-[1,2,4]triazol[4,3-a]chinazolin-5-on

1-Azepan-1-yl-7-brom-4-(4-fluor-benzyl)-4H-[1,2,4]triazol[4,3-a]chinazolin-5-on

4-(1-Azepan-1-yl-7-brom-5-oxo-5H-[1,2,4]triazal[4,3-a]chinazolin-4-ylmethyl)-benzonitril

1-Azepan-1-yl-7-brom-4-(3,4-dimethoxy-benzyl)-4H-[1,2,4]triazol[4,3-a]chinazolin-5-on

1-(Azepan-1-yl)-7-brom-4-(3-pyridinylmethyl)-4H-[1,2,4]triazol[4,3-a]chinazolin-5-on

1-(Azepan-1-yl)-7-brom-4-(3-phenylallyl)-4H-[1,2,4]triazol[4,3-a]chinazolin-5-on

1-Azepan-1-yl-7-brom-4-[3-(4-chlor-phenyl)-allyl]-4H-[1,2,4]triazol[4,3-a]chinazolin-5-on

1-Azepan-1-yl-7-brom-4-[3-(4-methoxy-phenyl)-allyl]-4H-[1,2,4]triazol[4,3-a]chinazolin-5-on

1-Azepan-1-yl-7-brom-4-((E)-3-pyridin-3-yl-allyl)-4H-[1,2,4]triazol[4,3-a]chinazolin-5-on

1-Azepan-1-yl-7-brom-4-(3-pyridin-4-yl-allyl)-4H-[1,2,4]triazol[4,3-a]chinazolin-5-on

7-Brom-4-(4-methyl-benzyl)-1-pyrrolidin-1-yl-4H-[1,2,4]triazol[4,3-a]chinazolin-5-on

7-Brom-4-(4-chlor-benzyl)-1-pyrrolidin-1-yl-4H-[1,2,4]triazol[4,3-a]chinazolin-5-on

7-Brom-4-(4-fluor-benzyl)-1-pyrrolidin-1-yl-4H-[1,2,4]triazol[4,3-a]chinazolin-5-on

3-(7-Brom-5-oxo-1-pyrrolidin-1-yl-5H-[1,2,4]triazol[4,3-a]chinazolin-4-ylmethyl)-benzonitril

4-(7-Brom-5-oxo-1-pyrrolidin-1-yl-5H-[1,2,4]triazol[4,3-a]chinazolin-4-ylmethyl)-benzonitril

4-(7-Brom-5-oxo-1-pyrrolidin-1-yl-5H-[1,2,4]triazol[4,3-a]chinazolin-4-ylmethyl)-benzoesäuremethylester

7-Brom-4-(4-nitro-benzyl)-1-pyrrolidin-1-yl-4H-[1,2,4]triazol[4,3-a]chinazolin-5-on

7-Brom-4-(4-methoxy-benzyl)-1-pyrrolidin-1-yl-4H-[1,2,4]triazol[4,3-a]chinazolin-5-on

Essigsäure4-(7-brom-5-oxo-1-pyrrolidin-1-yl-5H-[1,2,4]triazol[4,3-a]chinazolin-4-ylmethyl)-phenylester

7-Brom-4-(4-hydroxy-benzyl)-1-pyrrolidin-1-yl-4H-[1,2,4]triazol[4,3-a]chinazolin-5-on

7-Brom-4-(3,4-dimethoxy-benzyl)-1-pyrrolidin-1-yl-4H-[1,2,4]triazol[4,3-a]chinazolin-5-on

4-Benzo[1,3]dioxol-5-ylmethyl-7-brom-1-pyrrolidin-1-yl-4H-[1,2,4]triazol[4,3-a]chinazolin-5-on

7-Brom-4-(3,5-dimethoxy-benzyl)-1-pyrrolidin-1-yl-4H-[1,2,4]triazol[4,3-a]chinazolin-5-on

7-Brom-1-pyrrolidin-1-yl-4-(3,4,5-trimethoxy-benzyl)-4H-[1,2,4]triazol[4,3-a]chinazolin-5-on

[4-(7-Brom-5-oxo-1-pyrrolidin-1-yl-5H-[1,2,4]triazol[4,3-a]chinazolin-4-ylmethyl)-phenyl]-essigsäure

1-(pyrrolidin-1-yl)-7-brom-4-(3-phenylallyl)-4H-[1,2,4]triazol[4,3-a]chinazolin-5-on

7-Brom-4-(3-phenyl-allyl)-1-pyrrolidin-1-yl-4H-[1,2,4]triazol[4,3-a]chinazolin-5-on

7-Brom-4-[(E)-3-(4-chlor-phenyl)-allyl]-1-pyrrolidin-1-yl-4H-[1,2,4]triazol[4,3-a]chinazolin-5-on

7-Brom-4-[3-(4-methoxy-phenyl)-allyl]-1-pyrrolidin-1-yl-4H-[1,2,4]triazol[4,3-a]chinazolin-5-on

7-Brom-4-(3-pyridin-3-yl-allyl)-1-pyrrolidin-1-yl-4H-[1,2,4]triazol[4,3-a]chinazolin-5-on

7-Brom-4-((E)-3-pyridin-4-yl-allyl)-1-pyrrolidin-1-yl-4H-[1,2,4]triazol[4,3-a]chinazolin-5-on

7-Brom-4-(1H-imidazol-4-ylmethyl)-1-pyrrolidin-1-yl-4H-[1,2,4]triazol[4,3-a]chinazolin-5-on

7-Brom-4-(3,5-dimethyl-isoxazol-4-ylmethyl)-1-pyrrolidin-1-yl-4H-[1,2,4]triazolo[4,3-a]chinazolin-5-on

7-Brom-4-cyclopentylmethyl-1-pyrrolidin-1-yl-4H-[1,2,4]triazol[4,3-a]chinazolin-5-on

7-Brom-4-butyl-1-pyrrolidin-1-yl-4H-[1,2,4]triazol[4,3-a]chinazolin-5-on

7-Brom-1-pyrrolidin-1-yl-4-(2,2,2-trifluor-ethyl)-4H-[1,2,4]triazol[4,3-a]chinazolin-5-on

7-Brom-4-(2-hydroxy-ethyl)-1-pyrrolidin-1-yl-4H-[1,2,4]triazol[4,3-a]chinazolin-5-on

7-Brom-4-(2-diethylainin-ethyl)-1-pyrrolidin-1-yl-4H-[1,2,4]triazol[4,3-a]chinazolin-5-on

7-Brom-4-prop-2-ynyl-1-pyrrolidin-1-yl-4H-[1,2,4]triazol[4,3-a]chinazolin-5-on

7-Brom-4-(2-phenoxy-ethyl)-1-pyrrolidin-1-yl-4H-[1,2,4]triazol[4,3-a]chinazolin-5-on

7-Brom-4-(2-phenylsulfenyl-ethyl)-1-pyrrolidin-1-yl-4H-[1,2,4]triazol[4,3-a]chinazolin-5-on

(7-Brom-5-oxo-1-pyrrolidin-1-yl-5H-[1,2,4]triazol[4,3-a]chinazolin-4-yl)-phenylessigsäuremethylester

4-(7-Brom-5-oxo-1-piperidin-1-yl-5H-[1,2,4]triazol[4,3-a]chinazolin-4-ylmethyl)-benzonitril

7-Brom-4-(3,4-dimethoxy-benzyl)-1-piperidin-1-yl-4H-[1,2,4]triazol[4,3-a]chinazolin-5-on

1-(Piperidin-1-yl)-7-brom-4-(3-phenylallyl)-4H-[1,2,4]triazol[4,3-a]chinazolin-5-on

7-Brom-4-(3-pyridin-3-yl-allyl)-1-thiomorpholin-4-yl-4H-[1,2,4]triazol[4,3-a]chinazolin-5-on

7-Brom-1-dimethylamino-4-(4-methyl-benzyl)-4H-[1,2,4]triazol[4,3-a]chinazolin-5-on

4-(7-Brom-1-dimethylamino-5-oxo-5H-[1,2,4]triazol[4,3-a]chinazolin-4-ylmethyl)-benzonitril

7-Brom-1-dimethylamino-4-(4-hydroxy-benzyl)-4H-[1,2,4]triazol[4,3-a]chinazolin-5-on

4-(7-Brom-1-dimethylamino-5-oxo-5H-[1,2,4]triazol[4,3-a]chinazolin-4-ylmethyl)-benzoesäuremethylester

[4-(7-Brom-1-dimethylamino-5-oxo-5H-[1,2,4]triazol[4,3-a]chinazolin-4-ylmethyl)-phenyl]-essigsäure

[4-(7-Brom-1-dimethylamino-5-oxo-5H-[1,2,4]triazol[4,3-a]chinazolin-4-ylmethyl)-phenyl]-acetonitril

7-Brom-1-dimethylamino-4-(pyridin-3-ylmethyl)-4H-[1,2,4]triazol[4,3-a]chinazolin-5-on

7-Brom-1-dimethylamino-4-(3-phenyl-allyl)-4H-[1,2,4]triazol[4,3-a]chinazolin-5-on

7-Brom-1-dimethylamino-4-(3-phenyl-allyl)-4H-[1,2,4]triazol[4,3-a]chinazolin-5-on

7-Brom-1-dimethylamino-4-(3-pyridin-4-yl-allyl)-4H-[1,2,4]triazol[4,3-a]chinazolin-5-on

7-Brom-1-dimethylamino-4-prop-2-ynyl-4H-[1,2,4]triazol[4,3-a]chinazolin-5-on

7-Brom-1-dimethylamino-4-(3-phenyl-prop-2-ynyl)-4H-[1,2,4]triazol[4,3-a]chinazolin-5-on

4-(7-Brom-1-dimethylamino-5-oxo-5H-[1,2,4]triazol[4,3-a]chinazolin-4-yl)-phenyl-essigsäuremethylester

1-Azepan-1-yl-7-methyl-4-pyridin-3-ylmethyl-4H-[1,2,4]triazol[4,3-a]chinazolin-5-on

1-Azepan-1-yl-7-methyl-4-(3-phenyl-allyl)-4H-[1,2,4]triazol[4,3-a]chinazolin-5-on

4-(7-Methyl-5-oxo-1-pyrrolidin-1-yl-5H-[1,2,4]triazol[4,3-a]chinazolin-4-ylmethyl)-benzonitril

4-(3,4-Dimethoxy-benzyl)-7-methyl-1-pyrrolidin-1-yl-4H-[1,2,4]triazol[4,3-a]chinazolin-5-on

4-(7-Methyl-5-oxo-1-pyrrolidin-1-yl-5H-[1,2,4]triazol[4,3-a]chinazolin-4-ylmethyl)-benzoesäuremethylester

[4-(7-Methyl-5-oxo-1-pyrrolidin-1-yl-5H-[1,2,4]triazol[4,3-a]chinazolin-4-ylmethyl)-phenyl]-essigsäure

7-Methyl-4-pyridin-3-ylmethyl-1-pyrrolidin-1-yl-4H-[1,2,4]triazol[4,3-a]chinazolin-5-on

7-Methyl-4-(3-phenyl-allyl)-1-pyrrolidin-1-yl-4H-[1,2,4]triazol[4,3-a]chinazolin-5-on

[4-(7-Methyl-5-oxo-1-thiomorpholin-4-yl-5H-[1,2,4]triazol[4,3-a]chinazolin-4-ylmethyl)-phenyl]-essigsäure

7-Methyl-4-(3-pyridin-3-yl-allyl)-1-thiomorpholin-4-yl-4H-[1,2,4]triazol[4,3-a]chinazolin-5-on

4-(1-Dimethylamino-7-methyl-5-oxo-5H-[1,2,4]triazol[4,3-a]chinazolin-4-ylmethyl)-benzonitril

[4-(Dimethylamino-methyl-oxo-5H-[1,2,4]triazol[4,3-a]chinazolin-4-ylmethyl)phenyl]-essigsäure

1-Dimethylamino-7-methyl-4-((E)-3-phenyl-allyl)-4H-[1,2,4]triazol[4,3-a]chinazolin-5-on

1-Dimethylamino-7-methyl-4-(3-pyridin-3-yl-allyl)-4H-[1,2,4]triazol[4,3-a]chinazolin-5-on

1-Dimethylamino-7-methyl-4-(3-pyridin-4-yl-allyl)-4H-[1,2,4]triazol[4,3-a]chinazolin-5-on

1-(Azepan-1-yl)-8-methyl-4-(3-phenylallyl)-4H-[1,2,4]triazol[4,3-a]chinazolin-5-on

(4-Cyan-benzyl)-dimethylamino-oxo-4,5-dihydro-[1,2,4]triazol[4,3-a]chinazolin-7-carbonitril

7-Hydroxy-4-((E)-3-phenyl-allyl)-1-pyrrolidin-1-yl-4H-[1,2,4]triazol[4,3-a]chinazolin-5-on

1-(Azepan-1-yl)-3-(3-phenylallyl)-3H-[1,2,4]triazol[4,3-a]-chinazolin-5-on

3-Allyl-1-azepan-1-yl-7-chlor-3H-[1,2,4]triazol[4,3-a]chinazolin-5-on

1-(Azepan-1-yl)-7-chlor-3-benzyl-3H-[1,2,4]triazol[4,3-a]-chinazolin-5-on

1-Azepan-1-yl-7-chlor-3-(4-methyl-benzyl)-3H-[1,2,4]triazol[4,3-a]chinazolin-5-on

1-(Azepan-1-yl)-7-chlor-3-(2-chlorbenzyl)-3H-[1,2,4]triazol[4,3-a]chinazolin-5-on

1-(Azepan-1-yl)-7-chlor-3-(3-chlorbenzyl)-3H-[1,2,4]triazol[4,3-a]chinazolin-5-on

1-(Azepan-1-yl)-7-chlor-3-(4-chlorbenzyl)-3H-[1,2,4]triazol[4,3-a]chinazolin-5-on

1-(Azepan-1-yl)-7-chlor-3-(4-brombenzyl)-3H-[1,2,4]triazol[4,3-a]chinazolin-5-on

1-(Azepan-1-yl)-7-chlor-3-(4-fluorbenzyl)-3H-[1,2,4]triazol[4,3-a]chinazolin-5-on

1-(Azepan-1-yl)-7-chlor-3-(4-(trifluormethyl)benzyl)-3H-[1,2,4]triazol[4,3-a]chinazolin-5-on

1-(Azepan-1-yl)-7-chlor-3-(4-cyanbenzyl)-3H-[1,2,4]triazol[4,3-a]chinazolin-5-on

1-(Azepan-1-yl)-7-chlor-3-(2-methoxybenzyl)-3H-[1,2,4]triazol[4,3-a]chinazolin-5-on

1-(Azepan-1-yl)-7-chlor-3-(3-methoxybenzyl)-3H-[1,2,4]triazol[4,3-a]chinazolin-5-on

1-(Azepan-1-yl)-7-chlor-3-(4-methoxybenzyl)-3H-[1,2,4]triazol[4,3-a]chinazolin-5-on

1-(Azepan-1-yl)-7-chlor-3-(3,4-dichlorbenzyl)-3H-[1,2,4]triazol[4,3-a]chinazolin-5-on

1-(Azepan-1-yl)-7-chlor-3-(3,4-dimethoxybenzyl)-3H-[1,2,4]triazol[4,3-a]chinazolin-5-on

1-(Azepan-1-yl)-7-chlor-3-(2-pyridylmethyl)-3H-[1,2,4]triazol[4,3-a]chinazolin-5-on

1-(Azepan-1-yl)-7-ehloro-3-(3-pyridylmethyl)-3H-[1,2,4]triazol[4,3-a]chinazolin-5-on

1-(Azepan-1-yl)-7-chlor-3-(2-phenylethyl)-3H-[1,2,4]triazol[4,3-a]chinazolin-5-on

1-(Azepan-1-yl)-7-chlor-3-[2-(4-methoxyphenyl)ethyl]-3H-[1,2,4]triazol[4,3-a]chinazolin-5-on

1-(Azepan-1-yl)-7-chlor-3-(3-phenylpropyl)-3H-[1,2,4]triazol[4,3-a]chinazolin-5-on

1-Azepan-1-yl-7-chlor-3-(2-oxo-2-phenyl-ethyl)-3H-[1,2,4]triazol[4,3-a]chinazolin-5-on

1-(Azepan-1-yl)-7-chlor-3-[2-(4-methoxyphenyl)-2-oxoethyl]-3H-[1,2,4]triazol[4,3-a]chinazolin-5-on

1-(Azepan-1-yl)-7-chlor-3-[2-(4-chlorphenyl)-2-oxoethyl)-3H-[1,2,4]triazol[4,3-a]chinazolin-5-on

5-[(1-(Azepan-1-yl)-7-chlor-5-oxo-5H-[1,2,4]triazol[4,3-a]-chinazolin-3yl)acetyl]-2-methoxybenzoesäuremethylester

1-(Azepan-1-yl)-7-brom-3-(4-chlorbenzyl)-3H-[1,2,4]triazol[4,3-a]chinazolin-5-on

1-(Azepan-1-yl)-7-brom-3-(4-fluorbenzyl)-3H-[1,2,4]triazol[4,3-a]chinazolin-5-on

4-(1-(Azepan-1-yl)-7-brom-5-oxo-5H-[1,2,4]triazol[4,3-a]-chinazolin-3-ylmethyl)-benzonitril

1-(Azepan-1-yl)-7-brom-3-(3,4-dimethoxybenzyl)-3H-[1,2,4]triazol[4,3-a]chinazolin-5-on

[4-(7-Brom-5-oxo-1-perhydro-azepin-1-yl-5H-[1,2,4]triazol[4,3-a]chinazolin-3-ylmethyl)-phenyl]-essigsäure

1-(Azepan-1-yl)-7-brom-3-(pyridin-3-ylmethyl)-3H-[1,2,4]triazol[4,3-a]chinazolin-5-on

1-Azepan-1-yl-7-brom-3-((E)-3-phenyl-allyl)-3H-[1,2,4]triazol[4,3-a]chinazolin-5-on

7-Brom-3-((E)-3-phenyl-allyl)-1-piperidin-1-yl-3H-[1,2,4]triazol[4,3-a]chinazolin-5-on

7-Brom-3-(4-chlorbenzyl)-1-(pyrrolidin-1-yl)-3H-[1,2,4]triazol[4,3-a]chinazolin-5-on

7-Brom-3-(4-fluorbenzyl)-1-(pyrrolidin-1-yl)-3H-[1,2,4]triazol[4,3-a]chinazolin-5-on

4-(7-Brom-5-oxo-1-(pyrrolidin-1-yl)-5H-[1,2,4]triazol[4,3-a]-chinazolin-3-ylmethyl)-benzonitril

4-(7-Brom-5-oxo-1-(pyrrolidin-1-yl)-5H-[1,2,4]triazol[4,3-a]-chinazolin-3-ylmethyl)benzoesäuremethylester

7-Brom-3-(4-methoxy-benzyl)-1-pyrrolidin-1-yl-3H-[1,2,4]triazol[4,3-a]chinazolin-5-on

Essigsäure 4-(7-brom-5-oxo-1-pyrrolidin-1-yl-5H-[1,2,4]triazol[4,3-a]chinazolin-3-ylmethyl)-phenylester(?)

7-Brom-1-dimethylamino-3-(4-hydroxy-benzyl)-3H-[1,2,4]triazol[4,3-a]chinazolin-5-on

3-(Benzo[1,3]dioxol-5-ylmethyl)-7-brom-1-(pyrrolidin-1-yl)-3H-[1,2,4]triazol[4,3-a]chinazolin-5-on

7-Brom-3-(3,5-dimethoxy-benzyl)-1-(pyrrolidin-1-yl)-3H-[1,2,4]triazol-[4,3-a]chinazolin-5-on

7-Brom-1-(pyrrolidin-1-yl)-3-(3,4,5-trimethoxybenzyl)-3H-[1,2,4]triazol[4,3-a]chinazolin-5-on

7-Brom-3-(1H-imidazol-4-ylmethyl)-1-pyrrolidin-1-yl-3H-[1,2,4]triazol[4,3-a]chinazolin-5-on

7-Brom-3-(n-butyl)-1-(pyrrolidin-1-yl)-3H-[1,2,4]triazol[4,3-a]-chinazolin-5-on

(7-Brom-5-oxo-1-pyrrolidin-1-yl-5H-[1,2,4]triazol[4,3-a]chinazolin-3-yl)-phenylessigsäuremethylester

7-Brom-1-dimethylamino-3-(3-phenyl-allyl)-3H-[1,2,4]triazol[4,3-a]chinazolin-5-on

4-(7-Brom-1-dimethylamino-5-oxo-5H-[1,2,4]triazol[4,3-a]chinazolin-3-yl)-phenyl-essigsäuremethylester

1-(Azepan-1-yl)-7-methyl-3-(3-phenylallyl)-3H-[1,2,4]triazol[4,3-a]chinazolin-5-on

7-Methyl-3-(3-phenylallyl)-1-(pyrrolidin-1-y1)-3H-[1,2,4]triazol[4,3-a]chinazolin-5-on

1-(Azepan-1-yl)-3,8-dimethyl-3H-[1,2,4]triazol[4,3-a]-chinazolin-5-on

1-Azepan-1-yl-8-methyl-3-((E)-3-pheny]-allyl)-3H-[1,2,4]triazol[4,3-a]chinazolin-5-on

7-Hydroxy-3-(3-phenylallyl)-1-(pyrrolidin-1-yl)-3H-[1,2,4]triazol[4,3-a]chinazolin-5-on

1,8-bis(Azepan-1-yl)-3-(3-phenylallyl)-3H-[1,2,4]triazol[4,3-a]chinazolin-5-on

1-(Azepan-1-yl)-4-benzyl-7-brom-4H-[1,2,4]triazol[4,3-a]-chinazolin-5-on

4-Benzyl-7-brom-1-(pyrrolidin-1-yl)-4H-[1,2,4]triazol[4,3-a]-chinazolin-5-on

4-Benzyl-7-brom-1-(butyl-methyl-amino)-4H-[1,2,4]triazol[4,3-a]chinazolin-5-on

4-Benzyl-1-(pyrrolidin-1-yl)-4H-[1,2,4]triazol[4,3-a]chinazolin-5-on

7-Chlor-1-dibutylamino-4-methyl-4H-[1,2,4]triazol[4,3-a]-chinazolin-5-on

7-Chlor-4-methyl-1-(piperidin-1-yl)-4H-[1,2,4]triazol[4,3-a]-chinazolin-5-on

7-Chlor-4-methyl-1-(4-methyl-piperazin-1-yl)-4H-[1,2,4]triazol[4,3-a]chinazolin-5-on

7-Chlor-4-methyl-1-(1,8,8-trimethyl-3-aza-bicyclo[3.2.1]oct-3-yl)-4H-[1,2,4]triazol[4,3-a]chinazolin-5-on

1-(Azepan-1-yl)-7-chlor-4-phenyl-4H-[1,2,4]triazol[4,3-a]-chinazolin-5-on

1-(Azepan-1-yl)-4-benzyl-7-chlor-4H-[1,2,4]triazol[4,3-a]-chinazolin-5-on

4-Benzyl-7-chlor-1-(pyrrolidin-1-yl)-4H-[1,2,4]triazol[4,3-a]-chinazolin-5-on

4-Benzyl-7-chior-1-(piperidin-1-yl)-4H-[1,2,4]triazol[4,3-a]-chinazolin-5-on

1-(Azepan-1-yl)-8-chlor-4-methyl-4H-[1,2,4]triazol[4,3-a]-chinazolin-5-on

1-(Azepan-1-yl)-4-benzyl-8-chlor-4H-[1,2,4]triazol[4,3-a]-chinazolin-5-on

1-(Azepan-1-yl)-7-brom-4-methyl-4H-[1,2,4]triazol[4,3-a]-chinazolin-5-on

4-Benzyl-7-brom-1-(piperidin-1-yl)-4H-[1,2,4]triazol[4,3-a]-chinazolin-5-on

4-Benzyl-7-brom-1-dimethylamino-4H-[1,2,4]triazol[4,3-a]chinazolin-5-on

4-Benzyl-7-brom-1-morpholin-4-yl-4H-[1,2,4]triazol[4,3-a]chinazolin-5-on

4-Benzyl-7-brom-1-thiomorpholin-4-yl-4H-[1,2,4]triazol[4,3-a]chinazolin-5-on

4-Benzyl-7-brom-1-(4-methyl-piperazin-1-yl)-4H-[1,2,4]triazol[4,3-a]chinazolin-5-on

4-Benzyl-7-brom-1-(4-phenyl-piperazin-1-yl)-4H-[1,2,4]triazol[4,3-a]chinazolin-5-on

4-Benzyl-1-(4-benzyl-piperazin-1-yl)-7-brom-4H-[1,2,4]triazol[4,3-a]chinazolin-5-on

4-Benzyl-7-brom-1-(3,6-dihydro-2H-pyridin-1-yl)-4H-[1,2,4]triazol[4,3-a]chinazolin-5-on

4-Benzyl-7-brom-1-(2,5-dihydro-pyrrol-1-yl)-4H-[1,2,4]triazol[4,3-a]chinazolin-5-on

4-Benzyl-7-brom-1-(3-hydroxy-pyrrolidin-1-yl)-4H-[1,2,4]triazol[4,3-a]chinazolin-5-on

4-Benzyl-7-brom-1-methylamino-4H-[1,2,4]triazol[4,3-a]chinazolin-5-on

4-Benzyl-7-iod-1-pyrrolidin-1-yl-4H-[1,2,4]triazol[4,3-a]chinazolin-5-on

1-Azepan-1-yl-4-benzyl-7-methyl-4H-[1,2,4]triazol[4,3-a]chinazolin-5-on

4-Benzyl-7-methyl-1-pyrrolidin-1-yl-4H-[1,2,4]triazol[4,3-a]chinazolin-5-on

4-Benzyl-1-dimethylamino-7-methyl-4H-[1,2,4]triazol[4,3-a]chinazolin-5-on

4-Benzyl-7-methyl-1-thiomorpholin-4-yl-4H-[1,2,4]triazol[4,3-a]chinazolin-5-on

1-Azepan-1-yl-4-benzyl-8-methyl-4H-[1,2,4]triazol[4,3-a]chinazolin-5-on

1-Azepan-1-yl-4-benzyl-7-methoxy-4H-[1,2,4]triazol[4,3-a]chinazolin-5-on

4-Benzyl-7-methoxy-1-pyrrolidin-1-yl-4H-[1,2,4]triazol[4,3-a]chinazolin-5-on

4-Benzyl-5-oxo-1-pyrrolidin-1-yl-4,5-dihydro-[1,2,4]triazol[4,3-a]chinazolin-7-carbonitril

1-Azepan-1-yl-4-benzyl-7-nitro-4H-[1,2,4]triazol[4,3-a]chinazolin-5-on

1-(Azepan-1-yl)-4-benzyl-7-chlor-4H-[1,2,4]triazol[4,3-a]-chinazolin-5-on

1-(Azepan-1-yl)-4-methyl-4H-[1,2,4]triazol[4,3-a]chinazolin-5-on

1-(Azepan-1-yl)-4-benzyl-4H-[1,2,4]triazol[4,3-a]chinazolin-5-on

1-(Azepan-1-yl)-6-chlor-4-methyl-4H-[1,2,4]triazol[4,3-a]-chinazolin-5-on

1-(Azepan-1-yl)-7-chlor-4-methyl-4H-[1,2,4]triazol[4,3-a]-chinazolin-5-on

1-(Azepan-1-yl)-7-chlor-4-ethyl-4H-[1,2,4]triazol[4,3-a]-chinazolin-5-on

7-Chlor-4-methyl-1-(pyrrolidin-1-yl)-4H-[1,2,4]triazol[4,3-a]-chinazolin-5-on

7-Chlor-4-methyl-1-(morpholin-4-yl)-4H-[1,2,4]triazol[4,3-a]-chinazolin-5-on

1-(Azocan-1-yl)-7-chlor-4-methyl-4H-[1,2,4]triazol[4,3-a]-chinazolin-5-on

7-Chlor-1-(3,4-dihydro-2H-chinolin-1-yl)-4-methyl-4H-[1,2,4]triazol[4,3-a]chinazolin-5-on

7-Chlor-1-(3,4-dihydro-1H-isochinolin-2-yl)-4-methyl-4H-[1,2,4]triazol[4,3-a]chinazolin-5-on

1-(4-Benzylpiperidin-1-yl)-7-chlor-4-methyl-4H-[1,2,4]triazol[4,3-a]chinazolin-5-on

7-Chlor-4-methyl-1-(1,3,3-trimethyl-6-azabicyclo[3,2,1]oct-6-yl)-4H-[1,2,4]triazol[4,3-a]-chinazolin-5-on

1-(Azepan-1-yl)-7-fluor-4-methyl-4H-[1,2,4]triazol[4,3-a]-chinazolin-5-on

1-(Azepan-1-yl)-7-iod-4-methyl-4H-[1,2,4]triazol[4,3-a]-chinazolin-5-on

1-(Azepan-1-yl)-7-methoxy-4-methyl-4H-[1,2,4]triazol[4,3-a]-chinazolin-5-on

4-Benzyl-7-brom-1-(ethyl-methyl-amino)-4H-[1,2,4]triazol[4,3-a]chinazolin-5-on

4-Benzyl-1-diethylamino-7-methyl-4H-[1,2,4]triazol[4,3-a]chinazolin-5-on

4-Benzyl-7-brom-1-pyrrol-1-yl-4H-[1,2,4]triazol[4,3-a]chinazolin-5-on

4-(4-Amino-benzyl)-7-brom-1-pyrrolidin-1-yl-4H-[1,2,4]triazol[4,3-a]chinazolin-5-on

4-Benzyl-7-hydroxy-1-pyrrolidin-1-yl-4H-[1,2,4]triazol[4,3-a]chinazolin-5-on

4-(7-Hydroxy-5-oxo-1-pyrrolidin-1-yl-5H-[1,2,4]triazol[4,3-a]chinazolin-4-ylmethyl)-benzonitril

N-(4-Benzyl-5-oxo-1-pyrrolidin-1-yl-4,5-dihydro-[1,2,4]triazol[4,3-a]chinazolin-7yl)acetamid

N-[5-Oxo-4-(3-phenyl-allyl)-1-pyrrolidin-1-yl-4,5-dihydro-[1,2,4]triazol[4,3-a]chinazolin-7-yl]-acetamid

7-Amino-4-((E)-3-phenyl-allyl)-1-pyrrolidin-1-yl-4H-[1,2,4]triazol[4,3-a]chinazolin-5-on

7-Amino-1-azepan-1-yl-4-benzyl-4H-[1,2,4]triazol[4,3-a]chinazolin-5-on

7-Amino-4-benzyl-1-pyrrolidin-1-yl-4H-[1,2,4]triazol[4,3-a]chinazolin-5-on

4-(7-Amino-5-oxo-1-pyrrolidin-1-yl-5H-[1,2,4]triazol[4,3-a]chinazolin-4-ylmethyl)-benzonitril

7-Amino-4-((E)-3-pyridin-3-yl-allyl)-1-pyrrolidin-1-yl-4H-[1,2,4]triazol[4,3a]chinazolin-5-on

4-(Amino-dimethylamino-oxo-5H-[1,2,4]triazol[4,3-a]chinazolin-4-ylmethyl)-benzonitril

7-Amino-1-dimethylamino-4-((E)-3-phenyl-allyl)-4H-[1,2,4]triazol[4,3-a]chinazolin-5-on

4-Benzyl-7-methylamino-1-pyrrolidin-1-yl-4H-[1,2,4]triazol[4,3-a]chinazolin-5-on

4-(7-Methylamino-5-oxo-1-pyrrolidin-1-yl-5H-[1,2,4]triazol[4,3-a]chinazolin-4-ylmethyl)-benzonitril

4-Benzyl-8-methylamino-1-pyrrolidin-1-yl-4H-[1,2,4]triazol[4,3-a]chinazolin-5-on

4-Benzyl-7-ethylamino-1-pyrrolidin-1-yl-4H-[1,2,4]triazol[4,3-a]chinazolin-5-on

4-Benzyl-7-isopropylamino-1-pyrrolidin-1-yl-4H-[1,2,4]triazol[4,3-a]chinazolin-5-on

N-(4-Benzyl-5-oxo-1-pyrrolidin-1-yl-4,5-dihydro-[1,2,4]triazol[4,3-a]chinazolin-7-yl)-methansulfonamid

4-Benzyl-7-dimethylamino-1-pyrrolidin-1-yl-4H-[1,2,4]triazol[4,3-a]chinazolin-5-on

4-Benzyl-1-dimethylamino-5-oxo-4,5-dihydro-[1,2,4]triazol[4,3-a]chinazolin-7-carbonitril

4-Benzyl-5-oxo-1-pyrrolidin-1-yl-4,5-dihydro-[1,2,4]triazol[4,3-a]chinazolin-7-carbonsäure

[4-(7-Brom-5-oxo-1-pyrrolidin-1-yl-5H-[1,2,4]triazol[4,3-a]chinazolin-4-ylmethyl)-phenyl]-essigsäuremethylester

2-[4-(7-Brom-5-oxo-1-pyrrolidin-1-yl-5H-[1,2,4]triazol[4,3-a]chinazolin-4-ylmethyl)-phenyl]-N-methylacetamid

2-[4-(7-Brom-1-dimethylamino-5-oxo-5H-[1,2,4]triazol[4,3-a]chinazolin-4-ylmethyl)-phenyl]-acetamid

2-[4-(7-Brom-5-oxo-1-pyrrolidin-1-yl-5H-[1,2,4]triazol[4,3-a]chinazolin-4-ylmethyl)-phenyl]-N,N-dimethylacetamid

2-[4-(7-Brom-5-oxo-1-pyrrolidin-1-yl-5H-[1,2,4]triazol[4,3-a]chinazolin-4-ylmethyl)-phenyl]-N-hydroxyacetamid

4-(1-Dimethylamino-7-methyl-5-thioxo-5H-[1,2,4]triazol[4,3-a]chinazolin-4-ylmethyl)-benzonitril

4-(7-Brom-1-dimethylamino-5-thioxo-5H-[1,2,4]triazol[4,3-a]chinazolin-4-ylmethyl)-benzonitril

1-Dimethylamino-7-methyl-4-(3-pyridin-3-yl-allyl)-4H-[1,2,4]triazol[4,3-a]chinazolin-5-thion

4-Benzyl-7-(N,N-dimethylsulfonylamino)-1-(pyrrolidin-1-yl)-4H-[1,2,4]triazol[4,3-a]chinazolin-5-on

**8.** Triazol[4,3-a]chinazolin-5-one und/oder -5-thione nach einem der Ansprüche 1 bis 4, ausgewählt aus der Gruppe, welche umfaßt:

1-(Azepan-1-yl)-7-chlor-4-(3-phenylallyl)-4H-[1,2,4]triazol[4,3-a]chinazolin-5-on

7-Brom-1-dimethylamino-4-((E)-3-pyridin-3-yl-allyl)-4H-[1,2,4]triazol[4,3-a]chinazolin-5-on

1-(Azepan-1-yl)-7-chlor-4-(4-chlorbenzyl)-4H-[1,2,4]triazol[4,3-a]chinazolin-5-on

1-(Azepan-1-yl)-7-chlor-4-(4-fluorbenzyl)-4H-[1,2,4]triazol[4,3-a]chinazolin-5-on

1-(Azepan-1-yl)-7-chlor-4-(3,4-dimethoxybenzyl)-4H-[1,2,4]triazol[4,3-a]chinazolin-5-on

1-(Azepan-1-yl)-7-chlor-4-(3-pyridylmethyl)-4H-[1,2,4]triazol[4,3-a]chinazolin-5-on

1-(Azepan-1-yl)-7-brom-4-(4-chlorphenylmethyl)-4H-[1,2,4]triazol[4,3-a]chinazolin-5-on

1-(Azepan-1-yl-7-brom-5-oxo-5H-[1,2,4]triazol[4,3-a]chinazolin-4-ylmethyl)-benzonitril

1-Azepan-1-yl-7-brom-4-(3,4-dimethoxy-benzyl)-4H-[1,2,4]triazol[4,3-a]chinazolin-5-on

1-(Azepan-1-yl)-7-brom-4-(3-phenylallyl)-4H-[1,2,4]triazol[4,3-a]chinazolin-5-on

1-Azepan-1-yl-7-brom-4-((E)-3-pyridin-3-yl-allyl)-4H-[1,2,4]triazol[4,3-a]chinazolin-5-on

1-Azepan-1-yl-7-brom-4-(3-pyridin-4-yl-allyl)-4H-[1,2,4]triazol[4,3-a]chinazolin-5-on

7-Brom-4-(4-methyl-benzyl)-1-pyrrolidin-1-yl-4H-[1,2,4]triazol[4,3-a]chinazolin-5-on

7-Brom-4-(4-chlor-benzyl)-1-pyrrolidin-1-yl-4H-[1,2,4]triazol[4,3-a]chinazolin-5-on

7-Brom-4-(4-fluor-benzyl)-1-pyrrolidin-1-yl-4H-[1,2,4]triazol[4,3-a]chinazolin-5-on

4-(7-Brom-5-oxo-1-pyrrolidin-1-yl-5H-[1,2,4]triazol[4,3-a]chinazolin-4-ylmethyl)-benzonitril

4-(7-Brom-5-oxo-1-pyrrolidin-1-yl-5H-[1,2,4]triazol[4,3-a]chinazolin-4-ylmethyl)benzoesäuremethylester

7-Brom-4-(4-nitro-benzyl)-1-pyrrolidin-1-yl-4H-[1,2,4]triazol[4,3-a]chinazolin-5-on

7-Brom-4-(4-methoxy-benzyl)-1-pyrrolidin-1-yl-4H-[1,2,4]triazol[4,3-a]chinazolin-5-on

Essigsäure 4-(7-brom-5-oxo-1-pyrrolidin-1-yl-5H-[1,2,4]triazol[4,3-a]chinazolin-4-ylmethyl)-phenylester

7-Brom-4-(4-hydroxy-benzyl)-1-pyrrolidin-1-yl-4H-[1,2,4]triazol[4,3-a]chinazolin-5-on

7-Brom-4-(3,4-dimethoxy-benzyl)-1-pyrrolidin-1-yl-4H-[1,2,4]triazol[4,3-a]chinazolin-5-on

1-(Pyrrolidin-1-yl)-7-brom-4-(3-phenylallyl)-4H-[1,2,4]triazol[4,3-a]chinazolin-5-on

7-Brom-4-[(E)-3-(4-chlor-phenyl)-allyl]-1-pyrrolidin-1-yl-4H-[1,2,4]triazol[4,3a]chinazolin-5-on

7-Brom-4-[3-(4-methoxy-phenyl)-allyl]-1-pyrrolidin-1-yl-4H-[1,2,4]triazol[4,3-a]chinazolin-5-on

7-Brom-4-(3-pyridin-3-yl-allyl)-1-pyrrolidin-1-yl-4H-[1,2,4]triazol[4,3-a]chinazolin-5-on

7-Brom-4-((E)-3-pyridin-4-yl-allyl)-1-pyrrolidin-1-yl-4H-[1,2,4)triazol[4,3-a]chinazolin-5-on

7-Brom-4-(3,4-dimethoxy-benzyl)-1-piperidin-1-yl-4H-[1,2,4]triazol[4,3-a]chinazolin-5-on

1-(Piperidin-1-yl)-7-brom-4-(3-phenylallyl)-4H-[1,2,4]triazol[4,3-a]chinazolin-5-on

7-Brom-1-dimethylamino-4-(4-methyl-benzyl)-4H-[1,2,4]triazol[4,3-a]chinazolin-5-on

4-(7-Brom-1-dimethylamino-5-oxo-5H-[1,2,4]triazol[4,3-a]chinazolin-4-ylmethyl)-benzonitril

7-Brom-1-dimethylamino-4-(4-hydroxy-benzyl)-4H-[1,2,4]triazol[4,3-a]chinazolin-5-on

4-(7-Brom-1-dimethylamino-5-oxo-5H-[1,2,4]triazol[4,3-a]chinazolin-4-ylmethyl)benzoesäuremethylester

[4-(7-Brom-1-dimethylamino-5-oxo-5H-[1,2,4]triazol[4,3-a]chinazolin-4-ylmethyl)-phenyl]-acetonitril

7-Brom-1-dimethylamino-4-(3-phenyl-allyl)-4H-[1,2,4]triazol[4,3-a]chinazolin-5-on

7-Brom-1-dimethylamino-4-(3-phenyl-prop-2-ynyl)-4H-[1,2,4]triazol[4,3-a]chinazolin-5-on

1-Azepan-1-yl-7-methyl-4-(3-phenyl-allyl)-4H-[1,2,4]triazol[4,3-a]chinazolin-5-on

4-(3,4-Dimethoxy-benzyl)-7-methyl-1-pyrrolidin-1-yl-4H-[1,2,4]triazol[4,3-a]chinazolin-5-on

[4-(7-Methyl-5-oxo-1-pyrrolidin-1-yl-5H-[1,2,4]triazol[4,3-a]chinazolin-4-ylmethyl)phenyl]-essigsäure

7-Methyl-4-(3-phenyl-allyl)-1-pyrrolidin-1-yl-4H-[1,2,4]triazol[4,3-a]chinazolin-5-on

[4-(Dimethylamino-methyl-oxo-5H-[1,2,4]triazol[4,3-a]chinazolin-4-ylmethyl)-phenyl]essigsäure

1-Dimethylamino-7-methyl-4-((E)-3-phenyl-allyl)-4H-[1,2,4]triazol[4,3-a]chinazolin-5-on

1-Dimethylamino-7-methyl-4-(3-pyridin-3-yl-allyl)-4H-[1,2,4]triazol[4,3-a]chinazolin-5-on

[4-(7-Brom-5-oxo-1-perhydro-azepin-1-yl-5H-[1,2,4]triazol[4,3-a]chinazolin-3-ylmethyl)-phenyl]-essigsäure

4-Benzyl-7-brom-1-(pyrrolidin-1-yl)-4H-[1,2,4]triazol[4,3-a]-chinazolin-5-on

4-Benzyl-7-brom-1-(2,5-dihydro-pyrrol-1-yl)-4H-[1,2,4]triazol[4,3-a]chinazolin-5-on

4-Benzyl-7-iod-1-pyrrolidin-1-yl-4H-[1,2,4]triazol[4,3-a]chinazolin-5-on

1-Azepan-1-yl-4-benzyl-7-methyl-4H-[1,2,4]triazol[4,3-a]chinazolin-5-on

4-(4-Amino-benzyl)-7-brom-1-pyrrolidin-1-yl-4H-[1,2,4]triazol[4,3-a]chinazolin-5-on

7-Amino-4-((E)-3-phenyl-allyl)-1-pyrrolidin-1-yl-4H-[1,2,4]triazol[4,3-a]chinazolin-5-on

7-Amino-1-azepan-1-yl-4-benzyl-4H-[1,2,4]triazol[4,3-a]chinazolin-5-on

7-Amino-4-((E)-3-pyridin-3-yl-allyl)-1-pyrrolidin-1-yl-4H-[1,2,4]triazol[4,3-a]chinazolin-5-on

7-Amino-1-dimethylamino-4-((E)-3-phenyl-allyl)-4H-[1,2,4]triazol[4,3-a]chinazolin-5-on

4-Benzyl-7-methylamino-1-pyrrolidin-1-yl-4H-[1,2,4]triazol[4,3-a]chinazolin-5-on

4-(7-Methylamino-5-oxo-1-pyrrolidin-1-yl-5H-[1,2,4]triazol[4,3-a]chinazolin-4-ylmethyl)-benzonitril

4-Benzyl-8-methylamino-1-pyrrolidin-1-yl-4H-[1,2,4]triazol[4,3-a]chinazolin-5-on

4-Benzyl-7-ethylamino-1-pyrrolidin-1-yl-4H-[1,2,4]triazol[4,3-a]chinazolin-5-on

4-Benzyl-7-isopropylamino-1-pyrrolidin-1-yl-4H-[1,2,4]triazol[4,3-a]chinazolin-5-on

[4-(7-Brom-5-oxo-1-pyrrolidin-1-yl-5H-[1,2,4]triazol[4,3-a]chinazolin-4-ylmethyl)phenyl]-essigsäuremethylester

2-[4-(7-Brom-5-oxo-1-pyrrolidin-1-yl-5H-[1,2,4]triazol[4,3-a]chinazolin-4-ylmethyl)phenyl]-N-methylacetamid

2-[4-(7-Brom-1-dimethylamino-5-oxo-5H-[1,2,4]triazol[4,3-a]chinazolin-4-ylmethyl)phenyl]-acetamid

2-[4-(7-Brom-5-oxo-1-pyrrolidin-1-yl-5H-[1,2,4]triazol[4,3-a]chinazolin-4-ylmethyl)phenyl]-N,N-dimethylacetamid

2-[4-(7-Brom-5-oxo-1-pyrrolidin-1-yl-5H-[1,2,4]triazol[4,3-a]chinazolin-4-ylmethyl)phenyl]-N-hydroxyacetamid

1-Dimethylamino-7-methyl-4-(3-pyridin-3-yl-allyl)-4H-[1,2,4]triazol[4,3-a]chinazolin-5-thion

**9.** Triazol[4,3-a]chinazolin-5-one und/oder -5-thione nach einem der Ansprüche 1 bis 4, ausgewählt aus der Gruppe, welche umfaßt:

7-Brom-1-dimethylamino-4((E)-3-pyridin-3-yl-allyl)-4H-[1,2,4]triazol[4,3-a]chinazolin-5-on

1-(Azepan-1-yl)-7-chlor-4-(3,4-dimethoxybenzyl)-4H-[1,2,4]triazol[4,3-a]chinazolin-5-on

1-(Azepan-1-yl)-7-chlor-4-(3-pyridylmethyl)-4H-[1,2,4]triazol[4,3-a]chinazolin-5-on

4-(1-Azepan-1-yl-7-brom-5-oxo-5H-[1,2,4]triazol[4,3-a]chinazolin-4-ylmethyl)-benzonitril

1-Azepan-1-yl-7-brom-4-(3,4-dimethoxy-benzyl)-4H-[1,2,4]triazol[4,3-a]chinazolin-5-on

1-(Azepan-1-yl)-7-brom-4-(3-phenylallyl)-4H-[1,2,4]triazol[4,3-a]chinazolin-5-on

1-Azepan-1-yl-7-brow-4-((E)-3-pyridin-3-yl-allyl)-4H-[1,2,4]triazol[4,3-a]chinazolin-5-on

1-Azepan-1-yl-7-brom-4-(3-pyridin-4-yl-allyl)-4H-[1,2,4]triazol[4,3-a]chiazolin-5-on

7-Brom-4-(4-methyl-benzyl)-1-pyrrolidin-1-yl-4H-[1,2,4]triazol[4,3-a]chinazolin-5-on

7-Brom-4-(4-chlor-benzyl)-1-pyrrolidin-1-yl-4H-[1,2,4]triazol[4,3-a]chinazolin-5-on

7-Brom-4-(3,4-dimethoxy-benzyl)-1-pyrrolidin-1-yl-4H-[1,2,4]triazol[4,3-a]chinazolin-5-on

1-(Pyrrolidin-1-yl)-7-brom-4-(3-phenylallyl)-4H-[1,2,4]triazol[4,3-a]chinazolin-5-on

7-Brom-4-(3-pyridin-3-yl-allyl)-1-pyrrolidin-1-yl-4H-[1,2,4]triazol[4,3-a]chinazolin-5-on

1-(Piperidin-1-yl)-7-brom-4-(3-phenylallyl)-4H-[1,2,4]triazol[4,3-a]chinazolin-5-on

4-(7-Brom-1-dimethylamino-5-oxo-5H-[1,2,4]triazol[4,3-a]chinazolin-4-ylmethyl)-benzonitril

4-(Brom-dimethylamino-oxo-5H-[1,2,4]triazol[4,3-a]chinazolin-4-ylmethyl)-benzoesäuremethylester

7-Brom-1-dimethylamino-4-(3-phenyl-allyl)-4H-[1,2,4]triazol[4,3-a]chinazolin-5-on

7-Brom-1-dimethylamino-4-(3-phenyl-prop-2-ynyl)-4H-[1,2,4]triazol[4,3-a]chinazolin-5-on

1-Azepan-1-yl-7-methyl-4-(3-phenyl-allyl)-4H-[1,2,4]triazol[4,3-a]chinazolin-5-on

4-(3,4-Dimethoxy-benzyl)-7-methyl-1-pyrrolidin-1-yl-4H-[1,2,4]triazol[4,3a]chinazolin-5-on

7-Methyl-4-(3-phenyl-allyl)-1-pyrrolidin-1-yl-4H-[1,2,4]triazol[4,3-a]chinazolin-5-on

7-Amino-4-((E)-3-phenyl-allyl)-1-pyrrolidin-1-yl-4H-[1,2,4]triazol[4,3-a]chinazolin-5-on

7-Amino-4-((E)-3-pyridin-3-yl-allyl)-1-pyrrolidin-1-yl-4H-[1,2,4]triazol[4,3-a]chinazolin-5-on

4-Benzyl-7-methylamino-1-pyrrolidin-1-yl-4H-[1,2,4]triazol[4,3-a]chinazolin-5-on

4-(7-Methylamino-5-oxo-1-pyrrolidin-1-yl-5H-[1,2,4]triazol[4,3-a]chinazolin-4-ylmethyl)-benzonitril

[4-(7-Brom-5-oxo-1-pyrrolidin-1-yl-5H-[1,2,4]triazol[4,3-a]chinazolin-4-ylmethyl)phenyl]-essigsäuremethylester

2-[4-(7-Brom-5-oxo-1-pyrrolidin-1-yl-5H-[1,2,4]triazol[4,3-a]chinazolin-4-ylmethyl)phenyl]-N-methylacetamid

2-[4-(7-Brom-5-oxo-1-pyrrolidin-1-yl-5H-[1,2,4]triazol[4,3-a]chinazolin-4-ylmethyl)phenyl]-N,N-dimethylacetamid

1-Dimethylamino-7-methyl-4-(3-pyridin-3-yl-allyl)-4H-[1,2,4]triazol[4,3-a]chinazolin-5-thion

**10.** Intermediäre Verbindungen der allgemeinen Formel III:

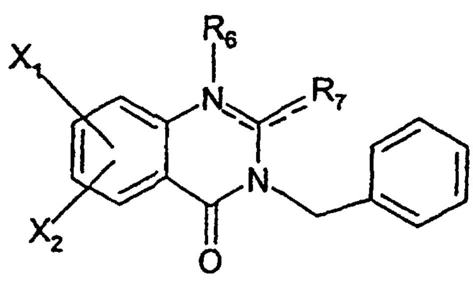

III

in der:

- $X_1$, $X_2$-und $A_1$ wie in Anspruch 1 definiert sind,
- wobei die gestrichelten Linien für optionale Doppelbindungen stehen,
- $R_6$ Wasserstoff ist; und
- $R_7$ S oder Hydrazin ist;

wobei $R_7$ an den Stickstoff an $R_6$ gebunden sein kann, um einen Ring zu bilden, insbesondere ein Triazol, der gegebenenfalls mit einer niederen Thioalkylgruppe, Mercapto oder Halogen substituiert sein kann, unter der Bedingung, daß, wenn $R_7$ S oder Hydrazin ist und $X_1$ Wasserstoff, Halogen, ein Alkylrest mit 1 bis 3 Kohlenstoffatomen, ein Alkoxyrest mit 1 bis 3 Kohlenstoffatomen oder ein Nitrorest ist, $X_2$ dann nicht Wasserstoff, Halogen, ein Alkylrest mit 1 bis 3 Kohlenstoffatomen, ein Alkoxyrest mit 1 bis 3 Kohlenstoffatomen oder ein Nitrorest ist.

**11.** Intermediäre Verbindungen der allgemeinen Formel IV:

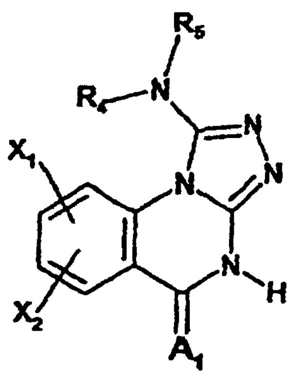

IV

in der $X_1$, $X_2$, $A_1$, $R_4$ und $R_5$ wie in Anspruch 1 definfert sind.

**12.** Intermediäre Verbindungen der allgemeinen Formel V:

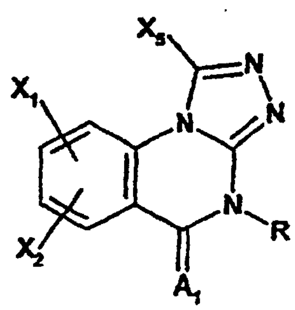

(V)

in der $X_1$, $X_2$, $A_1$ und R wie in Anspruch 1 definiert sind und $X_5$ eine Halogengruppe, insbesondere F, Br oder Cl, $-OCOX_7$, $-OSO_2X_7$ oder $-SO_2X_7$ ist, in denen $X_7$ eine niedere Alkyl- oder Arylgruppe ist.

**13.** Verfahren zur Herstellung der Verbindungen der allgemeinen Formel I und II:

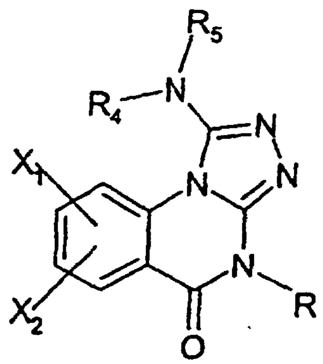

I

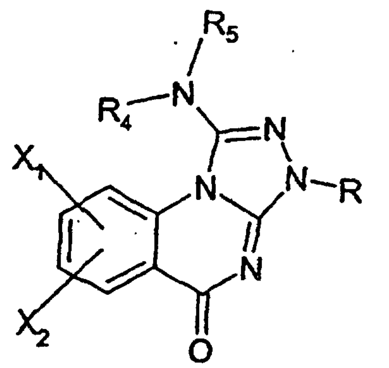

II

in denen $X_1$, $X_2$, R, $R_4$ und $R_5$ wie in Anspruch 1 definiert sind,
wobei das Verfahren **dadurch gekennzeichnet ist, daß** es die Umsetzung einer Verbindung der allgemeinen Formel IV:

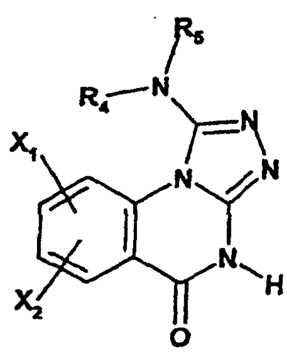

IV

in der $X_1$, $X_2$, $R_4$ und $R_5$ wie in Anspruch 1 definiert sind, mit einer Verbindung der allgemeinen Formel

R-X'

umfaßt, in der R wie vorstehend definiert ist, und X' eine Halogengruppe, insbesondere F, Br oder Cl, $-OCOX_7$ oder $-OSO_2X_7$ ist, wobei $X_7$ eine niedere Alkyl- oder eine Arylgruppe ist;
zr Herstellung einer Mischung der Verbindungen der allgemeinen Formel I und II, die daraufhin gegebenenfalls voneinander getrennt werden.

**14.** Verfahren zur Herstellung der Verbindungen der allgemeinen Formel I:

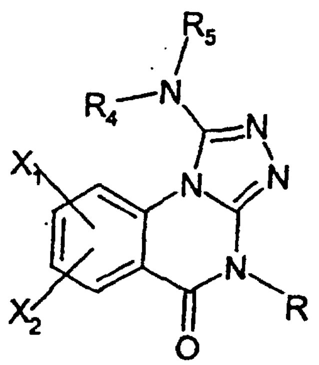

I

in denen $X_1$, $X_2$, R, $R_4$ und $R_5$ wie in Anspruch 1 definiert sind, wobei das Verfahren **dadurch gekennzeichnet ist, daß** es die Umsetzung einer Verbindung der allgemeinen Formel V:

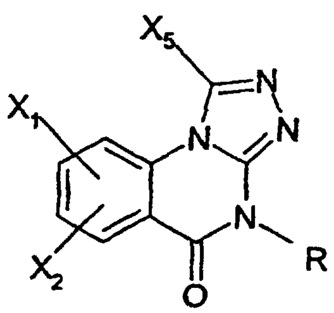

V

in der $X_1$, $X_2$ und R wie in Anspruch 1 definiert sind und $X_5$ eine Halogengruppe, insbesondere F, Br oder Cl, $-OCOX_7$, $-OSO_2X_7$ oder $-SO_2X_7$ ist, in denen $X_7$ eine niedere Alkylgruppe oder Arylgruppe ist, mit einer Verbindung der allgemeinen Formel:

$$HNR_4R_5$$

umfaßt, in der $R_4$ und $R_5$ wie in Anspruch 1 definiert sind, zum Herstellen einer Verbindung der allgemeinen Formel I.

**15.** Verfahren nach Anspruch 14, **dadurch gekennzeichnet, daß**, wenn $X_1$-$NR_2R_3$ ist und $-NR_2R_3$ und $-NR_4R_5$ identisch sind, die Verbindungen der Formel I hergestellt werden durch Umsetzen einer Verbindung der allgemeinen Formel VI:

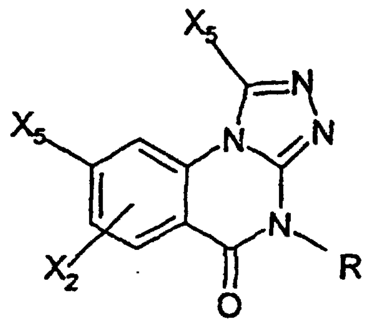

VI

in der $X_2$ und R wie in Ansprnch 1 definiert sind und $X_5$ wie in Anspruch 12 definiert ist, mit einer Verbindung der allgemeinen Formel:

$$HNR_2R_3$$

in der $R_2$ und $R_3$ wie in Anspruch 1 definiert sind,
zum Herstellen einer Verbindung der allgemeinen Formel (I):

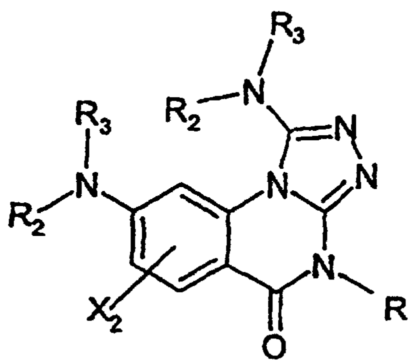

I

**16.** Verfahren nach Anspruch 14, **dadurch gekennzeichnet, daß**, wenn $X_1$ $-NR_2R_3$ ist und $-NR_2R_3$ und $-NR_4R_5$ verschieden sind, die Verbindungen der Formel I hergestellt werden durch Umsetzen einer Verbindung der allgemeinen Formel VII:

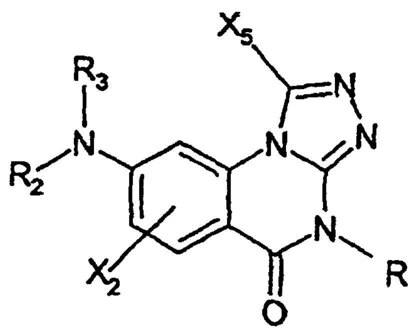

VII

in der $X_2$, $R_1$, $R_2$ und $R_3$ wie in Anspruch 1 definiert sind und $X_5$ wie in Anspruch 12 definiert ist, mit einer Verbindung der allgemeinen Formel

$$HNR_4R_5$$

in der $R_4$ und $R_5$ wie in Anspruch 1 definiert sind,zum Herstellen einer Verbindung der allgemeinen Formel (I):

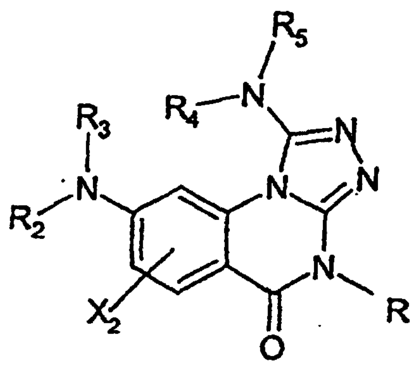

I

**17.** Pharmazeutische Zusammensetzung, welche eine Verbindung nach einem der Ansprüche 1 bis 9 und einen pharmazeutisch akzeptablen Exzipienten umfaßt.

**18.** Verwendung einer Verbindung nach einem der Ansprüche 1 bis 9 zur Herstellung eines Medikaments zur Behandlung einer Erkrankung oder eines Leidens, die bzw. das von einer Therapie mittels Inhibierung von Phosphodiesterasen und insbesondere von PDE4 herrührt.

**19.** Verwendung nach Anspruch 18, **dadurch gekennzeichnet, daß** es sich bei der Erkrankung um Asthma handelt.

**20.** Verwendung nach Anspruch 18, **dadurch gekennzeichnet, daß** es sich bei der Erkrankung um chronische Bronchitis oder ein akutes Lungenleiden handelt.

**21.** Verwendung nach Anspruch 18, **dadurch gekennzeichnet, daß** es sich bei der Erkrankung um atopische Dermatitis handelt.

**22.** Verwendung nach Anspruch 18, **dadurch gekennzeichnet, daß** es sich bei der Erkrankung um pulmonalen Hochdruck handelt.

23. Verwendung nach Anspruch 18, **dadurch gekennzeichnet, daß** es sich bei der Erkrankung um Herz- oder Lungeninsuffizienz handelt.

24. Verwendung nach Anspruch 18, **dadurch gekennzeichnet, daß** es sich bei der Erkrankung um Psoriasis handelt.

25. Verwendung nach Anspruch 18, **dadurch gekennzeichnet, daß** es sich bei der Erkrankung um eine entzündliche Erkrankung des Verdauungssystems wie hämorrhagische Proktokolitis oder Morbus Crohn handelt.

26. Verwendung nach Anspruch 18, **dadurch gekennzeichnet, daß** es sich bei der Erkrankung um Diabetes oder um eine Erkrankmg handelt, die mit einem erhöhten TNF-$\alpha$-Wert in Verbindung steht, wie etwa das Syndrom der akuten respiratorischen Insuffizienz und akute Pankreatitis.

27. Verwendung nach Anspruch 18, **dadurch gekennzeichnet, daß** es sich bei der Erkrankung um eine gutartige Prostatahypertrophie handelt.

28. Verwendung nach Anspruch 18, **dadurch gekennzeichnet, daß** die Erkrankung unter Gelenkrheumatismus und multipler Sklerose ausgewählt ist.

29. Verwendung nach Anspruch 18, **dadurch gekennzeichnet, daß** die Erkrankung unter Depression, durch Ischämie verursachter neuronaler Beeinträchtigung, und partieller zerebraler Ischämie ausgewählt ist.

30. Verwendung nach Anspruch 18, **dadurch gekennzeichnet, daß** es sich bei der Erkrankung um Krebs, insbesondere bösartige Tumore oder chronische lymphatische Leukämie handelt.

31. Verwendung einer Verbindung nach einem der Ansprüche 1 bis 9 zum Abschwächen der Ausbildung von Morphintoleranz- oder -abhängigkeitserscheinungen.

32. Verwendung einer Verbindung nach einem der Ansprüche 1 bis 9 zum Verringern von Verhaltensgedächtnisverlusten.

33. Verwendung einer Verbindung nach einem der Ansprüche 1 bis 9 zur Prävention von Frühgeburten.

34. Verwendung nach Anspruch 18, **dadurch gekennzeichnet, daß** es sich bei der Erkrankung um Septikämie oder polyviszerales Versagen handelt

35. Verwendung nach Anspruch 18, **dadurch gekennzeichnet, daß** es sich bei der Erkrankung um eine chronische obstruktive Lungenerkrankung (bzw. COPD) handelt.

36. Verwendung nach Anspruch 18, **dadurch gekennzeichnet, daß** es sich bei der Erkrankung um ein Emphysem handelt.

37. Verwendung nach Anspruch 18, **dadurch gekennzeichnet, daß** es sich bei der Erkrankung um Heuschnupfen handelt.

38. Verwendung nach Anspruch 18, **dadurch gekennzeichnet, daß** es sich bei der Erkrankung um kongestive Herzinsuffizienz handelt.

39. Verwendung nach Anspruch 18, **dadurch gekennzeichnet, daß** es sich bei der Erkrankung um Osteoporose handelt.

## Claims

1. Triazolo[4,3-a]quinazolin-5-ones and/or -5-thiones of formula I or II:

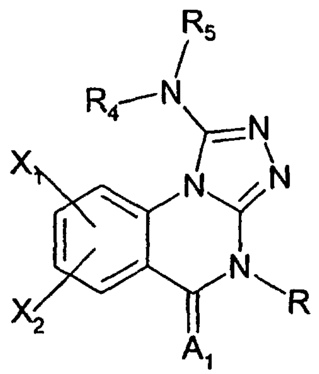

I

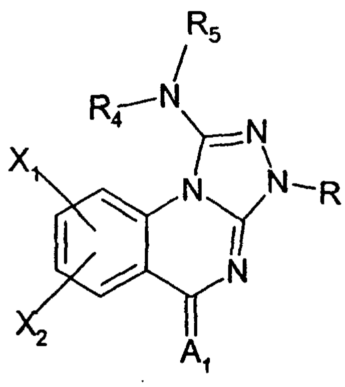

II

I and II being positional isomers of the group R on nitrogens 3 or 4, in which:

- $A_1$ is O or S;
- $X_1$ and $X_2$, which may be identical or different, represent:
  - hydrogen, hydroxyl, halogen, amino, nitro, mercapto, cyano, carboxyl,
  - lower alkyl, lower alkoxy or $-S(O)_mR_8$ in which m is 0, 1 or 2 and $R_8$ is a lower alkyl, which are optionally substituted with one or more halogen atoms,
  - $-CO-Q_1-Q_2-Q_3$ in which:
    - $Q_1$- is: a single valency bond, -O-,

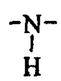 , 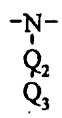 , or 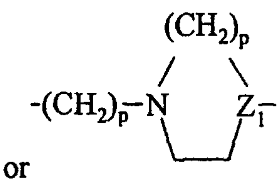 ,

in which p is an integer which can range from 0 to 3, and $Z_1$ is CH, N, O or S,

    - $Q_2$- is:

      a) $-(CH_2)_q-$, q being equal to 0, 1, 2, 3, or 4, or
      b) $-(CH_2-CH_2-O)_r-$, r being equal to 2, 3 or 4, and

    - $Q_3$ is: -H, -OH, lower alkoxy, -O-CO- $X_3$ $-NHX_3$ or

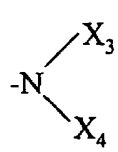

in which $X_3$ and $X_4$, which may be identical or different, represent a lower alkyl group, it being possible for $X_3$ and $X_4$ to be linked to form a ring, comprising one or more hetero atoms chosen from O, S and N,

  - $-NH-R_1$ in which $R_1$ represents a lower alkyl group, optionally substituted with one or more groups chosen from halogen, hydroxyl, cyano, lower alkoxy and $-CO-Q_1-Q_2-Q_3$, or
  - $-NR_2R_3$ in which $R_2$ and $R_3$, which may be identical or different, represent a lower alkyl, optionally substituted with one or more hydroxyl, halogen, cyano, lower alkoxy or $-CO-Q_1-Q_2-Q_3$ groups, it being possible for $R_2$ and $R_3$ to be linked to form a ring, comprising one or more hetero atoms chosen from O, S and N and optionally bridged with a lower alkyl, gem-dialkylated or substituted with one or more groups chosen from hydroxyl, keto, lower alkyl, lower alkoxy and $-CO-Q_1-Q_2-Q_3$:

- R represents:

  - lower alkyl, lower alkenyl, lower alkynyl, arylalkynyl, 2-, 3- or 4-pyridylalkyl optionally substituted with a lower alkyl, lower alkoxy,

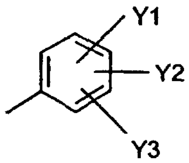 , 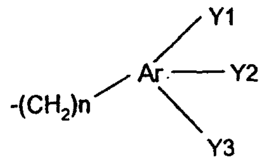 , 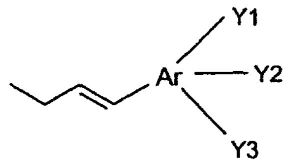

or

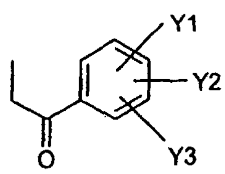

hydroxyl, halogen or amino group,
in which:

  - n is an integer from 1 to 5,
  - Ar is an aromatic ring comprising 5 or 6 atoms including from 0 to 3 hereto atoms chosen from O, S and N,
  - Y1, Y2 and Y3, which may be identical or different, represent:

    - hydrogen, hydroxyl, mercapto, amino, nitro, halogen, $NHR_1$, $NR_2R_3$, $-(CH_2)_s-CN$, or $-(CH_2)_sCO-Q_1-Q_2-Q_3$ in which s is an integer from 0 to 6;
    - lower alkyl, lower alkoxy or $-S(O)_mR_8$ in which case m is 0, 1 or 2 and $R_8$ is a lower alkyl, it being possible for each to be optionally substituted with one or more halogen atoms; and

- $R_4$ and $R_5$, represent:

  - lower alkyl when R4 and R5 are identical, aralkyl, cycloalkyl or cycloalkylalkyl, when $R_4$ and $R_5$ are different,
  - lower alkyl, it being possible for $R_4$ and $R_5$ to be linked to form a saturated ring or a ring including one or more double bonds comprising one or more hetero atoms chosen from O, S and N and optionally substituted with a lower alkyl, a hydroxyl or a lower alkoxy or bridged with a lower alkyl, gem-dialkylated or substituted with one or more groups chosen from hydroxyl, keto, lower alkyl, lower alkoxy, phenylalkyl or $CO-Q_1-Q_2-Q_3$, it also being possible for two of the atoms in the ring thus formed to form part of another ring chosen from phenyl and heteroaryl comprising from 4 to 8 atoms including 1 to 4 hetero atoms;

optionally the racemic forms thereof and the isomers thereof, as well as the pharmaceutically acceptable salts thereof.

**2.** Triazolo[4,3-a]quinazolin-5-ones and/or -5-thiones according to Claim 1, in which:

$A_1$ represents an oxygen atom;
$X_1$ represents a hydrogen atom and $X_2$ is a halogen, amino, lower alkyl, hydroxyl or $-NHR_1$ group, $R_1$ being as defined above,
R represents:

- a lower alkyl, lower alkenyl, arylalkynyl, 2-, 3- or 4-pyridylalkyl group optionally substituted on the pyridine nucleus with a lower alkyl, a halogen or a hydroxyl;

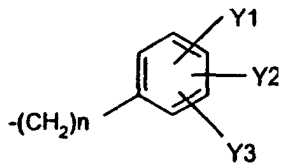

in which:

- n is an integer from 1 to 3,
- Y1, Y2 and Y3 each represent a hydrogen atom or a lower alkoxy group, more particularly methoxy,
- Y1 and Y2 each represent a hydrogen atom and Y3 represents a lower alkoxy group, an amino group, $NHR_1$, $NR_2R_3$, nitro, hydroxyl, a group $-(CH_2)_sCO-Q_1-Q_2-Q_3$, a group $(CH_2)_s-CN$ in which s, $Q_1$, $Q_2$, and $Q_3$ are as defined above, or a lower alkyl group optionally substituted with one or more halogen atoms, the position which is particularly preferred for the substituent Y3 being position 4, or
- Y1 represents a hydrogen atom and Y2 and Y3, which may be identical or different, represent a hydroxyl, halogen or lower alkoxy group, or

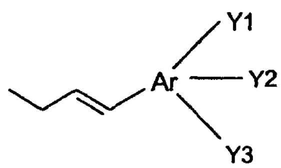

in which:

- Ar is as defined above;
- Y1, Y2 and Y3 each represent a hydrogen atom, or
- Y1 and Y2 each represent a hydrogen atom and Y3 is lower alkoxy or halogen;

$R_4$ and $R_5$ represent:

- lower alkyl when R4 and R5 are identical, aralkyl, cycloalkyl or cycloalkylalkyl when $R_4$ and $R_5$ are different,

lower alkyl, it being possible for $R_4$ and $R_5$ to be linked to form a saturated ring or a ring including one or more double bonds comprising one or more hetero atoms chosen from O, S and N and optionally substituted with a lower alkyl, a hydroxyl or a lower alkoxy or bridged with lower alkyl, gem-dialkylated or substituted with one or more groups chosen from hydroxyl, keto, lower alkyl, lower alkoxy, phenylalkyl or $CO-Q_1-Q_2-Q_3$, it being possible for two of the atoms of the ring thus formed also to form part of another ring chosen from phenyl and heteroaryl comprising from 4 to 8 atoms including 1 to 4 hetero atoms.

**3.** Triazolo[4,3-a]quinazolin-5-ones and/or -5-thrones according to Claim 1 or 2, in which:

$X_1$ represents a hydrogen atom,

$X_2$ represents a halogen, amino, lower alkyl, hydroxyl or $-NHR_1$ group;
R represents:

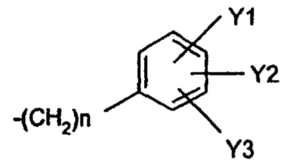

in which:

- n is an integer from 1 to 3,
- Y1, Y2 and Y3 each represent a hydrogen atom or a lower alkoxy group, more particularly methoxy and in particular 3, 4, 5-trimethoxy,
- Y1 and Y2 each represent a hydrogen atom and Y3 represents a lower alkoxy, amino, $NHR_1$, $NR_2R_3$, vitro, or hydroxyl group, a lower alkyl group optionally substituted with one or more halogen atoms, a group $-(CH_2)_sCO-Q_1-Q_2-Q_3$ in which s is 0 or I, $Q_1$ is O, -NH- or a valency bond, $Q_2$ is $-(CH_2)_q-$, q being equal to 0 1, 2, 3 or 4 and $Q_3$ is H, OH or $-NX_3X_4$ in which $X_3$ and $X_4$ are as defined above, a group $(CH_2)_s-CN$ in which s is 0 or 1, the position particularly preferred for the substituent Y3 being position 4 or
- Y1 represents a hydrogen atom and Y2 and Y3, which may be identical or different, represent a hydroxyl, halogen or lower alkoxy group; or

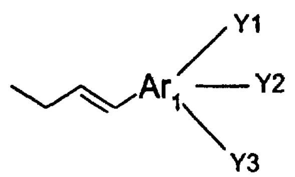

in which:

- $Ar_1$ is an aromatic ring comprising 6 atoms which can include a nitrogen atom in position 2, 3 or 4 and preferably in position 3;
- Y1, Y2 and Y3 each represent a hydrogen atom, or
- Y1 and Y2 each represent a hydrogen atom and Y3 is lower alkoxy group or a halogen group when $Ar_1$ does not comprise a nitrogen atom; and

$R_4$ and $R_5$, represent:

- lower alkyl, when R4 and R5 are identical, aralkyl, cycloalkyl or cycloalkylalkyl when $R_4$ and $R_5$ are different,
- lower alkyl, it being possible for $R_4$ and $R_5$ to be linked to form a saturated ring or a ring including one or more double bonds comprising one or more hetero atoms chosen from O, S and N and optionally substituted with a lower alkyl, a hydroxyl or a lower alkoxy or bridged with a lower alkyl, gem-dialkylated or substituted with one or more groups chosen from hydroxyl, keto, lower alkyl, lower alkoxy, phenylalkyl and $CO-Q_1-Q_2-Q_3$, it being possible for two of the atoms of the ring thus formed also to form part of another ring chosen from phenyl and heteroaryl comprising from 4 to 8 atoms including 1 to 4 hetero atoms.

4. Triazolo[4,3-a]quinazolin-5-ones and/or -5-thiones according to any one of Claims 1 to 3, in which:

- the halogen group is chosen from F, Cl, Br and I,
- the lower alkyl group is a linear or branched group containing from 1 to 6 carbon atoms,
- the lower alkoxy group is a linear or branched group containing from 1 to 5 carbon atoms,
- the lower alkylthio group is a linear or branched group containing from 1 to 5 carbon atoms,

- the lower alkenyl group contains from 3 to 6 carbon atoms,
- the lower alkynyl group contains from 3 to 6 carbon atoms,
- the 2-, 3- or 4-pyridylalkyl group comprises an alkyl containing 1 to 5 carbon atoms,
- the aryl group contains from 5 to 8 carbon atoms,
- the aralkyl group comprises an alkyl containing from 1 to 6 carbon atoms,
- the cycloalkyl group contains from 3 to 8 carbon atoms,
- the cycloalkylalkyl group comprises an alkyl containing from I to 6 carbon atoms,
- the lower alkyl, lower alkoxy or lower alkyithio groups substituted with one or more halogen atoms are chosen from the groups $-(CH_2)_p-CF_3$, $-O-(CH_2)_p-CF_3$ or $-S-(CH_2)_p-CF_3$, in which p is an integer from 0 to 3.

**5.** Triazolo[4,3-a]quinazolin-5-ones and/or -5-thiones according to Claim 1, in which:

- $A_1$ is O or S;
- $X_1$ and $X_2$, which may be identical or different, represent:
  - hydrogen, hydroxyl, halogen, amino, nitro, mercapto, cyano, carboxyl,
  - lower alkyl, lower alkoxy or $-S(O)_mR_8$ in which m is 0, 1 or 2 and $R_8$ is a lower alkyl, which are optionally substituted with one or more halogen atoms;
- R represents:

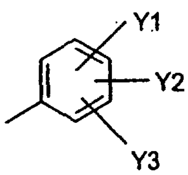
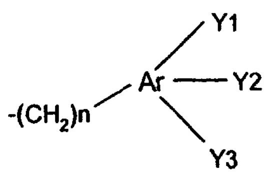
or
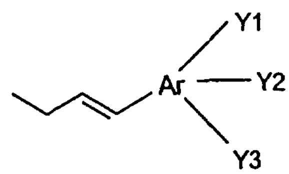

in which:

  - n is an integer from 1 to 5,
  - Ar is an aromatic ring comprising 5 or 6 atoms including from 0 to 3 hetero atoms chosen from O, S and N
  - Y1, Y2 and Y3, which may be identical or different, represent:
  - hydrogen, hydroxyl, mercapto, amino, $NHR_1$, $NR_2R_3$, nitro, halogen, $-(CH_2)_5CO-Q_1-Q_2-Q_3$, or $-(CH_2)_s-CN$ in which s is an integer from 0 to 6;
  - lower alkyl, lower alkoxy or $-S(O)_mR_8$ in which m is 0, 1 or 2 and $R_8$ is a lower alkyl, it being possible for each to be optionally substituted with one or more halogen atoms;

- $R_4$ and $R_5$, which may be identical or different, represent:

  lower alkyl, it being possible for $R_4$ and $R_5$ to be linked to form a saturated ring or a ring including one or more double bonds comprising one or more hetero atoms chosen from O, S and N and optionally bridged with a lower alkyl, gem-dialkylated or substituted with one or more groups chosen from hydroxyl, keto, lower alkyl, lower alkoxy, phenylalkyl and $CO-Q_1-Q_2-Q_3$.

**6.** Triazolo[4,3-a]quinazolin-5-ones and/or -5-thiones according to Claim 5, chosen from the group comprising:

7-Bromo-1-dimethylamino-4-((E)-3-pyrid-3-ylallyl)-4H-[1,2,4]triazolo[4,3-a]quinazolin-5-one
1-Dimethylamino-7-methyl-4-(3-pyrid-3-ylallyl)-4H-[1,2,4]triazolo[4,3-a]quinazolin-5-one
4-(3,4-Dimethoxybenzyl)-7-methyl-1-pyrrolidin-1-yl-4H-[1,2,4]triazolo[4,3-a]quinazolin-5-one
4-(1-Dimethylamino-7-methyl-5-oxo-5H-[1,2,4]triazolo[4,3-a]quinazolin-4-yl-methyl)benzonitrile
7-Bromo-1-dimethylamino-4-(3-phenylallyl)-4H-[1,2,4]triazolo[4,3-a]quinazolin-5-one
7-Methyl-4-(3-phenylallyl)-1-pyrrolidin-1-yl-4H-[1,2,4]triazolo[4,3-a]quinazolin-5-one
4-(7-Bromo-1-dimethylamino-5-oxo-5H-[1,2,4]triazolo[4,3-a]quinazolin-4-yl-methyl)benzonitrile
1-Azepan-1-yl-7-methyl-4-pyrid-3-ylmethyl-4H-[1,2,4]triazolo[4,3-a]quinazolin-5-one
4-(7-Methyl-5-oxo-1-pyrrolidin-1-yl-5H-[1,2,4]triazolo[4,3-a]quinazolin-4-yl-methyl)benzonitrile

1-Dimethylamino-7-methyl-4-((E)-3-phenylallyl)-4H-[1,2,4]triazolo[4,3-a]quinazolin-5-one
4-(7-Bromo-5-oxo-1-pyrrolidin-1-yl-5H-[1,2,4]triazolo[4,3-a]quinazolin-4-yl-methyl)benzonitrile
1-Azepan-1-yl-7-bromo-4-(3,4-dimethoxybenzyl)-4H-[1,2,4]triazolo[4,3-a]quinazolin-5-one

**7.** Triazolo[4,3-a]quinazolin-5-ones and/or -5-thrones according to Claim 1, 2, 3 or 4, chosen from the group comprising:

1-(Azepan-1-yl)-7-chloro-4-(3-phenylallyl)-4H-[1,2,4]triazolo[4,3-a]quinazolin-5-one

1-(Azepan-1-yl)-7-chloro-3-(3-phenylallyl)-3H-[1,2,4]triazolo[4,3-a]quinazolin-5-one

7-Bromo-1-dimethylamino-4-((E)-3-pyrid-3-ylallyl)-4H-[1,2,4]triazolo[4,3-a]quinazolin-5-one

7-Bromo-4-pyrid-3-ylmethyl-1-pyrrolidin-1-yl-4H-[1,2,4]triazolo[4,3-a]quinazolin-5-one

7-Bromo-3-pyrid-3-ylmethyl-1-pyrrolidin-1-yl-3H-[1,2,4]triazolo[4,3-a]quinazolin-5-one

1-Azepan-1-yl-4-(3-phenylallyl)-4H-[1,2,4]triazolo[4,3-a]quinazolin-5-one

1-(Azepan-1-yl)-7-chloro-4-allyl-4H-[1,2,4]triazolo[4,3-a]quinazolin-5-one

1-(Azepan-1-yl)-7-chloro-4-(4-methylbenzyl)-4H-[1,2,4]triazolo[4,3-a]quinazolin-5-one

1-(Azepan-1-yl)-7-chloro-4-(2-chlorobenzyl)-4H-[1,2,4]triazolo[4,3-a]quinazolin-5-one

1-(Azepan-1-yl)-7-chloro-4-(3-chlorobenzyl)-4H-[1,2,4]triazolo[4,3-a]quinazolin-5-one

1-(Azepan-1-yl)-7-chloro-4-(4-chlorobenzyl)-4H-[1,2,4]triazolo[4,3-a]quinazolin-5-one

1-(Azepan-1-yl)-7-chloro-4-(4-bromobenzyl)-4H-[1,2,4]triazolo[4,3-a]quinazolin-5-one

1-(Azepan-1-yl)-7-chloro-4-(4-fluorobenzyl)-4H-[1,2,4]triazolo[4,3-a]quinazolin-5-one

1-(Azepan-1-yl)-7-chloro-4-(4-(trifluoromethyl)benzyl)-4H-[1,2,4]triazolo[4,3-a]-quinazolin-5-one

1-(Azepan-1-yl)-7-chloro-4-(4-cyanobenzyl)-4H-[1,2,4]triazolo[4,3-a]quinazolin-5-one

1-(Azepan-1-yl)-7-chloro-4-(2-methoxybenzyl)-4H-[1,2,4]triazolo[4,3-a]quinazolin-5-one

1-(Azepan-1-yl)-7-chloro-4-(3-methoxybenzyl)-4H-[1,2,4]triazolo[4,3-a]quinazolin-5-one

1-(Azepan-1-yl)-7-chloro-4-(4-methoxybenzyl)-4H-[1,2,4]triazolo[4,3-a]quinazolin-5-one

1-(Azepan-1-yl)-7-chloro-4-(3,4-dichlorobenzyl)-4H-[1,2,4]triazolo[4,3-a]quinazolin-5-one

1-(Azepan-1-yl)-7-chloro-4-(3,4-dimethoxybenzyl)-4H-[1,2,4]triazolo[4,3-a]quinazolin-5-one

1-(Azepan-1-yl)-7-chloro-4-(2-pyridylmethyl)-4H-[1,2,4]triazolo[4,3-a]quinazolin-5-one

1-(Azepan-1-yl)-7-chloro-4-(3-pyridylmethyl)-4H-[1,2,4]triazolo[4,3-a]quinazolin-5-one

1-(Azepan-1-yl)-7-chloro-4-(4-pyridylmethyl)-4H-[1,2,4]triazolo[4,3-a]quinazolin-5-one

1-(Azepan-1-yl)-7-chloro-4-(2-phenylethyl)-4H-[1,2,4]triazolo[4,3-a]quinazolin-5-one

1-(Azepan-1-y1)-7-chloro-4-[2-(4-methoxyphenyl)ethyl]-4H-[1,2,4]triazolo[4,3-a]-quinazolin-5-one

1-(Azepan-1-yl)-7-chloro-4-(3-phenylpropyl)-4H-[1,2,4]triazolo[4,3-a]quinazolin-5-one

1-Azepan-1-yl-7-chloro-4-(2-oxo-2-phenylethyl)-4H-[1,2,4]triazolo[4,3-a]quinazolin-5-one

1-(Azepan-1-yl)-7-chloro-4-[2-(4-methoxyphenyl)-2-oxoethyl]-4H-[1,2,4]triazolo[4,3-a]-quinazolin-5-one

1-(Azepan-1-yl)-7-chloro-4-[2-(4-chlorophenyl)-2-oxoethyl]-4H-[1,2,4]triazolo[4,3-a]-quinazolin-5-one

5-[(1-(Azepan-1-yl)-7-chloro-5-oxo-5H-[1,2,4]triazolo[4,3-a]quinazolin-4-yl)acetyl]-2-methoxybenzoic acid methyl ester

7-Chloro-4-pyrid-3-ylmethyl-1-pyrrolidin-1-yl-4H-[1,2,4]triazolo[4,3-a]quinazolin-5-one

1-(Azepan-1-yl)-7-bromo-4-(4-chlorophenylmethyl)-4H-[1,2,4]triazolo[4,3-a]quinazolin-5-one

1-Azepan-1-yl-7-bromo-4-(4-fluorobenzyl)-4H-[1,2,4]triazolo[4,3-a]quinazolin-5-one

4-(1-Azepan-1-yl-7-bromo-5-oxo-5H-[1,2,4]triazolo[4,3-a]quinazolin-4-ylmethyl)-benzonitrile

1-Azepan-1-yl-7-bromo-4-(3,4-dimethoxybenzyl)-4H-[1,2,4]triazolo[4,3-a]quinazolin-5-one

1-(Azepan-1-yl)-7-bromo-4-(3-pyridylmethyl)-4H-[1,2,4]triazolo[4,3-a]quinazolin-5-one

1-(Azepan-1-yl)-7-bromo-4-(3-phenylallyl)-4H-[1,2,4]triazolo[4,3-a]quinazolin-5-one

1-Azepan-1-yl-7-bromo-4-[3-(4-chlorophenyl)allyl]-4H-[1,2,4]triazolo[4,3-a]quinazolin-5-one

1-Azepan-1-yl-7-bromo-4-[3-(4-methoxyphenyl)allyl]-4H-[1,2,4]triazolo[4,3-a]-quinazolin-5-one

1-Azepan-1-yl-7-bromo-4-((E)-3-pyrid-3-ylallyl)-4H-[1,2,4]triazolo[4,3-a]quinazolin-5-one

1-Azepan-1-yl-7-bromo-4-(3-pyrid-4-ylallyl)-4H-[1,2,4]triazolo[4,3-a]quinazolin-5-one

7-Bromo-4-(4-methylbenzyl)-1-pyrrolidin-1-yl-4H-[1,2,4]triazolo[4,3-a]quinazolin-5-one

7-Bromo-4-(4-chlorobenzyl)-1-pyrrolidin-1-yl-4H-[1,2,4]triazolo[4,3-a]quinazolin-5-one

7-Bromo-4-(4-fluorobenzyl)-1-pyrrolidin-1-yl-4H-[1,2,4]triazolo[4,3-a]quinazolin-5-one

3-(7-Bromo-5-oxo-1-pyrrolidin-1-yl-5H-[1,2,4]triazolo[4,3-a]quinazolin-4-ylmethyl)-benzonitrile

4-(7-Bromo-5-oxo-1-pyrrolidin-1-yl-5H-[1,2,4]triazolo[4,3-a]quinazolin-4-ylmethyl)-benzonitrile

Methyl 4-(7-bromo-5-oxo-1-pyrrolidin-1-yl-5H-[1,2,4]triazolo[4,3-a]quinazolin-4-ylmethyl)benzoate

7-Bromo-4-(4-nitrobenzyl)-1-pyrrolidin-1-yl-4H-[1,2,4]triazolo[4,3-a]quinazolin-5-one

7-Bromo-4-(4-methoxybenzyl)-1-pyrrolidin-1-yl-4H-[1,2,4]triazolo[4,3-a]quinazolin-5-one

Phenyl 4-(7-bromo-5-oxo-1-pyrrolidin-1-yl-5H-[1,2,4]triazolo[4,3-a]quinazolin-4-yl-methyl)acetate

7-Bromo-4-(4-hydroxybenzyl)-1-pyrrolidin-1-yl-4H-[1,2,4]triazolo[4,3-a]quinazolin-5-one

7-Bromo-4-(3,4-dimethoxybenzyl)-1-pyrrolidin-1-yl-4H-[1,2,4]triazolo[4,3-a]quinazolin-5-one

4-Benzo[1,3]dioxol-5-ylmethyl-7-bromo-1-pyrrolidin-1-yl-4H-[1,2,4]triazolo[4,3-a]-quinazolin-5-one

7-Bromo-4-(3,5-dimethoxybenzyl)-1-pyrrolidin-1-yl-4H-[1,2,4]triazolo[4,3-a]quinazolin-5-one

7-Bromo-1-pyrrolidin-1-yl-4-(3,4,5-trimethoxybenzyl)-4H-[1,2,4]triazolo[4,3-a]-quinazolin-5-one

[4-(7-Bromo-5-oxo-1-pyrrolidin-1-yl-5H-[1,2,4]triazolo[4,3-a]quinazolin-4-ylmethyl)-phenyl]acetic acid

1-(Pyrrolidin-1-yl)-7-bromo-4-(3-phenylallyl)-4H-[1,2,4]triazolo[4,3-a]quinazolin-5-one

7-Bromo-4-(3-phenylallyl)-1-pyrrolidin-1-yl-4H-[1,2,4]triazolo[4,3-a]quinazolin-5-one

7-Bromo-4-[(E)-3-(4-chlorophenyl)allyl]-1-pyrrolidin-1-yl-4H-[1,2,4]triazolo[4,3-a]-quinazolin-5-one

7-Bromo-4-[3-(4-methoxyphenyl)allyl]-1-pyrrolidin-1-yl-4H-[1,2,4]triazolo[4,3-a]-quinazolin-5-one

7-Bromo-4-(3-pyrid-3-ylallyl)-1-pyrrolidin-1-yl-4H-[1,2,4]triazolo[4,3-a]quinazolin-5-one

7-Bromo-4-((E)-3-pyrid-4-ylallyl)-1-pyrrolidin-1-yl-4H-[1,2,4]triazolo[4,3-a]quinazolin-5-one

7-Bromo-4-(1H-imidazol-4-ylmethyl)-1-pyrrolidin-1-yl-4H-[1,2,4]triazolo[4,3-a]-quinazolin-5-one

7-Bromo-4-(3,5-dimethylisoxazol-4-ylmethyl)-1-pyrrolidin-1-yl-4H-[1,2,4]triazolo[4,3-a]-quinazolin-5-one

7-Bromo-4-cyclopentylmethyl-1-pyrrolidin-1-yl-4H-[1,2,4]triazolo[4,3-a]quinazolin-5-one

7-Bromo-4-butyl-1-pyrrolidin-1-yl-4H-[1,2,4]triazolo[4,3-a]quinazolin-5-one

7-Bromo-1-pyrrolidin-1-yl-4-(2,2,2-trifluoroethyl)-4H-[1,2,4]triazolo[4,3-a]quinazolin-5-one

7-Bromo-4-(2-hydroxyethyl)-1-pyrrolidin-1-yl-4H-[1,2,4]triazolo[4,3-a]quinazolin-5-one

7-Bromo-4-(2-diethylaminoethyl)-1-pyrrolidin-1-yl-4H-[1,2,4]triazolo[4,3-a]quinazolin-5-one

7-Bromo-4-prop-2-ynyl-1-pyrrolidin-1-yl-4H-[1,2,4]triazolo[4,3-a]quinazolin-5-one

7-Bromo-4-(2-phenoxyethyl)-1-pyrrolidin-1-yl-4H-[1,2,4]triazolo[4,3-a]quinazolin-5-one

7-Bromo-4-(2-phenylsulphenylethyl)-1-pyrrolidin-1-yl-4H-[1,2,4]triazolo[4,3-a]-quinazolin-5-one

Methyl (7-bromo-5-oxo-1-pyrrolidin-1-yl-5H-[1,2,4]triazolo[4,3-a]quinazolin-4-yl)-phenylacetate

4-(7-Bromo-5-oxo-1-piperid-1-yl-5H-[1,2,4]triazolo[4,3-a]quinazolin-4-ylmethyl)-benzonitrile

7-Bromo-4-(3,4-dimethoxybenzyl)-1-piperid-1-yl-4H-[1,2,4]triazolo[4,3-a]quinazolin-5-one

1-(Piperid-1-yl)-7-bromo-4-(3-phenylallyl)-4H-[1,2,4]triazolo[4,3-a]quinazolin-5-one

7-Bromo-4-(3-pyrid-3-ylallyl)-1-thiomorpholin-4-yl-4H-[1,2,4]triazolo[4,3-a]quinazolin-5-one

7-Bromo-1-dimethylamino-4-(4-methylbenzyl)-4H-[1,2,4]triazolo[4,3-a]quinazolin-5-one

4-(7-Bromo-1-dimethylamino-5-oxo-5H-[1,2,4]triazolo[4,3-a]quinazolin-4-ylmethyl)-benzonitrile

7-Bromo-1-dimethylamino-4-(4-hydroxybenzyl)-4H-[1,2,4]triazolo[4,3-a]quinazalin-5-one

Methyl 4-(7-bromo-1-dimethylamino-5-oxo-5H-[1,2,4]triazolo[4,3-a]quinazolin-4-yl-methyl)benzoate

[4-(7-Bromo-1-dimethylamino-5-oxo-5H-[1,2,4]triazolo[4,3-a]quinazolin-4-ylmethyl)-phenyl]acetic acid

[4-(7-Bromo-1-dimethylamino-5-oxo-5H-[1,2,4]triazolo[4,3-a]quinazolin-4-ylmethyl)-phenyl]acetonitrile

7-Bromo-1-dimethylamino-4-(pyrid-3-ylmethyl)-4H-[1,2,4]triazolo[4,3-a]quinazolin-5-one

7-Bromo-1-dimethylamino-4-(3-phenylallyl)-4H-[1,2,4]triazolo[4,3-a]quinazolin-5-one

7-Bromo-1-dimethylamino-4-(3-phenylallyl)-4H-[1,2,4]triazolo[4,3-a]quinazolin-5-one

7-Bromo-1-dimethylamino-4-(3-pyrid-4-ylallyl)-4H-[1,2,4]triazolo[4,3-a]quinazolin-5-one

7-Bromo- 1-dimethylamino-4-prop-2-ynyl-4H-[1,2,4]triazolo[4,3-a]quinazolin-5-one

7-Bromo-1-dimethylamino-4-(3-phenylprop-2-ynyl)-4H-[1,2,4]triazolo[4,3-a]quinazolin-5-one

Methyl 4-(7-bromo-1-dimethylamino-5-oxo-5H-[1,2,4]triazolo[4,3-a]quinazolin-4-yl)-phenylacetate

1-Azepan-1-yl-7-methyl-4-pyrid-3-ylmethyl-4H-[1,2,4]triazolo[4,3-a]quinazolin-5-one

1-Azepan-1-yl-7-methyl-4-(3-phenylallyl)-4H-[1,2,4]triazolo[4,3-a]quinazolin-5-one

4-(7-Methyl-5-oxo-1-pyrrolidin-1-yl-5H-[1,2,4]triazolo[4,3-a]quinazolin-4-ylmethyl)-benzonitrile

4-(3,4-Dimethoxybenzyl)-7-methyl-1-pyrrolidin-1-yl-4H-[1,2,4]triazolo[4,3-a]quinazolin-5-one

Methyl 4-(7-methyl-5-oxo-1-pyrrolidin-1-yl-5H-[1,2,4]triazolo[4,3-a]quinazolin-4-yl-methyl)benzoate

[4-(7-Methyl-5-oxo-1-pyrrolidin-1-yl-5H-[1,2,4]triazolo[4,3-a]quinazolin-4-ylmethyl)-phenyl]acetic acid

7-Methyl-4-pyrid-3-ylmethyl-1-pyrrolidin-1-yl-4H-[1,2,4]triazolo[4,3-a]quinazolin-5-one

7-Methyl-4-(3-phenylallyl)-1-pyrrolidin-1-yl-4H-[1,2,4]triazolo[4,3-a]quinazolin-5-one

[4-(7-Methyl-5-oxo-1-thiomorpholin-4-yl-5H-[1,2,4]triazolo[4,3-a]quinazolin-4-yl-methyl)phenyl]acetic acid

7-Methyl-4-(3-pyrid-3-ylallyl)-1-thiomorpholin-4-yl-4H-[1,2,4]triazolo[4,3-a]quinazolin-5-one

4-(1-Dimethylamino-7-methyl-5-oxo-5H-[1,2,4]triazolo[4,3-a]quinazolin-4-ylmethyl)-benzonitrile

[4-(Dimethylaminomethyl-oxo-5H-[1,2,4]triazolo[4,3-a]quinazolin-4-ylmethyl)phenyl]-acetic acid

1-Dimethylamino-7-methyl-4-((E)-3-phenylallyl)-4H-[1,2,4]triazolo[4,3-a]quinazolin-5-one

1-Dimethylamino-7-methyl-4-(3-pyrid-3-ylallyl)-4H-[1,2,4]triazolo[4,3-a]quinazolin-5-one

1-Dimethylamino-7-methyl-4-(3-pyrid-4-ylallyl)-4H-[1,2,4]triazolo[4,3-a]quinazolin-5-one

1-(Azepan-1-yl)-8-methyl-4-(3-phenylallyl)-4H-[1,2,4]triazolo[4,3-a]quinazolin-5-one

(4-Cyanobenzyl)dimethylaminooxo-4,5-dihydro-[1,2,4]triazolo[4,3-a]quinazoline-7-carbonitrile

7-Hydroxy-4-((E)-3-phenylallyl)-1-pyrrolidin-1-yl-4H-[1,2,4]triazolo[4,3-a]quinazolin-5-one

1-(Azepan-1-yl)-3-(3-phenylallyl)-3H-[1,2,4]triazolo[4,3-a]-quinazolin-5-one

3-Allyl-1-azepan-1-yl-7-chloro-3H-[1,2,4]triazolo[4,3-a]quinazolin-5-one

1-(Azepan-1-yl)-7-chloro-3-benzyl-3H-[1,2,4]triazolo[4,3-a]quinazolin-5-one

1-Azepan-1-yl-7-chloro-3-(4-methylbenzyl)-3H-[1,2,4]triazolo[4,3-a]quinazolin-5-one

1-(Azepan-1-yl)-7-chloro-3-(2-chlorobenzyl)-3H-[1,2,4]triazolo[4,3-a]quinazolin-5-one

1-(Azepan-1-yl)-7-chloro-3-(3-chlorobenzyl)-3H-[1,2,4]triazolo[4,3-a]quinazolin-5-one

1-(Azepan-1-yl)-7-chloro-3-(4-chlorobenzyl)-3H-[1,2,4]triazolo[4,3-a]quinazolin-5-one

1-(Azepan-1-yl)-7-chloro-3-(4-bromobenzyl)-3H-[1,2,4]triazolo[4,3-a]quinazolin-5-one

1-(Azepan-1-yl)-7-chloro-3-(4-fluorobenzyl)-3H-[1,2,4]triazolo[4,3-a]quinazolin-5-one

1-(Azepan-1-yl)-7-chloro-3-(4-(trifluoromethyl)benzyl)-3H-[1,2,4]triazolo[4,3-a]-quinazolin-5-one

1-(Azepan-1-yl)-7-chloro-3-(4-cyanobenzyl)-3H-[1,2,4]triazolo[4,3-a]quinazolin-5-one

1-(Azepan-1-yl)-7-chloro-3-(2-methoxybenzyl)-3H-[1,2,4]triazolo[4,3-a]quinazolin-5-one

1-(Azepan-1-yl)-7-chloro-3-(3-methoxybenzyl)-3H-[1,2,4]triazolo[4,3-a]quinazolin-5-one

1-(Azepan-1-yl)-7-chloro-3-(4-methoxybenzyl)-3H-[1,2,4]triazolo[4,3-a]quinazolin-5-one

1-(Azepan-1-yl)-7-chloro-3-(3,4-dichlorobenzyl)-3H-[1,2,4]triazolo[4,3-a]quinazolin-5-one

1-(Azepan-1-yl)-7-chloro-3-(3,4-dimethoxybenzyl)-3H-[1,2,4]triazolo[4,3-a]quinazolin-5-one

1-(Azepan-1-yl)-7-chloro-3-(2-pyridylmethyl)-3H-[1,2,4]triazolo[4,3-a]quinazolin-5-one

1-(Azepan-1-yl)-7-chloro-3-(3-pyridylmethyl)-3H-[1,2,4]triazolo[4,3-a]quinazolin-5-one

1-(Azepan-1-yl)-7-chloro-3-(2-phenylethyl)-3H-[1,2,4]triazolo[4,3-a]quinazolin-5-one

1-(Azepan-1-yl)-7-chloro-3-[2-(4-methoxyphenyl)ethyl]-3H-[1,2,4]triazolo[4,3-a]-quinazolin-5-one

1-(Azepan-1-yl)-7-chloro-3-(3-phenylpropyl)-3H-[1,2,4]triazolo[4,3-a]quinazolin-5-one

1-Azepan-1-yl-7-chloro-3-(2-oxo-2-phenylethyl)-3H-[1,2,4]triazolo[4,3-a]quinazolin-5-one

1-(Azepan-1-yl)-7-chloro-3-[2-(4-methoxyphenyl)-2-oxoethyl]-3H-[1,2,4]triazolo[4,3-a]-quinazolin-5-one

1-(Azepan-1-yl)-7-chloro-3-[2-(4-chlorophenyl)-2-oxoethyl]-3H-[1,2,4]triazolo[4,3-a]-quinazolin-5-one

Methyl 5-[(1-(azepan-1-yl)-7-chloro-5-oxo-5H-[1,2,4]triazolo[4,3-a]quinazolin-3-yl)-acetyl]-2-methoxybenzoate

1-(Azepan-1-yl)-7-bromo-3-(4-chlorobenzyl)-3H-[1,2,4]triazolo[4,3-a]quinazolin-5-one

1-(Azepan-1-yl)-7-bromo-3-(4-fluorobenzyl)-3H-[1,2,4]triazolo[4,3-a]quinazolin-5-one

4-(1-(Azepan-1-yl)-7-bromo-5-oxo-5H-[1,2,4]triazolo[4,3-a]quinazolin-3-ylmethyl)-benzonitrile

1-(Azepan-1-yl)-7-bromo-3-(3,4-dimethoxybenzyl)-3H-[1,2,4]triazolo[4,3-a]quinazolin-5-one

[4-(7-Bromo-5-oxo-1-perhydroazepin-1-yl-5H-[1,2,4]triazolo[4,3-a]quinazolin-3-yl-methyl)phenyl]acetic acid

1-(Azepan-1-yl)-7-bromo-3-(pyrid-3-ylmethyl)-3H-[1,2,4]triazolo[4,3-a]quinazolin-5-one

1-Azepan-1-yl-7-bromo-3-((E)-3-phenylallyl)-3H-[1,2,4]triazolo[4,3-a]quinazolin-5-one

7-Bromo-3-((E)-3-phenylallyl)-1-piperid-1-yl-3H-[1,2,4]triazolo[4,3-a]quinazolin-5-one

7-Bromo-3-(4-chlorobenzyl)-1-(pyrrolidin-1-yl)-3H-[1,2,4]triazolo[4,3-a]quinazolin-5-one

7-Bromo-3-(4-fluorobenzyl)-1-(pyrrolidin-1-yl)-3H-[1,2,4]triazolo[4,3-a]quinazolin-5-one

4-(7-Bromo-5-oxo-1-(pyrrolidin-1-yl)-5H-[1,2,4]triazolo[4,3-a]quinazolin-3-ylmethyl)-benzonitrile

Methyl 4-(7-bromo-5-oxo-1-(pyrrolidin-1-yl)-5H-[1,2,4]triazolo[4,3-a]quinazolin-3-yl-methyl)benzoate

7-Bromo-3-(4-methoxybenzyl)-1-pyrrolidin-1-yl-3H-[1,2,4]triazolo[4,3-a]quinazolin-5-one

Phenyl 4-(7-bromo-5-oxo-1-pyrrolidin-1-yl-5H-[1,2,4]triazolo[4,3-a]quinazolin-3-yl-methyl)acetate

7-Bromo-1-dimethylamino-3-(4-hydroxybenzyl)-3H-[1,2,4]triazolo[4,3-a]quinazolin-5-one

3-(Benzo[1,3]dioxol-5-ylmethyl)-7-bromo-1-(pyrrolidin-1-yl)-3H-[1,2,4]triazolo[4,3-a]-quinazolin-5-one

7-Bromo-3-(3,5-dimethoxybenzyl)-1-(pyrrolidin-1-yl)-3H-[1,2,4]triazolo[4,3-a]quinazolin-5-one

7-Bromo-1-(pyrrolidin-1-yl)-3-(3,4,5-trimethoxybenzyl)-3H-[1,2,4]triazolo[4,3-a]-quinazolin-5-one

7-Bromo-3-(1H-imidazol-4-ylmethyl)-1-pyrrolidin-1-yl-3H-[1,2,4]triazolo[4,3-a]-quinazolin-5-one

7-Bromo-3-(n-butyl)-1-(pyrrolidin-1-yl)-3H-[1,2,4]triazolo[4,3-a]quinazolin-5-one

Methyl (7-bromo-5-oxo-1-pyrrolidin-1-yl-5H-[1,2,4]triazolo[4,3-a]quinazolin-3-yl)-phenylacetate

7-Bromo-1-dimethylamino-3-(3-phenylallyl)-3H-[1,2,4]triazolo[4,3-a]quinazolin-5-one

Methyl 4-(7-bromo-1-dimethylamino-5-oxo-5H-[1,2,4]triazolo[4,3-a]quinazolin-3-yl)-phenylacetate

1-(Azepan-1-yl)-7-methyl-3-(3-phenylallyl)-3H-[1,2,4]triazolo[4,3-a]quinazolin-5-one

7-Methyl-3-(3-phenylallyl)-1-(pyrrolidin-1-yl)-3H-[1,2,4]triazolo[4,3-a]quinazolin-5-one

1-(Azepan-1-yl)-3,8-dimethyl-3H-[1,2,4]triazolo[4,3-a]quinazolin-5-one

1-Azepan-1-yl-8-methyl-3-((E)-3-phenylallyl)-3H-[1,2,4]triazolo[4,3-a]quinazolin-5-one

7-Hydroxy-3-(3-phenylallyl)-1-(pyrrolidin-1-yl)-3H-[1,2,4]triazolo[4,3-a]quinazolin-5-one

1,8-Bis(azepan-1-yl)-3 -(3-phenylallyl)-3H-[1,2,4]triazolo[4,3-a]quinazolin-5-one

1-(Azepan-1-yl)-4-benzyl-7-bromo-4H-[1,2,4]triazolo[4,3-a]quinazolin-5-one

4-Benzyl-7-bromo-1-(pyrrolidin-1-yl)-4H-[1,2,4]triazolo[4,3-a]quinazolin-5-one

4-Benzyl-7-bromo-1-(butylmethylamino)-4H-[1,2,4]triazolo[4,3-a]quinazolin-5-one

4-Benzyl-1-(pyrrolidin-1-yl)-4H-[1,2,4]triazolo[4,3-a]quinazolin-5-one

7-Chloro-1-dibutylamino-4-methyl-4H-[1,2,4]triazolo[4,3-a]quinazolin-5-one

7-Chloro-4-methyl-1-(piperid-1-yl)-4H-[1,2,4]triazolo[4,3-a]quinazolin-5-one

7-Chloro-4-methyl-1-(4-methylpiperazin-1-yl)-4H-[1,2,4]triazolo[4,3-a]quinazolin-5-one

7-Chloro-4-methyl-1-(1,8,8-trimethyl-3-azabicyclo[3.2.1]oct-3-yl)-4H-[1,2,4]triazolo-[4,3-a]quinazolin-5-one

1-(Azepan-1-yl)-7-chloro-4-phenyl-4H-[1,2,4]triazolo[4,3-a]quinazolin-5-one

1-(Azepan-1-yl)-4-benzyl-7-chloro-4H-[1,2,4]triazolo[4,3-a]quinazolin-5-one

4-Benzyl-7-chloro-1-(pyrrolidin-1-yl)-4H-[1,2,4]triazolo[4,3-a]quinazolin-5-one

4-Benzyl-7-chloro-1-(piperid-1-yl)-4H-[1,2,4]triazolo[4,3-a]quinazolin-5-one

1-(Azepan-1-yl)-8-chloro-4-methyl-4H-[1,2,4]triazolo[4,3-a]quinazolin-5-one

1-(Azepan-1-yl)-4-benzyl-8-chloro-4H-[1,2,4]triazolo[4,3-a]quinazolin-5-one

1-(Azepan-1-yl)-7-bromo-4-methyl-4H-[1,2,4]triazolo[4,3-a]quinazolin-5-one

4-Benzyl-7-bromo-1-(piperid-1-yl)-4H-[1,2,4]triazolo[4,3-a]quinazolin-5-one

4-Benzyl-7-bromo-1-dimethylamino-4H-[1,2,4]triazolo[4,3-a]quinazolin-5-one

4-Benzyl-7-bromo-1-morpholin-4-yl-4H-[1,2,4]triazolo[4,3-a]quinazolin-5-one

4-Benzyl-7-bromo-1-thiomorpholin-4-yl-4H-[1,2,4]triazolo[4,3-a]quinazolin-5-one

4-Benzyl-7-bromo-1-(4-methylpiperazin-1-yl)-4H-[1,2,4]triazolo[4,3-a]quinazolin-5-one

4-Benzyl-7-bromo-1-(4-phenylpiperazin-1-yl)-4H-[1,2,4]triazolo[4,3-a]quinazolin-5-one

4-Benzyl-1-(4-benzylpiperazin-1-yl)-7-bromo-4H-[1,2,4]triazolo[4,3-a]quinazolin-5-one

4-Benzyl-7-bromo-1-(3,6-dihydro-2H-pyrid-1-yl)-4H-[1,2,4]triazolo[4,3-a]quinazolin-5-one

4-Benzyl-7-bromo-1-(2,5-dihydropyrrol-1-yl)-4H-[1,2,4]triazolo[4,3-a]quinazolin-5-one

4-Benzyl-7-bromo-1-(3-hydroxypyrrolidin-1-yl)-4H-[1,2,4]triazolo[4,3-a]quinazolin-5-one

4-Benzyl-7-bromo-1-methylamino-4H-[1,2,4]triazolo[4,3-a]quinazolin-5-one

4-Benzyl-7-iodo-1-pyrrolidin-1-yl-4H-[1,2,4]triazolo[4,3-a]quinazolin-5-one

1-Azepan-1-yl-4-benzyl-7-methyl-4H-[1,2,4]triazolo[4,3-a]quinazolin-5-one

4-Benzyl-7-methyl-1-pyrrolidin-1-yl-4H-[1,2,4]triazolo[4,3-a]quinazolin-5-one

4-Benzyl-1-dimethylamino-7-methyl-4H-[1,2,4]triazolo[4,3-a]quinazolin-5-one

4-Benzyl-7-methyl-1-thiomorpholin-4-yl-4H-[1,2,4]triazolo[4,3-a]quinazolin-5-one

1-Azepan-1-yl-4-benzyl-8-methyl-4H-[1,2,4]triazolo[4,3-a]quinazolin-5-one

1-Azepan-1-yl-4-benzyl-7-methoxy-4H-[1,2,4]triazolo[4,3-a]quinazolin-5-one

4-Benzyl-7-methoxy-1-pyrrolidin-1-yl-4H-[1,2,4]triazolo[4,3-a]quinazolin-5-one

4-Benzyl-5-oxo-1-pyrrolidin-1-yl-4,5-dihydro[1,2,4]triazolo[4,3-a]quinazoline-7-carbonitrile

1-Azepan-1-yl-4-benzyl-7-nitro-4H-[1,2,4]triazolo[4,3-a]quinazolin-5-one

1-(Azepan-1-yl)-4-benzyl-7-chloro-4H-[1,2,4]triazolo[4,3-a]quinazolin-5-one

1-(Azepan-1-yl)-4-methyl-4H-[1,2,4]triazolo[4,3-a]quinazolin-5-one

1-(Azepan-1-yl)-4-benzyl-4H-[1,2,4]triazolo[4,3-a]quinazolin-5-one

1-(Azepan-1-yl)-6-chloro-4-methyl-4H-[1,2,4]triazolo[4,3-a]quinazolin-5-one

1-(Azepan-1-yl)-7-chloro-4-methyl-4H-[1,2,4]triazolo[4,3-a]quinazolin-5-one

1-(Azepan-1-yl)-7-chloro-4-ethyl-4H-[1,2,4]triazolo[4,3-a]quinazolin-5-one

7-Chloro-4-methyl-1-(pyrrolidin-1-yl)-4H-[1,2,4]triazolo[4,3-a]quinazolin-5-one

7-Chloro-4-methyl-1-(morpholin-4-yl)-4H-[1,2,4]triazolo[4,3-a]quinazolin-5-one

1-(Azocan-1-yl)-7-chloro-4-methyl-4H-[1,2,4]triazolo[4,3-a]quinazolin-5-one

7-Chloro-1-(3,4-dihydro-2H-quinolin-1-yl)-4-methyl-4H-[1,2,4]triazolo[4,3-a]quinazolin-5-one

7-Chloro-1-(3,4-dihydro-1H-isoquinolin-2-yl)-4-methyl-4H-[1,2,4]triazolo[4,3-a]-quinazolin-5-one

1-(4-Benzylpiperid-1-yl)-7-chloro-4-methyl-4H-[1,2,4]triazolo[4,3-a]quinazolin-5-one

7-Chloro-4-methyl-1-(1,3,3-trimethyl-6-azabicyclo[3,2,1]oct-6-yl)-4H-[1,2,4]triazolo-[4,3-a]quinazolin-5-one

1-(Azepan-1-yl)-7-fluoro-4-methyl-4H-[1,2,4]triazolo[4,3-a]quinazolin-5-one

1-(Azepan-1-yl)-7-iodo-4-methyl-4H-[1,2,4]triazolo[4,3-a]quinazolin-5-one

1-(Azepan-1-yl)-7-methoxy-4-methyl-4H-[1,2,4]triazolo[4,3-a]quinazolin-5-one

4-Benzyl-7-bromo-1-(ethylmethylamino)-4H-[1,2,4]triazolo[4,3-a]quinazolin-5-one

4-Benzyl-1-diethylamino-7-methyl-4H-[1,2,4]triazolo[4,3-a]quinazolin-5-one

4-Benzyl-7-bromo-1-pyrrol-1-yl-4H-[1,2,4]triazolo[4,3-a]quinazolin-5-one

4-(4-Aminobenzyl)-7-bromo-1-pyrrolidin-1-yl-4H-[1,2,4]triazolo[4,3-a]quinazolin-5-one

4-Benzyl-7-hydroxy-1-pyrrolidin-1-yl-4H-[1,2,4]triazolo[4,3-a]quinazolin-5-one

4-(7-Hydroxy-5-oxo-1-pyrrolidin-1-yl-5H-[1,2,4]triazolo[4,3-a]quinazolin-4-ylmethyl)-benzonitrile

N-(4-Benzyl-5-oxo-1-pyrrolidin-1-yl-4,5-dihydro[1,2,4]triazolo[4,3-a]quinazolin-7-yl)-acetamide

N-[5-Oxo-4-(3-phenylallyl)-1-pyrrolidin-1-yl-4,5-dihydro[1,2,4]triazolo[4,3-a]quinazolin-7-yl]acetamide

7-Amino-4-((E)-3-phenylallyl)-1-pyrrolidin-1-yl-4H-[1,2,4]triazolo[4,3-a]quinazolin-5-one

7-Amino-1-azepan-1-yl-4-benzyl-4H-[1,2,4]triazolo[4,3-a]quinazolin-5-one

7-Amino-4-benzyl-1-pyrrolidin-1-yl-4H-[1,2,4]triazolo[4,3-a]quinazolin-5-one

4-(7-Amino-5-oxo-1-pyrrolidin-1-yl-5H-[1,2,4]triazolo[4,3-a]quinazolin-4-ylmethyl)-benzonitrile

7-Amino-4-((E)-3-pyrid-3-ylallyl)-1-pyrrolidin-1-yl-4H-[1,2,4]triazolo[4,3-a]quinazolin-5-one

4-(Aminodimethylaminooxo-5H-[1,2,4]triazolo[4,3-a]quinazolin-4-ylmethyl)benzonitrile

7-Amino-1-dimethylamino-4-((E)-3-phenylallyl)-4H-[1,2,4]triazolo[4,3-a]quinazolin-5-one

4-Benzyl-7-methylamino-1-pyrrolidin-1-yl-4H-[1,2,4]triazolo[4,3-a]quinazolin-5-one

4-(7-Methylamino-5-oxo-1-pyrrolidin-1-yl-5H-[1,2,4]triazolo[4,3-a]quinazolin-4-yl-methyl)benzonitrile

4-Benzyl-8-methylamino-1-pyrrolidin-1-yl-4H-[1,2,4]triazolo[4,3-a]quinazolin-5-one

4-Benzyl-7-ethylamino-1-pyrrolidin-1-yl-4H-[1,2,4]triazolo[4,3-a]quinazolin-5-one

4-Benzyl-7-isopropylamino-1-pyrrolidin-1-yl-4H-[1,2,4]triazolo[4,3-a]quinazolin-5-one

N-(4-Benzyl-5-oxo-1-pyrrolidin-1-yl-4,5-dihydro[1,2,4]triazolo[4,3-a]quinazolin-7-yl)-methanesulphonamide

4-Benzyl-7-dimethylamino-1-pyrrolidin-1-yl-4H-[1,2,4]triazolo[4,3-a]quinazolin-5-one

4-Benzyl-1-dimethylamino-5-oxo-4,5-dihydro[1,2,4]triazolo[4,3-a]quinazoline-7-carbonitrile

4-Benzyl-5-oxo-1-pyrrolidin-1-yl-4,5-dihydro[1,2,4]triazolo[4,3-a]quinazoline-7-carboxylic acid

Methyl [4-(7-bromo-5-oxo-1-pyrrolidin-1-yl-5H-[1,2,4]triazolo[4,3-a]quinazolin-4-yl-methyl)phenyl]acetate

2-[4-(7-Bromo-5-oxo-1-pyrrolidin-1-yl-5H-[1,2,4]triazolo[4,3-a]quinazolin-4-ylmethyl)-phenyl]-N-methylacetamide

2-[4-(7-Bromo-1-dimethylamino-5-oxo-5H-[1,2,4]triazolo[4,3-a]quinazolin-4-ylmethyl)-phenyl]acetamide

2-[4-(7-Bromo-5-oxo-1-pyrralidin-1-yl-5H-[1,2,4]triazolo[4,3-a]quinazolin-4-ylmethyl)-phenyl]-N,N-dimethylacetamide

2-[4-(7-Bromo-5-oxo-1-pyrrolidin-1-yl-5H-[1,2,4]triazolo[4,3-a]quinazolin-4-ylmethyl)-phenyl]-N-hydroxyacetamide

4-(1-Dimethylamino-7-methyl-5-thioxo-5H-[1,2,4]triazolo[4,3-a]quinazolin-4-ylmethyl)-benzonitrile

4-(7-Bromo-1-dimethylamino-5-thioxo-5H-[1,2,4]triazolo[4,3-a]quinazolin-4-ylmethyl)-benzonitrile

1-Dimethylamino-7-methyl-4-(3-pyrid-3-ylallyl)-4H-[1,2,4]triazolo[4,3-a]quinazoline-5-thione

4-Benzyl-7-(N,N-dimethylsulphonylamino)-1-(pyrrolidin-1-yl)-4H-[1,2,4]triazolo [4,3-a]quinazolin-5-one

**8.** Triazolo[4,3-a]quinazolin-5-ones and/or -5-thrones according to any one of Claims 1 to 4, chosen from the group comprising:

1-(Azepan-1-yl)-7-chloro-4-(3-phenylallyl)-4H-[1,2,4]triazolo[4,3-a]quinazolin-5-one

7-Bromo-1-dimethylamino-4-((E)-3-pyrid-3-ylallyl)-4H-[1,2,4]triazolo[4,3-a]quinazolin-5-one

1-(Azepan-1-yl)-7-chloro-4-(4-chlorobenzyl)-4H-[1,2,4]triazolo[4,3-a]quinazolin-5-one

1-(Azepan-1-yl)-7-chloro-4-(4-fluorobenzyl)-4H-[1,2,4]triazolo[4,3-a]quinazolin-5-one

1-(Azepan-1-yl)-7-chloro-4-(3,4-dimethoxybenzyl)-4H-[1,2,4]triazolo[4,3-a]quinazolin-5-one

1-(Azepan-1-yl)-7-chloro-4-(3-pyridylmethyl)-4H-[1,2,4]triazolo[4,3-a]quinazolin-5-one

1-(Azepan-1-yl)-7-bromo-4-(4-chlorophenylmethyl)-4H-[1,2,4]triazolo[4,3-a]quinazolin-5-one

4-(1-Azepan-1-yl-7-bromo-5-oxo-5H-[1,2,4]triazolo[4,3-a]quinazolin-4-ylmethyl)-benzonitrile

1-Azepan-1-yl-7-bromo-4-(3,4-dimethoxybenzyl)-4H-[1,2,4]triazolo[4,3-a]quinazolin-5-one

1-(Azepan-1-yl)-7-bromo-4-(3-phenylallyl)-4H-[1,2,4]triazolo[4,3-a]quinazolin-5-one

1-Azepan-1-yl-7-bromo-4-((E)-3-pyrid-3-ylallyl)-4H-[1,2,4]triazolo[4,3-a]quinazolin-5-one

1-Azepan-1-yl-7-bromo-4-(3-pyrid-4-ylallyl)-4H-[1,2,4]triazolo[4,3-a]quinazolin-5-one

7-Bromo-4-(4-methylbenzyl)-1-pyrrolidin-1-yl-4H-[1,2,4]triazolo[4,3-a]quinazolin-5-one

7-Bromo-4-(4-chlorobenzyl)- 1-pyrrolidin-1-yl-4H-[1,2,4]triazolo[4,3-a]quinazolin-5-one

7-Bromo-4-(4-fluorobenzyl)-1-pyrrolidin-I -yl-4H-[1,2,4]triazolo[4,3-a]quinazolin-5-one

4-(7-Bromo-5-oxo-1-pyrrolidin-1-yl-5H-[1,2,4]triazolo[4,3-a]quinazolin-4-ylmethyl)-benzonitrile

Methyl 4-(7-bromo-5-oxo-1-pyrrolidin-1-yl-5H-[1,2,4]triazolo[4,3-a]quinazolin-4-yl-methyl)benzoate

7-Bromo-4-(4-nitrobenzyl)-1-pyrrolidin-1-yl-4H-[1,2,4]triazolo[4,3-a]quinazolin-5-one

7-Bromo-4-(4-methoxybenzyl)-1-pyrrolidin-1-yl-4H-[1,2,4]triazolo[4,3-a]quinazolin-5-one

Phenyl 4-(7-bromo-5-oxo-1-pyrrolidin-1-yl-5H-[1,2,4]triazolo[4,3-a]quinazolin-4-yl-methyl)acetate

7-Bromo-4-(4-hydroxybenzyl)-1-pyrrolidin-1-yl-4H-[1,2,4]triazolo[4,3-a]quinazolin-5-one

7-Bromo-4-(3,4-dimethoxybenzyl)-1-pyrrolidin-1-yl-4H-[1,2,4]triazolo[4,3-a]quinazolin-5-one

1-(Pyrrolidin-1-yl)-7-bromo-4-(3-phenylallyl)-4H-[1,2,4]triazolo[4,3-a]quinazolin-5-one

7-Bromo-4-[(E)-3-(4-chlorophenyl)allyl]-1-pyrrolidin-1-yl-4H-[1,2,4]triazolo[4,3-a]-quinazolin-5-one

7-Bromo-4-[3-(4-methoxyphenyl)allyl]-1-pyrrolidin-1-yl-4H-[1,2,4]triazolo[4,3-a]-quinazolin-5-one

7-Bromo-4-(3-pyrid-3-ylallyl)-1-pyrrolidin-1-yl-4H-[1,2,4]triazolo[4,3-a]quinazolin-5-one

7-Bromo-4-((E)-3-pyrid-4-ylallyl)-1-pyrrolidin-1-yl-4H-[1,2,4]triazolo[4,3-a]quinazolin-5-one

7-Bromo-4-(3,4-dimethoxybenzyl)-1-piperid-1-yl-4H-[1,2,4]triazolo[4,3-a]quinazolin-5-one

1-(Piperid-1-yl)-7-bromo-4-(3-phenylallyl)-4H-[1,2,4]triazolo[4,3-a]quinazolin-5-one

7-Bromo-1-dimethylamino-4-(4-methylbenzyl)-4H-[1,2,4]triazolo[4,3-a]quinazolin-5-one

4-(7-Bromo-1-dimethylamino-5-oxo-5H-[1,2,4]triazolo[4,3-a]quinazolin-4-ylmethyl)-benzonitrile

7-Bromo-1-dimethylamino-4-(4-hydroxybenzyl)-4H-[1,2,4]triazolo[4,3-a]quinazolin-5-one

Methyl 4-(7-bromo-1-dimethylamino-5-oxo-5H-[1,2,4]triazolo[4,3-a]quinazolin-4-ylmethyl)benzoate

[4-(7-Bromo-1-dimethylamino-5-oxo-5H-[1,2,4]triazolo[4,3-a]quinazolin-4-ylmethyl)-phenyl]acetonitrile

7-Bromo-1-dimethylamino-4-(3-phenylallyl)-4H-[1,2,4]triazolo[4,3-a]quinazolin-5-one

7-Bromo-1-dimethylamino-4-(3-phenylprop-2-ynyl)-4H-[1,2,4]triazolo[4,3-a]quinazolin-5-one

1-Azepan-1-yl-7-methyl-4-(3-phenylallyl)-4H-[1,2,4]triazolo[4,3-a]quinazolin-5-one

4-(3,4-Dimethoxybenzyl)-7-methyl-1-pyrrolidin-1-yl-4H-[1,2,4]triazolo[4,3-a]quinazolin-5-one

[4-(7-Methyl-5-oxo-1-pyrrolidin-1-yl-5H-[1,2,4]triazolo[4,3-a]quinazolin-4-ylmethyl)-phenyl]acetic acid

7-Methyl-4-(3-phenylallyl)-1-pyrrolidin-1-yl-4H-[1,2,4]triazolo[4,3-a]quinazolin-5-one

[4-(Dimethylaminomethyloxo-5H-[1,2,4]triazolo[4,3-a]quinazolin-4-ylmethyl)phenyl]acetic acid

1-Dimethylamino-7-methyl-4-((E)-3-phenylallyl)-4H-[1,2,4]triazolo[4,3-a]quinazolin-5-one

1-Dimethylamino-7-methyl-4-(3-pyrid-3-ylallyl)-4H-[1,2,4]triazolo[4,3-a]quinazolin-5-one

[4-(7-Bromo-5-oxo-1-perhydroazepin-1-yl-5H-[1,2,4]triazolo[4,3-a]quinazolin-3-yl-methyl)phenyl]acetic acid

4-Benzyl-7-bromo-1-(pyrrolidin-1-yl)-4H-[1,2,4]triazolo[4,3-a]quinazolin-5-one

4-Benzyl-7-bromo-1-(2,5-dihydropyrrol-1-yl)-4H-[1,2,4]triazolo[4,3-a]quinazolin-5-one

4-Benzyl-7-iodo-1-pyrrolidin-1-yl-4H-[1,2,4]triazolo[4,3-a]quinazolin-5-one

1-Azepan-1-yl-4-benzyl-7-methyl-4H-[1,2,4]triazolo[4,3-a]quinazolin-5-one

4-(4-Aminobenzyl)-7-bromo-1-pyrrolidin-1-yl-4H-[1,2,4]triazolo[4,3-a]quinazolin-5-one

7-Amino-4-((E)-3-phenylallyl)-1-pyrrolidin-1-yl-4H-[1,2,4]triazolo[4,3-a]quinazolin-5-one

7-Amino-1-azepan-1-yl-4-benzyl-4H-[1,2,4]triazolo[4,3-a]quinazolin-5-one

7-Amino-4-((E)-3-pyrid-3-ylallyl)-1-pyrrolidin-1-yl-4H-[1,2,4]triazolo[4,3-a]quinazolin-5-one

7-Amino-1-dimethylamino-4-((E)-3-phenylallyl)-4H-[1,2,4]triazolo[4,3-a]quinazolin-5-one

4-Benzyl-7-methylamino-1-pyrrolidin-1-yl-4H-[1,2,4]triazolo[4,3-a]quinazolin-5-one

4-(7-Methylamino-5-oxo-1-pyrrolidin-1-yl-5H-[1,2,4]triazolo[4,3-a]quinazolin-4-ylmethyl)-benzonitrile

4-Benzyl-8-methylamino-1-pyrrolidin-1-yl-4H-[1,2,4]triazolo[4,3-a]quinazolin-5-one

4-Benzyl-7-ethylamino-1-pyrrolidin-1-yl-4H-[1,2,4]triazolo[4,3-a]quinazolin-5-one

4-Benzyl-7-isopropylamino-1-pyrrolidin-1-yl-4H-[1,2,4]triazolo[4,3-a]quinazolin-5-one

Methyl [4-(7-bromo-5-oxo-1-pyrrolidin-1-yl-5H-[1,2,4]triazolo[4,3-a]quinazolin-4-yl-methyl)phenyl]acetate

2-[4-(7-Bromo-5-oxo-1-pyrrolidin-1-yl-5H-[1,2,4]triazolo[4,3-a]quinazolin-4-ylmethyl)-phenyl]-N-methylacetamide

2-[4-(7-Bromo-1-dimethylamino-5-oxo-5H-[1,2,4]triazolo[4,3-a]quinazolin-4-ylmethyl)-phenyl]acetamide

2-[4-(7-Bromo-5-oxo-1-pyrrolidin-1-yl-5H-[1,2,4]triazolo[4,3-a]quinazolin-4-ylmethyl)-phenyl]-N,N-dimethylacetamide

2-[4-(7-Bromo-5-oxo-1-pyrrolidin-1-yl-5H-[1,2,4]triazolo[4,3-a]quinazolin-4-ylmethyl)-phenyl]-N-hydroxyacetamide

1-Dimethylamino-7-methyl-4-(3-pyrid-3-ylallyl)-4H-[1,2,4]triazolo[4,3-a]quinazoline-5-thione

**9.** Triazolo[4,3-a]quinazolin-5-ones and/or -5-thrones according to any one of Claims 1 to 4, chosen from the group

comprising:

7-Bromo-1-dimethylamino-4-((E)-3-pyrid-3-ylallyl)-4H-[1,2,4]triazolo[4,3-a]quinazolin-5-one
1-(Azepan-1-yl)-7-chloro-4-(3,4-dimethoxybenzyl)-4H-[1,2,4]triazolo[4,3-a]quinazolin-5-one
1-(Azepan-1-yl)-7-chloro-4-(3-pyridylmethyl)-4H-[1,2,4]triazolo[4,3-a]quinazolin-5-one
4-(1-Azepan-1-yl-7-bromo-5-oxo-5H-[1,2,4]triazolo[4,3-a]quinazolin-4-ylmethyl)-benzonitrile
1-Azepan-1-yl-7-bromo-4-(3,4-dimethoxybenzyl)-4H-[1,2,4]triazolo[4,3-a]quinazolin-5-one
1-(Azepan-1-yl)-7-bromo-4-(3-phenylallyl)-4H-[1,2,4]triazolo[4,3-a]quinazolin-5-one
1-Azegan-1-yl-7-bromo-4-((E)-3-pyrid-3-ylallyl)-4H-[1,2,4]triazolo[4,3-a]quinazolin-5-one
1-Azepan-1-yl-7-bromo-4-(3-pyrid-4-ylallyl)-4H-[1,2,4]triazolo[4,3-a]quinazolin-5-one
7-Bromo-4-(4-methylbenzyl)-1-pyrrolidin-1-yl-4H-[1,2,4]triazolo[4,3-a]quinazolin-5-one
7-Bromo-4-(4-chlorobenzyl)-1-pyrrolidin-1-yl-4H-[1,2,4]triazolo[4,3-a]quinazolin-5-one
7-Bromo-4-(3,4-dimethoxybenzyl)-1-pyrrolidin-1-yl-4H-[1,2,4]triazolo[4,3-a]quinazolin-5-one
1-(Pyrrolidin-1-yl)-7-bromo-4-(3-phenylallyl)-4H-[1,2,4]triazolo[4,3-a]quinazolin-5-one
7-Bromo-4-(3-pyrid-3-ylallyl)-1-pyrrolidin-1-yl-4H-[1,2,4]triazolo[4,3-a]quinazolin-5-one
1-(Piperid-1-yl)-7-bromo-4-(3-phenylallyl)-4H-[1,2,4]triazolo[4,3-a]quinazolin-5-one
4-(7-Bromo-1-dimethylamino-5-oxo-5H-[1,2,4]triazolo[4,3-a]quinazolin-4-ylmethyl)-benzonitrile
Methyl 4-(Bromodimethylaminooxo-5H-[1,2,4]triazolo[4,3-a]quinazolin-4-ylmethyl)-benzoate
7-Bromo-1-dimethylamino-4-(3-phenylallyl)-4H-[1,2,4]triazolo[4,3-a]quinazolin-5-one
7-Bromo-1-dimethylamino-4-(3-phenylprop-2-ynyl)-4H-[1,2,4]triazolo[4,3-a]quinazolin-5-one
1-Azepan-1-yl-7-methyl-4-(3-phenylallyl)-4H-[1,2,4]triazolo[4,3-a]quinazolin-5-one
4-(3,4-Dimethoxybenzyl)-7-methyl-1-pyrrolidin-1-yl-4H-[1,2,4]triazolo[4,3-a]quinazolin-5-one
7-Methyl-4-(3-phenylallyl)-1-pyrrolidin-1-yl-4H-[1,2,4]triazolo[4,3-a]quinazolin-5-one
7-Amino-4-((E)-3-phenylallyl)-1-pyrrolidin-1-yl-4H-[1,2,4]triazolo[4,3-a]quinazolin-5-one
7-Amino-4-((E)-3-pyrid-3-ylallyl)-1-pyrrolidin-1-yl-4H-[1,2,4]triazolo[4,3-a]quinazolin-5-one
4-Benzyl-7-methylamino-1-pyrrolidin-1-yl-4H-[1,2,4]triazolo[4,3-a]quinazolin-5-one
4-(7-Methylamino-5-oxo-1-pyrrolidin-1-yl-5H-[1,2,4]triazolo[4,3-a]quinazolin-4-yl-methyl)benzonitrile
Methyl [4-(7-bromo-5-oxo-1-pyrrolidin-1-yl-5H-[1,2,4]triazolo[4,3-a]quinazolin-4-yl-methyl)phenyl]acetate
2-[4-(7-Bromo-5-oxo-1-pyrrolidin-1-yl-5H-[1,2,4]triazolo[4,3-a]quinazolin-4-ylmethyl)-phenyl]-N-methylacetamide
2-[4-(7-Bromo-5-oxo-1-pyrrolidin-1-yl-5H-[1,2,4]triazolo[4,3-a]quinazolin-4-ylmethyl)-phenyl]-N,N-dimethylacetamide
1-Dimethylamino-7-methyl-4-(3-pyrid-3-ylallyl)-4H-[1,2,4]triazolo[4,3-a]quinazoline-5-thione

**10.** Intermediate compounds of general formula III:

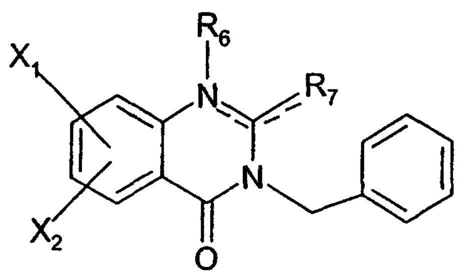

III

in which:

- $X_1$, $X_2$ and $A_1$ are as defined in Claim 1;
- the dashed lines represent optional double bonds;
- $R_6$ is hydrogen; and
- $R_7$ is S or hydrazino;

it being possible for $R_7$ to be linked to the nitrogen in $R_6$ to form a ring, particularly a triazole, optionally substituted with a lower thioalkyl, mercapto or halogen group, with the proviso that when $R_7$ is S or hydrazino and $X_1$ is

hydrogen, halogen, alkyl group comprising from 1 to 3 carbon atoms, alkoxy group comprising from 1 to 3 carbon atom or vitro, then $X_2$ is not hydrogen, halogen, alkyl group comprising from 1 to 3 carbon atoms, alkoxy group comprising from 1 to 3 carbon atom or vitro.

**11.** Intermediate compounds of general formula IV:

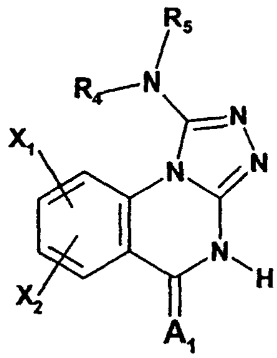

IV

in which $X_1$, $X_2$, $A_1$, $R_4$ and $R_5$ are as defined in Claim 1.

**12.** Intermediate compounds of general formula V:

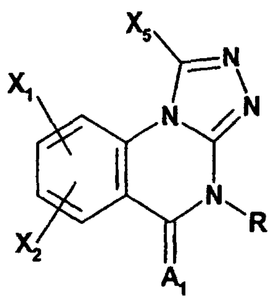

(V)

in which $X_1$, $X_2$, $A_1$ and R are as defined in Claim 1 and $X_5$ is a halogen, particularly F, Br or Cl, $-OCOX_7$, $-OSO_2X_7$ or $-SO_2X_7$ group in which $X_7$ is a lower alkyl or aryl group.

**13.** Process for manufacturing the compounds of general formulae I and II:

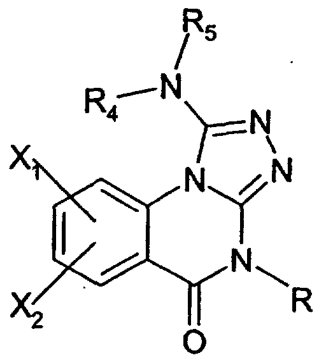

, 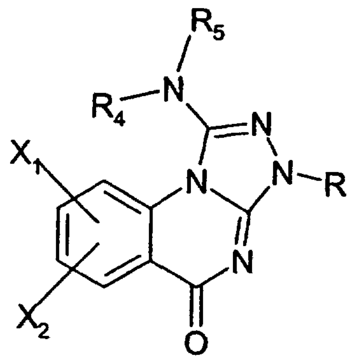

I II

in which $X_1$, $X_2$, R, $R_4$ and $R_5$ are as defined in Claim 1, the said process being **characterized in that** it comprises the reaction of a compound of general formula IV:

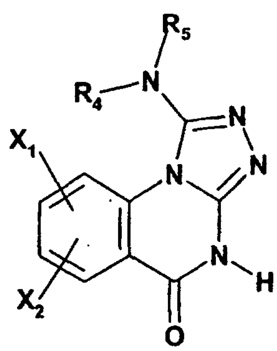

IV

in which $X_1$, $X_2$, $R_4$ and $R_5$ are as defined in Claim 1, with a compound of general formula

R-X'

in which R is as defined above and X' is a halogen, particularly F, Br or Cl, $-OCOX_7$ or $-OSO_2X_7$ group in which $X_7$ is a lower alkyl or aryl group; to give a mixture of the compounds of general formulae I and II, which are then optionally separated.

**14.** Process for manufacturing the compounds of general formula I:

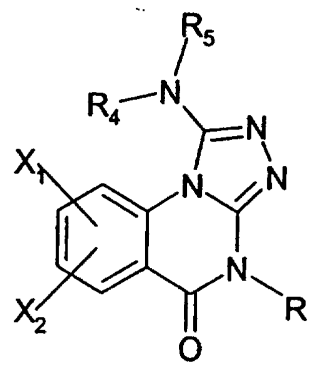

I

in which $X_1$, $X_2$, R, $R_4$ and $R_5$ are as defined in Claim 1,
the said process being **characterized in that** it comprises the reaction of a compound of general formula V:

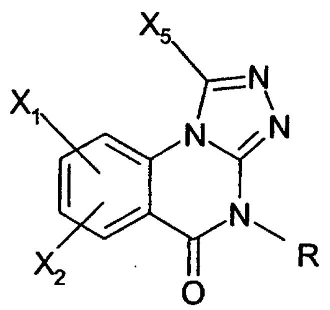

V

in which $X_1$, $X_2$ and R are as defined in Claim 1 and $X_5$ is halogen, particularly F, Br or Cl, $-OCOX_7$, $-OSO_2X_7$ or $-SO_2X_7$ group in which $X_7$ is a lower alkyl or aryl group;
with a compound of general formula:

$$HNR_4R_5$$

in which $R_4$ and $R_5$ are as defined in Claim 1,
to give a compound of general formula I.

**15.** Process according to Claim 14, **characterized in that** when $X_1$ is $-NR_2R_3$ and $-NR_2R_3$ and $-NR_4R_5$ are identical, the compounds of formula I are obtained by reacting a compound of general formula VI:

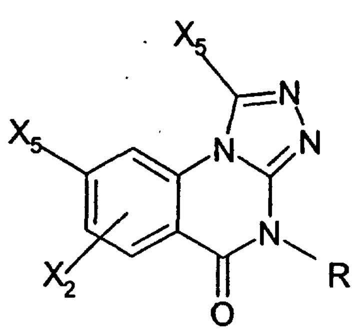

VI

in which $X_2$ and R are as defined in Claim 1 and $X_5$ is as defined in claim 12 with a compound of general formula:

$$HNR_2R_3$$

in which $R_2$ and $R_3$ are as defined in Claim 1, to give a compound of general formula (I):

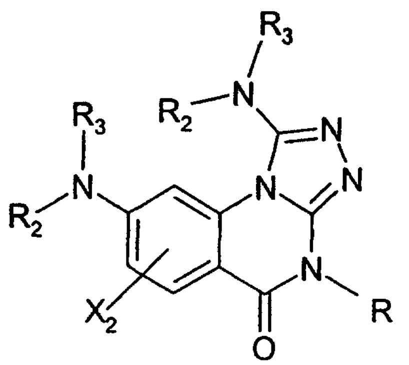

I

**16.** Process according to Claim 14, **characterized in that** when $X_1$ is $-NR_2R_3$ and $-NR_2R_3$ and $-NR_4R_5$ are different, the compounds of formula I are obtained by reacting a compound of general formula VII:

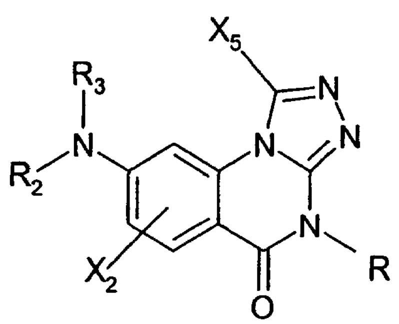

VII

in which $X_2$, R, $R_2$ and $R_3$ are as defined in Claim 1 and $X_5$ is as defined in claim 12, with a compound of general formula:

$$HNR_4R_5$$

in which $R_4$ and $R_5$ are as defined in Claim 1, to give a compound of general formula (I):

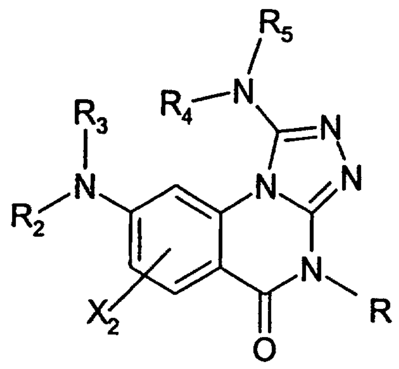

I

17. Pharmaceutical composition comprising a compound according to any one of Claims 1 to 9 and a pharmaceutically acceptable excipient .

18. Use of a compound according to any one of Claims 1 to 9 for the preparation of a medicinal product intended for treating a condition or complaint involving a treatment by inhibition of phosphodiesterases, and more particularly of PDE4.

19. Use according to Claim 18, **characterized in that** the condition is asthma.

20. Use according to Claim 18, **characterized in that** the condition is chronic bronchitis or acute pulmonary attack.

21. Use according to Claim 18, **characterized in that** the condition is atopic dermatitis.

22. Use according to Claim 18, **characterized in that** the condition is pulmonary hypertension.

23. Use according to Claim 18, **characterized in that** the condition is cardiac or pulmonary insufficiency.

24. Use according to Claim 18, **characterized in that** the condition is psoriasis.

25. Use according to Claim 18, **characterized in that** the condition is an inflammatory condition of the digestive system such as haemorrhagic rectocolitis or Crohn's disease.

26. Use according to Claim 18, **characterized in that** the condition is diabetes or a condition associated with a high level of TNF-$\alpha$, such as acute respiratory distress syndrome or acute pancreatitis.

27. Use according to Claim 18, **characterized in that** the condition is benign hypertrophy of the prostate.

28. Use according to Claim 18, **characterized in that** the condition is chosen from rheumatoid arthritis and multiple sclerosis.

29. Use according to Claim 18, **characterized in that** the condition is chosen from depression, ischaemia-mediated neuronal attack and partial cerebral ischaemia.

30. Use according to Claim 18, **characterized in that** the condition is cancer, more particularly malignant tumours or chronic lymphoid leukaemia.

31. Use of a compound according to any one of Claims 1 to 9 to attenuate the development of tolerance or morphine-dependency phenomena.

32. Use of a compound according to any one of Claims 1 to 9 to reduce behavioural memory losses.

**33.** Use of a compound according to any one of Claims I to 9 to prevent premature labour.

**34.** Use according to Claim 18, **characterized in that** the condition is septicaemia or multiple organ failure syndrome.

**35.** Use according to Claim 18, **characterized in that** the condition is chronic obstructive pulmonary disease (COPD).

**36.** Use according to Claim 18, **characterized in that** the condition is emphysema.

**37.** Use according to Claim 18, **characterized in that** the condition is allergic rhinitis.

**38.** Use according to Claim 18, **characterized in that** the condition is congestive cardiac insufficiency.

**39.** Use according to Claim 18, **characterized in that** the condition is osteoporosis.